# EUROPEAN PATENT SPECIFICATION

(11) **EP 0 948 522 B1**
(45) Date of publication and mention of the grant of the patent: **11.06.2008**
(21) Application number: 97909070.1
(22) Date of filing: 31.10.1997
(51) Int. Cl.: C07K 2/00

(54) **THERAPEUTIC AND DIAGNOSTIC AGENTS CAPABLE OF MODULATING CELLULAR RESPONSIVENESS TO CYTOKINES**
THERAPEUTIKA UND DIAGNOSTIKA, DIE DIE ZELLULÄRE ANSPRECHBARKEIT AUF CYTOKINE REGULIEREN
AGENTS THERAPEUTIQUES ET DIAGNOSTIQUES CAPABLES DE MODULER LA RECEPTIVITE CELLULAIRE AUX CYTOKINES

(30) Priority: 01.11.1996 AU PO338496; 14.02.1997 AU PO511797
(43) Date of publication of application: 13.10.1999
(60) Divisional application: 08006605.3
(73) Proprietor: THE WALTER AND ELIZA HALL INSTITUTE OF MEDICAL RESEARCH, Parkville, VIC 3052 (AU)
(72) Inventor: HILTON, Douglas, J., Warrandyte, VIC 3113 (AU); ALEXANDER, Warren, S., Moonee Ponds, VIC 3039 (AU); VINEY, Elizabeth, M., Bundoora, VIC 3083 (AU); WILLSON, Tracy, A., North Balwyn, VIC 3104 (AU); RICHARDSON, Rachael, T., East Brighton, VIC 3187 (AU); STARR, Robyn, Carlton, VIC 3053 (AU); NICHOLSON, Sandra, E., Newport, VIC 3015 (AU); METCALF, Donald, Balwyn, VIC 3103 (AU); NICOLA, Nicos, A., Mont Albert, VIC 3127 (AU)
(74) Representative: Dzieglewska, Hanna Eva
(86) International application number: PCT/AU1997/000729
(87) International publication number: WO 1998/020023

(56) References cited:
- WO-A-96/39427
- WO-A-98/30688
- WO-A-99/03993
- AU-A- 2 792 495
- DATABASE EM_RO [Online] EMBL; Accession number Z47352; ID : MMPRMGNS, 1 July 1995 (1995-07-01) XP002165867 -& SCHLÜTER, G. ET AL.: "Sequence analysis of the conserved protamine gene cluster shows that it contains a fourth expressed gene" MOL. REPROD. DEV., vol. 43, January 1996 (1996-01), pages 1-6, XP000993037
- DATABASE EM_HUM [Online] EMBL; Accession number : Z46940; ID : HSPRMTNP2, 1 July 1995 (1995-07-01) XP002165868
- DATABASE EM_RO [Online] EMBL; Accession number : Z46939; ID : BTPRMTNP2, 1 July 1995 (1995-07-01) XP002165869
- A YOSHIMURA ET AL: "A novel cytokine-inducible gene CIS encodes an SH2-containing protein that binds to Tyrosine-phosphorylated interleukin-3 and erythropoietin" EMBO JOURNAL, vol. 14, no. 12, 1995, pages 2816-2826, XP002088456 ISSN: 0261-4189
- SEIJIRO MINAMOTO ET AL: "Cloning and functional analysis of new members of STAT induced STAT inhibitor (SSI) family: SSI.2 and SSI-3" BIOCHEMICAL AND BIOPHYSICAL RESEARCH COMMUNICATIONS, vol. 237, no. 1, 8 August 1997 (1997-08-08), pages 79-83, XP002095406 ISSN: 0006-291X
- DATABASE EM_RO [Online] EMBL; Accession number : U72673 (ID : MMU72673), 12 March 1997 (1997-03-12) XP002172403 -& FU. X. ET AL.: "E2a-Pbx1 induces aberrant expression of tissue-specific and developmentally regulated genes when expressed in NIH 3T3 fibroblasts" MOLECULAR AND CELLULAR BIOLOGY, vol. 17, March 1997 (1997-03), pages 1503-1512, XP002172402
- R STARR ET AL: "A family of cytokine-inducible inhibitors of signalling" NATURE, vol. 387, 26 June 1997 (1997-06-26), pages 917-921, XP002085491 ISSN: 0028-0836
- T A ENDO ET AL: "A new protein containing an SH2 domain that inhibits JAK kinases" NATURE, vol. 387, 26 June 1997 (1997-06-26), pages 921-924, XP002085492 ISSN: 0028-0836
- T NAKA ET AL: "Structure and function of a new STAT-induced STAT inhibitor" NATURE, vol. 387, no. 6636, 26 June 1997 (1997-06-26), pages 924-929, XP002088455 ISSN: 0028-0836
- DATABASE EM_EST [Online] EMBL; Accession number : AA189608 (ID : MMAA81160), 20 January 1997 (1997-01-20) MARRA, M. ET AL.: "mt98e04.r1 Soares mouse 3NbMS Mus musculus cDNA clone IMAGE:637950 5' " XP002172404
- D J HILTON ET AL: "Twenty proteins containing a C-terminal SOCS box form five structural classes" PROCEEDINGS OF THE NATIONAL ACADEMY OF SCIENCES OF USA, vol. 95, January 1998 (1998-01), pages 114-119, XP002085497 ISSN: 0027-8424
- YEAST, Vol. 12, No. 15, issued 1996, DELAVEAU Th. et al., "Analysis of a 23 KB Region on the Left Arm of Yeast Chromosome IV", pages 1587-1592, XP001052778
- SCIENCE, Vol. 270, No. 5234, issued 1995, LABEIT S. et al., "Titins: Giant Proteins in Charge of Muscle Ultrastructure and Elasticity", pages 293-6. XP002906289
- THE EMBO JOURNAL, Vol. 14, No. 12, issued 1995, YOSHIMURA A. et al., "A Novel Cytokine - Inducable Gene CIS Encodes and SH2 - Containing Protein That Binds to Tyrosine - Phosphorylated Interleukin 3 and Erythropoietin Receptors", pages 2816-2826, XP002088456
- BIOCHEMISTRY, Vol. 34, No. 8, issued 1995, WEBER A. et al., "The 2-Oxoglutarate/Malate Translocator of Chloroplast Envelope Membranes: Molecular Cloning of a Transporter Containing a 12-Helix Motif and Expression of the Functional Protein in Yeast Cells", pages 2621-7. XP002906290
- JOURNAL OF BACTERIOLOGY, Vol. 176, No. 24, issued 1994, IWAI A. et al., "Molecular Cloning and Expression of an Isomalto-Dextranase Gene from Arthrobacter Globiformis T6", pages 7730-4, XP001052783
- NUCLEIC ACIDS RESEARCH, Vol. 122, No. 11, issued 1994, ALTHOFF S. et al., "Molecular Evolution of SRP Cycle Components: Functional Implications", pages 1933-47. XP002906291
- NATURE, Vol. 368, No. 6466, issued 1994, WILSON R. et al., "2.2 MB of Contiguous Nucleotide Sequence from Chromosome III of C Elegans", pages 32-38, XP002910426
- THE EMBO JOURNAL, Vol. 11, No. 5, issued 1992, LABEIT S. et al., "Towards a Molecular Understanding of Titin", pages 1711-16. XP002906292
- ADVANCES IN BIOPHYSICS, Vol. 33, (Muscle Elastic Proteins), issued 1996, KOLMERER B. et al., "A Systematic Search of the Data Bases for Sequences Homologous to Titin/Connection", pages 3-11, XP001052780
- MICROBIOLOGY, Vol. 142, No. 8, issued 1996, YONEYAMA H., "Protein C (OprC) of the Outer Membrane of Pseudomonas Aeruginosa is a Copper-Regulated Channel Protein", pages 2137-2144, XP001052781
- JOURNAL OF BACTERIOLOGY, Vol. 178, No. 15, issued 1996, LIMBERGER R. et al., "Organisation, Transcription and Expression 'of the 5' Region of the Fla Operon of Treponema Phagedenis and Treponema Pallidum", pages 4628-4634, XP001052777
- THE JOURNAL OF CELL BIOLOGY, Vol. 133, No. 6, issued 1996, GOODSON H.V. et al., "Synthetic Lethality Screen Identifies a Novel Yest Myosin I Gene (MY05): Myosin I Protein are Required for Polarisation of the Actin Cytoskeleton", pages 1277-1291, XP001052779
- GENES AND DEVELOPMENT, Vol. 9, No. 24, issued 1995, HERRSCHER R.F. et al., "The Immunoglobulin Heavy-Chain Matrix-Associating Regions are Bound by Bright: A B Cell-Specific Trans-Activator That Describes a New DNA-Binding Protein Family", pages 3067-82, XP001052782

### SEQUENCE LISTING

(1) GENERAL INFORMATION:
   (i) APPLICANT: (Other than US) THE WALTER AND ELIZA HALL INSTITUTE OF MEDICAL RESEARCH (US Only)
   (ii) TITLE OF INVENTION: THERAPEUTIC AND DIAGNOSTIC AGENTS
   (iii) NUMBER OF SEQUENCES: 49
   (iv) CORRESPONDENCE ADDRESS:
      (A) ADDRESSEE: DAVIES COLLISON CAVE
      (B) STREET: 1 LITTLE COLLINS STREET
      (C) CITY: MELBOURNE
      (D) STATE: VICTORIA
      (E) COUNTRY: AUSTRALIA
      (F) ZIP: 3000
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: PCT INTERNATIONAL
      (B) FILING DATE: 31-OCT-1997
   (vi) PRIOR APPLICATION DATA:
      (A) APPLICATION NUMBER: PO5117
      (B) FILING DATE: 14-FEB-1997
   (vii) PRIOR APPLICATION DATA:
      (A) APPLICATION NUMBER: PO 3384
      (B) FILING DATE: 01-NOV-1996
   (viii) ATTORNEY/AGENT INFORMATION:
      (A) NAME: HUGHES DR, E JOHN L
      (C) REFERENCE/DOCKET NUMBER: EJH/EK
   (ix) TELECOMMUNICATION INFORMATION:
      (A) TELEPHONE: +61 3 9254 2777
      (B) TELEFAX: +61 3 9254 2770
(2) INFORMATION FOR SEQ ID NO:1:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID N0:1:
      CACGCCGCCC ACGTGAAGGC 20
(2) INFORMATION FOR SEQ ID NO:2:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:2:
      TTCGCCAATG ACAAGACGCT 20
(2) INFORMATION FOR SEQ ID NO:3:
   (i) SEQUENCE CHARACTERISTICS:
      (A) LENGTH: 1236 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS
      (B) LOCATION: 1..636
   (xi) SEQUENCE DESCRIPTION: SEQ ID NO:3:
(2) INFORMATION FOR SEQ ID NO:4:
   (i) SEQUENCE CHARACTERISTICS:
      (A) LENGTH: 212 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Met Val Ala Arg Asn Gln Val Ala Ala Asp Asn Ala Ile Ser Pro Ala
    1               5                  10                  15
   Ala Glu Pro Arg Arg Arg Ser Glu Pro Ser Ser Ser Ser Ser Ser Ser
               20                  25                  30
   Ser Pro Ala Ala Pro Val Arg Pro Arg Pro Cys Pro Ala Val Pro Ala
           35                  40                  45
   Pro Ala Pro Gly Asp Thr His Phe Arg Thr Phe Arg Ser His Ser Asp
       50                  55                  60
   Tyr Arg Arg Ile Thr Arg Thr Ser Ala Leu Leu Asp Ala Cys Gly Phe
   65                  70                  75                  80
   Tyr Trp Gly Pro Leu Ser Val His Gly Ala His Glu Arg Leu Arg Ala
                   85                  90                  95
   Glu Pro Val Gly Thr Phe Leu Val Arg Asp Ser Arg Gln Arg Asn Cys
               100                 105                 110
   Phe Phe Ala Leu Ser Val Lys Met Ala Ser Gly Pro Thr Ser Ile Arg
           115                 120                 125
   Val His Phe Gln Ala Gly Arg Phe His Leu Asp Gly Ser Arg Glu Thr
       130                 135                 140
   Phe Asp Cys Leu Phe Glu Leu Leu Glu His Tyr Val Ala Ala Pro Arg
   145                 150                 155                 160
   Arg Met Leu Gly Ala Pro Leu Arg Gln Arg Arg Val Arg Pro Leu Gln
                   165                 170                 175
   Glu Leu Cys Arg Gln Arg Ile Val Ala Ala Val Gly Arg Glu Asn Leu
               180                 185                 190
   Ala Arg Ile Pro Leu Asn Pro Val Leu Arg Asp Tyr Leu Ser Ser Phe
           195                 200                 205
   Pro Phe Gln Ile
       210
(2) INFORMATION FOR SEQ ID NO:5:
   (i) SEQUENCE CHARACTERISTICS:
      (A) LENGTH: 1121 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS
      (B) LOCATION: 223..819
   (xi) SEQUENCE DESCRIPTION: SEQ ID NO:5:
   GCGATCTGTG GGTGACAGTG TCTGCGAGAG ACTTTGCCAC ACCATTCTGC CGGAATTTGG       60
   AGAAAAAGAA CCAGCCGCTT CCAGTCCCCT CCCCCTCCGC CACCATTTCG GACACCCTGC      120
   ACACTCTCGT TTTGGGGTAC CCTGTGACTT CCAGGCAGCA CGCGAGGTCC ACTGGCCCCA      180
   GCTCGGGCGA CCAGCTGTCT GGGACGTGTT GACTCATCTC CC ATG ACC CTG CGG         234
                                                  Met Thr Leu Arg
                                                   1
   TGC CTG GAG CCC TCC GGG AAT GGA GCG GAC AGG ACG CGG AGC CAG TGG        282
   Cys Leu Glu Pro Ser Gly Asn Gly Ala Asp Arg Thr Arg Ser Gln Trp
     5                  10                  15                  20
   GGG ACC GCG GGG TTG CCG GAG GAA CAG TCC CCC GAG GCG GCG CGT CTG        330
   Gly Thr Ala Gly Leu Pro Glu Glu Gln Ser Pro Glu Ala Ala Arg Leu
                    25                  30                  35
   GCG AAA GCC CTG CGC GAG CTC AGT CAA ACA GGA TGG TAC TGG GGA AGT        378
   Ala Lys Ala Leu Arg Glu Leu Ser Gln Thr Gly Trp Tyr Trp Gly Ser
                40                  45                  50
   ATG ACT GTT AAT GAA GCC AAA GAG AAA TTA AAA GAG GCT CCA GAA GGA        426
   Met Thr Val Asn Glu Ala Lys Glu Lys Leu Lys Glu Ala Pro Glu Gly
            55                  60                  65
   ACT TTC TTG ATT AGA GAT AGT TCG CAT TCA GAC TAC CTA CTA ACT ATA        474
   Thr Phe Leu Ile Arg Asp Ser Ser His Ser Asp Tyr Leu Leu Thr Ile
        70                  75                  80
   TCC GTT AAG ACG TCA GCT GGA CCG ACT AAC CTG CGG ATT GAG TAC CAA        522
   Ser Val Lys Thr Ser Ala Gly Pro Thr Asn Leu Arg Ile Glu Tyr Gln
    85                  90                  95                 100
   GAT GGG AAA TTC AGA TTG GAT TCT ATC ATA TGT GTC AAG TCC AAG CTT        570
   Asp Gly Lys Phe Arg Leu Asp Ser Ile Ile Cys Val Lys Ser Lys Leu
                   105                 110                 115
   AAA CAG TTT GAC AGT GTG GTT CAT CTG ATT GAC TAC TAT GTC CAG ATG        618
   Lys Gln Phe Asp Ser Val Val His Leu Ile Asp Tyr Tyr Val Gln Met
               120                 125                 130
   TGC AAG GAT AAA CGG ACA GGC CCA GAA GCC CCA CGG AAT GGG ACT GTT        666
   Cys Lys Asp Lys Arg Thr Gly Pro Glu Ala Pro Arg Asn Gly Thr Val
           135                 140                 145
   CAC CTG TAC CTG ACC AAA CCT CTG TAT ACA TCA GCA CCC ACT CTG CAG        714
   His Leu Tyr Leu Thr Lys Pro Leu Tyr Thr Ser Ala Pro Thr Leu Gln
       150                 155                 160
   CAT TTC TGT CGA CTC GCC ATT AAC AAA TGT ACC GGT ACG ATC TGG GGA        762
   His Phe Cys Arg Leu Ala Ile Asn Lys Cys Thr Gly Thr Ile Trp Gly
   165                 170                 175                 180
   CTG CCT TTA CCA ACA AGA CTA AAA GAT TAC TTG GAA GAA TAT AAA TTC        810
   Leu Pro Leu Pro Thr Arg Leu Lys Asp Tyr Leu Glu Glu Tyr Lys Phe
                   185                 190                 195
   CAG GTA TAAGTATTTC TCTCTCTTTT TCGTTTTTTT TTAAAAAAAA AAAAACACAT         866
   Gln Val
   GCCTCATATA GACTATCTCC GAATGCAGCT ATGTGAAAGA GAACCCAGAG GCCCTCCTCT      926
   GGATAACTGC GCAGAATTCT CTCTTAAGGA CAGTTGGGCT CAGTCTAACT TAAAGGTGTG      986
   AAGATGTAGC TAGGTATTTT AAAGTTCCCC TTAGGTAGTT TTAGCTGAAT GATGCTTTCT    1046
   TTCCTATGGC TGCTCAAGAT CAAATGGCCC TTTTAAATGA AACAAAACAA AACAAAACAA    1106
   AAAAAAAAAA AAAAA                                                     1121
(2) INFORMATION FOR SEQ ID NO:6:
   (i) SEQUENCE CHARACTERISTICS:
      (A) LENGTH: 198 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Met Thr Leu Arg Cys Leu Glu Pro Ser Gly Asn Gly Ala Asp Arg Thr
    1               5                  10                  15
   Arg Ser Gln Trp Gly Thr Ala Gly Leu Pro Glu Glu Gln Ser Pro Glu
               20                  25                  30
   Ala Ala Arg Leu Ala Lys Ala Leu Arg Glu Leu Ser Gln Thr Gly Trp
           35                  40                  45
   Tyr Trp Gly Ser Met Thr Val Asn Glu Ala Lys Glu Lys Leu Lys Glu
       50                  55                  60
   Ala Pro Glu Gly Thr Phe Leu Ile Arg Asp Ser Ser His Ser Asp Tyr
   65                  70                  75                  80
   Leu Leu Thr Ile Ser Val Lys Thr Ser Ala Gly Pro Thr Asn Leu Arg
                   85                  90                  95
   Ile Glu Tyr Gln Asp Gly Lys Phe Arg Leu Asp Ser Ile Ile Cys Val
               100                 105                 110
   Lys Ser Lys Leu Lys Gln Phe Asp Ser Val Val His Leu Ile Asp Tyr
           115                 120                 125
   Tyr Val Gln Met Cys Lys Asp Lys Arg Thr Gly Pro Glu Ala Pro Arg
       130                 135                 140
   Asn Gly Thr Val His Leu Tyr Leu Thr Lys Pro Leu Tyr Thr Ser Ala
   145                 150                 155                 160
   Pro Thr Leu Gln His Phe Cys Arg Leu Ala Ile Asn Lys Cys Thr Gly
                   165                 170                 175
   Thr Ile Trp Gly Leu Pro Leu Pro Thr Arg Leu Lys Asp Tyr Leu Glu
               180                 185                 190
   Glu Tyr Lys Phe Gln Val
           195
(2) INFORMATION FOR SEQ ID NO:7:
   (i) SEQUENCE CHARACTERISTICS:
      (A) LENGTH: 2187 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS
      (B) LOCATION: 18..695
   (xi) SEQUENCE DESCRIPTION: SEQ ID NO:7:
   CGCTGGCTCC GTGCGCC ATG GTC ACC CAC AGC AAG TTT CCC GCC GCC GGG          50
                      Met Val Thr His Ser Lys Phe Pro Ala Ala Gly
                       1               5                  10
   ATG AGC CGC CCC CTG GAC ACC AGC CTG CGC CTC AAG ACC TTC AGC TCC         98
   Met Ser Arg Pro Leu Asp Thr Ser Leu Arg Leu Lys Thr Phe Ser Ser
               15                  20                  25
   AAA AGC GAG TAC CAG CTG GTG GTG AAC GCC GTG CGC AAG CTG CAG GAG        146
   Lys Ser Glu Tyr Gln Leu Val Val Asn Ala Val Arg Lys Leu Gln Glu
           30                  35                  40
   AGC GGA TTC TAC TGG AGC GCC GTG ACC GGC GGC GAG GCG AAC CTG CTG        194
   Ser Gly Phe Tyr Trp Ser Ala Val Thr Gly Gly Glu Ala Asn Leu Leu
       45                  50                  55
   CTC AGC GCC GAG CCC GCG GGC ACC TTT CTT ATC CGC GAC AGC TCG GAC        242
   Leu Ser Ala Glu Pro Ala Gly Thr Phe Leu Ile Arg Asp Ser Ser Asp
   60                  65                  70                  75
   CAG CGC CAC TTC TTC ACG TTG AGC GTC AAG ACC CAG TCG GGG ACC AAG        290
   Gln Arg His Phe Phe Thr Leu Ser Val Lys Thr Gln Ser Gly Thr Lys
                   80                  85                  90
   AAC CTA CGC ATC CAG TGT GAG GGG GGC AGC TTT TCG CTG CAG AGT GAC        338
   Asn Leu Arg Ile Gln Cys Glu Gly Gly Ser Phe Ser Leu Gln Ser Asp
               95                  100                 105
   CCC CGA AGC ACG CAG CCA GTT CCC CGC TTC GAC TGT GTA CTC AAG CTG        386
   Pro Arg Ser Thr Gln Pro Val Pro Arg Phe Asp Cys Val Leu Lys Leu
           110                 115                 120
   GTG CAC CAC TAC ATG CCG CCT CCA GGG ACC CCC TCC TTT TCT TTG CCA        434
   Val His His Tyr Met Pro Pro Pro Gly Thr Pro Ser Phe Ser Leu Pro
       125                 130                 135
   CCC ACG GAA CCC TCG TCC GAA GTT CCG GAG CAG CCA CCT GCC CAG GCA        482
   Pro Thr Glu Pro Ser Ser Glu Val Pro Glu Gln Pro Pro Ala Gln Ala
   140                 145                 150                 155
   CTC CCC GGG AGT ACC CCC AAG AGA GCT TAC TAC ATC TAT TCT GGG GGC        530
   Leu Pro Gly Ser Thr Pro Lys Arg Ala Tyr Tyr Ile Tyr Ser Gly Gly
                   160                 165                 170
   GAG AAG ATT CCG CTG GTA CTG AGC CGA CCT CTC TCC TCC AAC GTG GCC        578
   Glu Lys Ile Pro Leu Val Leu Ser Arg Pro Leu Ser Ser Asn Val Ala
               175                 180                 185
   ACC CTC CAG CAT CTT TGT CGG AAG ACT GTC AAC GGC CAC CTG GAC TCC        626
   Thr Leu Gln His Leu Cys Arg Lys Thr Val Asn Gly His Leu Asp Ser
           190                 195                 200
   TAT GAG AAA GTG ACC CAG CTG CCT GGA CCC ATT CGG GAG TTC CTG GAT        674
   Tyr Glu Lys Val Thr Gln Leu Pro Gly Pro Ile Arg Glu Phe Leu Asp
       205                 210                 215
   CAG TAT GAT GCT CCA CTT TAAGGAGCAA AAGGGTCAGA GGGGGGCCTG               722
   Gln Tyr Asp Ala Pro Leu
   220                 225
   GGTCGGTCGG TCGCCTCTCC TCCGAGGCAC ATGGCACAAG CACAAAAATC CAGCCCCAAC    782
   GGTCGGTAGC TCCCAGTGAG CCAGGGGCAG ATTGGCTTCT TCCTCAGGCC CTCCACTCCC    842
   GCAGAGTAGA GCTGGCAGGA CCTGGAATTC GTCTGAGGGG AGGGGGAGCT GCCACCTGCT    902
   TTCCCCCCTC CCCCAGCTCC AGCTTCTTTC AAGTGGAGCC AGCCGGCCTG GCCTGGTGGG    962
   ACAATACCTT TGACAAGCGG ACTCTCCCCT CCCCTTCCTC CACACCCCCT CTGCTTCCCA   1022
   AGGGAGGTGG GGACACCTCC AAGTGTTGAA CTTAGAACTG CAAGGGGAAT CTTCAAACTT   1082
   TCCCGCTGGA ACTTGTTTGC GCTTTGATTT GGTTTGATCA AGAGCAGGCA CCTGGGGGAA   1142
   GGATGGAAGA GAAAAGGGTG TGTGAAGGGT TTTTATGCTG GCCAAAGAAA TAACCACTCC   1202
   CACTGCCCAA CCTAGGTGAG GAGTGGTGGC TCCTGGCTCT GGGGAGAGTG GCAAGGGGTG   1262
   ACCTGAAGAG AGCTATACTG GTGCCAGGCT CCTCTCCATG GGGCAGCTAA TGAAACCTCG   1322
   CAGATCCCTT GCACCCCAGA ACCCTCCCCG TTGTGAAGAG GCAGTAGCAT TTAGAAGGGA   1382
   GACAGATGAG GCTGGTGAGC TGGCCGCCTT TTCCAACACC GAAGGGAGGC AGATCAACAG   1442
   ATGAGCCATC TTGGAGCCCA GGTTTCCCCT GGAGCAGATG GAGGGTTCTG CTTTGTCTCT   1502
   CCTATGTGGG GCTAGGAGAC TCGCCTTAAA TGCCCTCTGT CCCAGGGATG GGGATTGGCA   1562
   CACAAGGAGC CAAACACAGC CAATAGGCAG AGAGTTGAGG GATTCACCCA GGTGGCTACA   1622
   GGCCAGGGGA AGTGGCTGCA GGGGAGAGAC CCAGTCACTC CAGGAGACTC CTGAGTTAAC   1682
   ACTGGGAAGA CATTGGCCAG TCCTAGTCAT CTCTCGGTCA GTAGGTCCGA GAGCTTCCAG   1742
   GCCCTGCACA GCCCTCCTTT CTCACCTGGG GGGAGGCAGG AGGTGATGGA GAAGCCTTCC   1802
   CATGCCGCTC ACAGGGGCCT CACGGGAATG CAGCAGCCAT GCAATTACCT GGAACTGGTC   1862
   CTGTGTTGGG GAGAAACAAG TTTTCTGAAG TCAGGTATGG GGCTGGGTGG GGCAGCTGTG   1922
   TGTTGGGGTG GCTTTTTTCT CTCTGTTTTG AATAATGTTT ACAATTTGCC TCAATCACTT   1982
   TTATAAAAAT CCACCTCCAG CCCGCCCCTC TCCCCACTCA GGCCTTCGAG GCTGTCTGAA   2042
   GATGCTTGAA AAACTCAACC AAATCCCAGT TCAACTCAGA CTTTGCACAT ATATTTATAT   2102
   TTATACTCAG AAAAGAAACA TTTCAGTAAT TTATAATAAA AGAGCACTAT TTTTTAATGA   2162
   AAAAAAAAAA AAAAAAAAAA AAAAA                                         2187
(2) INFORMATION FOR SEQ ID NO:8:
   (i) SEQUENCE CHARACTERISTICS:
      (A) LENGTH: 225 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Met Val Thr His Ser Lys Phe Pro Ala Ala Gly Met Ser Arg Pro Leu
   1 5 10 15
   Asp Thr Ser Leu Arg Leu Lys Thr Phe Ser Ser Lys Ser Glu Tyr Gln
   20 25 30
   Leu Val Val Asn Ala Val Arg Lys Leu Gln Glu Ser Gly Phe Tyr Trp
   35 40 45
   Ser Ala Val Thr Gly Gly Glu Ala Asn Leu Leu Leu Ser Ala Glu Pro
   50 55 60
   Ala Gly Thr Phe Leu Ile Arg Asp Ser Ser Asp Gln Arg His Phe Phe
   65 70 75 80
   Thr Leu Ser Val Lys Thr Gln Ser Gly Thr Lys Asn Leu Arg Ile Gln
   85 90 95
   Cys Glu Gly Gly Ser Phe Ser Leu Gln Ser Asp Pro Arg Ser Thr Gln
   100 105 110
   Pro Val Pro Arg Phe Asp Cys Val Leu Lys Leu Val His His Tyr Met
   115 120 125
   Pro Pro Pro Gly Thr Pro Ser Phe Ser Leu Pro Pro Thr Glu Pro Ser
   130 135 140
   Ser Glu Val Pro Glu Gln Pro Pro Ala Gln Ala Leu Pro Gly Ser Thr
   145 150 155 160
   Pro Lys Arg Ala Tyr Tyr Ile Tyr Ser Gly Gly Glu Lys Ile Pro Leu
   165 170 175
   Val Leu Ser Arg Pro Leu Ser Ser Asn Val Ala Thr Leu Gln His Leu
   180 185 190
   Cys Arg Lys Thr Val Asn Gly His Leu Asp Ser Tyr Glu Lys Val Thr
   195 200 205
   Gln Leu Pro Gly Pro Ile Arg Glu Phe Leu Asp Gln Tyr Asp Ala Pro
   210 215 220
   Leu
   225
(2) INFORMATION FOR SEQ ID NO:9:
   (i) SEQUENCE CHARACTERISTICS:
      (A) LENGTH: 1094 base pairs
      (B) TYPE: nucleic acid
      (D) TOPOLOGY: linear
   (ii) MOLECULE TYPE: protein
   (xi) SEQUENCE DESCRIPTION: SEQ ID NO:9:
   CTCCGGCTGG CCCCTTCTGT AGGATGGTAG CACACAACCA GGTGGCAGCC GACAATGCAG     60
   TCTCCACAGC AGCAGAGCCC CGACGGCGGC CAGAACCTTC CTCCTCTTCC TCCTCCTCGC    120
   CCGCGGCCCC CGCGCGCCCG CGGCCGTGCC CCGCGGTCCC GGCCCCGGCC CCCGGCGACA    180
   CGCACTTCCG CACATTCCGT TCGCACGCCG ATTACCGGCG CATCACGCGC GCCAGCGCGC    240
   TCCTGGACGC CTGCGGATTC TACTGGGGGC CCCTGAGCGT GCACGGGGCG CACGAGCGGC    300
   TGCGCGCCGA GCCCGTGGGC ACCTTCCTGG TGCGCGACAG CCGCCAGCGG AACTGCTTTT    360
   TCGCCCTTAG CGTGAAGATG GCCTCGGGAC CCACGAGCAT CCGCGTGCAC TTTCAGGCCG    420
   GCCGCTTTCA CCTGGATGGC AGCCGCGAGA GCTTCGACTG CCTCTTCGAG CTGCTGGAGC    480
   ACTACGTGGC GGCGCCGCGC CGCATGCTGG GGGCCCCGCT GCGCCAGCGC CGCGTGCGGC    540
   CGCTGCAGGA GCTGTGCCGC CAGCGCATCG TGGCCACCGT GGGCCGCGAG AACCTGGCTC    600
   GCATCCCCCT CAACCCCGTC CTCCGCGACT ACCTGAGCTC CTTCCCCTTC CAGATTTGAC    660
   CGGCAGCGCC CGCCGTGCAC GCAGCATTAA CTGGGATGCC GTGTTATTTT GTTATTACTT    720
   GCCTGGAACC ATGTGGGTAC CCTCCCCGGC CTGGGTTGGA GGGAGCGGAT GGGTGTAGGG    780
   GCGAGGCGCC TCCCGCCCTC GGCTGGAGAC GAGGCCGCAG ACCCCTTCTC ACCTCTTGAG    840
   GGGGTCCTCC CCCTCCTGGT GCTCCCTCTG GGTCCCCCTG GTTGTTGTAG CAGCTTAACT    900
   GTATCTGGAG CCAGGACCTG AACTCGCACC TCCTACCTCT TCATGTTTAC ATATACCCAG    960
   TATCTTTGCA CAAACCAGGG GTTGGGGGAG GGTCTCTGGC TTTATTTTTC TGCTGTGCAG   1020
   AATCCTATTT TATATTTTTT AAAGTCAGTT TAGGTAATAA ACTTTATTAT GAAAGTTTTT   1080
   TTTTTTAAAA AAAA                                                     1094
(2) INFORMATION FOR SEQ ID NO:10:
   (i) SEQUENCE CHARACTERISTICS:
      (A) LENGTH: 211 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:10:
   Met Val Ala His Asn Gln Val Ala Ala Asp Asn Ala Val Ser Thr Ala
   1               5                   10                  15
   Ala Glu Pro Arg Arg Arg Pro Glu Pro Ser Ser Ser Ser Ser Ser Ser
               20                  25                  30
   Pro Ala Ala Pro Ala Arg Pro Arg Pro Cys Pro Ala Val Pro Ala Pro
           35                  40                  45
   Ala Pro Gly Asp Thr His Phe Arg Thr Phe Arg Ser His Ala Asp Tyr
       50                  55                  60
   Arg Arg Ile Thr Arg Ala Ser Ala Leu Leu Asp Ala Cys Gly Phe Tyr
   65                  70                  75                  80
   Trp Gly Pro Leu Ser Val His Gly Ala His Glu Arg Leu Arg Ala Glu
                   85                  90                  95
   Pro Val Gly Thr Phe Leu Val Arg Asp Ser Arg Gln Arg Asn Cys Phe
               100                 105                 110
   Phe Ala Leu Ser Val Lys Met Ala Ser Gly Pro Thr Ser Ile Arg Val
           115                 120                 125
   His Phe Gln Ala Gly Arg Phe His Leu Asp Gly Ser Arg Glu Ser Phe
       130                 135                 140
   Asp Cys Leu Phe Glu Leu Leu Glu His Tyr Val Ala Ala Pro Arg Arg
   145                 150                 155                 160
   Met Leu Gly Ala Pro Leu Arg Gln Arg Arg Val Arg Pro Leu Gln Glu
                   165                 170                 175
   Leu Cys Arg Gln Arg Ile Val Ala Thr Val Gly Arg Glu Asn Leu Ala
               180                 185                 190
   Arg Ile Pro Leu Asn Pro Val Leu Arg Asp Tyr Leu Ser Ser Phe Pro
           195                 200                 205
   Phe Gln Ile
       210
(2) INFORMATION FOR SEQ ID NO:11:
   (i) SEQUENCE CHARACTERISTICS:
      (A) LENGTH: 2807 base pairs
      (B) TYPE: nucleic acid
      (D) TOPOLOGY: linear
   (ii) MOLECULE TYPE: protein
   (xi) SEQUENCE DESCRIPTION: SEQ ID NO:11:
   GGAAACCGAG GCGGGGAGAC CAGGAGGCCT TGGCCTCAGA GCTTCAGAGT CGCGTGGCAG     60
   CAAACAGAGA AACCTGTAGA GGGCAGTGTG CGTCACTTAG CTCAGGGAAG CTGCACGCGA    120
   AACTCACCCG CCTTCATTCA TAAACATCGT CAGCTAGGCA CCTACTCCTG GGCTTTCAGG    180
   ACAAACTGAA TCACGAAACC ACAGTGTCCT TAAAATAGGT CTGACCGCCT GAATCCCTGG    240
   CCAAGGTGTG TACGGGGCAT GGGAGCCCTT GTGCAGAGAT GCTTGCAGGA GCCTTGAGGG    300
   GCTCTGTAAG ACAGAGGCTA GGAAGACAAA GTTGGGGGCT ACAGCTTCTT GTCCTGCCCG    360
   GGGCCTCAGT TTCTTCGGTT GCCCACGTAG GAGTGCAGAG AGTCCAGCCC CTGGGGACCC    420
   AACCCAACCC CGCCCAGTTT CCGAGGAACT CGTCCGGGAG CGGGGGCGCC CCTCCCGCAC    480
   CGCCTTAGGC TTCCTTTGAA GCCTCTGCGG TCAGGCCACC GCTTCCTGGG AAGCCCAAGC    540
   CAAGGCCAGG CCGAGTGGCC AACGGGAGGG GCCCGCGCGC GATTCTGGAG GAGGGCGGCG      600
   GCCCCACAGG TCTCCAGGGC TGGCTAGCCG GGCTCCTAGA GCGGAGACTG CCAAGGCCTT      660
   CGGGTCCTGG GCAGGAAGGA TCCTGGCAGG GAGGAGTTGC TTGGGGGGTG GGGGGGAAAG      720
   GCTCCAGGCG CGGTGGAGCT CTGACCAGGA GAATGCACAC ACTCGGAGGG GAGGAGGCGT      780
   GTCAGCCCCA AGCTAGCATC CCACCCGGGG AGCAGCGATG TGGGGCGAAG GTAGCCAGAG      840
   CAAAAGAGCA GGCACCAGGT GACACGAAAC AGAAGATTCC GGGTAGAGCC AGAACCCCAG      900
   AAGTCCCATT CAGGGAAGGT GCGAGGCGAG AACGAGTTAG GTGGACCCTC TCCAGGGGCA      960
   GCCAAAGAAA TCTAAAGAGA ACCCGAAGGA CTTGCCGGAA AGAGAAACCG AAAGCGGCGG     1020
   TGGGCGGGAT CGGTGGGCGG GGCCTCCCTG GTTTAAGAGC TTGATGCAGG GGCGGGCAGC     1080
   AGCAGAGAGA ACTGCGGCCG TGGCAGCGGC ACGGCTCCCG GCCCCGGAGC ATGCGCGACA     1140
   GCAGCCCCGG AACCCCCAGC CGCGGCGCCC CGCGTCCCGC CGCCAGGTGA GCCGAGGCAG     1200
   CTGCGAAGGA GCAGGCGGGA GGGGATGGGA GGAAGGGGAG CAGAGCCTGG CAGGACTATC     1260
   CTCGCAGACT GCATGGCGGG GTCGTGGATG CTATGCCTCT GGCGCCCGCC CCACCGGCTG     1320
   GCCCAGGCGG CCCCTCGCGC GCGCGGGGCG CCGTCAGCCC CTCCTCTCCG GCCCTGAGCC     1380
   CGGATCGTCC GCCCGGGTTC CAGTTCCCGG CGTGGCCAGT AGGCGGCAAC CGCGAGGCGG     1440
   CAAGCCACCC AGCGGGGACG GCCTGGAGTC GGGCCCCTCT CCACGCCCCC TTCTCCACGC     1500
   GCGCGGGGAG GCAGGGCTCC ACCGCCAGTC TGGAAGGGTT CCACATACAG GAACGGCCTA     1560
   CTTCGCAGAT GAGCCCACCG AGGCTCAGGC TCCGGGCGGA TTCTGCGTGT CACCCTCGCT     1620
   CCTTGGGGTC CGCTGGCCGG CCTGTGCCAC CCGGACGCCC GGTTCACTGC CTCTGTCTCC     1680
   CCCATCAGCG CAGCCCCGGA CGCTATGGCC CACCCCTCCA GCTGGCCCCT CGAGTAGGAT     1740
   GGTAGCACGT AACCAGGTGG AAGCCGACAA TGCGATCTCC CCGGCATCAG AGCCCCGACG     1800
   GCGGCCAGAG CCATCCTCGT CCTCGTCTTC GTCCTCGCCG GCGGCCCCGG CGCGTCCCCG     1860
   GCCCTGCCCG GTGGTCCCGG CCCCGGCTCC GGGCGACACT CACTTCCGCA CCTTCCGCTC     1920
   CCACTCTGAT TACCGGCGCA TCACGCGGAC CAGCGCTCTC CTGGACGCCT GCGGCTTCTA     1980
   CTGGGGACCC CTGAGCGTGC ATGGGGCGCA CGAACGGCTG CGTTCCGAAC CCGTGGGCAC     2040
   CTTCTTGGTG CGCGACAGTC GCCAGCGGAA CTGCTTCTTC GCGCTCAGCG TGAAGATGGC     2100
   TTCGGGCCCC ACGAGCATTC GTGTGCACTT CCAGGCCGGC CGCTTCCACC TGGACGGCAA     2160
   CCGCGAGACC TTCGACTGCC TCTTCGAGCT GCTGGAGCAC TACGTGGCGG CGCCGCGCCG     2220
   CATGTTGGGG GCCCCACTGC GCCAGCGCCG CGTGCGGCCG CTGCAGGAGC TGTGTCGCCA     2280
   GCGCATCGTG GCCGCCGTGG GTCGCGAGAA CCTGGCACGC ATCCCTCTTA ACCCGGTACT     2340
   CCGTGACTAC CTGAGTTCCT TCCCCTTCCA GATCTGACCG GCTGCCGCCG TGCCCGCAGA     2400
   ATTAAGTGGG AGCGCCTTAT TATTTCTTAT TATTAATTAT TATTATTTTT CTGGAACCAC     2460
   GTGGGAGCCC TCCCCGCCTA GGTCGGAGGG AGTGGGTGTG GAGGGTGAGA TCCCTCCCAC     2520
   TTCTGGCTGG AGACCTTATC CCGCCTCTCG GGGGGCCTCC CCTCCTGGTG CTCCCTCCCG     2580
   GTCCCCCTGG TTGTAGCAGC TTGTGTCTGG GGCCAGGACC TGAACTCCAC GCCTACCTCT     2640
   CCATGTTTAC ATGTTCCCAG TATCTTTGCA CAAACCAGGG GTGGGGGAGG GTCTCTGGCT     2700
   TCATTTTTCT GCTGTGCAGA ATATTCTATT TTATATTTTT ACATCCAGTT TAGATAATAA     2760
   ACTTTATTAT GAAAGTTTTT TTTTTTAAAG AAACAAAGAT TTCTAGA                   2807
(2) INFORMATION FOR SEQ ID NO:12:
   (i) SEQUENCE CHARACTERISTICS:
      (A) LENGTH: 212 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:12:
   Met Val Ala Arg Asn Gln Val Glu Ala Asp Asn Ala Ile Ser Pro Ala
   1               5                   10                  15
   Ser Glu Pro Arg Arg Arg Pro Glu Pro Ser Ser Ser Ser Ser Ser Ser
               20                  25                  30
   Ser Pro Ala Ala Pro Ala Arg Pro Arg Pro Cys Pro Val Val Pro Ala
           35                  40                  45
   Pro Ala Pro Gly Asp Thr His Phe Arg Thr Phe Arg Ser His Ser Asp
       50                  55                  60
   Tyr Arg Arg Ile Thr Arg Thr Ser Ala Leu Leu Asp Ala Cys Gly Phe
   65                  70                  75                  80
   Tyr Trp Gly Pro Leu Ser Val His Gly Ala His Glu Arg Leu Arg Ser
                   85                  90                  95
   Glu Pro Val Gly Thr Phe Leu Val Arg Asp Ser Arg Gln Arg Asn Cys
               100                 105                 110
   Phe Phe Ala Leu Ser Val Lys Met Ala Ser Gly Pro Thr Ser Ile Arg
           115                 120                 125
   Val His Phe Gln Ala Gly Arg Phe His Leu Asp Gly Asn Arg Glu Thr
       130                 135                 140
   Phe Asp Cys Leu Phe Glu Leu Leu Glu His Tyr Val Ala Ala Pro Arg
   145                 150                 155                 160
   Arg Met Leu Gly Ala Pro Leu Arg Gln Arg Arg Val Arg Pro Leu Gln
                   165                 170                 175
   Glu Leu Cys Arg Gln Arg Ile Val Ala Ala Val Gly Arg Glu Asn Leu
               180                 185                 190
   Ala Arg Ile Pro Leu Asn Pro Val Leu Arg Asp Tyr Leu Ser Ser Phe
           195                 200                 205
   Pro Phe Gln Ile
       210
(2) INFORMATION FOR SEQ ID NO:13:
   (i) SEQUENCE CHARACTERISTICS:
      (A) LENGTH: 1611 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS
      (B) LOCATION: 263..1529
   (xi) SEQUENCE DESCRIPTION: SEQ ID NO:13:
   CGAATTCCGG GCGGGCTGTG TGAGTCTGTG AGTGGAAGGC GCGCCGGCTC TTTTGTCTGA       60
   GTGTGACCCG GTGGCTTTGT TCCAGGCATT CCGGTGATTT CCTCCGGGCA GTCCGCAGAA      120
   GCCGCAGCGG CCGCCCGCGC TCTCTCTGCA GTCTCCACAC CCGGGAGAGC CTGAGCCCGC      180
   GTCACGCCCC TCAGCCCCCG CTGAGTCCCT TCTCTGTTGT CGCGTCCGAA TCGAGTTCCC      240
   GGAATCAGAC GGTGCCCCAT AG ATG GCC AGC TTT CCC CCG AGG GTT AAC GAG      292
                            Met Ala Ser Phe Pro Pro Arg Val Asn Glu
                             1               5                  10
   AAA GAG ATC GTG AGA TCA CGT ACT ATA GGG GAA CTC TTG GCT CCA GCA      340
   Lys Glu Ile Val Arg Ser Arg Thr Ile Gly Glu Leu Leu Ala Pro Ala
                   15                  20                  25
   GCT CCT TTT GAC AAG AAA TGT GGT GGT GAG AAC TGG ACG GTT GCT TTT      388
   Ala Pro Phe Asp Lys Lys Cys Gly Gly Glu Asn Trp Thr Val Ala Phe
               30                  35                  40
   GCT CCT GAT GGT TCC TAC TTT GCG TGG TCA CAA GGA TAT CGC ATA GTG     436
   Ala Pro Asp Gly Ser Tyr Phe Ala Trp Ser Gln Gly Tyr Arg Ile Val
               45                  50                  55
   AAG CTT GTC CCG TGG TCC CAG TGC CGT AAG AAC TTT CTT TTG CAT GGT     484
   Lys Leu Val Pro Trp Ser Gln Cys Arg Lys Asn Phe Leu Leu His Gly
           60                  65                  70
   TCC AAA AAT GTT ACC AAT TCA AGC TGT CTA AAA TTG GCA AGA CAA AAC     532
   Ser Lys Asn Val Thr Asn Ser Ser Cys Leu Lys Leu Ala Arg Gln Asn
    75                  80                  85                  90
   AGT AAT GGT GGT CAG AAA AAC AAG CCT CCT GAG CAC GTT ATA GAC TGT     580
   Ser Asn Gly Gly Gln Lys Asn Lys Pro Pro Glu His Val Ile Asp Cys
                    95                 100                 105
   GGA GAC ATA GTC TGG AGT CTT GCT TTT GGG TCT TCA GTT CCA GAA AAA     628
   Gly Asp Ile Val Trp Ser Leu Ala Phe Gly Ser Ser Val Pro Glu Lys
               110                 115                 120
   CAG AGT CGT TGC GTT AAT ATA GAA TGG CAT CGG TTC CGA TTT GGA CAG     676
   Gln Ser Arg Cys Val Asn Ile Glu Trp His Arg Phe Arg Phe Gly Gln
           125                 130                 135
   GAT CAG CTA CTC CTT GCC ACA GGA TTA AAC AAT GGT CGC ATC AAA ATC     724
   Asp Gln Leu Leu Leu Ala Thr Gly Leu Asn Asn Gly Arg Ile Lys Ile
       140                 145                 150
   TGG GAT GTA TAT ACA GGA AAA CTC CTC CTT AAT TTG GTA GAC CAC ATT     772
   Trp Asp Val Tyr Thr Gly Lys Leu Leu Leu Asn Leu Val Asp His Ile
   155                 160                 165                 170
   GAA ATG GTT AGA GAT TTA ACT TTT GCT CCA GAT GGG AGC TTA CTC CTT     820
   Glu Met Val Arg Asp Leu Thr Phe Ala Pro Asp Gly Ser Leu Leu Leu
                   175                 180                 185
   GTA TCA GCT TCA AGA GAC AAA ACT CTA AGA GTG TGG GAC CTG AAA GAT     868
   Val Ser Ala Ser Arg Asp Lys Thr Leu Arg Val Trp Asp Leu Lys Asp
               190                 195                 200
   GAT GGA AAC ATG GTG AAA GTA TTG CGG GCA CAT CAG AAT TGG GTG TAC     916
   Asp Gly Asn Met Val Lys Val Leu Arg Ala His Gln Asn Trp Val Tyr
           205                 210                 215
   AGT TGT GCA TTC TCT CCC GAC TGT TCT ATG CTG TGT TCA GTG GGC GCC     964
   Ser Cys Ala Phe Ser Pro Asp Cys Ser Met Leu Cys Ser Val Gly Ala
       220                 225                 230
   AGT AAA GCA GTT TTC CTT TGG AAT ATG GAT AAA TAC ACC ATG ATT AGG      1012
   Ser Lys Ala Val Phe Leu Trp Asn Met Asp Lys Tyr Thr Met Ile Arg
   235                 240                 245                 250
   AAG CTG GAA GGT CAT CAC CAT GAT GTT GTA GCT TGT GAC TTT TCT CCT      1060
   Lys Leu Glu Gly His His His Asp Val Val Ala Cys Asp Phe Ser Pro
                   255                 260                 265
   GAT GGA GCA TTG CTA GCT ACT GCA TCC TAT GAC ACT CGT GTG TAT GTC      1108
   Asp Gly Ala Leu Leu Ala Thr Ala Ser Tyr Asp Thr Arg Val Tyr Val
               270                 275                 280
   TGG GAT CCA CAC AAT GGA GAC CTT CTG ATG GAG TTT GGG CAC CTG TTT      1156
   Trp Asp Pro His Asn Gly Asp Leu Leu Met Glu Phe Gly His Leu Phe
           285                 290                 295
   CCC TCG CCC ACT CCA ATA TTT GCT GGA GGA GCA AAT GAC CGA TGG GTG      1204
   Pro Ser Pro Thr Pro Ile Phe Ala Gly Gly Ala Asn Asp Arg Trp Val
       300                 305                 310
   AGA GCT GTG TCT TTC AGT CAT GAT GGA CTG CAT GTT GCC AGC CTT GCT      1252
   Arg Ala Val Ser Phe Ser His Asp Gly Leu His Val Ala Ser Leu Ala
   315                 320                 325                 330
   GAT GAT AAA ATG GTG AGG TTC TGG AGA ATC GAT GAG GAT TGT CCG GTA      1300
   Asp Asp Lys Met Val Arg Phe Trp Arg Ile Asp Glu Asp Cys Pro Val
                   335                 340                 345
   CAA GTT GCA CCT TTG AGC AAT GGT CTT TGC TGT GCC TTT TCT ACT GAT      1348
   Gln Val Ala Pro Leu Ser Asn Gly Leu Cys Cys Ala Phe Ser Thr Asp
               350                 355                 360
   GGC AGT GTT TTA GCT GCT GGG ACA CAT GAT GGA AGT GTG TAT TTT TGG      1396
   Gly Ser Val Leu Ala Ala Gly Thr His Asp Gly Ser Val Tyr Phe Trp
           365                 370                 375
   GCC ACT CCA AGG CAA GTC CCT AGC CTT CAA CAT ATA TGT CGC ATG TCA      1444
   Ala Thr Pro Arg Gln Val Pro Ser Leu Gln His Ile Cys Arg Met Ser
       380                 385                 390
   ATC CGA AGA GTG ATG TCC ACC CAA GAA GTC CAA AAA CTG CCT GTT CCT      1492
   Ile Arg Arg Val Met Ser Thr Gln Glu Val Gln Lys Leu Pro Val Pro
   395                 400                 405                 410
   TCC AAA ATA TTG GCG TTT CTC TCC TAC CGC GGT TAG A CTGAAGACTG         1539
   Ser Lys Ile Leu Ala Phe Leu Ser Tyr Arg Gly  *
                   415                 420
   CCTTTCCTGG TAGGCCTGCC AGACAGAGCG CCCTTTACAA GACACACCTC AAGCTTTACC    1599
   TCGTGCCGAA TT                                                        1611
(2) INFORMATION FOR SEQ ID NO:14:
   (i) SEQUENCE CHARACTERISTICS:
      (A) LENGTH: 422 amino acids
      (B) TYPE: amino acid
      (D) TOPOLOGY: linear
   (ii) MOLECULE TYPE: protein
   (xi) SEQUENCE DESCRIPTION: SEQ ID NO:14:
   Met Ala Ser Phe Pro Pro Arg Val Asn Glu Lys Glu Ile Val Arg Ser
     1               5                  10                  15
   Arg Thr Ile Gly Glu Leu Leu Ala Pro Ala Ala Pro Phe Asp Lys Lys
                20                  25                  30
   Cys Gly Gly Glu Asn Trp Thr Val Ala Phe Ala Pro Asp Gly Ser Tyr
            35                  40                  45
   Phe Ala Trp Ser Gln Gly Tyr Arg Ile Val Lys Leu Val Pro Trp Ser
        50                  55                  60
   Gln Cys Arg Lys Asn Phe Leu Leu His Gly Ser Lys Asn Val Thr Asn
    65                  70                  75                  80
   Ser Ser Cys Leu Lys Leu Ala Arg Gln Asn Ser Asn Gly Gly Gln Lys
                    85                  90                  95
   Asn Lys Pro Pro Glu His Val Ile Asp Cys Gly Asp Ile Val Trp Ser
               100                 105                 110
   Leu Ala Phe Gly Ser Ser Val Pro Glu Lys Gln Ser Arg Cys Val Asn
           115                 120                 125
   Ile Glu Trp His Arg Phe Arg Phe Gly Gln Asp Gln Leu Leu Leu Ala
       130                 135                 140
   Thr Gly Leu Asn Asn Gly Arg Ile Lys Ile Trp Asp Val Tyr Thr Gly
   145                 150                 155                 160
   Lys Leu Leu Leu Asn Leu Val Asp His Ile Glu Met Val Arg Asp Leu
                   165                 170                 175
   Thr Phe Ala Pro Asp Gly Ser Leu Leu Leu Val Ser Ala Ser Arg Asp
               180                 185                 190
   Lys Thr Leu Arg Val Trp Asp Leu Lys Asp Asp Gly Asn Met Val Lys
           195                 200                 205
   Val Leu Arg Ala His Gln Asn Trp Val Tyr Ser Cys Ala Phe Ser Pro
       210                 215                 220
   Asp Cys Ser Met Leu Cys Ser Val Gly Ala Ser Lys Ala Val Phe Leu
   225                 230                 235                 240
   Trp Asn Met Asp Lys Tyr Thr Met Ile Arg Lys Leu Glu Gly His His
                   245                 250                 255
   His Asp Val Val Ala Cys Asp Phe Ser Pro Asp Gly Ala Leu Leu Ala
               260                 265                 270
   Thr Ala Ser Tyr Asp Thr Arg Val Tyr Val Trp Asp Pro His Asn Gly
           275                 280                 285
   Asp Leu Leu Met Glu Phe Gly His Leu Phe Pro Ser Pro Thr Pro Ile
       290                 295                 300
   Phe Ala Gly Gly Ala Asn Asp Arg Trp Val Arg Ala Val Ser Phe Ser
   305                 310                 315                 320
   His Asp Gly Leu His Val Ala Ser Leu Ala Asp Asp Lys Met Val Arg
                   325                 330                 335
   Phe Trp Arg Ile Asp Glu Asp Cys Pro Val Gln Val Ala Pro Leu Ser
               340                 345                 350
   Asn Gly Leu Cys Cys Ala Phe Ser Thr Asp Gly Ser Val Leu Ala Ala
           355                 360                 365
   Gly Thr His Asp Gly Ser Val Tyr Phe Trp Ala Thr Pro Arg Gln Val
   370 375 380
   Pro Ser Leu Gln His Ile Cys Arg Met Ser Ile Arg Arg Val Met Ser
   385 390 395 400
   Thr Gln Glu Val Gln Lys Leu Pro Val Pro Ser Lys Ile Leu Ala Phe
   405 410 415
   Leu Ser Tyr Arg Gly *
   420
(2) INFORMATION FOR SEQ ID NO:15:
   (i) SEQUENCE CHARACTERISTICS:
      (A) LENGTH: 783 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:15:
   CTGTCTTCCT CCGCAGCGCG AGGCTGGGTA CAGGGTCTAT TGTCTGTGGT TGACTCCGTA 60
   CTTTGGTCTG AGGCCTTCGG GAGCTTTCCC GAGGCAGTTA GCAGAAGCCG CAGCGACCGC 120
   CCCCGCCCGT CTCCTCTGTC CCTGGGCCCG GGAGACAAAC TTGGCGTCAC GCCCTCAGCG 180
   GTCGCCACTC TCTTCTCTGT TGTTGGGTCC GCATCGTATT CCCGGAATCA GACGGTGCCC 240
   CATAGATGGC CAGCTTTCCC CCGAGGGTCA ACGAGAAAGA GATCGTGAGA TCACGTACTA 300
   TAGGTGAACT TTTAGCTCCT GCAGCTCCTT TTGACAAGAA ATGTGGTCGT GAAAATTGGA 360
   CTGTTGCTTT TGCTCCAGAT GGTTCATACT TTGCTTGGTC ACAAGGACAT CGCACAGTAA 420
   AGCTTGTTCC GTGGTCCCAG TGCCTTCAGA ACTTTCTCTT GCATGGCACC AAGAATGTTA 480
   CCAATTCAAG CAGTTTAAGA TTGCCAAGAC AAAATAGTGA TGGTGGTCAG AAAAATAAGC 540
   CTCGTGACAT ATTATAGACT GTGGAGATAT AGTCTGGAGT CTTGCTTTTG GGTCATCAGT 600
   TCCAGAAAAA CAGAGTCGCT GTGTAAATAT AGAATGGCAT CGCTTCAGAT TTGGACAAGA 660
   TCAGCTACTT CTTGCTACAG GGTTGAACAA TGGGCGTATC AAAATATGGG ATGTATATCA 720
   GGAAACTCCT CCTTAACTTG GTAGATCATA CTGAAGTGGT CAGAGATTTA ACTTTTGCTC 780
   CAG 783
(2) INFORMATION FOR SEQ ID NO:16:
   (i) SEQUENCE CHARACTERISTICS:
      (A) LENGTH: 1122 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:16:
   CTCTGTATGT CTGAATGAAG CTATAACATT TGCCTTTTTA TTGCAGGTTT TCCTTTGGAA 60
   TATGGATAAA TACACCATGA TACGGAAACT AGAAGGACAT CACCATGATG TGGTAGCTTG 120
   TGACTTTTCT CCTGATGGAG CATTACTGGC TACTGCATCT TATGATACTC GAGTATATAT 180
   CTGGGATCCA CATAATGGAG ACATTCTGAT GGAATTTGGG CACCTGTTTC CCCCACCTAC 240
   TCCAATATTT GCTGGAGGAG CAAATGACCG GTGGGTACGA TCTGTATCTT TTAGCCATGA 300
   TGGACTGCAT GTTGCAAGCC TTGCTGATGA TAAAATGGTG AGGTTCTGGA GAATTGATGA 360
   GGATTATCCA GTGCAAGTTG CACCTTTGAG CAATGGTCTT TGCTGTGCCT TCTCTACTGA 420
   TGGCAGTGTT TTAGCTGCTG GGACACATGA CGGAAGTGTG TATTTTTGGG CCACTCCACG 480
   GCAGGTCCCT AGCCTGCAAC ATTTATGTCG CATGTCAATC CGAAGAGTGA TGCCCACCCA 540
   AGAAGTTCAG GAGCTGCCGA TTCCTTCCAA GCTTTTGGAG TTTCTCTCGT ATCGTATTTA 600
   GAAGATTCTG CCTTCCCTAG TAGTAGGGAC TGACAGAATA CACTTAACAC AAACCTCAAG 660
   CTTTACTGAC TTCAATTATC TGTTTTTAAA GACGTAGAAG ATTTATTTAA TTTGATATGT 720
   TCTTGTACTG CATTTTGATC AGTTGAGCTT TTAAAATATT ATTTATAGAC AATAGAAGTA 780
   TTTCTGAACA TATCAAATAT AAATTTTTTT AAAGATCTAA CTGTGAAAAC ATACATACCT 840
   GTACATATTT AGATATAAGC TGCTATATGT TGAATGGACC CTTTTGCTTT TCTGATTTTT 900
   AGTTCTGACA TGTATATATT GCTTCAGTAG AGCCACAATA TGTATCTTTG CTGTAAAGTG 960
   CAAGGAAATT TTAAATTCTG GGACACTGAG TTAGATGGTA AATACTGACT TACGAAAGTT 1020
   GAATTGGGTG AGGCGGGCAA ATCACCTGAG GTCAGCAGTT TGAGACTAGC CTGGCAAACA 1080
   TGATGAAACC CTGTCTCTAC TAAAAATACA AAAAAAAAAA AA 1122
(2) INFORMATION FOR SEQ ID NO:17:
   (i) SEQUENCE CHARACTERISTICS:
      (A) LENGTH: 2537 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS.
      (B) LOCATION: 422..2029
   (xi) SEQUENCE DESCRIPTION: SEQ ID NO:17:
   CGGCACGAGC CGGGCTCCGT CCGGAGGAAG CGAGGCTGCG CCGCCGGCCC GGCAGGAGCG 60
   GAGGACGGGA GCGCGGGCGG TCGCGCTCGC CCTGTCGCTG ACTGCGCTGC CCCGGCCCAT 120
   CCTTGCCTGG CCGCAGGTGC CCTGGATGAG GCCGCCGCGC GTGTCCCGGC CGCTGAGTGT 180
   CCCCCGCGGT CGCCCGGCGC CTGCCCTCAA GCGGCCGCCT CTCCTTGCCC GGGTCCCCGT 240
   TTTCCCCCGG CGCAGTCCTC CTCCGGTGGG CGCCTCCGCA CCTCGGCGCA GGCGGCACGG 300
   CCCTCGGGCC GGGATGGATC CGCCGGGAAG AGGAAGACAA GCCGGGGCGT TGAGCCCCTG 360
   CGCACGGTGC CGCCGCGCGT AGTGGGAGCT TACTCGCAGT AGGCTCTCGC TCTTCTAATC      420
   A ATG GAT AAA GTG GGG AAA ATG TGG AAC AAC TTA AAA TAC AGA TGC          466
     Met Asp Lys Val Gly Lys Met Trp Asn Asn Leu Lys Tyr Arg Cys
      1               5                  10                  15
   CAG AAT CTC TTC AGC CAC GAG GGA GGA AGC CGT AAT GAG AAC GTG GAG        514
   Gln Asn Leu Phe Ser His Glu Gly Gly Ser Arg Asn Glu Asn Val Glu
                       20                  25                  30
   ATG AAC CCC AAC AGA TGT CCG TCT GTC AAA GAG AAA AGC ATC AGT CTG        562
   Met Asn Pro Asn Arg Cys Pro Ser Val Lys Glu Lys Ser Ile Ser Leu
                   35                  40                  45
   GGA GAG GCA GCT CCC CAG CAA GAG AGC AGT CCC TTA AGA GAA AAT GTT        610
   Gly Glu Ala Ala Pro Gln Gln Glu Ser Ser Pro Leu Arg Glu Asn Val
               50                  55                  60
   GCC TTA CAG CTG GGA CTG AGC CCT TCC AAG ACC TTT TCC AGG CGG AAC        658
   Ala Leu Gln Leu Gly Leu Ser Pro Ser Lys Thr Phe Ser Arg Arg Asn
           65                  70                  75
   CAA AAC TGT GCC GCA GAG ATC CCT CAA GTG GTT GAA ATC AGC ATC GAG        706
   Gln Asn Cys Ala Ala Glu Ile Pro Gln Val Val Glu Ile Ser Ile Glu
    80                  85                  90                  95
   AAA GAC AGT GAC TCG GGT GCC ACC CCA GGA ACG AGG CTT GCA CGG AGA        754
   Lys Asp Ser Asp Ser Gly Ala Thr Pro Gly Thr Arg Leu Ala Arg Arg
                       100                 105                 110
   GAC TCC TAC TCG CGG CAC GCC CCG TGG GGA GGA AAG AAG AAA CAT TCC        802
   Asp Ser Tyr Ser Arg His Ala Pro Trp Gly Gly Lys Lys Lys His Ser
                   115                 120                 125
   TGT TCC ACA AAG ACC CAG AGT TCA TTG GAT ACC GAG AAA AAG TTT GGT        850
   Cys Ser Thr Lys Thr Gln Ser Ser Leu Asp Thr Glu Lys Lys Phe Gly
               130                 135                 140
   AGA ACT CGA AGC GGC CTT CAG AGG CGA GAG CGG CGC TAT GGA GTC AGC        898
   Arg Thr Arg Ser Gly Leu Gln Arg Arg Glu Arg Arg Tyr Gly Val Ser
           145                 150                 155
   TCC ATG CAG GAC ATG GAC AGC GTT TCT AGC CGC GCG GTC GGG AGC CGC        946
   Ser Met Gln Asp Met Asp Ser Val Ser Ser Arg Ala Val Gly Ser Arg
   160                 165                 170                 175
   TCC CTG AGG CAG AGG CTC CAG GAC ACG GTG GGT TTG TGT TTT CCC ATG        994
   Ser Leu Arg Gln Arg Leu Gln Asp Thr Val Gly Leu Cys Phe Pro Met
                   180                 185                 190
   AGA ACT TAC AGC AAG CAG TCA AAG CCA CTC TTT TCC AAT AAA AGA AAA       1042
   Arg Thr Tyr Ser Lys Gln Ser Lys Pro Leu Phe Ser Asn Lys Arg Lys
               195                 200                 205
   ATA CAT CTT TCT GAA TTA ATG CTG GAG AAA TGC CCT TTT CCT GCT GGC       1090
   Ile His Leu Ser Glu Leu Met Leu Glu Lys Cys Pro Phe Pro Ala Gly
           210                 215                 220
   TCG GAT TTA GCA CAA AAG TGG CAT TTG ATT AAA CAG CAT ACC GCC CCT       1138
   Ser Asp Leu Ala Gln Lys Trp His Leu Ile Lys Gln His Thr Ala Pro
       225                 230                 235
   GTG AGC CCA CAC TCA ACA TTT TTT GAT ACA TTT GAT CCA TCA CTG GTG       1186
   Val Ser Pro His Ser Thr Phe Phe Asp Thr Phe Asp Pro Ser Leu Val
   240                 245                 250                 255
   TCT ACA GAA GAT GAA GAA GAT AGG CTT CGC GAG AGA AGA CGG CTT AGT       1234
   Ser Thr Glu Asp Glu Glu Asp Arg Leu Arg Glu Arg Arg Arg Leu Ser
                   260                 265                 270
   ATC GAA GAA GGG GTG GAT CCC CCT CCC AAC GCA CAA ATA CAC ACC TTT       1282
   Ile Glu Glu Gly Val Asp Pro Pro Pro Asn Ala Gln Ile His Thr Phe
               275                 280                 285
   GAA GCT ACT GCA CAG GTC AAC CCA TTG TAT AAG CTG GGA CCA AAG TTA       1330
   Glu Ala Thr Ala Gln Val Asn Pro Leu Tyr Lys Leu Gly Pro Lys Leu
           290                 295                 300
   GCT CCT GGG ATG ACA GAG ATA AGT GGA GAT GGT TCT GCA ATT CCA CAA       1378
   Ala Pro Gly Met Thr Glu Ile Ser Gly Asp Gly Ser Ala Ile Pro Gln
       305                 310                 315
   GCA ATT GTG ACT CAG AAG AGG ATT CAA CCA CCC TAT GTC TGC AGT CAC       1426
   Ala Ile Val Thr Gln Lys Arg Ile Gln Pro Pro Tyr Val Cys Ser His
   320                 325                 330                 335
   GGA GGC AGA AGC AGC GCC AGG TGT CCG GGG ACA GCC ACG CGC ACG TTA       1474
   Gly Gly Arg Ser Ser Ala Arg Cys Pro Gly Thr Ala Thr Arg Thr Leu
                   340                 345                 350
   GCA GAC AGG GAG CTT GGA AAG TTC ATA CGC AGA TCG ATT ACA TAC ACT       1522
   Ala Asp Arg Glu Leu Gly Lys Phe Ile Arg Arg Ser Ile Thr Tyr Thr
               355                 360                 365
   GCC TCG TGC CAG ATT TGC TTC AGA TCA CAG GGA ATC CCT GTT ACT GGG      1570
   Ala Ser Cys Gln Ile Cys Phe Arg Ser Gln Gly Ile Pro Val Thr Gly
           370                 375                 380
   GCG TGA TGG ACC GAT ACG AGG CCG AAG CCC TTC TAG AAG GGA AAC CGG      1618
   Ala  *  Trp Thr Asp Thr Arg Pro Lys Pro Phe  *  Lys Gly Asn Arg
       385                 390                 395
   AAG GCA CGT TCT TGC TCA GGG ACT CTG CAC AGG AGG ACT ACC TCT TCT      1666
   Lys Ala Arg Ser Cys Ser Gly Thr Leu His Arg Arg Thr Thr Ser Ser
   400                 405                 410                 415
   CTG TGA GCT TCC GCC GCT ACA ACA GGT CTC TGC ACG CCC GGA TCG AGC      1714
   Leu  *  Ala Ser Ala Ala Thr Thr Gly Leu Cys Thr Pro Gly Ser Ser
                   420                 425                 430
   AGT GGA ACC ACA ACT TCA GCT TCG ATG CCC ATG ACC CCT GCG TGT TTC      1762
   Ser Gly Thr Thr Thr Ser Ala Ser Met Pro Met Thr Pro Ala Cys Phe
               435                 440                 445
   ACT CCT CCA CGT CAC GGG GCT TCT CGA ACA CTA TAA AGA CCC CAG CTC      1810
   Thr Pro Pro Arg His Gly Ala Ser Arg Thr Leu  *  Arg Pro Gln Leu
           450                 455                 460
   TTG CAT GTT TTT TGA ACC GTT GCT AAC GAT ATC ACT GAA TAG AAC TTT      1858
   Leu His Val Phe  *  Thr Val Ala Asn Asp Ile Thr Glu  *  Asn Phe
       465                 470                 475
   CCC TTT CAG CCT GCA GTA TAT CTG CCG CGC AGT GAT CTG CAG ATG CAC      1906
   Pro Phe Gln Pro Ala Val Tyr Leu Pro Arg Ser Asp Leu Gln Met His
   480                 485                 490                 495
   TAC GTA TGA TGG GAT TGA CGG GCT CCC GCT ACC GTC GAT GTT ACA GGA      1954
   Tyr Val  *  Trp Asp  *  Arg Ala Pro Ala Thr Val Asp Val Thr Gly
                   500                 505                 510
   TTT TTT AAA AGA GTA TCA TTA TAA ACA AAA AGT TAG GGT TCG CTG GTT      2002
   Phe Phe Lys Arg Val Ser Leu  *  Thr Lys Ser  *  Gly Ser Leu Val
               515                 520                 525
   AGA ACG AGA CCA GTC AAA GCA AAG TAACTCCTGT CCCCAAAGGG CACTAACTAA     2056
   Arg Thr Arg Pro Val Lys Ala Lys
   530 535
   GTCTGCTCCT CCCGTGCATC GAACTGCACC CATAGGAGGC AGTCAGCTGC TAGGATTTCC 2116
   CACCCAGAAT GGGAGCTTAG TCATTAGCCT CTGCCCTATG GGGTCCGCTG TTCCTCAGAC 2176
   AAAGGTGCCT AGGGACAGCA AGATGGCTTG CAGGTGTTCG GTGGGCTGTG ACAACTGAGG 2236
   GAGGCAACTC TGGGGCATTT GCTATGAAGA ATTCTATTTC TTACCGAAGA ACAAATTATT 2296
   AATATTGGAT GGGTATTTCA ATAGTGTGAC TAATGTTTGA AATTATTTTT TCTAAGAATT 2356
   TTTCTATAAC CTTCAGAAAA AGTAGTGATG TTTGTAGTTA CTATAAATCA AGCTTTGAAA 2416
   GTTCAAAACA AACAAGTTAA ATAAAAGACT ACCTTCCTTT TAGAGAAAAC AAATGCAAGT 2476
   TTTCCCAGCC ACAGGCATTG TGCACTGTTA ATGTTGCTTG TTATCAGCTC CTTTCTCCTC 2536
   C 2537
(2) INFORMATION FOR SEQ ID NO:18:
   (i) SEQUENCE CHARACTERISTICS:
      (A) LENGTH: 535 amino acids
      (B) TYPE: amino acid
      (D) TOPOLOGY: linear
   (ii) MOLECULE TYPE: protein
   (xi) SEQUENCE DESCRIPTION: SEQ ID NO:18:
   Met Asp Lys Val Gly Lys Met Trp Asn Asn Leu Lys Tyr Arg Cys Gln
   1 5 10 15
   Asn Leu Phe Ser His Glu Gly Gly Ser Arg Asn Glu Asn Val Glu Met
   20 25 30
   Asn Pro Asn Arg Cys Pro Ser Val Lys Glu Lys Ser Ile Ser Leu Gly
   35 40 45
   Glu Ala Ala Pro Gln Gln Glu Ser Ser Pro Leu Arg Glu Asn Val Ala
   50 55 60
   Leu Gln Leu Gly Leu Ser Pro Ser Lys Thr Phe Ser Arg Arg Asn Gln
   65                  70                  75                  80
   Asn Cys Ala Ala Glu Ile Pro Gln Val Val Glu Ile Ser Ile Glu Lys
                   85                  90                  95
   Asp Ser Asp Ser Gly Ala Thr Pro Gly Thr Arg Leu Ala Arg Arg Asp
               100                 105                 110
   Ser Tyr Ser Arg His Ala Pro Trp Gly Gly Lys Lys Lys His Ser Cys
           115                 120                 125
   Ser Thr Lys Thr Gln Ser Ser Leu Asp Thr Glu Lys Lys Phe Gly Arg
       130                 135                 140
   Thr Arg Ser Gly Leu Gln Arg Arg Glu Arg Arg Tyr Gly Val Ser Ser
   145                 150                 155                 160
   Met Gln Asp Met Asp Ser Val Ser Ser Arg Ala Val Gly Ser Arg Ser
                   165                 170                 175
   Leu Arg Gln Arg Leu Gln Asp Thr Val Gly Leu Cys Phe Pro Met Arg
               180                 185                 190
   Thr Tyr Ser Lys Gln Ser Lys Pro Leu Phe Ser Asn Lys Arg Lys Ile
           195                 200                 205
   His Leu Ser Glu Leu Met Leu Glu Lys Cys Pro Phe Pro Ala Gly Ser
       210                 215                 220
   Asp Leu Ala Gln Lys Trp His Leu Ile Lys Gln His Thr Ala Pro Val
   225                 230                 235                 240
   Ser Pro His Ser Thr Phe Phe Asp Thr Phe Asp Pro Ser Leu Val Ser
                   245                 250                 255
   Thr Glu Asp Glu Glu Asp Arg Leu Arg Glu Arg Arg Arg Leu Ser Ile
               260                 265                 270
   Glu Glu Gly Val Asp Pro Pro Pro Asn Ala Gln Ile His Thr Phe Glu
           275                 280                 285
   Ala Thr Ala Gln Val Asn Pro Leu Tyr Lys Leu Gly Pro Lys Leu Ala
       290                 295                 300
   Pro Gly Met Thr Glu Ile Ser Gly Asp Gly Ser Ala Ile Pro Gln Ala
   305                 310                 315                 320
   Ile Val Thr Gln Lys Arg Ile Gln Pro Pro Tyr Val Cys Ser His Gly
                   325                 330                 335
   Gly Arg Ser Ser Ala Arg Cys Pro Gly Thr Ala Thr Arg Thr Leu Ala
               340                 345                 350
   Asp Arg Glu Leu Gly Lys Phe Ile Arg Arg Ser Ile Thr Tyr Thr Ala
           355                 360                 365
   Ser Cys Gln Ile Cys Phe Arg Ser Gln Gly Ile Pro Val Thr Gly Ala
       370                 375                 380
    *  Trp Thr Asp Thr Arg Pro Lys Pro Phe  *  Lys Gly Asn Arg Lys
   385                 390                 395                 400
   Ala Arg Ser Cys Ser Gly Thr Leu His Arg Arg Thr Thr Ser Ser Leu
                   405                 410                 415
   *  Ala Ser Ala Ala Thr Thr Gly Leu Cys Thr Pro Gly Ser Ser Ser
              420                 425                 430
   Gly Thr Thr Thr Ser Ala Ser Met Pro Met Thr Pro Ala Cys Phe Thr
           435                 440                 445
   Pro Pro Arg His Gly Ala Ser Arg Thr Leu  *  Arg Pro Gln Leu Leu
       450                 455                 460
   His Val Phe  *  Thr Val Ala Asn Asp Ile Thr Glu  *  Asn Phe Pro
   465                 470                 475                 480
   Phe Gln Pro Ala Val Tyr Leu Pro Arg Ser Asp Leu Gln Met His Tyr
                   485                 490                 495
   Val  *  Trp Asp  *  Arg Ala Pro Ala Thr Val Asp Val Thr Gly Phe
               500                 505                 510
   Phe Lys Arg Val Ser Leu  *  Thr Lys Ser  *  Gly Ser Leu Val Arg
           515                 520                 525
   Thr Arg Pro Val Lys Ala Lys
       530                 535
(2) INFORMATION FOR SEQ ID NO:19:
   (i) SEQUENCE CHARACTERISTICS:
      (A) LENGTH: 1221 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:19:
   GATTAAACAG CATACAGCTC CTGTGAGCCC ACATTCAACA TTTTTTGATA CTTTGATCCA     60
   TCTTTGGTTT CTACAGAAGA TGAAGAAGAT AGGCTTAGAG AGAGAAGGCG GCTTAGTATT    120
   GAAGAAGGGG TTGATCCCCC TCCCAATGCA CAAATACATA CATTTGAAGC TACTGCACAG    180
   GTTAATCCAT TATTAAACTG GGACCAAAAT TAGCTCCTGG AATGACTGAA ATAAGTGGGG    240
   ACAGTTCTGC AATTCCACAA GCTAATTGTG ACTCGGAAGA GGATACAACC ACCCTGTGTT    300
   GCAGTCACGG AGGCAGAAGC AGCGTCAGAT ATCTGGAGAC AGCCATACCC ATGTTAGCAG    360
   ACAGGGAGCT TGGAAAGTCC ACACACAGAT TGATTACATA CACTGCTTCG TGCCTGATTT    420
   GCTTCAAATT ACAGGGAATC CCTGTTACTG GGGAGTGATG GACCGTTATG AAGCAGAAGC    480
   CCTTCTCGAA GGGAAACCTG AAGGCACGTT TTTGCTCAGG GACTCTGCGC AAGAGGACTA    540
   CTTCTTCTCT GTGAGCTTCC GCCGATACAA CAGATCCCTG CATGCCCGAA TTGAGCAGTG    600
   GAATCACAAC TTTAGTTTCG ACGCCCATGA CCCGTGTGTA TTTCACTCCT CCACTGTAAC    660
   GGGACTTTTA GAACATTATA AAGATCCCAG TTCGTGCATG TTTTTTGAAC CATTGCTTAC    720
   TATATCACTA AATAGGACTT TCCCTTTTAG CCTGCAGTAT ATCTGTCGCG CGGTAATCTG    780
   CAGGTGCACT ACGTATGATG GAATTGATGG GCTCCCTCTA CCCTCAATGT TACAGGATTT    840
   TTTAAAAGAG TATCATTATA AACAAAAAGT TAGAGTTCGC TGGTTGGAAC GAGAACCAGT    900
   CAAGGCAAAG TAAACTCTCC GGTCCCCAAA GGGTGTTAAC TAGGTCCGCT TTCATGTGCA    960
   TCAGACAGTA CACCTATAGC AAGCACACGT AGCAGTGTTA GGCTTTTTCA TACAGTATGT     1020
   AAGCTTAGTG TTAGTATCTG TCAGATGCTA CCTGCTGTTA CTTATTCAGA TAAACATGGT     1080
   GCCTATTGGA ACAATAGCGG ATAGAGCTAC AGGTGTTCAG TAAGACTACA AAAACATTTT     1140
   GCCTATTTCG CTAACAGTTT GGTTTTTAAT GGCTGTGGTA TTTGAGTGAG GCAACTCTGG     1200
   GGCATTTGTT ATGAAGAAAT G                                               1221
(2) INFORMATION FOR SEQ ID NO:20:
   (i) SEQUENCE CHARACTERISTICS:
      (A) LENGTH: 2369 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS
      (B) LOCATION: 116..1330
   (xi) SEQUENCE DESCRIPTION: SEQ ID NO:20:
   GGCACGAGGC GGTGGTGGCG GCGGCGGGCG CGGCCGCGGC GGGGCGGGCG CGGAATGAAG      60
   GCCCACGGCC CTGGGGGCTG AGGCGCCCGC CGCCTGGGGC GGGCCGCGCG TCCTC ATG      118
                                                                 Met
                                                                   1
   GAG GCC GGA GAG GAG CCG CTG CTG CTG GCT GAA CTC AAG CCT GGG CGC      166
   Glu Ala Gly Glu Glu Pro Leu Leu Leu Ala Glu Leu Lys Pro Gly Arg
                 5                  10                  15
   CCC CAC CAG TTC GAC TGG AAG TCA AGC TGC GAG ACC TGG AGC GTG GCC      214
   Pro His Gln Phe Asp Trp Lys Ser Ser Cys Glu Thr Trp Ser Val Ala
            20                  25                  30
   TTC TCG CCA GAC GGT TCC TGG TTC GCC TGG TCT CAA GGA CAC TGC GTG      262
   Phe Ser Pro Asp Gly Ser Trp Phe Ala Trp Ser Gln Gly His Cys Val
        35                  40                  45
   GTC AAG CTG GTC CCC TGG CCC TTA GAG GAA CAG TTC ATC CCT AAA GGA        310
   Val Lys Leu Val Pro Trp Pro Leu Glu Glu Gln Phe Ile Pro Lys Gly
    50                  55                  60                  65
   TTC GAA GCC AAG AGC CGA AGC AGC AAG AAT GAC CCA AAA GGA CGG GGC        358
   Phe Glu Ala Lys Ser Arg Ser Ser Lys Asn Asp Pro Lys Gly Arg Gly
                    70                  75                  80
   AGT CTG AAG GAG AAG ACG CTG GAC TGT GGC CAG ATT GTG TGG GGG CTG        406
   Ser Leu Lys Glu Lys Thr Leu Asp Cys Gly Gln Ile Val Trp Gly Leu
                85                  90                  95
   GCC TTC AGC CCG TGG CCC TCT CCA CCC AGC AGG AAA CTC TGG GCA CGT        454
   Ala Phe Ser Pro Trp Pro Ser Pro Pro Ser Arg Lys Leu Trp Ala Arg
           100                 105                 110
   CAC CAT CCC CAG GCG CCT GAT GTT TCT TGC CTG ATC CTG GCC ACA GGT        502
   His His Pro Gln Ala Pro Asp Val Ser Cys Leu Ile Leu Ala Thr Gly
       115                 120                 125
   CTC AAC GAT GGG CAG ATC AAG ATT TGG GAG GTA CAG ACA GGC CTC CTG        550
   Leu Asn Asp Gly Gln Ile Lys Ile Trp Glu Val Gln Thr Gly Leu Leu
   130                 135                 140                 145
   CTT CTG AAT CTT TCT GGC CAC CAA GAC GTC GTG AGA GAT CTG AGC TTC        598
   Leu Leu Asn Leu Ser Gly His Gln Asp Val Val Arg Asp Leu Ser Phe
                   150                 155                 160
   ACG CCC AGC GGC AGT TTG ATT TTG GTC TCT GCA TCC CGG GAT AAG ACA        646
   Thr Pro Ser Gly Ser Leu Ile Leu Val Ser Ala Ser Arg Asp Lys Thr
               165                 170                 175
   CTT CGA ATT TGG GAC CTG AAT AAA CAC GGT AAG CAG ATC CAG GTG TTA        694
   Leu Arg Ile Trp Asp Leu Asn Lys His Gly Lys Gln Ile Gln Val Leu
           180                 185                 190
   TCC GGC CAT CTG CAG TGG GTT TAC TGC TGC TCC ATC TCC CCT GAC TGT        742
   Ser Gly His Leu Gln Trp Val Tyr Cys Cys Ser Ile Ser Pro Asp Cys
       195                 200                 205
   AGC ATG CTG TGC TCT GCA GCT GGG GAG AAG TCG GTC TTT CTG TGG AGC        790
   Ser Met Leu Cys Ser Ala Ala Gly Glu Lys Ser Val Phe Leu Trp Ser
   210                 215                 220                 225
   ATG CGG TCC TAC ACA CTA ATC CGG AAA CTA GAA GGC CAC CAA AGC AGT      838
   Met Arg Ser Tyr Thr Leu Ile Arg Lys Leu Glu Gly His Gln Ser Ser
                   230                 235                 240
   GTT GTC TCC TGT GAT TTC TCT CCT GAT TCA GCC TTG CTT GTC ACA GCT      886
   Val Val Ser Cys Asp Phe Ser Pro Asp Ser Ala Leu Leu Val Thr Ala
               245                 250                 255
   TCG TAT GAC ACC AGT GTG ATT ATG TGG GAC CCC TAC ACC GGC GCG AGG      934
   Ser Tyr Asp Thr Ser Val Ile Met Trp Asp Pro Tyr Thr Gly Ala Arg
           260                 265                 270
   CTG AGG TCA CTT CAT CAC ACA CAA CTT GAA CCC ACC ATG GAT GAC AGT      982
   Leu Arg Ser Leu His His Thr Gln Leu Glu Pro Thr Met Asp Asp Ser
       275                 280                 285
   GAC GTC CAC ATG AGC TCC CTG AGG TCC GTG TGC TTC TCA CCT GAA GGC     1030
   Asp Val His Met Ser Ser Leu Arg Ser Val Cys Phe Ser Pro Glu Gly
   290                 295                 300                 305
   TTG TAT CTC GCT ACG GTG GCA GAT GAC AGG CTG CTC AGG ATC TGG GCT     1078
   Leu Tyr Leu Ala Thr Val Ala Asp Asp Arg Leu Leu Arg Ile Trp Ala
                   310                 315                 320
   CTG GAA CTG AAG GCT CCG GTT GCC TTT GCT CCG ATG ACC AAT GGT CTT     1126
   Leu Glu Leu Lys Ala Pro Val Ala Phe Ala Pro Met Thr Asn Gly Leu
               325                 330                 335
   TGC TGC ACG TTC TTC CCA CAC GGT GGA ATT ATT GCC ACA GGG ACG AGA     1174
   Cys Cys Thr Phe Phe Pro His Gly Gly Ile Ile Ala Thr Gly Thr Arg
           340                 345                 350
   GAT GGC CAT GTC CAG TTC TGG ACA GCT CCC CGG GTC CTG TCC TCA CTG     1222
   Asp Gly His Val Gln Phe Trp Thr Ala Pro Arg Val Leu Ser Ser Leu
       355                 360                 365
   AAG CAC TTA TGC AGG AAA GCC CTC CGA AGT TTC CTG ACA ACG TAT CAA     1270
   Lys His Leu Cys Arg Lys Ala Leu Arg Ser Phe Leu Thr Thr Tyr Gln
   370                 375                 380                 385
   GTC CTA GCA CTG CCA ATC CCC AAG AAG ATG AAA GAG TTC CTC ACA TAC     1318
   Val Leu Ala Leu Pro Ile Pro Lys Lys Met Lys Glu Phe Leu Thr Tyr
                   390                 395                 400
   AGG ACT TTC TAGCAGTGCC GGCTCCCCCA CCTCCTGCAG CAGCAGCAGT          1367
   Arg Thr Phe
           405
   ACAAGGGACT GGCTAGGATG GAGTCAGGCA GCTCACACTG GACCAGTGTG GACCTTCCTT  1427
   CCTCCCATGG CATGTGCAAG TAGGTCTGCG TGACCCCACT TCTGTGGTGC CGGCCTTACC  1487
   TCGTCTTCAT CCGTGGTGAG CAGCCTTCGT CAGTCTAGTT GTGTTGAAGC CAAGTGCAGT  1547
   TGTGGATGTT GCTGGGGTAA TAAAGGCAAG CGGGCTCCAG AGCCTCTCTG GTGGCGGCCA  1607
   AGCCACACTC CCTTAACTGG GAAGTACCTG CCACGTAGGG CATTTCTGCT GCCTATTTCC  1667
   AGCCAGCGGC TGCATGGTTT GAAGTTCCTC CGTTGTGGTC AGAAGAACTC TGGTGTTTGG  1727
   TTCCCTGCTC AGCTGCGCGT GGACTGGGCT GAGCTCCTCA CCATACACTA GTGCCGGCTT  1787
   TTGTTTCCTG TAAACAGTGG TTGCATGTGT AGAGAAGTAA CAAGCGAGTA TTCAGATCAT  1847
   ACGAGGAGGC GTTCCTCGGT GCATGACGGT CAGATGGCCA TTTATCAGCA TATTTATTTG  1907
   TATTTTCTCA GCACATAGTA AGGTACAACT GTGTTTTCTC AATTGTCTCG AAAAAACAGA  1967
   GTTCTTAAGT GGCCCAGTTG TGGAGCCAAG TCTAAGTCGT GTGGAGTCAG TGCTGACATC  2027
   ACTGGCTTGT GCTGTCTGTC ACATGTGTTT GTCTCTGCTG CTTGACCTCA TGGGATGTAC  2087
   CCTCCAGTTC AACTGCCCAA AACAGACAGC CCCTTCCAAG CACCGTTCTT TGACAGCGGT  2147
   AGCAGCTACC TATTCAAGAC GCCTCACACA AAATCTGCCT TAGAAAGTTA ATATATTTTA  2207
   AATTATTTTA AAAGAAACTC AACATCTTAT TCTTTGGCCT TTCTTAATTG ATGCTTTATG  2267
   GAGGCAGTGT TAACATTGTA CAGTGTATGC ATAGAGGAGT CTCCTCTATT TGAAGAACAA  2327
   TGCAAAATGA GGCTTTCATT GAAGGGAAAA AAAAAAAAAA AA                     2369
(2) INFORMATION FOR SEQ ID NO:21:
   (i) SEQUENCE CHARACTERISTICS:
      (A) LENGTH: 404 amino acids
      (B) TYPE: amino acid
      (D) TOPOLOGY: linear
   (ii) MOLECULE TYPE: protein
   (xi) SEQUENCE DESCRIPTION: SEQ ID NO:21:
   Met Glu Ala Gly Glu Glu Pro Leu Leu Leu Ala Glu Leu Lys Pro Gly
    1               5                  10                  15
   Arg Pro His Gln Phe Asp Trp Lys Ser Ser Cys Glu Thr Trp Ser Val
               20                  25                  30
   Ala Phe Ser Pro Asp Gly Ser Trp Phe Ala Trp Ser Gln Gly His Cys
           35                  40                  45
   Val Val Lys Leu Val Pro Trp Pro Leu Glu Glu Gln Phe Ile Pro Lys
       50                  55                  60
   Gly Phe Glu Ala Lys Ser Arg Ser Ser Lys Asn Asp Pro Lys Gly Arg
   65                  70                  75                  80
   Gly Ser Leu Lys Glu Lys Thr Leu Asp Cys Gly Gln Ile Val Trp Gly
                   85                  90                  95
   Leu Ala Phe Ser Pro Trp Pro Ser Pro Pro Ser Arg Lys Leu Trp Ala
               100                 105                 110
   Arg His His Pro Gln Ala Pro Asp Val Ser Cys Leu Ile Leu Ala Thr
           115                 120                 125
   Gly Leu Asn Asp Gly Gln Ile Lys Ile Trp Glu Val Gln Thr Gly Leu
       130                 135                 140
   Leu Leu Leu Asn Leu Ser Gly His Gln Asp Val Val Arg Asp Leu Ser
   145                 150                 155                 160
   Phe Thr Pro Ser Gly Ser Leu Ile Leu Val Ser Ala Ser Arg Asp Lys
                   165                 170                 175
   Thr Leu Arg Ile Trp Asp Leu Asn Lys His Gly Lys Gln Ile Gln Val
               180                 185                 190
   Leu Ser Gly His Leu Gln Trp Val Tyr Cys Cys Ser Ile Ser Pro Asp
           195                 200                 205
   Cys Ser Met Leu Cys Ser Ala Ala Gly Glu Lys Ser Val Phe Leu Trp
       210                 215                 220
   Ser Met Arg Ser Tyr Thr Leu Ile Arg Lys Leu Glu Gly His Gln Ser
   225                 230                 235                 240
   Ser Val Val Ser Cys Asp Phe Ser Pro Asp Ser Ala Leu Leu Val Thr
                   245                 250                 255
   Ala Ser Tyr Asp Thr Ser Val Ile Met Trp Asp Pro Tyr Thr Gly Ala
               260                 265                 270
   Arg Leu Arg Ser Leu His His Thr Gln Leu Glu Pro Thr Met Asp Asp
           275                 280                 285
   Ser Asp Val His Met Ser Ser Leu Arg Ser Val Cys Phe Ser Pro Glu
       290                 295                 300
   Gly Leu Tyr Leu Ala Thr Val Ala Asp Asp Arg Leu Leu Arg Ile Trp
   305                 310                 315                 320
   Ala Leu Glu Leu Lys Ala Pro Val Ala Phe Ala Pro Met Thr Asn Gly
                   325                 330                 335
   Leu Cys Cys Thr Phe Phe Pro His Gly Gly Ile Ile Ala Thr Gly Thr
               340                 345                 350
   Arg Asp Gly His Val Gln Phe Trp Thr Ala Pro Arg Val Leu Ser Ser
           355                 360                 365
   Leu Lys His Leu Cys Arg Lys Ala Leu Arg Ser Phe Leu Thr Thr Tyr
       370                 375                 380
   Gln Val Leu Ala Leu Pro Ile Pro Lys Lys Met Lys Glu Phe Leu Thr
   385                 390                 395                 400
   Tyr Arg Thr Phe
(2) INFORMATION FOR SEQ ID NO:22:
   (i) SEQUENCE CHARACTERISTICS:
      (A) LENGTH: 1246 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:22:
   GACACTGCAT CGTCAAACTG ATCCCCTGGC CGTTGGAGGA GCAGTTCATC CCTAAAGGGT 60
   TTGAAGCCAA AAGCCGAAGT AGCAAAAATG AGACGAAAGG GCGGGGCAGC CCAAAAGAGA 120
   AGACGCTGGA CTGTGGTCAG ATTGTCTGGG GGCTGGCCTT CAGCCTGTGC TTTCCCCACC 180
   CAGCAGGAAG CTCTGGGCAC GCCACCACCC CCAAGTGCCC GATGTCTCTT GCCTGGTTCT 240
   TGCTACGGGA CTCAACGATG GGCAGATCAA GATCTGGGAG GTGCAGACAG GGCTCCTGCT 300
   TTTGAATCTT TCCGGCCACC AAGATGTCGT GAGAGATCTG AGCTTCACAC CCAGTGGCAG 360
   TTTGATTTTG GTCTCCGCGT CACGGGATAA GACTCTTCGC ATCTGGGACC TGAATAAACA 420
   CGGTAAACAG ATTCAAGTGT TATCGGGCCA CCTGCAGTGG GTTTACTGCT GTTCCATCTC 480
   CCCAGACTGC AGCATGCTGT GCTCTGCAGC TGGAGAGAAG TCGGTCTTTC TATGGAGCAT 540
   GAGGTCCTAC ACGTTAATTC GGAAGCTAGA GGGCCATCAA AGCAGTGTTG TCTCTTGTGA 600
   CTTCTCCCCC GACTCTGCCC TGCTTGTCAC GGCTTCTTAC GATACCAATG TGATTATGTG 660
   GGACCCCTAC ACCGGCGAAA GGCTGAGGTC ACTCCACCAC ACCCAGGTTG ACCCCGCCAT 720
   GGATGACAGT GACGTCCACA TTAGCTCACT GAGATCTGTG TGCTTCTCTC CAGAAGGCTT 780
   GTACCTTGCC ACGGTGGCAG ATGACAGACT CCTCAGGATC TGGGCCCTGG AACTGAAAAC 840
   TCCCATTGCA TTTGCTCCTA TGACCAATGG GCTTTGCTGG CACATTTTTT CCACATGGTG 900
   GAGTCATTGC CACAGGGACA AGAGATGGCC ACGTCCAGTT CTGGACAGCT CCTAGGGTCC 960
   TGTCCTCACT GAAGCACTTA TGCCGGAAAG CCCTTCGAAG TTTCCTAACA ACTTACCAAG 1020
   TCCTAGCACT GCCAATCCCC AAGAAAATGA AAGAGTTCCT CACATACAGG ACTTTTTAAG 1080
   CAACACCACA TCTTGTGCTT CTTTGTAGCA GGGTAAATCG TCCTGTCAAA GGGAGTTGCT 1140
   GGAATAATGG GCCAAACATC TGGTCTTGCA TTGAAATAGC ATTTCTTTGG GATTGTGAAT 1200
   AGAATGTAGC AAAACCAGAT TCCAGTGTAC TAGTCATGGA TTTTTC 1246
(2) INFORMATION FOR SEQ ID NO:23:
   (i) SEQUENCE CHARACTERISTICS:
      (A) LENGTH: 422 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:23:
   ACCATGGTTC CAAGTCCTCT CCCCTGTGGT CAAGTTGCCC GAATGTTGGG CCCAAGTGCC    60
   TTTTCCTCCT TGGGCCTCCC CTTCTGACCT GCAGGACAGT TTTCCGGAGC CCATTTGGTA   120
   TGAGGTATTA ATTAGCCTTA ACTAAATTAC AGGGGACTCA GAGGCCGTGC TCCTGACCGA   180
   TCCAGACACT ATTTTTTTTT TTTTTTTTTA ACAATGGTGT GCATGTGCAG GAAATGACAA   240
   ATTTGTATGT CAGATTATAC AAGGATGTAT TCTTAAACCG CATGACTATT CAGATGGCTA   300
   CTGAGTTATC AGTGGCCATT TATTAGCATC ATATTTATTT GTATTTTCTC AACAGATGTT   360
   AAGGTACAAC TGTGTTTTTC TCGATTATCT AAAAACCATA GTACTTAAAT TGAAAAAAAA   420
   AA                                                                  422
(2) INFORMATION FOR SEQ ID NO:24:
   (i) SEQUENCE CHARACTERISTICS:
      (A) LENGTH: 2019 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:24:
   GGCACGAGGC GGGGTCAGGG CGGAGGCTGA GGACCAAGTA GGCATGGCGG AGGGCGGGAC 60
   CGGCCCCGAT GGACGGGCCG GCCCGGGACC CGCAGGTCCT AATCTGAAGG AGTGGCTGAG 120
   GGAGCAGTTC TGTGACCATC CACTGGAGCA CTGTGACGAT ACAAGACTCC ATGATGCAGC 180
   CTATGTAGGG GACCTCCAGA CCCTCAGGAA CCTACTGCAA GAGGAGAGCT ACCGGAGCCG 240
   CATCAATGAG AAGTCTGTCT GGTGCTGCGG CTGGCTTCCC TGCACACCAC TGAGGATCGC 300
   AGCCACTGCA GGCCATGGGA ACTGTGTGGA CTTCCTCATA CGCAAAGGGG CCGAGGTGGA 360
   CCTGGTGGAT GTCAAGGGGC AGACTGCCCT GTATGTGGCT GTAGTGAACG GGCACTTGGA 420
   GAGCACTGAG ATCCTTTTGG AAGCTGGTGC TGATCCCAAC GGCAGCCGGC ACCACCGCAG 480
   CACTCCTGTG TACCATGCCT YTCGTGTGGG TAGGGACGAC ATCCTGAAGG CTCTTATCAG 540
   GTATGGGGCA GATGTTGATG TCAACCATCA TCTGAATTCT GACACCCGGC CCCCTTTTTC 600
   ACGGCGGCTA ACCTCCTTGG TGGTCTGTCC TCTATACATC AGTGCTGCCT ACCATAACCT 660
   TCAGTGCTTC AGGCTGCTCT TGCAGGCTGG GGCAAATCCT GACTTCAATT GCAATGGCCC 720
   TGTCAACACC CAGGAGTTCT ACAGGGGATC CCCTGGGTGT GTCATGGATG CTGTCCTGCG 780
   CCATGGCTGT GAAGCAGCCT TCGTGAGTCT GTTGGTAGAG TTTGGAGCCA ACCTGAACCT 840
   GGTGAAGTGG GAATCCCTGG GCCCAGAGGC AAGAGGCAGA AGAAAGATGG ATCCTGAGGC 900
   CTTGCAGGTC TTTAAAGAGG CCAGAAGTAT TCCCAGGACC TTGCTGAGTT TGTGCCGGGT 960
   GGCTGTGAGA AGAGCTCTTG GCAAATACCG ACTGCATCTG GTTCCCTCGC TGCCGCTGCC 1020
   AGACCCCATA AAGAAGTTTT TGCTTTATGA GTAGCATTCA CATGCAGTGC TGACTGCAAT 1080
   GTGGAAGCCG ATCACCTGCA GTGAAAACTG ACACAGACTC TGGCATCCTG GGAACCATGG 1140
   CCTGTGCTGC CAGCTTGATC CTTGGCTGTC AGTGAAGAAA AAACGGCTGT GTTCTCTTGG 1200
   ACTGTGATTC TATCTCAGGT GCTTGGGCCA TCGAACGCTC CTTGAGTCAT TGTCAACTGA 1260
   GAGGCACATA CAAACTTAAT TTTGTTCCTC TTCAGTCTCT CTGTTTTGGA TTCTTCCTGG 1320
   CAATGTGTGC AGCATGGGCT GAGCCTGGTG ATTGCCCTAG TGGGGAAGGC TTTTTTCTCC 1380
   AGGCTATGCA TCTATTTATG TTCCTACTTT GCAATTTATT GTTCTTTTAA GGCTTGATAT 1440
   CAAAACAGAA AGAGGTTTGT TAAGAAAAGA TATAGGGAGA AAGGAATTCC GGTTCCGTGC 1500
   ACTTGCTAGC CTGCTTTCCT TGCCTGGGTT TGTCTGTCTA TGCTGCCTGG TGCACATCCC 1560
   TTCTCTTTGC TGCCACTGTT CTATTTTGGG AGTTGTCTTC CGTCTAAGAT GGCTTCTGGG 1620
   GTTCTATCTT ATTGCACAGA GGTCCCAGAA CAGTGTTCAT AGGGCACCAT CTGCTCTGCC 1680
   AAGGGTTTTC TGATGTCTTA CCCTGGGGAT CTTCAGACAG TGGTTACCTT TAGGAGACCC 1740
   ACCTGGAACT AACCATTAAG TGACTGCCCA CATTCAGATC AGGGACCATC TTAATAGTAC 1800
   TCACTGCCAG TCCTCACAAG AGAAGATGAC ACGGGTGCTC TCTTCAGACA CTCCCATACA 1860
   GGAAGTTGGA AAATGTCTTG GTCACCTGGG TTGTTCCCAG GCTACAACTT CTTGGTGTTC 1920
   CACTAARACC AGRATATCCT AGTTTTTTGG GTTGACTGTT CCCTCCCCAC TTTCCTTGAA 1980
   NCCCAATGCC CNTTTGTKTN GGTTGCTTCC CTAAAAKTT 2019
(2) INFORMATION FOR SEQ ID NO:25:
   (i) SEQUENCE CHARACTERISTICS:
      (A) LENGTH: 350 amino acids
      (B) TYPE: amino acid
      (C) STRANDEDNESS: single
      (D) TOPOLOGY: linear
   (ii) MOLECULE TYPE: DNA
   (xi) SEQUENCE DESCRIPTION: SEQ ID NO:25:
   Ala Arg Gly Gly Val Arg Ala Glu Ala Glu Asp Gln Val Gly Met Ala
   1 5 10 15
   Glu Gly Gly Thr Gly Pro Asp Gly Arg Ala Gly Pro Gly Pro Ala Gly
   20 25 30
   Pro Asn Leu Lys Glu Trp Leu Arg Glu Gln Phe Cys Asp His Pro Leu
   35 40 45
   Glu His Cys Asp Asp Thr Arg Leu His Asp Ala Ala Tyr Val Gly Asp
   50 55 60
   Leu Gln Thr Leu Arg Asn Leu Leu Gln Glu Glu Ser Tyr Arg Ser Arg
   65 70 75 80
   Ile Asn Glu Lys Ser Val Trp Cys Cys Gly Trp Leu Pro Cys Thr Pro
   85 90 95
   Leu Arg Ile Ala Ala Thr Ala Gly His Gly Asn Cys Val Asp Phe Leu
   100 105 110
   Ile Arg Lys Gly Ala Glu Val Asp Leu Val Asp Val Lys Gly Gln Thr
   115 120 125
   Ala Leu Tyr Val Ala Val Val Asn Gly His Leu Glu Ser Thr Glu Ile
   130 135 140
   Leu Leu Glu Ala Gly Ala Asp Pro Asn Gly Ser Arg His His Arg Ser
   145 150 155 160
   Thr Pro Val Tyr His Ala Xaa Arg Val Gly Arg Asp Asp Ile Leu Lys
   165 170 175
   Ala Leu Ile Arg Tyr Gly Ala Asp Val Asp Val Asn His His Leu Asn
   180 185 190
   Ser Asp Thr Arg Pro Pro Phe Ser Arg Arg Leu Thr Ser Leu Val Val
   195 200 205
   Cys Pro Leu Tyr Ile Ser Ala Ala Tyr His Asn Leu Gln Cys Phe Arg
   210 215 220
   Leu Leu Leu Gln Ala Gly Ala Asn Pro Asp Phe Asn Cys Asn Gly Pro
   225 230 235 240
   Val Asn Thr Gln Glu Phe Tyr Arg Gly Ser Pro Gly Cys Val Met Asp
   245 250 255
   Ala Val Leu Arg His Gly Cys Glu Ala Ala Phe Val Ser Leu Leu Val
               260                 265                 270
   Glu Phe Gly Ala Asn Leu Asn Leu Val Lys Trp Glu Ser Leu Gly Pro
           275                 280                 285
   Glu Ala Arg Gly Arg Arg Lys Met Asp Pro Glu Ala Leu Gln Val Phe
       290                 295                 300
   Lys Glu Ala Arg Ser Ile Pro Arg Thr Leu Leu Ser Leu Cys Arg Val
   305                 310                 315                 320
   Ala Val Arg Arg Ala Leu Gly Lys Tyr Arg Leu His Leu Val Pro Ser
                   325                 330                 335
   Leu Pro Leu Pro Asp Pro Ile Lys Lys Phe Leu Leu Tyr Glu
               340                 345                 350
(2) INFORMATION FOR SEQ ID NO:26:
   (i) SEQUENCE CHARACTERISTICS:
      (A) LENGTH: 419 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:26:
   GCATCCATGG CGGAGGGCGG CAGCACGACG GGCGGGCAGG GCCGGGCTCC GCAGGTCGTA     60
   ATCTGAAGGA GTGGCTGAGG GAGCAATTTT GTGATCATCC GCTGGAGCAC TGTGAGGACA    120
   CGAGGCTCCA TGATGCAGCT TACGTCGGGG ACCTCCAGAC CCTCAGGAGC CTATTGCAAG    180
   AGGAGAGCTA CCGGAGCCGC ATCAACGAGA AGTCTGTCTG GTGCTGTGGC TGGCTCCCCT    240
   GCACACCGTT GCGAATCGCG GCCACTGCAG GCCATGGGAG CTGTGTGGAC TTCCTCATCC    300
   GGAAGGGGGC CGAGGTGGAT CTGGTGGACG TAAAAGGACA GACGGCCCTG TATGTGGCTG    360
   TGGTGAACGG GCACCTAGAG AGTACCCAGA TCCTTCTCGA AGCTGGCGCG GACCCCAAC     419
(2) INFORMATION FOR SEQ ID NO:27:
   (i) SEQUENCE CHARACTERISTICS:
      (A) LENGTH: 595 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:27:
   GAGGAAGAAG AAAAGTGGAC CCTGAGGCCT TGCAGGTCTT TAAAGAGGCC AGAAGTGTTC     60
   CCAGAACCTT GCTGTGTCTG TGCCGTGTGG CTGTGAGAAG AGCTCTTGGC AAAACCGGCT    120
   TCATCTGATT CCTTCGCTGC CTCTGCCAGA CCCCATAAAG AAGTTTCTAC TCCATGAGTA    180
   GACTCCAAGT GCTGCGGTTG ATTCCAGTGA GGGAGAAAGT GATCTGCAGG GAGGTGGACA    240
   CCGAGCCCTG AGTGCTGTGC TGCTGCTGGT CTCCTGATGG CTGTTGCTGC AGAAGATGTC    300
   CTCGTAGACT GTCATTGCTC CTCAGGTGCC TGGGCCGCTG AACAGTCCTT GGGTCATTGT    360
   CAGCTGAGAG GCTTATACTA AAGTTATTAT TGTTTTTCCC AAGTTCTCTG TTCTGGATTT    420
   TCAGTTGCAT ATTAATGTAA CGGGCCATGG GGTATGTACA TGTAGGGGCT GAGGTTGGAG    480
   GCCTACTAAT TTCCTGTAGG GAAGACTCCC AGCACTTCTG GAACTGTGCT TCTCTTTATT    540
   TTTCTACTTC TCAATTTGAT GGTTCGATTA AAGCCTTCTA GTATCTCAAT GAAAA         595
(2) INFORMATION FOR SEQ ID NO:28:
   (i) SEQUENCE CHARACTERISTICS:
      (A) LENGTH: 896 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS
      (B) LOCATION: 4..396
   (xi) SEQUENCE DESCRIPTION: SEQ ID NO:28:
   CTG ATG TCC GCA ATT CTG AAG GTT GGA CAC CAC TGC TGG CTG CCT GTG          48
       Met Ser Ala Ile Leu Lys Val Gly His His Cys Trp Leu Pro Val
         1               5                  10                  15
   ACA TCC GCT GTC AAT CCC CAA AGG ATG CTG AGG CCA CCA CCA ACC GCT          96
   Thr Ser Ala Val Asn Pro Gln Arg Met Leu Arg Pro Pro Pro Thr Ala
                     20                  25                  30
   GTT TTC AAC TGT GCC GCT TGC TGC TGT CTG TGG GGG CAG ATG CTG ATG         144
   Val Phe Asn Cys Ala Ala Cys Cys Cys Leu Trp Gly Gln Met Leu Met
                 35                  40                  45
   AAT ACA TAC CGT GTA GTT CAG CTT CCT GAG GAG GCC AAG GGC TTG GTG         192
   Asn Thr Tyr Arg Val Val Gln Leu Pro Glu Glu Ala Lys Gly Leu Val
             50                  55                  60
   CCA CCA GAG ATT CTA CAG AAG TAC CAT GGA TTC TAC TCT TCC CTC TTT         240
   Pro Pro Glu Ile Leu Gln Lys Tyr His Gly Phe Tyr Ser Ser Leu Phe
         65                  70                  75
   GCC TTG GTG AGG CAG CCC AGG TCG CTG CAG CAT CTC TGC CGT TGT GCG         288
   Ala Leu Val Arg Gln Pro Arg Ser Leu Gln His Leu Cys Arg Cys Ala
    80                  85                  90                  95
   CTC CGC AGT CAC CTG GAG GGC TGT CTG CCC CAT GCA CTA CCG CGC CTT         336
   Leu Arg Ser His Leu Glu Gly Cys Leu Pro His Ala Leu Pro Arg Leu
                    100                 105                 110
   CCC CTG CCA CCG CGC ATG CTC CGC TTT CTG CAG CTG GAC TTT GAG GAT         384
   Pro Leu Pro Pro Arg Met Leu Arg Phe Leu Gln Leu Asp Phe Glu Asp
                115                 120                 125
   CTG CTC TAC TAGGCTTGCT GCCCTGTGAA CAAAGCAGAC CCCACCCCCA                 433
   Leu Leu Tyr
           130
   CCCCAAGGGC ATCTCTCAGC AATGAATGAT GCAAGGCGGT CTGTCTTCAA GTCAGGAGTG     493
   GACGCCTTGA TCCACACTTG AGAGAAGAGG CCAGATCAGC ACCYGGCTGG TAGTGATNGC     553
   AGAGGGCACC TGTGCAGATC TGTGTGCGCA CTGGAAATCT CTAGGCTGAA GGCYAGAGCA     613
   AATGGTGCAR GTGTTAGTCC TTGGGANGAG AGACAGANGG TGAGAAAGCA AGACAGAGGT     673
   GAGAGTGCAC ATGTCAAGTG GTAGATTGCC TTAAAAGAAA GCTAAAAAAA GAAAAAGATT     733
   CGGGCGAACT TCTTTAGGGG TAATGCTGCA GCGTGTTAAA CTGACTGACC AGCGTCCATA     793
   TCTTTGGACC CTTCCCGGGT GAAAAAGCCC CTTCATCCTC CAGCGCTCCC CAAGGGTGCT     853
   TAGCAATACC GGGTGCTTTT CTGCCGCAAA GTGAGTTACC AAA                       896
(2) INFORMATION FOR SEQ ID NO:29:
   (i) SEQUENCE CHARACTERISTICS:
      (A) LENGTH: 130 amino acids
      (B) TYPE: amino acid
      (D) TOPOLOGY: linear
   (ii) MOLECULE TYPE: protein
   (xi) SEQUENCE DESCRIPTION: SEQ ID NO:29:
   Met Ser Ala Ile Leu Lys Val Gly His His Cys Trp Leu Pro Val Thr
     1               5                  10                  15
   Ser Ala Val Asn Pro Gln Arg Met Leu Arg Pro Pro Pro Thr Ala Val
                20                  25                  30
   Phe Asn Cys Ala Ala Cys Cys Cys Leu Trp Gly Gln Met Leu Met Asn
            35                  40                  45
   Thr Tyr Arg Val Val Gln Leu Pro Glu Glu Ala Lys Gly Leu Val Pro
        50                  55                  60
   Pro Glu Ile Leu Gln Lys Tyr His Gly Phe Tyr Ser Ser Leu Phe Ala
    65                  70                  75                  80
   Leu Val Arg Gln Pro Arg Ser Leu Gln His Leu Cys Arg Cys Ala Leu
                    85                  90                  95
   Arg Ser His Leu Glu Gly Cys Leu Pro His Ala Leu Pro Arg Leu Pro
               100                 105                 110
   Leu Pro Pro Arg Met Leu Arg Phe Leu Gln Leu Asp Phe Glu Asp Leu
           115                 120                 125
   Leu Tyr
       130
(2) INFORMATION FOR SEQ ID NO:30:
   (i) SEQUENCE CHARACTERISTICS:
      (A) LENGTH: 436 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:30:
   GTGGGGGCGT CATCATGACC TCCTCTAGGG CTCTGCAACA TGACTCCTGT GGTGCAAATC     60
   AACAAATTGT TCACTGATGA ATCCACAAGG ATCTCTGGGC CTACAACCAG GTCCTGGTCC    120
   ACATGACTGT CGTCTTCGGA GAAGGCACCA CTCGCCCCCG GCAGGTACGG CTGACACCTC    180
   CATGGGAGAA GACGTATCCA GGCAGCAGCT GCGCGGCCCT TCAAGAGGGC ACATCCCGTC    240
   ATCTAAAGGC ACGGTGTACT GAAGGTAGTC CTGAGACATG AGTCCGATTA CTACAGGCAC    300
   GTGTTCCTCC AGGTGGAGGC TCAGGTCCCC GGGTGAGCTG GGCTGCAGCC GGGACTCAGG    360
   GCGCGGCTCT GGCTGCAGGT CTCGCAGCTC CCTGGGCTGT AGCTCCCGCA GATCCTTGCG    420
   CACACCGTTG ACTGGT                                                    436
(2) INFORMATION FOR SEQ ID NO:31:
   (i) SEQUENCE CHARACTERISTICS:
      (A) LENGTH: 2180 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:31:
   TTAATAGTAC CTACATAGTA GAAAATTATA ACTCCACTTT AAAACAATGT TTTCTTTCTA 60
   TTCAAATCAA TTTAAAACTT TTTATAAACA TTAATGTTGC AAGAGAATCC AGTCCATTTA 120
   TGAAAATTAG TTGACAATCA AGTTCACCCA AGAAAATGTT GACTAAGCTA AAGAAATCAC 180
   AGATAAAACA TTTTACCAAA AGGATAGGTA ACACACAAAA AAATGCTATC ACAGGAAGCT 240
   ATGATCATCT AATATTTCTT TAATAATAAT TCTAGTTCCA TAGGTTTTCA TGTTATGCCA 300
   ATTTGTACCC GAGTTTAATT ACAGAAAAGG CAACAATTTC TAAATTGGTG GTATACATTT 360
   CTTTACAATT TTTTAATGTA AGGCCATTTA TTAAAATAGA CAAACTAGAA GATGAAAACG 420
   AAGGCAACAG AAAAATTCAA CTTTTCACAA CCAAAAGAAT TAGCACAACC TTAGAAATAA 480
   TTTAGAAAAA AGTGTTGTTA AAAGATATGT TGCAGATCTC CGTTCCATTA CCCAAGATTA 540
   TGTCAATTCA CGATTCTAAA TAAATCTTTT TAAAGTAAGA GATTAAAAAC TCATCTTCAG 600
   TGTATATGTA AATTCCGTGG TTTTATCACA CAGGTATGTT TATTCAACAC TGCTTTGGAA 660
   ATGGACCATT TAAAAGGACA TGGCAATTTC CATTCTGTTA AGTTTCATTC AACCTTTACT 720
   TAGGGGTTGA TTACCACATG AAATGTGCTT TTAATGCATA AAAATCACAG TGGATTAGCC 780
   AGCAAAAGGG ACTGGGCGGG GGGGGCATTG AGGAGAATTT GATAATTCAC ATTGTGATTA 840
   TTCTGCACAT TGATGAAACA TAATTCACAC CTCTAAAACC TCAAGACTTC CCTTTTTTAA 900
   AGAACCAAAA TAAACCCAAG ACACCTTGCT GACACTTCCC CACCCCTAAA CAAACTGATG 960
   ACTCTTTTAC ACATAAAACT GAAATAGTTA TGGCAGCAAA AGATTTTGAT GGCAATGAAA 1020
   GTTTGTAAAC TGTATTTCAA TCTCTTGTTC TTATTCCCAA AGTGCAAGAT GCAGGGTTCT 1080
   CAATCTTTCA GTAGTGCTTC TCCTGTAAAT AATCCTTCAT TTTGTTTGGC AAAGGCAGTT 1140
   TCTGAATTAA GTCTATTCTG GTATACTGAC GTATAACAAA ACGACACAGG TACTGCAACG 1200
   AGCGCACCTA TGAACCCCGG AACACTGGTT GGCAAGTTCT GACGGAAGTG CAGATTCCAG 1260
   GCAGCGAGAC CTTGAATAAC AAAAAGCTCC CATTTTCAGA GTCCCTGATT GAATGCTCCA 1320
   ATTAGATCAA CTATGGACGT ATGTCCTTCC ACATCGGCTG TTCATAAAAG CTAAACCTAC 1380
   CATTTGAGTG CTCAATTCTA GTGTGAAGTG TTTTACCATG GGAGCGAAAG TCACAGCTTA 1440
   AAAGGTAACG GTCGTCAGAA CTGTCCCGAA CAAGAAAAGA ACCATCTGGC ACGTTTGCTA 1500
   GCTTCCCTTC TGCCTCCCAA CGTGTGATTG GTCCCCAGTA CCATCCTTGC TTTGCAAGTT 1560
   TTTTCAGCTC CTCTGTAAGG CTTGTCACAA CCATGGGACC ACTACTTTGC ACTGAGTCAT 1620
   AAACTCTTGC AACCCCAGGA GCAGAGTTCG GATCAAAATT CAAATGACAG CGCATAACTT 1680
   TCAGCCACGT GGGGCTTTCT GTCCAGTGAG TCCACTGAAA GTTCCCCTTT GGGATTTGGA 1740
   TTATTCCTGC ATTGGAGTAA CCAATGGTGA AGATTGGAGG GACATCCATC GTGAACCCGC 1800
   TCTCCGGGGT TCTGCAACAT GACTCCCGTG GTGCCAATCA ACAAGCCATT CACCGGACTG 1860
   ATCCACGAAG ATCTCTGGGG CGACAACTAG GTCCTGGTCT ACCTGACTCT CATCCTCGGG 1920
   GAAAGCGCGC CCTCCCACTT GAGGAGGAAC CGCAGAGACT TCCATGGGAG AAGAGCTGTC 1980
   CAGACAATAG CTCCGTGATC CTTCCAAAGG ATACATCCCC TCATCTAAAG GCACAGTATA 2040
   CTGAATGTAG TCCTGAGGCA TAAGTCCAAT AACGACAGGC ACATGTTCAT CCAGGTGAAG 2100
   ATGCAGGTCT CCATTATGAG AAGCCGAGCT CTTCAGTGAA TTGGCTTGCT CCTGGCACGT 2160
   GGTCTCAGAC TGGAGGTCGT 2180
(2) INFORMATION FOR SEQ ID NO:32:
   (i) SEQUENCE CHARACTERISTICS:
      (A) LENGTH: 2649 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:32:
   GGCACGAGGC TGTGTCCAGC ACACAGAGAG GGCCCGGCCA TCTGCTTTGG TTCAGAGCCC      60
   TGTGTCTGTC TGTCACTTAG ACTCTTCCTC CCGGCTCGCA GCTCACCCTC CATCCTCCTT     120
   ACTGGCTCCA GCATGACTCG CTTCTCTTAT GCAGAGTACT TTGCTCTGTT TCACTCTGGC     180
   TCTGCACCTT CCAGGTCCCC TTCGTCTCCC GAGAACCCAC CGGCCCGCGC ACCCCTGGGT     240
   CTGTTCCAAG GGGTCATGCA GAAGTATAGC AGCAACCTGT TCAAGACCTC CCAGATGGCG     300
   GCTATGGACC CCGTGCTGAA GGCCATCAAG GAAGGGGATG AAGAGGCCTT GAAGATCATG     360
   ATCCAGGATG GGAAGAATCT TGCAGAGCCC AACAAGGAGG GCTGGCTGCC GCTCCACGAG     420
   GCTGCCTACT ATGGCCAGCT GGGCTGCCTG AAAGTCCTGC AGCAAGCCTA CCCAGGGACC     480
   ATTGACCAAC GCACACTGCA GGAAGAGACA GCATTATACC TGGCCACATG CAGAGAACAC     540
   CTGGATTGCC TCCTGTCGCT GCTCCAGGCG GGGGCAGAGC CTGACATCTC TAACAAATCC     600
   AGGGAGACTC CACTTTACAA AGCCTGTGAG CGCAAGAACG CGGAGGCGGT GAGGATATTG     660
   GTGCGATACA ACGCAGACGC CAACCACCGC TGTAACAGGG GCTGGACCGC ACTGCACGAG     720
   TCTGTCTCCC GCAATGACCT GGAGGTCATG GAGATCCTAG TGAGTGGCGG GGCCAAGGTG     780
   GAGGCCAAGA ATGTCTACAG CATCACCCCT TTGTTTGTGG CTGCCCAGAG TGGGCAGCTG     840
   GAGGCCCTGA GGTTCCTGGC CAAGCATGGT GCAGACATCA ACACGCAGGC CAGTGACAGT     900
   GCATCAGCCC TCTACGAGGC CAGCAAGAAT GAGCATGAAG ACGTGGTAGA GTTTCTTCTC     960
   TCTCAGGGCG CCGATGCTAA CAAAGCCAAC AAGGACGGCC TGCTCCCCCT GCATGTTGCC    1020
   TCCAAGAAGG GCAACTATAG AATAGTGCAG ATGCTGCTGC CTGTGACCAG CCGCACGCGC 1080
   GTGCGCCGTA GCGGCATCAG CCCGCTGCAC CTAGCGGCCG AGCGCAACCA CGACGCGGTG 1140
   CTGGAGGCGC TGCTGGCCGC GCGCTTCGAC GTGAACGCAC CTCTGGCTCC CGAGCGCGCC 1200
   CGCCTCTACG AGGACCGCCG CAGTTCTGCG CTCTACTTCG CTGTGGTCAA CAACAATGTG 1260
   TACGCCACCG AGCTGTTGCT GCTGGCGGGC GCGGACCCCA ACCGCGATGT CATCAGCCCT 1320
   CTGCTCGTGG CCATCCGCCA CGGCTGCCTG CGCACCATGC AGCTGCTGTT GGACCATGGC 1380
   GCCAACATCG ACGCCTACAT CGCCACTCAC CCCACCGCCT TTCCAGCCAC CATCATGTTT 1440
   GCCATGAAGT GCCTGTCGTT ACTCAAGTTC CTTATGGACC TCGGCTGCGA TGGCGAGCCC 1500
   TGCTTCTCCT GCCTGTACGG CAACGGGCCG CACCACCCGC CCCGCGACCT GGCCGCTTCC 1560
   ACGACGCACC CGTGGACGAC AAGGCACCTA GCGTGGTGCA GTTCTGTGAG TTCCTGTCGG 1620
   CCCCGGAAGT GAGCCGCTGG GCGGGACCCA TCATCGATGT CCTCCTGGAC TATGTGGGCA 1680
   ACGTGCAGCT GTGCTCCCGG CTGAAGGAGC ACATCGACAG CTTTGAGGAC TGGGCTGTCA 1740
   TCAAGGAGAA GGCAGAACCT CCGAGACCTC TGGCTCACCT CTGCCGGCTG CGGGTTCGGA 1800
   AGGCCATAGG AAAATACCGG ATAAAACTCC TGGACACACT GCCGCTTCCC GGCAGGCTAA 1860
   TCAGATACTT GAAATATGAG AATACACAGT AACCAGCCTG GAGAGGAGAT GTGGCCTTCA 1920
   GACTGTTTCC GGGACGCCCC AGGTGGCCTG CATCCAGGAC CCCCTGGGGT CAGAACAGGT 1980
   GTGACCTTGC TGGTTCTTTG CTGGAGCTTC ACCCAAAGTG AGAACCTGAT GTGGGGAGTG 2040
   GACGTGGAAC CTCTGCTTTC ACACTGTCAG CGGATCGCAG ACCCGCTCTG CTTCTGGCCA 2100
   TAGCCAGAGA CCTTCAACCT GGGGCCAGGG GAGAGCTGGT CTGGGCAAGG TGGCCCAGGC 2160
   AGGAATCCTG GCCTTAAGCT GGAGAACTTG TAGGAATCCC TCACTGGACC CTCAGCTTTC 2220
   AGGCTGCGAG GGAGACGCCC AGCCCAAGTA TTTTATTTCC GTGACACAAT AACGTTGTAT 2280
   CAGAAAAAAA AAAAAACATG GGCGCAGCTT ATTCCTTAGT AGGGTATTTA CTTGCATGCG 2340
   CGCTTAAAGC TACTGGAAAC ATGCGTTCCA CTATGCTTGA GAATCCCCTT GCACTGGTAA 2400
   ACGAGAGCCG ACGTGCTTCA AGGTTGGATT TTTGGTTGCC CCTTTGGCGT TCCGCGGGTT 2460
   TGTCCGACGT AATTGACCCC GTGTTTTGTC ACTTTCGAGT GTTCCGACTA TTGGGGGGCT 2520
   TTTGGTTGTC CCCAAAATTG TGGGTGGTGT GCGGACGCCA CGAGAAGTGG TTCATGGGCG 2580
   ATAATCATTA CTGGAGAATG TAGAGCGGCG GTTTTACGAA TAAATATTTT TTAAGCCGCC 2640
   TTCCCAAAA 2649
(2) INFORMATION FOR SEQ ID NO:33:
   (i) SEQUENCE CHARACTERISTICS:
      (A) LENGTH: 495 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:33:
   CCTCCTGAGA GTTCGCCGGC CCGGGCCCAA TGGGTTGTTC CAAGGGGTCA TGCAGAAATA 60
   CAGCAGCAGC TTGTTCAAGA CCTCCCAGCT GGCGCCTGCG GACCCCTTGA TAAAGGCCAT 120
   CAAGGATGCG ATGAAGAGGC CTTGAAGACC ATGATCAAGG AAGGGAAGAA TCTCGCAGAG 180
   CCCAACAAGG AGGGCTGGCT GCCGCTGCAC GAGGCCGCAT ACTATGGCCA GGTGGGCTGC 240
   CTGAAAGTCC TGCAGCGAGC GTACCCAGGG ACCATCGACC AGCGCACCCT GCAGGAGGAA 300
   ACAGCCGTTT ACTTGGCAAC GTGCAGGGGC CACCTGGACT GTCTCCTGTC ACTGCTCCAA 360
   GCAGGGGCAG AGCGGGACAT CTCCAACAAA TCCCGAGAGA ACCGCTCTAC AAAGCCTGTG 420
   AGCGCAAGAA CGCGGAAGCC GTGAAGATTC TTGGTGCAGC ACAACGCAGA CACCAACAAC 480
   GCTGCAACCG GGCTG 495
(2) INFORMATION FOR SEQ ID NO:34:
   (i) SEQUENCE CHARACTERISTICS:
      (A) LENGTH: 709 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:34:
   GTGCAGCTCT GCTCGCGGCT GAAGGAACAC ATCGACAGCT TTGAGGACTG GGCCGTCATC     60
   AAGGAGAAGG CAGAACCTCC AAGACCTCTG GCTCACCTTT GCCGACTGCG GGTTCGAAAG    120
   GCCATTGGGA AATACCGTAT AAAACTCCTA GACACCTTGC CGCTCCCAGG CAGGCTGATT    180
   AGATACCTGA AATACGAGAA CACCCAGTAA CTGGGGCCAC GGGGAGAGAG GAGTAGCCCC    240
   TCAGACTCTT CTTACTAAGT CTCAGGACGT CGGTGTTCCC AACTCCAAGG GGACCTGGTG    300
   ACAGACGAGG CTGCAGGCTG CCTCCCTCTC AGCCTGGACA GCTACCAGGA TCTCACTGGG    360
   TCTCAGGGCC CAGAGCTTTG GCCAGAGCAG AGAACAGAAT GTGTCAAGGA GAAGAATCAT    420
   TTGTTTACAA ACTGATGAGC AGATCCCAGA CCTTCTCTAC CTTCAGGAAT GGCAGAAACC    480
   TCTATTCCTG GGGCCAGGGC AGAGCTTGAG GTGTTCTGGG GAAGGTGGTG CTCAGAGCCT    540
   TCCCTGTGCC CCTCCACTTG TTCTGGAAAA CTCACCACTT GACTTCAGAG CTTTCTCTCC    600
   AAAGACTAAG ATGAAGACGT GGCCCAAGGT AGGGGGTAGG GGGAGCCTGG GTCTTGGAGG    660
   GCTTTGTTAA GTATTAATAT AATAAATGTT ACACATGTGA AAAAAAAAA               709
(2) INFORMATION FOR SEQ ID NO:35:
   (i) SEQUENCE CHARACTERISTICS:
      (A) LENGTH: 848 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS
      (B) LOCATION: 1..624
   (xi) SEQUENCE DESCRIPTION: SEQ ID NO:35:
   TTG GAG AAG TGT GGT TGG TAT TGG GGG CCA ATG AAT TGG GAA GAT GCA      48
   Leu Glu Lys Cys Gly Trp Tyr Trp Gly Pro Met Asn Trp Glu Asp Ala
     1               5                  10                  15
   GAG ATG AAG CTG AAA GGG AAA CCA GAT GGT TCT TTC CTG GTA CGA GAC      96
   Glu Met Lys Leu Lys Gly Lys Pro Asp Gly Ser Phe Leu Val Arg Asp
                20                  25                  30
   AGT TCT GAT CCT CGT TAC ATC CTG AGC CTC AGT TTC CGA TCA CAG GGT     144
   Ser Ser Asp Pro Arg Tyr Ile Leu Ser Leu Ser Phe Arg Ser Gln Gly
            35                  40                  45
   ATC ACC CAC CAC ACT AGA ATG GAG CAC TAC AGA GGA ACC TTC AGC CTG     192
   Ile Thr His His Thr Arg Met Glu His Tyr Arg Gly Thr Phe Ser Leu
        50                  55                  60
   TGG TGT CAT CCC AAG TTT GAG GAC CGC TGT CAA TCT GTT GTA GAG TTT     240
   Trp Cys His Pro Lys Phe Glu Asp Arg Cys Gln Ser Val Val Glu Phe
    65                  70                  75                  80
   ATT AAG AGA GCC ATT ATG CAC TCC AAG AAT GGA AAG TTT CTC TAT TTC     288
   Ile Lys Arg Ala Ile Met His Ser Lys Asn Gly Lys Phe Leu Tyr Phe
                    85                  90                  95
   TTA AGA TCC AGG GTT CCA GGA CTG CCA CCA ACT CCT GTC CAG CTG CTC     336
   Leu Arg Ser Arg Val Pro Gly Leu Pro Pro Thr Pro Val Gln Leu Leu
               100                 105                 110
   TAT CCA GTG TCC CGA TTC AGC AAT GTC AAA TCC CTC CAG CAC CTT TGC     384
   Tyr Pro Val Ser Arg Phe Ser Asn Val Lys Ser Leu Gln His Leu Cys
           115                 120                 125
   AGA TTC CGG ATA CGA CAG CTC GTC AGG ATA GAT CAC ATC CCA GAT CTC     432
   Arg Phe Arg Ile Arg Gln Leu Val Arg Ile Asp His Ile Pro Asp Leu
       130                 135                 140
   CCA CTG CCT AAA CCT CTG ATC TCT TAT ATC CGA AAG TTC TAC TAC TAT        480
   Pro Leu Pro Lys Pro Leu Ile Ser Tyr Ile Arg Lys Phe Tyr Tyr Tyr
   145                 150                 155                 160
   GAT CCT CAG GAA GAG GTA TAC CTG TCT CTA AAG GAA GCG CAG CGT CAG        528
   Asp Pro Gln Glu Glu Val Tyr Leu Ser Leu Lys Glu Ala Gln Arg Gln
                   165                 170                 175
   TTT CCA AAC AGA AGC AAG AGG TGG AAC CCT CCA CGT AGC GAG GGG CTC        576
   Phe Pro Asn Arg Ser Lys Arg Trp Asn Pro Pro Arg Ser Glu Gly Leu
               180                 185                 190
   CCT GCT GGT CAC CAC CAA GGG CAT TTG GTT GCC AAG CTC CAG CTT TGAAGAACCA
   631
   Pro Ala Gly His His Gln Gly His Leu Val Ala Lys Leu Gln Leu
           195                 200                 205
   AATTAAGCTA CCATGAAAAG AAGAGGAAAA GTGAGGGAAC AGGAAGGTTG GGATTCTCTG      691
   TGCAGAGACT TTGGTTCCCC ACGCAAGCCC TGGGGCTTGG AAGAAGCACA TGACCGTACT      751
   CTGCGTGGGG CTCCACCTCA CACCCACCCC TGGGCATCTT AGGACTGGAG GGGCTCCTTG      811
   GAAAACTGGA AGAAGTCTCA ACACTGTTTC TTTTTCA                               848
(2) INFORMATION FOR SEQ ID NO:36:
   (i) SEQUENCE CHARACTERISTICS:
      (A) LENGTH: 207 amino acids
      (B) TYPE: amino acid
      (D) TOPOLOGY: linear
   (ii) MOLECULE TYPE: protein
   (xi) SEQUENCE DESCRIPTION: SEQ ID NO:36:
   Leu Glu Lys Cys Gly Trp Tyr Trp Gly Pro Met Asn Trp Glu Asp Ala
    1               5                  10                  15
   Glu Met Lys Leu Lys Gly Lys Pro Asp Gly Ser Phe Leu Val Arg Asp
               20                  25                  30
   Ser Ser Asp Pro Arg Tyr Ile Leu Ser Leu Ser Phe Arg Ser Gln Gly
           35                  40                  45
   Ile Thr His His Thr Arg Met Glu His Tyr Arg Gly Thr Phe Ser Leu
        50                  55                  60
   Trp Cys His Pro Lys Phe Glu Asp Arg Cys Gln Ser Val Val Glu Phe
    65                  70                  75                  80
   Ile Lys Arg Ala Ile Met His Ser Lys Asn Gly Lys Phe Leu Tyr Phe
                    85                  90                  95
   Leu Arg Ser Arg Val Pro Gly Leu Pro Pro Thr Pro Val Gln Leu Leu
               100                 105                 110
   Tyr Pro Val Ser Arg Phe Ser Asn Val Lys Ser Leu Gln His Leu Cys
           115                 120                 125
   Arg Phe Arg Ile Arg Gln Leu Val Arg Ile Asp His Ile Pro Asp Leu
       130                 135                 140
   Pro Leu Pro Lys Pro Leu Ile Ser Tyr Ile Arg Lys Phe Tyr Tyr Tyr
   145                 150                 155                 160
   Asp Pro Gln Glu Glu Val Tyr Leu Ser Leu Lys Glu Ala Gln Arg Gln
                   165                 170                 175
   Phe Pro Asn Arg Ser Lys Arg Trp Asn Pro Pro Arg Ser Glu Gly Leu
               180                 185                 190
   Pro Ala Gly His His Gln Gly His Leu Val Ala Lys Leu Gln Leu
           195                 200                 205
(2) INFORMATION FOR SEQ ID NO:37:
   (i) SEQUENCE CHARACTERISTICS:
      (A) LENGTH: 464 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:37:
   GTTCCAAGCC TAACCCATCT TTGTCGTTTG GAAATTCGGG CCAGTCTAAA AGCAGAGCAC 60
   CTTCACTCTG ACATTTTCAT CCATCAGTTG CCACTTCCCA GAAGTCTGCA GAACTATTTG 120
   CTCTATGAAG AGGTTTTAAG AATGAATGAG ATTCTAGAAC CAGCAGCTAA TCAGGATGGA 180
   GAAACCAGCA AGGCCACCTG ACACAGGTCC TTTAATTCTG TTTAGTCACA AAAGACGGCT 240
   TGTGTGACTG TTTGGATTTG GTGATCAAAT GTCCATGTTT ACAGTTGCTT TTCCCAGTTT 300
   GTGTCTTTCC CAATATTGTG AACCTTATCC ATCTTGCCTT ACTCAGTTTT ATTTCTAGTG 360
   CACTTTGTTG TGTATTATTT GTTTACCTGA CCATTTTCTA CTTTATTCTG CTAATAAACT 420
   GTAATTCTGA AAAAAAAAAA AAAAAAAAAA AAAAAAAAAA AAAA 464
(2) INFORMATION FOR SEQ ID NO:38:
   (i) SEQUENCE CHARACTERISTICS:
      (A) LENGTH: 747 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:38:
   GGGGATCGAA AGCGGGGGCT TCTGGGACGC AGCTCTGGAG ACGCGGCCTC GGACCAGCCA 60
   TTTCGGTGTA GAAGTGGCAG CACGGCAGAC TGGTCAAACA AATGGATTTT ACAGAGGCTT 120
   ACGCGGACAC GTGCTCTACA GTTGGACTTG CTGCCAGGGA AGGCAATGTT AAAGTCTTAA 180
   GGAAACTGCT CAAAAAGGGC CGAAGTGTCG ATGTTGCTGA TAACAGGGGA TGGATGCCAA 240
   TTCATGAAGC AGCTTATCAC AACTCTGTAG AATGTTTGCA AATGTTAATT AATGCAGATT 300
   CATCTGAAAA CTACATTAAG ATGAAGACCT TTGAAGGTTT CTGTGCTTTG CATCTCGCTG 360
   CAAGTCAAGG ACATTGGAAA ATCGTACAGA TTCTTTTAGA AGCTGGGGCA GATCCTAATG 420
   CAACTACTTT AGAAGAAACG ACACCATTGT TTTTAGCTGT TGAAAATGGA CAGATAGATG 480
   TGTTAAGGCT GTTGCTTCAA CACGGAGCAA ATGTTAATGG ATCCCATTCT ATGTGTGGAT 540
   GGAACTCCTT GCACCAGGCT TCTTTTCAGG AAAATGCTGA GATCATAAAA TTGCTTCTTA 600
   GAAAAGGAGC AAACAAGGAA TGCCAGGATG ACTTTGGAAT CACACCTTTA TTTGTGGCTG 660
   CTCAGTATGG CCAAGCTAGA AAGCTTTGAA GCATACTTAT TTCATCCGGG TGCAAATGTC 720
   AATTGTCAAG CCTTGGACAA AGCTACC 747
(2) INFORMATION FOR SEQ ID NO:39:
   (i) SEQUENCE CHARACTERISTICS:
      (A) LENGTH: 1018 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:39:
   CACAAATGGG ACCATACAAA AATCTTGGAC TTGTTAATAA CCACTTACTA ACCGGGACCT 60
   GTGACACTGG GCTAAACAAA GTAAGTCCCT GTTTACTCAG CAGTGTTTGG GGGACATGAA 120
   GGATTGCCTA GAAATATTAC TCCGGAATGG TCTACAGCCC AGACGCCCAG GCGTGCCTTG 180
   TTTTTGGATT CAGTTCTCCT GTGTGCATGG CTTTCCAAAA GGAGGTGGAG CTGTAGTTCT 240
   TTGGAATTGT GAACATTCTT TTGAAATATG GAGCCCAGAT AAATGAACTT CATTTGGCAT 300
   ACTGCCTGAA GTACGAGAAG TTTTCGATAT TTCGCTACTT TTTGAGGAAA GGTTGCTCAT 360
   TGGGACCATG GAACCATATA TATGAATTTG TAAATCATGC AATTAAAGCA CAAGCAAAAT 420
   ATAAGGAGTG GTTGCCACAT CTTCTGGTTG CTGGATTTGA CCCACTGATT CTACTGTGCA 480
   ATTCTTGGAT TGACTCAGTC AGCATTGACA CCCTTATCTT CACTTTGGAG TTTACTAATT 540
   GGAAGACACT TGCACCAGCT GTTGAAAGGA TGCTCTCTGC TCGTGCCTCA AACGCTTGGA 600
   TTCTACAGCA ACATATTGCC CACTGTTCCA TCCCTGACCC ATCTTTGTCG TTTGGAAATT 660
   CGGTCCAGTC TAAAATCAGA ACGTCTACGG TCTGACAGTT ATATTAGTCA GCTGCCACTT 720
   CCCAGAAGCC TACATAATTA TTTGCTCTAT GAAGACGTTC TGAGGATGTA TGAAGTTCCA 780
   GAACTGGCAG CTATTCAAGA TGGATAAATC AGTGAAACTA CTTAACACAG CTAATTTTTT 840
   TCTCTGAAAA ATCATCGAGA CAAAAGAGCC ACAGAGTACA AGTTTTTATG ATTTTATAGT 900
   CAAAAGATGA TTATTGATTG TCAGATAGGT TAGGTTTTGG GGGGCCAGTA GTTCAGTGAG 960
   AATGTTTATG TTTACAACTA GCCTTCCCAG TAAAAAAAAA AAAAAAAAAA AAAAAAAA 1018
(2) INFORMATION FOR SEQ ID NO:40:
   (i) SEQUENCE CHARACTERISTICS:
      (A) LENGTH: 1897 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:40:
   CGGGGGGCTG GGACCTGGGG CGTAACCGTC TCTACCACGA CGGCAAGAAC CAGCCAAGTA 60
   AAACATACCC AGCCTTTCTG GAGCCGGACG AGACATTCAT TGTCCCTGAC TCCTTTTTCG 120
   TGGCCCTGGA CATGRATGAT GGGACCTTAA GTTTCATCGT GGATGGACAG TACATGGGAG 180
   TGGCTTTCCG GGGACTCAAG GGTAAAAAGC TGTATCCTGT AGTGAGTGCC GTCTGGGGCC 240
   ACTGTGAGAT CCGCATGCGC TACTTGAACG GACTTGATCC TGAGCCCCTG CCACTCATGG 300
   ACCTGTGCCG GCGTTCGGTG CGCCTAGCGC TGGGAAAAGA GCGCCTGGGT GCCATCCCCG 360
   CTCTGCCGCT ACCTGCCTCC CTCAAAGCCT ACCTCCTCTA CCAGTGATCC ACATCCCAGG 420
   ACCGCCATAC GACAGCCATC TGGTGCCAAR TCACTGAGCC CGTTGGGGTC CGCCGACCCC 480
   TGCGCCTGGG ATGGAAGCCC ACCTCAGCCA TGGGCAGACG TGCCCCCTCA TCCTACCGGC 540
   TGCCTCTGCT GGGGGAACCT ATGCCAACGG ACTTCTCCCT TCCCAACACT GGCTGAAGCA 600
   GCAGCACCCA GGCCCTTCCC TGAACCAGAT GCAGAGAATA AACTATGAAA ACCTCTCTCA 660
   GGCGCCTTCT GCTCTCAGGT GGAGTGGGCT GCCCCCCACT CTCTGCAGAG AGAGGCTACA 720
   CCCACCTGGG GGGTCCTGGG AGGTAAGACT AGTAGGAGGT GCCAGGGCTG ARTCCAAAAG 780
   CAGGAATGGC CAGGAMCAGG CCATACAGAT GAAGCTCAGG ATGTCACATA CCATGGACAM 840
   TGAGACAGAA CCCCAGGTTG GAMTTCCCTT GGGCCAACGA GTGCCAGCTT TAATGTCAGC 900
   TGCMGGTGCT CTGTGGCCTG TATTTATTCT TTAAACAGTA GCAAAGGCCA TTTATTTATT 960
   CCACTTAGAA AGGAAACCTT GGTGGGTGGY TTCCCTCGAT GTGCTTTCCC CCACCTCCCT 1020
   GGAATGTGTG TGCCACACCT GTCCTTGTCC CAGGCCAGGA CTGTGGCACA TGAGCTGGTG 1080
   TGCACAGATA CACGTATGTC GTCGTGCATG ACCCCTGACT AGTTCCTAAG TAGCCCTGCA 1140
   CCAAGCACCA GAGCAGACCC CAAGAGAGGC CCGTGCAAGT CCCCATGTCC CCAGGTCCCT 1200
   GCTTCTGTTG CCTTGGGACT CATACACCGG CACACGTGTT TCAGCCTCTT GACTTCCATG 1260
   AGCTTCGAAT TTTGCCCCCG ATTCTTCTGA TATTTCCCAT TGGCATCCTC CAAAGCTCTG 1320
   GGCCTGGAGG GCATTAGGAC ACATGGAATG AGTGGGGTCT CCAGCCCCTG GGAAAGCCAC 1380
   TGGCAAGGCA GGATTAGAAA GACCAAGAGC AGGGTGGGGC GCCATGAAGC CTGTATGCCT 1440
   CTCAGGCTCA AGACCCCGCC ACACACCCAC TCAAGCCTCA GAAGTGGTGT GTAGGGCAGC 1500
   CCCAGGAGAG GAATGCCTGT CCTAGCAGCA CGTACATGGA GCACCCCACA TGTGCTCCAG 1560
   CCCTCTGGCT GTTTCTCTTG CTCTAGAATC AACTCCCTAC ATTGGGAATG TAGCCATTTG 1620
   GTAGAGGACT TGCCTAGCCT GCAGGAAGCT CACGTTCCAT CCCCTGCACC AAGGAGAATC 1680
   AAAGCTCAGG AGGCTGAGGC AGGAGGATTG CTGTCAGTGG TGTACAGAGG TCATGGCCAT 1740
   CCTGGGCTAT ATTAAACCTT GTCCTTTAAG AAAAAGAAAA GAAATCAACT TCCATTGAAT 1800
   CTGAGTTCTG CTCATTTCTG CACAGGTACA ATAGATGACT TKATTTGTTG AAAAATGKTT 1860
   AATATATTTA CMTATATATA TATTTGTAAG AAGCATT 1897
(2) INFORMATION FOR SEQ ID NO:41:
   (i) SEQUENCE CHARACTERISTICS:
      (A) LENGTH: 134 amino acids
      (B) TYPE: amino acid
      (C) STRANDEDNESS: single
      (D) TOPOLOGY: linear
   (ii) MOLECULE TYPE: DNA
   (xi) SEQUENCE DESCRIPTION: SEQ ID NO:41:
   Gly Gly Trp Asp Leu Gly Arg Asn Arg Leu Tyr His Asp Gly Lys Asn
   1               5                   10                  15
   Gln Pro Ser Lys Thr Tyr Pro Ala Phe Leu Glu Pro Asp Glu Thr Phe
               20                  25                  30
   Ile Val Pro Asp Ser Phe Phe Val Ala Leu Asp Met Xaa Asp Gly Thr
           35                  40                  45
   Leu Ser Phe Ile Val Asp Gly Gln Tyr Met Gly Val Ala Phe Arg Gly
       50                  55                  60
   Leu Lys Gly Lys Lys Leu Tyr Pro Val Val Ser Ala Val Trp Gly His
   65                  70                  75                  80
   Cys Glu Ile Arg Met Arg Tyr Leu Asn Gly Leu Asp Pro Glu Pro Leu
                   85                  90                  95
   Pro Leu Met Asp Leu Cys Arg Arg Ser Val Arg Leu Ala Leu Gly Lys
               100                 105                 110
   Glu Arg Leu Gly Ala Ile Pro Ala Leu Pro Leu Pro Ala Ser Leu Lys
           115                 120                 125
   Ala Tyr Leu Leu Tyr Gln
       130
(2) INFORMATION FOR SEQ ID NO:42:
   (i) SEQUENCE CHARACTERISTICS:
      (A) LENGTH: 265 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:42:
   AAGGGTAAAA AACTGTATCC TGTAGTGAGT GCCGTCTGGG GCCACTGTAG ATCCGAATGC 60
   GCTACTTGAA CGGACTCGAT CCCGAGACTG CCGCTCATGG ATTTGTGCCG TCGCTCGGTG 120
   CGCCTGGCCC TGGGGAGGGA GCGCCTGGGG GAGAACCACA CCTGCCGCTG CCGGCTTCCC 180
   TCAAGGCCTA CCTCCTCTAC CAGTGACGTT CGCCATCATA CCGCCAGCGC GACAGCCACC 240
   TGGTGCCAAC TCACTGAGCC GCCTG 265
(2) INFORMATION FOR SEQ ID NO:43:
   (i) SEQUENCE CHARACTERISTICS:
      (A) LENGTH: 2438 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:43:
   AAGTGGCGGC GGTCCCTGGA GAGCAGGCGG AGGCAGCGGC AAGTCTGACT CTGGGCTGAC 60
   CGTGGAGCCG GGGCGGGGGC TGACAGCCAG GCCTCCGCCT GGCGGGAGCC GCACGAGGAG 120
   CGGGAGTGGC CGGGCCTCTC TTCCGCGCTT GAGCGAGCGC CGGGTGATGG CGGTGGTGAT 180
   GGCGGCAGGC GCTCGGACAG CTCCGCTTGA GCTGAGCTCG GAGAGATCCG TCCAGAAAGT 240
   GCCCAGAAGA AACTTCCTCT TAGAAAAGCT GAAAAACACA RTATTTATAA CACTGGAAAT 300
   TGTAAAGAAT TTGTTTAAAA TGGCTGAAAA CAATAGTAAA AATGTAGATG TACGGCCTAA 360
   AACAAGTCGG AGTCGAAGTG CTGACAGGAA GGATGGTTAT GTGTGGAGTG GAAAGAAGTT 420
   GTCTTGGTCC AAAAAGAGTG AGAGTTGTTC TGAATCTGAA GCCATAGGTA CTGTTGAGAA 480
   TGTTGAAATT CCTCTAAGAA GCCAAGAAAG GCAGCTTAGC TGTTCGTCCA TTGAGTTGGA 540
   CTTAGATCAT TCCTGTGGGC ATAGATTTTT AGGCCGATCC CTTAAACAGA AACTGCAAGA 600
   TGCGGTGGGG CAGTGTTTTC CAATAAAGAA TTGTAGTGGC CGACACTCTC CAGGGCTTCC 660
   ATCTAAAAGA AAGATTCATA TCAGTGAACT CATGTTAGAT AAGTGCCCTT TCCCACCTCG 720
   CTCAGATTTA GCCTTTAGGT GGCATTTTAT TAAACGACAC ACTGTTCCTA TGAGTCCCAA 780
   CTCAGATGAA TGGGTGAGTG CAGACCTGTC TGAGAGGAAA CTGAGAGATG CTCAGCTGAA 840
   ACGAAGAAAC ACAGAAGATG ACATACCCTG TTTCTCACAT ACCAATGGCC AGCCTTGTGT 900
   CATAACTGCC AACAGTGCTT CGTGTACAGG TGGTCACATA ACTGGTTCTA TGATGAACTT 960
   GGTCACAAAC AACAGCATAG AAGACAGTGA CATGGATTCA GAGGATGAAA TTATAACGCT 1020
   GTGCACAAGC TCCAGAAAAA GGAATAAGCC CAGGTGGGAA ATGGAAGAGG AGATCCTGCA 1080
   GTTGGAGGCA CCTCCTAAGT TCCACACCCA GATCGACTAC GTCCACTGCC TTGTTCCAGA 1140
   CCTCCTTCAG ATCAGTAACA ATCCGTGCTA CTGGGGTGTC ATGGACAAAT ATGCAGCCGA 1200
   AGCTCTGCTG GAAGGAAAGC CAGAGGGCAC CTTTTTACTT CGAGATTCAG CGCAGGAAGA 1260
   TTATTTATTC TCTGTTAGTT TTAGACGCTA CAGTCGTTCT CTTCATGCTA GAATTGAGCA 1320
   GTGGAATCAT AACTTTAGCT TTGATGCCCA TGATCCTTGT GTCTTCCATT CTCCTGATAT 1380
   TACTGGGCTC CTGGAACACT ATAAGGACCC CAGTGCCTGT ATGTTCTTTG AGCCGCTCTT 1440
   GTCCACTCCC TTAATCCGGA CGTTCCCCTT TTCCTTGCAG CATATTTGCA GAACGGTTAT 1500
   TTGTAATTGT ACGACTTACG ATGGCATCGA TGCCCTTCCC ATTCCTTCGC CTATGAAATT 1560
   GTATCTGAAG GAATACCATT ATAAATCAAA AGTTAGGTTA CTCAGGATTG ATGTGCCAGA 1620
   GCAGCAGTGA TGCGGAGAGG TTAGAATGTC GACCTGCATA CATATTTTCA TTTAATATTT 1680
   TATTTTTCTT ATGCCTCTTT GAATTTTTGT ACAAAGGCAG TTGAATCAAA TAAAACTGTG 1740
   CCCTAAGTTT TAATTCCAGA TCAATTTATT TTTTTTATGA TACACTTGTT ATATATTTTT 1800
   AAGCAGGTGT TTGGTTTTGT TTTTACCATA TAAATTTACA TATGGTCCAG GCATATTTAC 1860
   AATTTCAAGG CATTGCATAT ACATTTGAAT ATTCTGTATT TTTTAAATAA TCTTTTGTTC 1920
   TTTCCTATGT GTGAAATATT TTGCTAATCT ATGCTATCAG TATTCTTGTA TGACCGAATA 1980
   GTTACCTATT CTCTTTTCAT CTTGAAGATT TTCAGTAAAG AGTGTTGTAA TCAATCCATT 2040
   ATAATGTAAT TGACTTTTGT AATTTGCCAA TAGGAGTGTT AAACAACAAA ATGATTTAAA 2100
   ATGAAACTTA ATGTATTTTC ATTTTAAATA TTAACTAAAC CAAGTTTGTT TGTTAGTTAT 2160
   TCTAGCCAAT AAGAAAAGAG AATGTAGCAT CCTAGAGGTG TATTTGTTCT GCAGTTTGGC 2220
   AGGACCGTCA GTTAGTCCAA ATAAACATCC CCTCAGCGTG GAGGCGAATG GAACCTGTGC 2280
   TCCTTTCTTA CGGGAAGCTT TGCAAAGCAA AATAGCAGGG TTACAAGCTT GGAGTTGTTA 2340
   AGGCAACTAG AGTTTTCTCT ATTAATTTAT AGACTGTTGT TGCACCTACT TAGCTCTTTT 2400
   TTGGGAACTC TAGTTCCCAG GGGAAAATAC CTCGTGCC 2438
(2) INFORMATION FOR SEQ ID NO:44:
   (i) SEQUENCE CHARACTERISTICS:
      (A) LENGTH: 542 amino acids
      (B) TYPE: amino acid
      (C) STRANDEDNESS: single
      (D) TOPOLOGY: linear
   (ii) MOLECULE TYPE: DNA
   (xi) SEQUENCE DESCRIPTION: SEQ ID NO:44:
   Ser Gly Gly Gly Pro Trp Arg Ala Gly Gly Gly Ser Gly Lys Ser Asp
   1 5 10 15
   Ser Gly Leu Thr Val Glu Pro Gly Arg Gly Leu Thr Ala Arg Pro Pro
   20 25 30
   Pro Gly Gly Ser Arg Thr Arg Ser Gly Ser Gly Arg Ala Ser Leu Pro
   35 40 45
   Arg Leu Ser Glu Arg Arg Val Met Ala Val Val Met Ala Ala Gly Ala
   50 55 60
   Arg Thr Ala Pro Leu Glu Leu Ser Ser Glu Arg Ser Val Gln Lys Val
   65 70 75 80
   Pro Arg Arg Asn Phe Leu Leu Glu Lys Leu Lys Asn Thr Xaa Phe Ile
   85 90 95
   Thr Leu Glu Ile Val Lys Asn Leu Phe Lys Met Ala Glu Asn Asn Ser
   100 105 110
   Lys Asn Val Asp Val Arg Pro Lys Thr Ser Arg Ser Arg Ser Ala Asp
   115 120 125
   Arg Lys Asp Gly Tyr Val Trp Ser Gly Lys Lys Leu Ser Trp Ser Lys
   130 135 140
   Lys Ser Glu Ser Cys Ser Glu Ser Glu Ala Ile Gly Thr Val Glu Asn
   145 150 155 160
   Val Glu Ile Pro Leu Arg Ser Gln Glu Arg Gln Leu Ser Cys Ser Ser
   165 170 175
   Ile Glu Leu Asp Leu Asp His Ser Cys Gly His Arg Phe Leu Gly Arg
   180 185 190
   Ser Leu Lys Gln Lys Leu Gln Asp Ala Val Gly Gln Cys Phe Pro Ile
   195 200 205
   Lys Asn Cys Ser Gly Arg His Ser Pro Gly Leu Pro Ser Lys Arg Lys
   210 215 220
   Ile His Ile Ser Glu Leu Met Leu Asp Lys Cys Pro Phe Pro Pro Arg
   225 230 235 240
   Ser Asp Leu Ala Phe Arg Trp His Phe Ile Lys Arg His Thr Val Pro
   245 250 255
   Met Ser Pro Asn Ser Asp Glu Trp Val Ser Ala Asp Leu Ser Glu Arg
   260 265 270
   Lys Leu Arg Asp Ala Gln Leu Lys Arg Arg Asn Thr Glu Asp Asp Ile
   275 280 285
   Pro Cys Phe Ser His Thr Asn Gly Gln Pro Cys Val Ile Thr Ala Asn
   290 295 300
   Ser Ala Ser Cys Thr Gly Gly His Ile Thr Gly Ser Met Met Asn Leu
   305 310 315 320
   Val Thr Asn Asn Ser Ile Glu Asp Ser Asp Met Asp Ser Glu Asp Glu
   325 330 335
   Ile Ile Thr Leu Cys Thr Ser Ser Arg Lys Arg Asn Lys Pro Arg Trp
   340 345 350
   Glu Met Glu Glu Glu Ile Leu Gln Leu Glu Ala Pro Pro Lys Phe His
   355 360 365
   Thr Gln Ile Asp Tyr Val His Cys Leu Val Pro Asp Leu Leu Gln Ile
   370 375 380
   Ser Asn Asn Pro Cys Tyr Trp Gly Val Met Asp Lys Tyr Ala Ala Glu
   385 390 395 400
   Ala Leu Leu Glu Gly Lys Pro Glu Gly Thr Phe Leu Leu Arg Asp Ser
   405 410 415
   Ala Gln Glu Asp Tyr Leu Phe Ser Val Ser Phe Arg Arg Tyr Ser Arg
   420 425 430
   Ser Leu His Ala Arg Ile Glu Gln Trp Asn His Asn Phe Ser Phe Asp
   435 440 445
   Ala His Asp Pro Cys Val Phe His Ser Pro Asp Ile Thr Gly Leu Leu
   450 455 460
   Glu His Tyr Lys Asp Pro Ser Ala Cys Met Phe Phe Glu Pro Leu Leu
   465 470 475 480
   Ser Thr Pro Leu Ile Arg Thr Phe Pro Phe Ser Leu Gln His Ile Cys
   485 490 495
   Arg Thr Val Ile Cys Asn Cys Thr Thr Tyr Asp Gly Ile Asp Ala Leu
   500 505 510
   Pro Ile Pro Ser Pro Met Lys Leu Tyr Leu Lys Glu Tyr His Tyr Lys
   515 520 525
   Ser Lys Val Arg Leu Leu Arg Ile Asp Val Pro Glu Gln Gln
   530 535 540
(2) INFORMATION FOR SEQ ID NO:45:
   (i) SEQUENCE CHARACTERISTICS:
      (A) LENGTH: 4999 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:45:
(2) INFORMATION FOR SEQ ID NO:46:
   (i) SEQUENCE CHARACTERISTICS:
      (A) LENGTH: 264 amino acids
      (B) TYPE: amino acid
      (C) STRANDEDNESS: single
      (D) TOPOLOGY: linear
   (ii) MOLECULE TYPE: DNA
   (xi) SEQUENCE DESCRIPTION: SEQ ID NO:46:
   Met Gly Gln Thr Ala Leu Ala Arg Gly Ser Ser Ser Thr Pro Thr Ser
   1 5 10 15
   Gln Ala Leu Tyr Ser Asp Phe Ser Pro Pro Glu Gly Leu Glu Glu Leu
   20 25 30
   Leu Ser Ala Pro Pro Pro Asp Leu Val Ala Gln Arg His His Gly Trp
   35 40 45
   Asn Pro Lys Asp Cys Ser Glu Asn Ile Asp Val Lys Glu Gly Gly Leu
   50 55 60
   Cys Phe Glu Arg Arg Pro Val Ala Gln Ser Thr Asp Gly Val Arg Gly
   65 70 75 80
   Lys Arg Gly Tyr Ser Arg Gly Leu His Ala Trp Glu Ile Ser Trp Pro
   85 90 95
   Leu Glu Gln Arg Gly Thr His Ala Val Val Gly Val Ala Thr Ala Leu
                   100                 105                 110
   Ala Pro Leu Gln Ala Asp His Tyr Ala Ala Leu Leu Gly Ser Asn Ser
           115                 120                 125
   Glu Ser Trp Gly Trp Asp Ile Gly Arg Gly Lys Leu Tyr His Gln Ser
       130                 135                 140
   Lys Gly Leu Glu Ala Pro Gln Tyr Pro Ala Gly Pro Gln Gly Glu Gln
   145                 150                 155                 160
   Leu Val Val Pro Glu Arg Leu Leu Val Val Leu Asp Met Glu Glu Gly
                   165                 170                 175
   Thr Leu Gly Tyr Ser Ile Gly Gly Thr Tyr Leu Gly Pro Ala Phe Arg
               180                 185                 190
   Gly Leu Lys Gly Arg Thr Leu Tyr Pro Ser Val Ser Ala Val Trp Gly
           195                 200                 205
   Gln Cys Gln Val Arg Ile Arg Tyr Met Gly Glu Arg Arg Val Glu Glu
       210                 215                 220
   Pro Gln Ser Leu Leu His Leu Ser Arg Leu Cys Val Arg His Ala Leu
   225                 230                 235                 240
   Gly Asp Thr Arg Leu Gly Gln Ile Ser Thr Leu Pro Leu Pro Pro Ala
                   245                 250                 255
   Met Lys Arg Tyr Leu Leu Tyr Lys
               260
(2) INFORMATION FOR SEQ ID NO:47:
   (i) SEQUENCE CHARACTERISTICS:
      (A) LENGTH: 5615 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:47:
   GTACTTTCTT TATATCTCCA TAATTTTATT TACTATTACT ACATGATACA TTATTTTATA 60
   AAAGTCTTTG TAACCTCCTT AAGGATTCAC TGCTTAATCT CCAGTGCTTA GCACAAATCA 120
   TTAAATGCGA ACCAGAAACT CTTCCAAATG TGTTACATCT ATAACCTCAT TGGATTCTCA 180
   CTACCAACCC CATGCAATAG ATACTAATGT GATCTCTGTC TTACAGAGGA AGAAACAGGC 240
   ACAGGGAGGT TCAGTAATTT GCCCAAGGTC ATACACACAC TGGCCTTCAG GTATTCATGC 300
   CCGGGGAGTC TGGTCCCACA GCTGGCATGT TTGCCATTAT ATTATATTGC CTCCTTATAG 360
   TGTCGGCACT CATTAAGCAC ATTGACAGCT ATGCTTGGTG AGTGACTACT ATGTACCCAG 420
   CTCTGTGCTA CATGCTTTAC CTGGATTATT TCAACTGCAC AACAACCCTG TGAGGTAACT 480
   ACCATCATTG CTCCTATTTT ACATAACAGA AAACTACAGA AATCTGGGGC TGGGCGTAGT 540
   GGCTCATGCC TGAAATCCCA GCACTTTGGG AGACCCTGTC TCTAAAAAAA ATTTTTTTTT 600
   GGCCGGACGT GGTGGCTCAC ACCTGTAATC TCAGCACTTT GGGAGGCTAA GGCAGGCAGA 660
   TCACAAGGTC AGGAGTTCTA GACCAGCCTG GCCAACATGG CAAAACCCTG TGTCTACTAA 720
   AAATACAAAA AATAGCTAGG CGTGGTGGCA GGTGCCTGTA ATCCCAGCTA CTCAGGAGGC 780
   TGAGGCAGGA GAATCCCCTG AACCTGGGAG ATGGAGGTTA CAGAGAGCCG AGATCGTGCC 840
   GCTGCACTCC AGCCTGGGCA ACAAGAGCAA GACTCTGTCT CGAAAAAAAT AAAAATAAAA 900
   ATAAAAATAT TTTTTTAAAA ATTAGCTGGG TGTGGTAGCA CATGCCTGTA GTCCCAGCTA 960
   CTTGGGAGGC TGAGGTAGGA GGATCACTTG AGCCCAGGAG GTCAAGGCTG CAGTGGGCTG 1020
   TGATGGCGCC ACTGCACTCT AGCCTTGGTG ACAGCAAGAC CCTGTCTCAA AAAAAAAAAA 1080
   AAGAGAAATC GGGCAACTTC CCCAAGATCG CGCAGTTAAC TAGTGGCATA GCTTCACTCA 1140
   AACTCGAAGT CTTAATCAGG ACACTCTACC AAATGAGATC AACGGCTCAG TAATGGATTG 1200
   GCATCCAGTA TGAAGACTGG ACCAGCAGGG AGAACTATGA TGCGTACAGC CTAGAGCCTG 1260
   AAGCAGATTT CACAGCCTCA GAGGTGGCAC AGGCTGACTC ACAACCCGGG GCAGAAAGGG 1320
   ACCAGCCCAG AAACAGTGAC CCAGAATCAC AGGGAAGTAG AAATGGGATT CGGCACAATG 1380
   AAGCCCCTCC TTGACCCCAT GCTCCTTACC CTCAGGGGCG CAGGAGTTAG TCGCTCAGGC 1440
   GGCTCAAAGG TCTTGACGGT GGAGAACACC ATCCCCAGGG ATTCCCGACG CGGTGATGCC 1500
   ATCAAAGCGT TAATTCTGAG ATGGGCCTGC CCGGGTGCGG ACTCTGCCGC AGCAAGAGAA 1560
   GGGTTAACTG CCCCGGGCCT TCGCCGTGGG GGCGGGGCCT CGGGGAGGGT CACAGCCCGG 1620
   GACTGAGACC CGAGGTTAAC CGCCCGGGGT GGGCTCCACG GGGGCGGGGC ATGCTCTCCG 1680
   CGGCTGCTGC CGGTATAGAG CGGTAACTGC CCAGGAGGGG GCGGGGCCCC ACAGGGGCGT 1740
   GGCCTCGGAG CTGCACGGCC GTGGGCGGCG ATGAGAGGGT TAAGCCCCAG AGGGCCCTGG 1800
   AGGGGCGGGG CCGCGGGACG GGCTCGGCCC AAGGGAGGAG CTGGGGGCGG AAGCGGCCGG 1860
   CGGTCTGCGC CCTGCGCGCC TCGGCTTCTT TCCGCCCGGC TCCTTCAGAG GCCCGGCGAC 1920
   CTCCAGGGCT GGGAAGTCAA CCGAGGTTCG GGGGCAGCGG CGAGGGCTCC GGGCGAGTAA 1980
   GGGGGATGGT CCATGCTGAG GCCCAAATGG GGCGAACTCG CGAGAGTCTC TGGCGACCTG 2040
   GATCAGATGG GGCGAGGGCA GATGAAGGGC CCAGGAGCTT TGGGGCAGCG AGGAGGGAGG 2100
   AGCGGGCCCG TTGGCAAACT TGGGTGAAAG GATGGGGTAC CTGGGTGACG AGCCCCCGCC 2160
   AGGATTCTGC TCTTCACGCC CCTTTTCTCC CAGCTCCCTT CCAGGTCAAT CCAAACTGGA 2220
   GCTCAACTTT CAGAAGAGAA AGACGCCCCA GCAAGCCTCT TTCGGGGAGT CCTCTAGCTC 2280
   CTCACCTCCA TGGGCCAGAC AGCTCTGGCA GGGGGCAGCA GCAGCACCCC CACGCCACAG 2340
   GCCCTGTACC CTGACCTCTC CTGTCCCGAG GGCTTGGAAG AGCTGCTGTC TGCACCCCCT 2400
   CCTGACCTGG GGGCCCAGCG GCGCCACGGT TGGAACCCCA AAGACTGTTC AGAGAACATC 2460
   GAGGTCAAGG AAGGAGGGTT GTACTTTGAG CGGCGGCCCG TGGCCCAGAG CACTGATGGG 2520
   GCCCGGGGTA AGAGGGGCTA TTCAAGGGGC CTGCACGCCT GGGAGATCAG CTGGCCCCTA 2580
   GAGCAGAGGG GCACGCATGC CGTGGTGGGC GTGGCCACGG CCCTCGCCCC GCTGCAGACT 2640
   GACCACTACG CGGCGCTGCT GGGCAGCAAC AGCGAGTCGT GGGGCTGGGA CATCGGGCGG 2700
   GGGAAGCTGT ACCATCAGAG CAAGGGGCCC GGAGCCCCCC AGTATCCAGC GGGAACTCAG 2760
   GGTGAGCAGC TGGAGGTGCC AGAGAGACTG CTGGTGGTTC TGGACATGGA GGAGGGAACT 2820
   CTGGGCTACG CTATTGGGGG CACCTACCTG GGGCCAGCAT TCCGCGGACT GAAGGGCAGG 2880
   ACCCTCTATC CGGCAGTAAG CGCTGTCTGG GGCCAGTGCC AGGTCCGCAT CCGCTACCTG 2940
   GGCGAAAGGA GAGGTGAGGC CTGGGGCAGA CGTGGGGAGA ACTTTCTGTC CCTGGTGGCA 3000
   GTGGTTTGGG ATGGAAACTC TTCTGACAAG AGCAGAGGGG ATGGACCTTC ATCCAGCCTG 3060
   CCTCAACCTC TGTTCAGTGC TGGGAAAGGC TAGGGGTCTT CACAGCTGTT ATTTAATTTA 3120
   ACCCAACAGC AATAGAGGTG AAACAGGCTT GAGAAAGCAA CTTTCTCAAG TTCTCTTGGC 3180
   CAGTAAATGG TGAACCTTCA GAATGGAGGG AGGAACTGCA GGGATGAGAG AATTCAGGAG 3240
   ATATCAACCC CTGAGCAAGA GGTGCAAAGC GTTAGGTACT GGGTTTGATG TACAGGTCCA 3300
   AAAGAAGGAT GGGCAGAGCC AGGTACCCAG GCTGTATACC GGATTCCCTG GGCTCTAACC 3360
   TGTCTCTGTG CCACATACCT ACTTCCTTCC TCAGCCACAC CTCTGGATGG AGACACTGGG 3420
   GCCCTGGGCA CCAGGGAGGA GAGCAGTGGA GGAGGCAGGG CCTTAGGGTG GGGCAGCAGG 3480
   GGAGGAGCCT CCCCAGGAAC TGACTGGGTC CAGGGCTTGG AGCTGCTCTC TGCAGTTGTG 3540
   TGGGCTGTAG AGTGGAGGGC CATCCCTCCT CACCTCAGCC CCAGCTCCCA AGCCTCTGGA 3600
   GTCAAAGCCT GGGCCAGCTC CACCACTGTC AGAGCCACCT TGGCCTGTTG TTTAGAGGGC 3660
   CTTAGCCAGC TCTTCACCCC CAGCTCTGAC TAGGGATGTG TGAAATCTTA TCTGGGAGGC 3720
   AGAACTTCCG GGTATCTCAA ATTCCCCTTT CAGCCAGGTG GGCACACTCG AAGCAGGAAA 3780
   GCAGAAAGGC ATCTGAGTAG GACCCCGTAG TTTGAGGACA TCTGGCTGGT GGCTGCACCC 3840
   ATACTTACAT TCCCCTCCTT CTCTCTCCCA GCGGAGCCAC ACTCCCTTCT GCACCTGAGC 3900
   CGCCTGTGTG TGCGCCACAA CCTGGGGGAT ACCCGGCTCG GCCAGGTGTC TGCCCTGCCC 3960
   TTGCCCCCTG CCATGAAGCG CTACCTGCTC TACCAGTGAG CCCTGTGATA CCACAGACTG 4020
   TGCTGAGGTC TTGCCACCAC CCCTCCCCTT GGGGAGGTGG GGAGGCACTG CTGGCCTAGA 4080
   CCAGCTGCTG AAAGCTGGTG AGGCTGAGCC CCTACCCCAA CCCAAGCTCT GCGGAAATCA 4140
   ACAGCCCCAG AGCCACTTGG AGGGAGGAAG AAAGGGAGCC GGCGTTCAAG GCTATGACAG 4200
   TCTGCTACGC AAAACATTTT TTCAAGTAAA AATAGTAAGA GATGTTGTTA TAGAAACCTG 4260
   TTCTTGTTTT TTTTTTTTTC TTGCACAAAT GATCATTTAT ATAGCTGCCT CAAAAAGGAA 4320
   GATTATCTGG GCAAGTCCAG TGAAGGCAGA CAAACCACAA GACCTAGTGC CAGGTTTATT 4380
   CCCTCACATG GGTGGTTCAC ATACACAGCA CAGAGGCACG GGCACCATGG GAGAGGGCAG 4440
   CACTCCTGCC TTCTGAGGGG ATCTTGGCCT CACGGTGTAA GAAGGGAGAG GATGGTTTCT 4500
   CTTCTGCCCT CACTAGGGCC TAGGGAACCC AGGAGCAAAT CCCACCACGC CTTCCATCTC 4560
   TCAGCCAAGG AGAAGCCACC TTGGTGACGT TTAGTTCCAA CCATTATAGT AAGTGGAGAA 4620
   GGGATTGGCC TGGTCCCAAC CATTACAGGG TGAAGATATA AACAGTAAAG GAAGATACAG 4680
   TTTGGATGAG GCCACAGGAA GGAGCAGATG ACACCATCAG AAGCATATGC AGGGAAAGGG 4740
   CAGTTACTGG GCTTCTGGGC TGCTTAGTCC CTGGCTTGGC AGGAAGGGTA GGGAAGATGG 4800
   ATGGGGCTCA TTGTTTGGCA TTGATGATGT CCACGAATTC GGGCTTGAGG GAAGCACCAC 4860
   CCACAAGGAA GCCATCCACA TCAGGCTGGC TGGCCAGCTC CTTGCAGGTT GCCCCAGTCA 4920
   CAGAGCCTGG GAAGGGAGCA GAACAAGGGC TTGGTCAAGA ATGGGATGAG TCTGCCCCAT 4980
   CCCCACCTCC ATGTCCGAGG GCTCAGTCTA GTCCTCAGCC CACTCCACCT CAGCCGGGAA 5040
   CCAAAGCCAC TCACCTCCAT AAATGATACG GGTGCTCTGA GCCACCGCAT CAGAGACGTT 5100
   GGACTTCAGC CATCCTCGGA GCTTCTCGTG TACTTCCTGG GCCTAGAACA AGAAGCTGGC 5160
   CTAAGTAAGA CCTTTTCTGC CTCTCTAAGA GGAAAAATCA CTGGCACCAG TGGACACTTA 5220
   GTGTGGTTTC TGACTGAGTC AGAGTACCAG GGCTCTGATC CAAGCCAGGC CCTGGACTGG 5280
   ATGCCCTTGG ACAAGTCACT GTCTCTGGGT TCAAGGTCTC TGTGTCTTTG AAATAAGGGG 5340
   TTGCCCCATG TGGGCTGTGT CTGTCCAAAC CTATTGAGGC AGGCTGGGAT GAGGGCAGGG 5400
   CTCCTGGGCC CGGTTACCTG TTGGGGTGTT GCAGTCTTGC CAGTACCAAT GGCCCACACA 5460
   GGCTCATAGG CCAGGACGAC CTTGCTCCAG TCCTTCACGT TATCTGCAGG GCAGAGATAC     5520
   AGATGGAGGG AAGGGTGAAC AAGAAAGAGC TCTCCAGCCA GGTTCTCCGG AGTACGAAGA     5580
   ACGGTGGCCT ACTGCCCCCT AGTGGACATT GGGGG                                5615
(2) INFORMATION FOR SEQ ID NO:48:
   (i) SEQUENCE CHARACTERISTICS:
      (A) LENGTH: 263 amino acids
      (B) TYPE: amino acid
      (C) STRANDEDNESS: single
      (D) TOPOLOGY: linear
   (ii) MOLECULE TYPE: DNA
   (xi) SEQUENCE DESCRIPTION: SEQ ID NO:48:
   Met Gly Gln Thr Ala Leu Ala Gly Gly Ser Ser Ser Thr Pro Thr Pro
   1               5                   10                  15
   Gln Ala Leu Tyr Pro Asp Leu Ser Cys Pro Glu Gly Leu Glu Glu Leu
               20                  25                  30
   Leu Ser Ala Pro Pro Pro Asp Leu Gly Ala Gln Arg Arg His Gly Trp
           35                  40                  45
   Asn Pro Lys Asp Cys Ser Glu Asn Ile Glu Val Lys Glu Gly Gly Leu
       50                  55                  60
   Tyr Phe Glu Arg Arg Pro Val Ala Gln Ser Thr Asp Gly Ala Arg Gly
   65                  70                  75                  80
   Lys Arg Gly Tyr Ser Arg Gly Leu His Ala Trp Glu Ile Ser Trp Pro
                   85                  90                  95
   Leu Glu Gln Arg Gly Thr His Ala Val Val Gly Val Ala Thr Ala Leu
               100                 105                 110
   Ala Pro Leu Gln Thr Asp His Tyr Ala Ala Leu Leu Gly Ser Asn Ser
           115                 120                 125
   Glu Ser Trp Gly Trp Asp Ile Gly Arg Gly Lys Leu Tyr His Gln Ser
       130                 135                 140
   Lys Gly Pro Gly Ala Pro Gln Tyr Pro Ala Gly Thr Gln Gly Glu Gln
   145                 150                 155                 160
   Leu Glu Val Pro Glu Arg Leu Leu Val Val Leu Asp Met Glu Glu Gly
                   165                 170                 175
   Thr Leu Gly Tyr Ala Ile Gly Gly Thr Tyr Leu Gly Pro Ala Phe Arg
               180                 185                 190
   Gly Leu Lys Gly Arg Thr Leu Tyr Pro Ala Val Ser Ala Val Trp Gly
           195                 200                 205
   Gln Cys Gln Val Arg Ile Arg Tyr Leu Gly Glu Arg Arg Ala Glu Pro
       210                 215                 220
   His Ser Leu Leu His Leu Ser Arg Leu Cys Val Arg His Asn Leu Gly
   225                 230                 235                 240
   Asp Thr Arg Leu Gly Gln Val Ser Ala Leu Pro Leu Pro Pro Ala Met
                   245                 250                 255
   Lys Arg Tyr Leu Leu Tyr Gln
               260
(2) INFORMATION FOR SEQ ID NO:49:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:49:
      AGCTAGATCT GGACCCTACA ATGGCAGC 28
(2) INFORMATION FOR SEQ ID NO:50:
   (i) SEQUENCE CHARACTERISTICS:
      (A) LENGTH: base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:50:
      AGCTAGATCT GCCATCCTAC TCGAGGGGCC AGCTGG 36

## Description

### FIELD OF THE INVENTION

The present invention relates generally to therapeutic and diagnostic agents. More particularly, the present invention provides therapeutic molecules capable of modulating signal transduction such as but not limited to cytokine-mediated signal transduction. The molecules of the present invention are useful, therefore, in modulating cellular responsiveness to cytokines as well as other mediators of signal transduction such as endogenous or exogenous molecules, antigens, microbes and microbial products, viruses or components thereof, ions, hormones and parasites.

Bibliographic details of the publications referred to in this specification by author are collected at the end of the description. Sequence Identity Numbers (SEQ ID NOs.) for the nucleotide and amino acid sequences referred to in the specification are defined after the bibliography. A summary of the SEQ ID NOs is given in Table 1.

Throughout this specification and the claims which follow, unless the context requires otherwise, the word "comprise", or variations such as "comprises" or "comprising", will be understood to imply the inclusion of a stated integer or group of integers but not the exclusion of any other integer or group of integers.

### BACKGROUND OF THE INVENTION

Cells continually monitor their environment in order to modulate physiological and biochemical processes which in turn affects future behaviour. Frequently, a cell's initial interaction with its surroundings occurs via receptors expressed on the plasma membrane. Activation of these receptors, whether through binding endogenous ligands (such as cytokines) or exogenous ligands (such as antigens), triggers a biochemical cascade from the membrane through the cytoplasm to the nucleus.

Of the endogenous ligands, cytokines represent a particularly important and versatile group. Cytokines are proteins which regulate the survival, proliferation, differentiation and function of a variety of cells within the body [Nicola, 1994]. The haemopoietic cytokines have in common a four-alpha helical bundle structure and the vast majority interact with a structurally related family of cell surface receptors, the type I and type II cytokine receptors [Bazan, 1990; Sprang, 1993]. In all cases, ligand-induced receptor aggregation appears to be a critical event in initiating intracellular signal transduction cascades. Some cytokines, for example growth hormone, erythropoietin (Epo) and granulocyte-colony-stimulating factor (G-CSF), trigger receptor homodimerisation, while for other cytokines, receptor heterodimerisation or heterotrimerisation is crucial. In the latter cases, several cytokines share common receptor subunits and on this basis can be grouped into three subfamilies with similar patterns of intracellular activation and similar biological effects [Hilton, 1994]. Interleukin-3 (IL-3). IL-5 and granulocyte-macrophage colony-stimulating factor (GM-CSF) use the common β-receptor subunit (βc) and each cytokine stimulates the production and functional activity of granulocytes and macrophages. IL-2, IL-4, IL-7, IL-9, and IL-15 each use the common γ-chain (γc), while IL-4 and IL-13 share an alternative γ-chain (γ'c or IL-13 receptor α-chain). Each of these cytokines plays an important role in regulating acquired immunity in the lymphoid system. Finally, IL-6, IL-11, leukaemia inhibitory factor (LIF), oncostatin-M (OSM), ciliary neurotrophic factor (CNTF) and cardiotrophin (CT) share the receptor subunit gp130. Each of these cytokines appears to be highly pleiotropic, having effects both within and outside the haemopoietic system [Nicola, 1994].

In all of the above cases at least one subunit of each receptor complex contains the conserved sequence elements, termed box1 and box2, in their cytoplasmic tails [Murakami. 1991]. Box1 is a proline-rich motif which is located more proximal to the transmembrane domain than the - acidic box 2 element. The box-1 region serves as the binding site for a class of cytoplasmic tyrosine kinases termed JAKs (Janus kinases). Ligand-induced receptor dimerisation serves to increase the catalytic activity of the associated JAKs through cross-phosphorylation. Activated JAKs then tyrosine phosphorylate several substrates, including the receptors themselves. Specific phosphotyrosine residues on the receptor then serve as docking sites for SH2-containing proteins, the best characterised of which are the signal transducers and activators of transcription (STATs) and the adaptor protein, shc. The STATs are then phosphorylated on tyrosines, probably by JAKs, dissociate from the receptor and form either homodimers or heterodimers through the interaction of the SH2 domain of one STAT with the phosphotyrosine residue of the other. STAT dimers then translocate to the nucleus where they bind to specific cytokine-responsive promoters and activate transcription [Darnell, 1994; Ihle, 1995; Ihle, 1995]. In a separate pathway, tyrosine phosphorylated she interacts with another SH2 domain-containing protein, Grb-2, leading ultimately to activation of members of the MAP kinase family and in turn transcription factors such as fos and jun [Sato, 1993; Cutler, 1993]. These pathways are not unique to members of the cytokine receptor family since cytokines that bind receptor tyrosine kinases also being able to activate STATs and members of the MAP kinase family [David, 1996; Leaman, 1996; Shual, 1993; Sato, 1993; Cutler, 1993].

Four members of the JAK family of cytoplasmic tyrosine kinases have been described, JAK1, JAK2, JAK3 and TYK2, each of which binds to a specific subset of cytokine receptor subunits. Six STATs have been described (STAT1 through STAT6), and these too are activated by distinct cytokine/receptor complexes. For example, STAT 1 appears to be functionally specific to the interferon system, STAT4 appears to be specific to IL-12, while STAT6 appears to be specific for IL-4 and IL-13. Thus, despite common activation mechanisms some degree of cytokine specificity may be achieved through the use of specific JAKs and STATs [Thierfelder, 1996; Kaplan, 1996; Takeda, 1996; Shimoda, 1996; Meraz, 1996; Durbin, 1996].

In addition to those described above, there are clearly other mechanisms of activation of these pathways. For example, the JAK/STAT pathway appears to be able to activate MAP kinases independent of the she-induced pathway [David, 1995] and the STATs themselves can be activated without binding to the receptor, possibly by direct interaction with JAKs [Gupta, 1996]. Conversely, full activation of STATS may require the action of MAP kinase in addition to that of JAKs [David, 1995; Wen, 1995].

While the activation of these signalling pathways is becoming better understood, little is known of the regulation of these pathways, including employment of negative or positive feedback loops. This is important since once a cell has begun to respond to a stimulus, it is critical that the intensity and duration of the response is regulated and that signal transduction is switched off. It is likewise desirable to increase the intensity of a response systemically or even locally as the situation requires.

In work leading up to the present invention, the inventors sought to isolate negative regulators of signal transduction. The inventors have now identified a new family of proteins which are capable of acting as regulators of signalling. The new family of proteins is defined as the suppressor of cytokine signalling (SOCS) family based on the ability of the initially identified SOCS molecules to suppress cytokine-mediated signalling. It should be noted, however, that not all members of the SOCS family need necessarily share suppressor function nor target solely cytokine mediated signalling. The SOCS family comprises at least three classes of protein molecules based on amino acid sequence motifs located N-terminal of a C-terminal motif called the SOCS box. The identification of this new family of regulatory molecules permits the generation of a range of effector or modulator molecules capable of modulating signal transduction and, hence, cellular responsiveness to a range of molecules including cytokines. The present invention, therefore, provides therapeutic and diagnostic agents based on SOCS proteins, derivatives, homologues, analogues and mimetics thereof as well as agonists and antagonists of SOCS proteins.

### SUMMARY OF THE INVENTION

The present invention concerns *inter alia* nucleic acid molecules encoding members of the SOCS family of proteins as well as the proteins themselves. Reference hereinafter to "SOCS" encompasses any or all members of the SOCS family. Specific SOCS molecules are defined numerically such as, for example, SOCS1, SOCS2 and SOCS3. The species from which the SOCS has been obtained may be indicated by a preface of a single letter abbreviation where "h" is human, "m" is murine and "r" is rat. Accordingly, "mSOCS1" is a specific SOCS from a murine animal. Reference herein to "SOCS" is not to imply that the protein solely suppresses cytokine-mediated signal transduction, as the molecule may modulate other effector-mediated signal transductions such as by hormones or other endogenous or exogenous molecules, antigens, microbes and microbial products, viruses or components thereof, ions, hormones and parasites. The term "modulates" encompasses up-regulation, down-regulation as well as maintenance of particular levels. The invention claimed herein is particularly concerned with a SOCS-3 protein.

One aspect of the present invention accordingly provides a nucleic acid molecule comprising a nucleotide sequence encoding, or complementary to a sequence encoding, a protein which comprises the amino acid sequence of SEQ ID NO. 8 or an amino acid sequence having at least 70% similarity to SEQ ID NO. 8, wherein said protein comprises a SOCS box in its C-terminal region and modulates signal transduction.

Such a nucleic acid molecule may encode a protein further comprising a protein:molecule interacting region located in a region N-terminal of the SOCS box.

Preferably, the protein : molecule interacting region is a protein:DNA or protein:protein binding region.

In particular, the nucleic acid molecule of the present invention may encode a protein comprising an SH2 domain N-terminal of the SOCS box.

The protein comprises a SOCS box in its C-terminal region wherein the SOCS box comprises the amino acid sequence:

X₁ X₂ X₃ X₄ X₅ X₆ X₇ X₈ X₉ X₁₀ X₁₁ X₁₂ X₁₃ X₁₄ X₁₅ X₁₆ [Xⱼ]ₙ X₁₇ X₁₈ X₁₉ X₂₀ X₂₁ X₂₂ X₂₃ [Xⱼ]ₙ X₂₄ X₂₅ X₂₆ X₂₇ X₂₈

wherein:
X₁ is L, I, V, M, A or P;
X₂ is any amino acid residue;
X₃ is P,T or S;
X₄ is L,I, V, M, A or P;
X₅ is any amino acid;
X₆ is any amino acid;
X₇ is L, I, V, M, A, F, Y or W;
X₈ is C, T or S;
X₉ is R, K or H;
X₁₀ is any amino acid;
X₁₁ is any amino acid;
X₁₂ is L, I, V, M, A or P;
X₁₃ is any amino acid;
X₁₄ is any amino acid;
X₁₅ is any amino acid;
X₁₆ is L, I, V, M, A, P, G, C, T or S;
[Xᵢ]ₙ is a sequence of n amino acids wherein n is from 1 to 50 amino acids and wherein the sequence Xᵢ may comprise the same or different amino acids selected from any amino acid residue;
X₁₇ is L, I, V, M, A or P;
X₁₈ is any amino acid;
X₁₉ is any amino acid;
X₂₀L, I, V, M, A or P;
X₂₁ is P;
X₂₂ is L, I, V, M, A, P or G;
X₂₃ is P or N;
[Xⱼ]ₙ is a sequence of n amino acids wherein n is from 1 to 50 amino acids and wherein the sequence Xj may comprise the same or different amino acids selected from any amino acid residue;
X₂₄ is L, I, V, M, A or P;
X₂₅ is any amino acid;
X₂₆ is any amino acid;
X₂₇ is Y or F;
X₂₈ is L, I, V, M, A or P.
and a protein : molecule interacting region such as an SH2 domain N-terminal of the SOCS box.

Preferably, the SOCS molecules modulate signal transduction such as from a cytokine or hormone or other endogenous or exogenous molecule, a microbe or microbial product, an antigen or a parasite.

More preferably, the SOCS molecules modulate cytokine mediated signal transduction.

Accordingly the nucleic acid molecule of the present invention is further defined as encoding a protein being capable of modulating signal transduction.

Preferably, the signal transduction is mediated by a cytokine such as one or more of EPO, TPO, G-CSF, GM-CSF, IL-3, IL-2, IL-4, IL-7, IL-13, IL-6, LIF, IL-12, IFNα, TNFα, IL-1 and/or M-CSF.

Preferably, the signal transduction is mediated by one or more of Interleukin 6 (IL-6), Leukaemia Inhibitory Factor (LIF).

Preferably, the signal transduction is mediated by IL-6.

Particularly preferred nucleic acid molecules comprise nucleotide sequences substantially set forth in SEQ ID NO:7 (mSOCS3) or a nucleotide sequence having at least 70% similarity to SEQ ID NO:7 or a nucleic acid molecule capable of hybridizing to SEQ ID NO:7 under medium stringency conditions at 42°C.

Another aspect of the present invention relates to a protein comprising the amino acid sequence of SEQ ID NO. 8 or an amino acid sequence having at least 70% similarity to SEQ ID NO. 8, wherein the protein comprises a SOCS box in its C-terminal region and modulates signal transduction.

The protein of the present invention may further comprise a protein:molecule interacting region, which may be located in a region N-terminal of the SOCS box.

Preferably, the protein: molecule interacting region is a protein:DNA or a protein:protein binding region.

In particular, the protein of the present invention may comprise a SH2 domain N-terminal of the SOCS box.

The protein modulates signal transduction such as cytokine-mediated signal transduction.

Preferred cytokines are EPO, TPO, G-CSF, GM-CSF, IL-3, IL-2, IL-4, IL-7, IL-13, IL-6, LIF, IL-12, IFNγ, TNFα, IL-1 and/or M-CSF.

A particularly preferred cytokine is IL-6.

Another aspect of the present invention contemplates an *in vitro* method of modulating levels of a SOCS-3 protein in a cell said method comprising contacting a cell containing a SOCS-3 gene with an effective amount of a modulator of SOCS-3 gene expression or SOCS-3 protein activity for a time and under conditions sufficient to modulate levels of said SOCS-3 protein.

A related aspect of the present invention provides an *in vitro* method of modulating signal transduction in a cell containing a SOCS-3 gene comprising contacting said cell with an effective amount of a modulator of SOCS-3 gene expression or SOCS-3 protein activity for a time sufficient to modulate signal transduction.

Yet a further related aspect of the present invention is directed to an *in vitro* method of influencing interaction between cells wherein at least one cell carries a SOCS-3 gene, said method comprising contacting the cell carrying the SOCS-3 gene with an effective amount of a modulator of SOCS-3 gene expression or SOCS-3 protein activity for a time sufficient to modulate signal transduction.

The invention also provides use of a modulator of the expression of a SOCS-3 gene comprising a nucleotide sequence as hereinbefore defined, or the activity of a SOCS-3 protein as hereinbefore defined, in the manufacture of a medicament for use of a modulator of the expression of a SOCS-3 gene comprising a nucleotide sequence as defined in any one of claims 1, 2 or 4 to 7 or the activity of a SOCS-3 protein as defined in any one of claims 3 to 7, in the manufacture of a medicament for treatment of a condition by modulating levels of a said SOCS-3 protein, modulating signal transduction or influencing interaction between cells.

In accordance with the present invention, n in [Xᵢ]ₙ and [Xⱼ]ₙ may, in addition from being 1-50, be from 1-30,1-20,1-10 and 1-5.

A summary of the SEQ ID NOs referred to in the subject specification is given in Table 1.

**TABLE 1**

| **SUMMARY OF SEQUENCE IDENTITY NUMBERS** | |
|---|---|
| **SEQUENCE** | **SEQ ID NO.** |
| PCR Primer | 1 |
| PCR Primer | 2 |
| Mouse SOCS 1 (nucleotide) | 3 |
| Mouse SOCS1 (amino acid) | 4 |
| Mouse SOCS2 (nucleotide) | 5 |
| Mouse SOCS2 (amino acid) | 6 |
| Mouse SOCS3 (nucleotide) | 7 |
| Mouse SOCS3 (amino acid) | 8 |
| Human SOCS 1 (nucleotide) | 9 |
| Human SOCS 1 (amino acid) | 10 |
| Rat SOCS 1 (nucleotide) | 11 |
| Rat SOCS1 (amino acid) | 12 |
| nucleotide sequence of murine SOCS4 | 13 |
| amino acid sequence of murine SOCS4 | 14 |
| nucleotide sequence of SOCS4 cDNA human contig 4.1 | 15 |
| nucleotide sequence of SOCS4 cDNA human contig 4.2 | 16 |
| nucleotide sequence of murine SOCS5 | 17 |
| amino acid sequence of murine SOCS5 | 18 |
| nucleotide sequence of human SOCS5 | 19 |
| nucleotide sequence of murine SOCS6 | 20 |
| amino acid of murine SOCS6 | 21 |
| nucleotide sequence of human SOCS6 contig h6.1 | 22 |
| nucleotide sequence of human SOCS6 contig h6.2 | 23 |
| nucleotide sequence of murine SOCS7 | 24 |
| amino acid sequence of murine SOCS7 | 25 |
| nucleotide sequence of human SOCS7 contig h7.1 | 26 |
| nucleotide sequence of human SOCS7 contig 17.2 | 27 |
| nucleotide sequence of murine SOCS8 | 28 |
| amino acid sequence of murine SOCS8 | 29 |
| nucleotide sequence of murine SOCS9 | 30 |
| nucleotide sequence of human SOCS9 | 31 |
| nucleotide sequence of murine SOCS10 | 32 |
| nucleotide sequence of human SOCS10 contig h10.1 | 33 |
| nucleotide sequence of human SOCS10 contig h10.2 | 34 |
| nucleotide sequence of human SOCS11 | 35 |
| amino acid sequence of human SOCS11 | 36 |
| nucleotide sequence of mouse SOCS12 | 37 |
| nucleotide sequence of human SOCS12 contig h12.1 | 38 |
| nucleotide sequence of human SOCS12 contig h12.2 | 39 |
| nucleotide sequence of murine SOCS13 | 40 |
| amino acid sequence of murine SOCS13 | 41 |
| nucleotide sequence of human SOCS13 cDNA contig h 13.1 | 42 |
| nucleotide sequence of murine SOCS 14 cDNA | 43 |
| amino acid sequence of murine SOCS14 | 44 |
| nucleotide sequence of murine SOCS15 cDNA | 45 |
| amino acid sequence of murine SOCS15 | 46 |
| nucleotide sequence of human SOCS15 | 47 |
| amino acid sequence of human SOCS15 | 48 |

Single and three letter abbreviations are used to denote amino acid residues and these are summarized in Table 2.

**TABLE 2**

| Amino Acid | Three-letter Abbreviation | One-letter Symbol |
|---|---|---|
| Alanine | Ala | A |
| Arginine | Arg | R |
| Asparagine | Asn | N |
| Aspartic acid | Asp | D |
| Cysteine | Cys | C |
| Glutamine | Gln | Q |
| Glutamic acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |
| Any residue | Xaa | X |

### BRIEF DESCRIPTION OF THE DRAWINGS

In some of the Figures, abbreviations are used to denote SOCS proteins with certain binding motifs. SOCS proteins which contain WD-40 repeats are referred to as WSB1-WSB4. SOCS proteins with ankyrin repeats are referred to as ASB 1-ASB3.

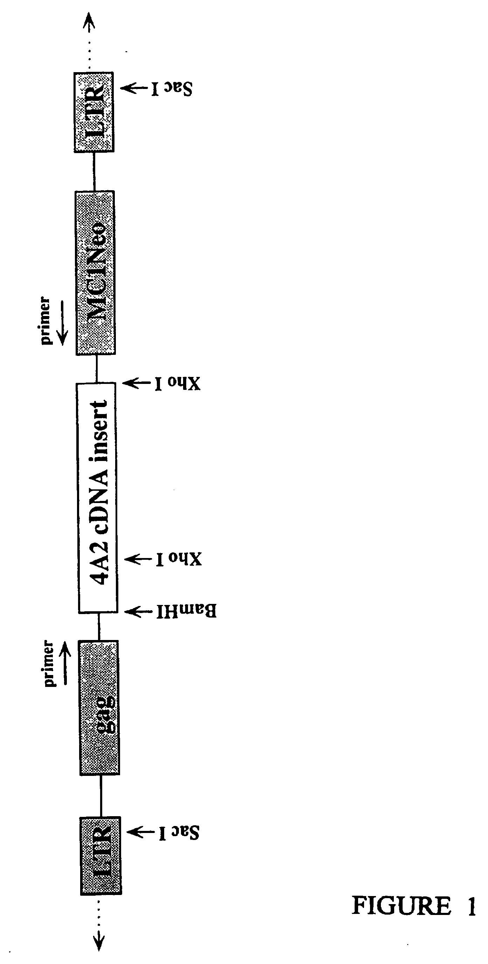

**Figure 1** is a diagrammatic representation showing generation of an IL-6-unresponsive M1 clone by retroviral infection. The RUFneo retrovirus, showing the position of landmark restriction endonuclease cleavage sites, the 4A2 cDNA insert and the position of PCR primer sequences.

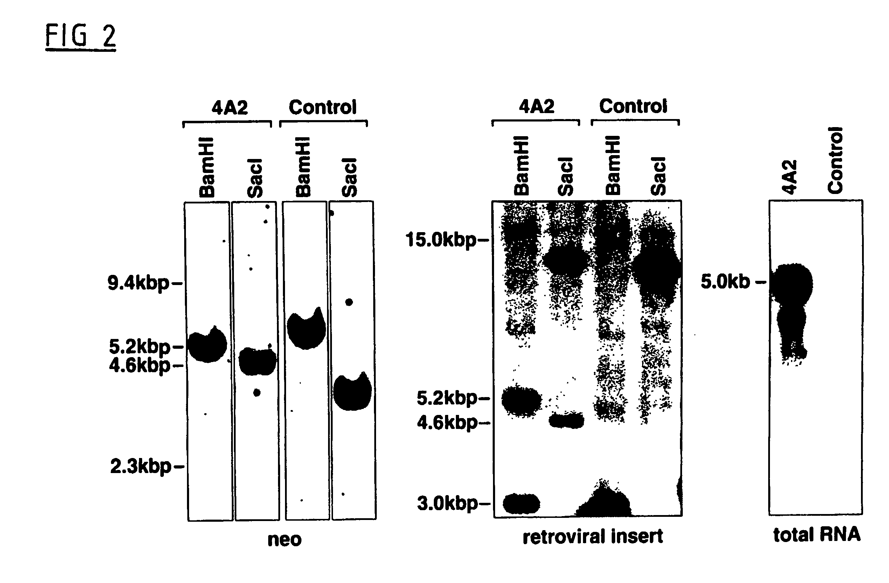

**Figure 2** is a photographic representation of Southern and Northern analysis. (Left and Middle Panels) Southern blot analysis of genomic DNA from clone 4A2 and a control infected M 1 clone. DNA was digested with BamH I, to reveal the number of retroviruses carried by each clone, and Sac I, to estimate the size of the retroviral cDNA insert. Left panel; probed with neo. Right panel; probed with the Xho 1-digested 4A2 PCR product. (Right Panel) . Northern blot analysis of total RNA from clone 4A2 and a control infected M 1 clone, probed with the Xho I-digested 4A2 PCR product. The two bands represent unspliced and spliced retroviral transcripts, resulting from splice donor and acceptor sites in the retroviral genome.

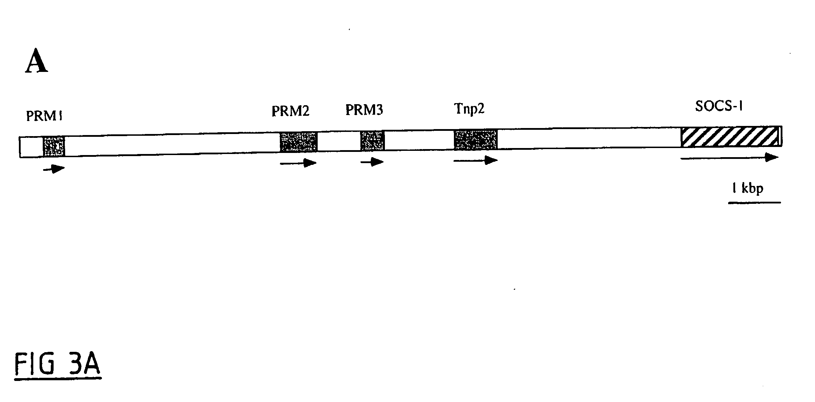

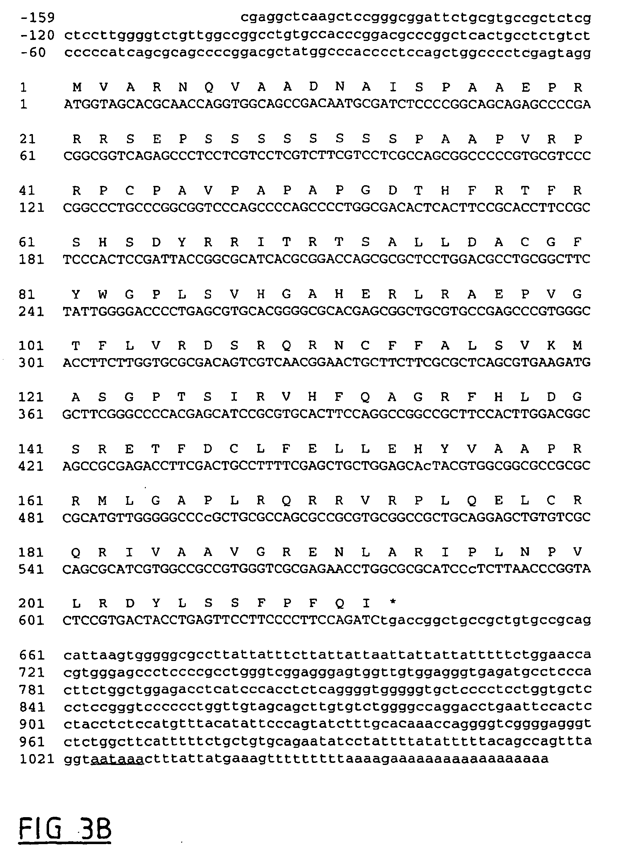

**Figure 3** is a representation of the nucleotide sequence and structure of the SOCS1 gene. A. The genomic context of SOCS1 in relation to the protamine gene cluster on murine chromosome 16. The accession number of this locus is MMPRMGNS (direct submission; G. Schlueter, 1995) for the mouse and BTPRMTNP2 for the rat (direct submission; G. Schlueter, 1996). B. The nucleotide sequence of the SOCS 1 cDNA and deduced amino acid sequence. Conventional one letter abbreviations are used for the amino acid sequence and the asterisk indicates the stop codon. The polyadenylation signal sequence is underlined. The coding region is shown in uppercase and the untranslated region is shown in lower case.

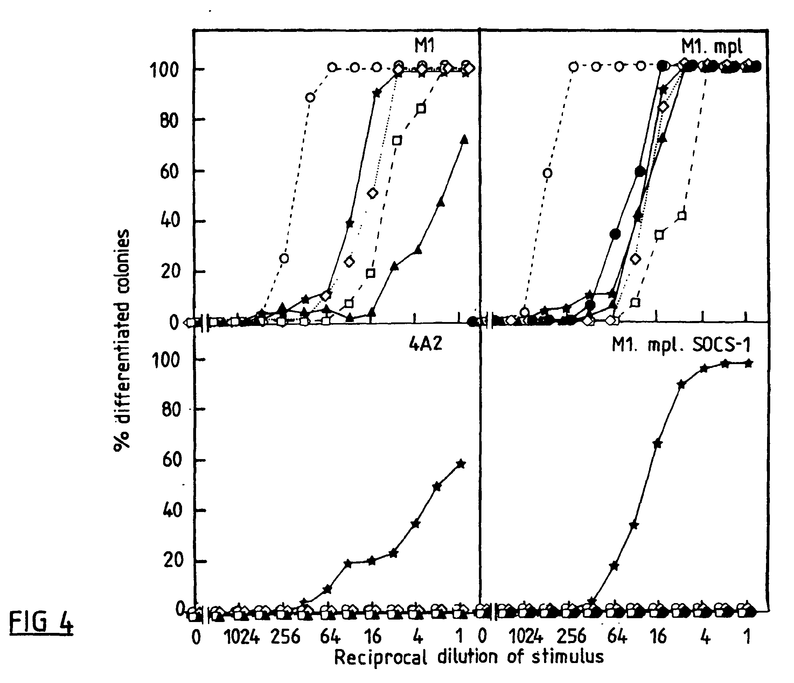

**Figure 4** is a graphical representation of cell differentiation in the presence of cytokines. Semi-solid agar cultures of parental M1 cells (M1 and M1.mpl) and M1 cells expressing SOCS 1 (4A2 and M1.mpl.SOCS1), were used and the percentage of colonies which differentiated in response to a titration of 1 mg/ml IL-6 (●), 100 ng/ml LIF (◇), 1 mg/ml OSM (□), 100 ng/ml IFN-γ (▲), 500 ng/ml TPO (●), or 3x10⁻⁶ M dexamethasone ( 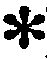 ) determined.

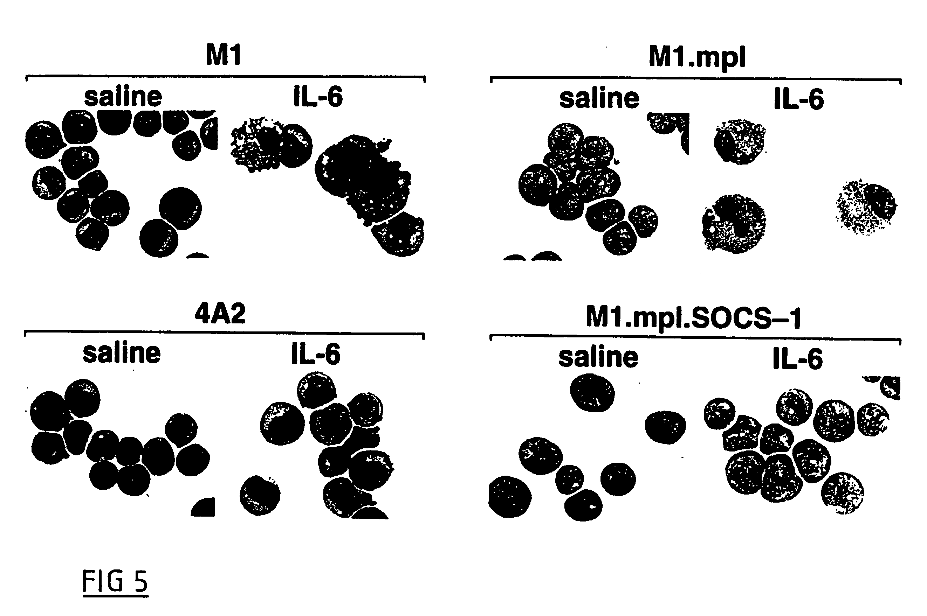

**Figure 5** is a photographic representation of cytospins of liquid cultures of parental M1 cells (M1 and M1.mpl)) and M1 cells expressing SOCS1 (4A2 and M1.mpl-SOCS1) cultured for 4 days in the presence of 10 ng/ml IL-6 or saline. Unlike parental M1 cells, morphological features consistent with macrophage differentiation are not observed in M1 cells constitutively expressing SOCS 1 (4A2 and M1.mpl.SOCS1) when cultured in IL-6.

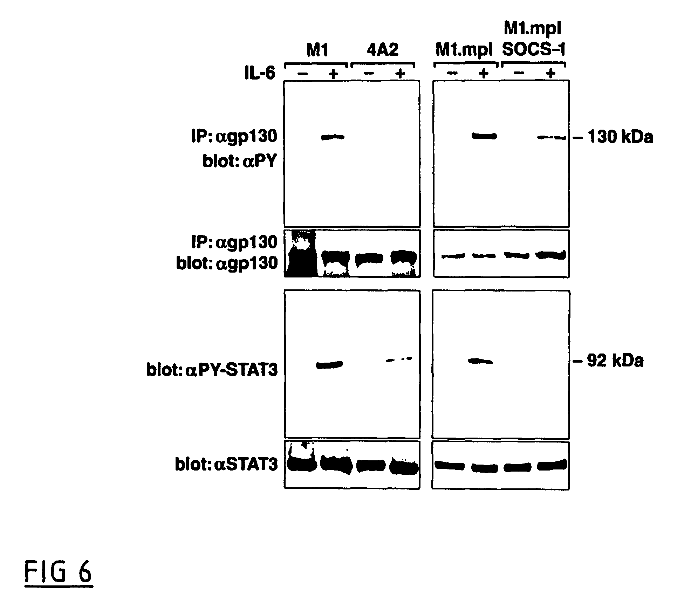

**Figure 6** is a photographic representation showing inhibition of phosphorylation of signalling molecules by SOCS1. Parental M1 cells (M1 and M1.mpl) and M1 cells expressing SOCS1 (4A2 and M1.mpLSOCS1) were incubated in the absence (-) or presence (+) of 10 ng/ml of IL-6 for 4 minutes at 37°C . Cells were then lysed and extracts were either immunopreciptated using anti-mouse gp130 antibody prior to SDS-PAGE (two upper panels) or were electrophoresed directly (two lower panels). Gels were blotted and the filters were then probed with anti-phosphotyrosine (upper panel), anti-gp130 antibody (second top panel), anti-phospho-STAT3 (second bottom panel) or anti-STAT3 (lower panel). Blots were visualised using peroxidase-conjugated secondary antibodies and Enhanced Chemiluminescence (ECL) reagents.

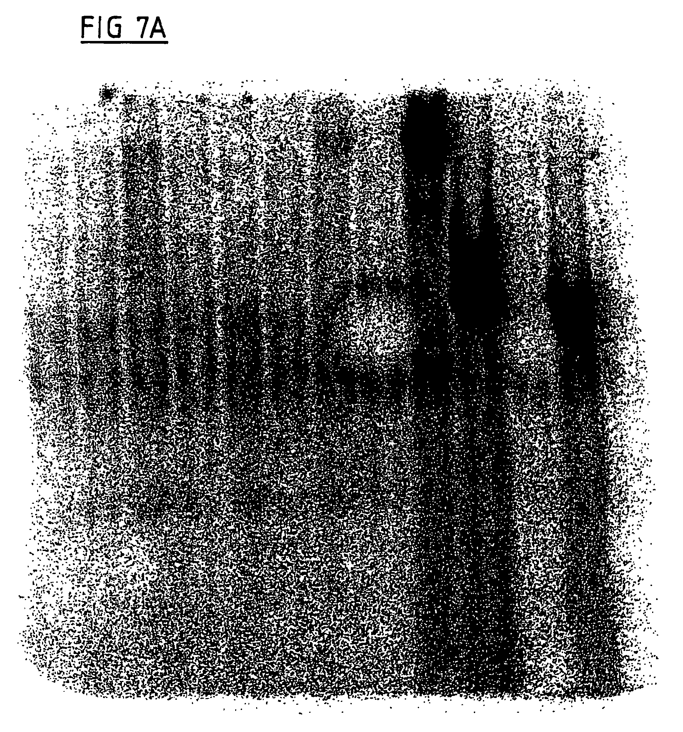

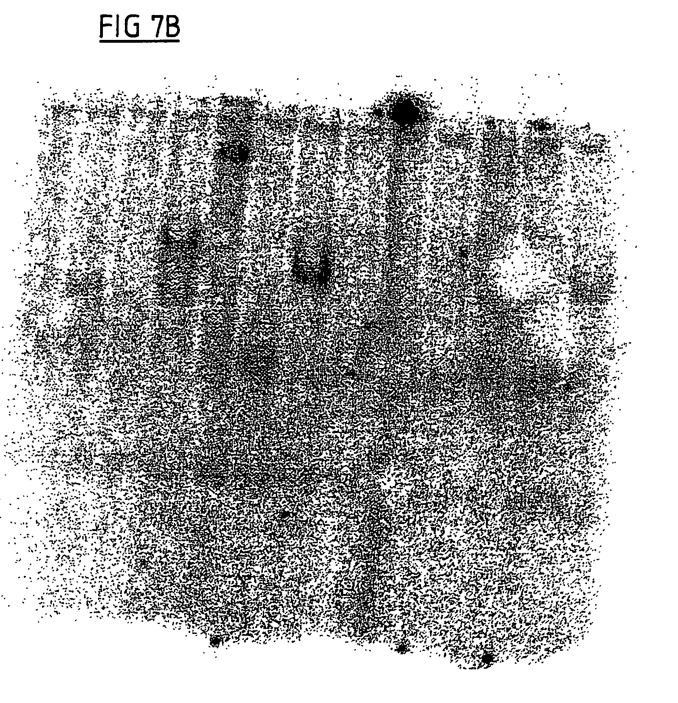

**Figure 7** is a representation of protein extracts prepared from (A) M1 cells or M1 cells expressing SOCS 1 (4A2) and (B) M1.mpl cells or M1.mpl.SOCSl cells incubated for 10 min at 37°C in 10 ml serum free DME containing either saline, 100 ng/ml IL-6 or 100 ng/ml IFN-γ. The binding reactions contained 4-6 µg protein (constant within a given experiment), 5 ng ³²P-labelled m67 oligonucleotide encoding the high affinity SIF (c*-sis-* inducible factor) binding site, and 800 ng sonicated salmon sperm DNA. For certain experiments, protein samples were preincubated with an excess of unlabelled m67 oligonucleotide, or antibodies specific for either STAT1 or STAT3.

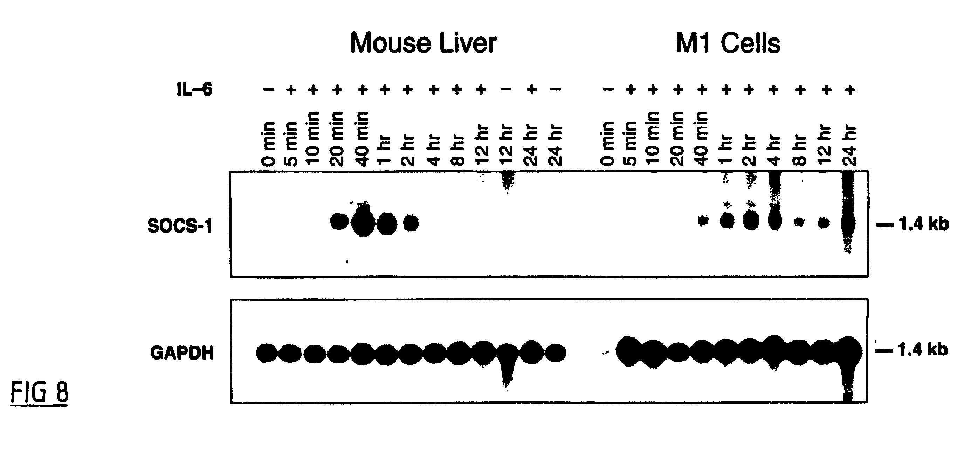

**Figure 8** is a photographic representation of Northern hybridisation. Mice were injected intravenously with 2 µg and after various periods of time, the livers were removed and polyA+ mRNA was purified. M1 cells were stimulated for various lengths of time with 500 ng/ml of IL-6, after which polyA+ mRNA was isolated. mRNA was fractionated by electrophoresis and immobilized on nylon filters. Northern blots were prehybridized, hybridized with random-primed ³²P-labelled SOCS 1 or GAPDH DNA fragments, washed and exposed to film overnight.

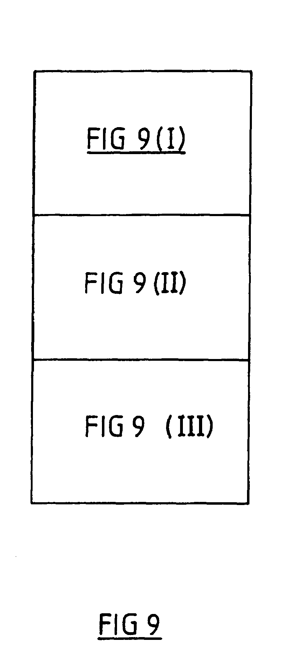

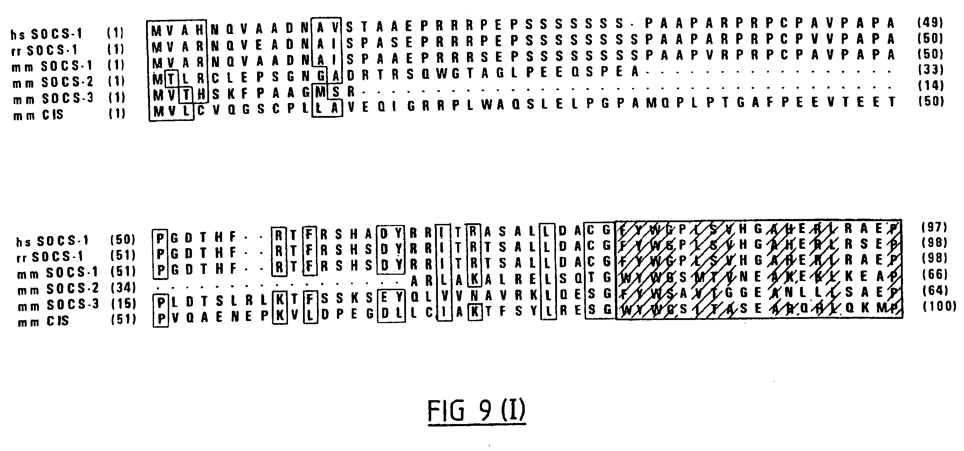

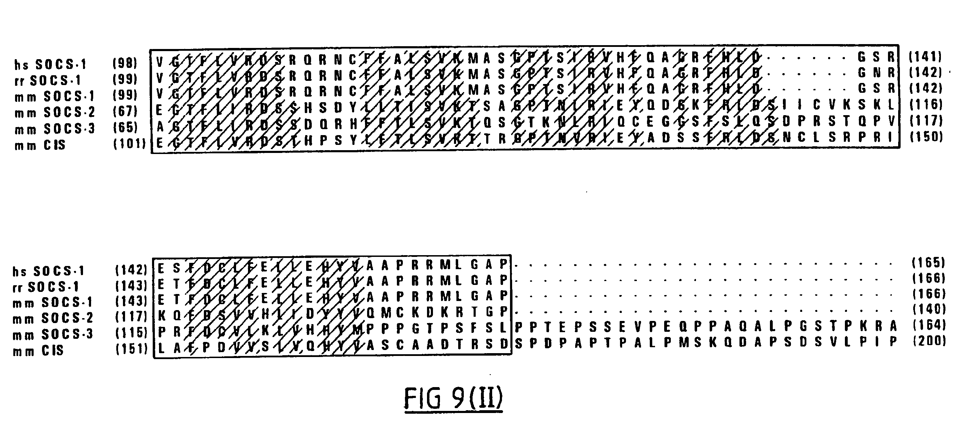

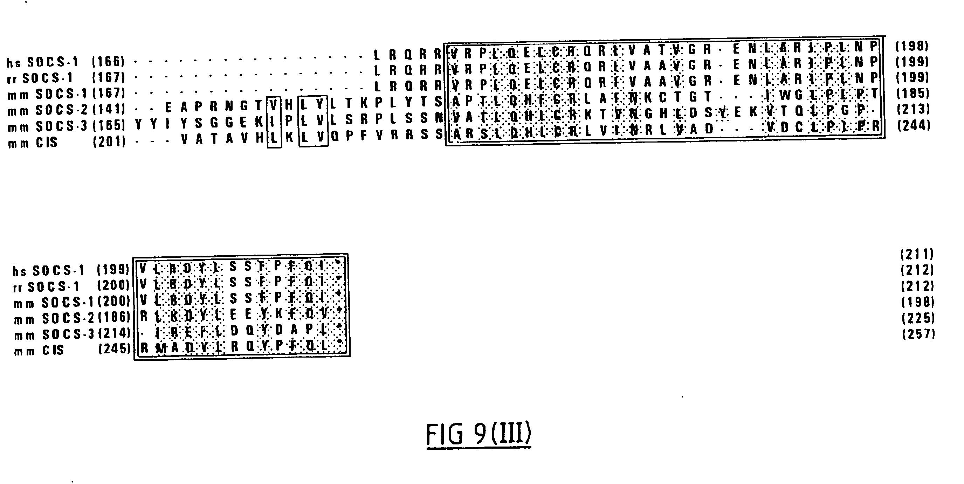

**Figure 9** is a representation of a comparison of the amino acid sequences of SOCS 1, SOCS2, SOCS3 and CIS. Alignment of the predicted amino acid sequence of mouse (mm), human (hs) and rat (rr) SOCS1, SOCS2, SOCS3 and CIS. Those residues shaded are conserved in three or four mouse SOCS family members. The SH2 domain is boxed in solid lines, while the SOCS box is bounded by double lines.

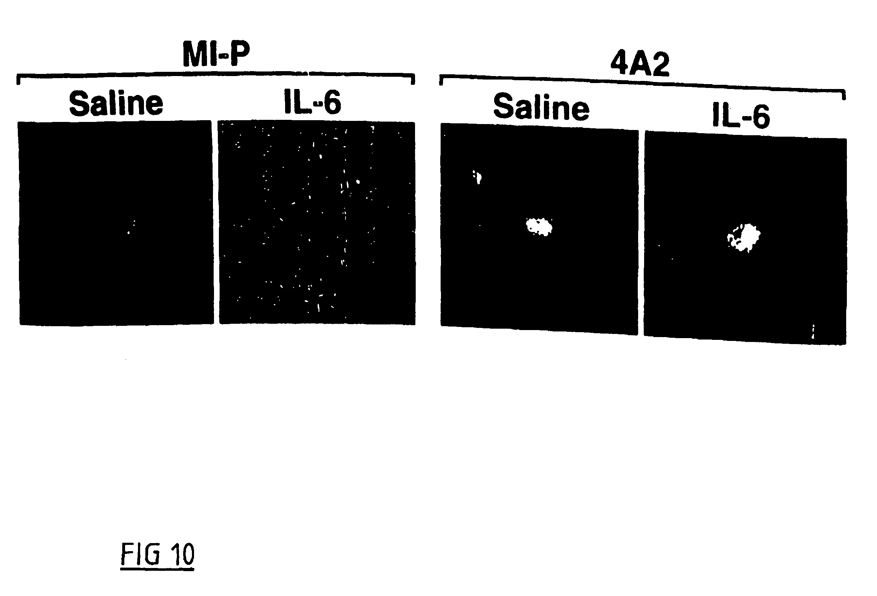

**Figure 10** is a photographic representation showing the phenotype of IL-6 unresponsive M 1 cell clone, 4A2. Colonies of parental M 1 cells (left panel) and clone 4A2 (right panel) cultured in semi-solid agar for 7 days in saline or 100 ng/ml IL-6.

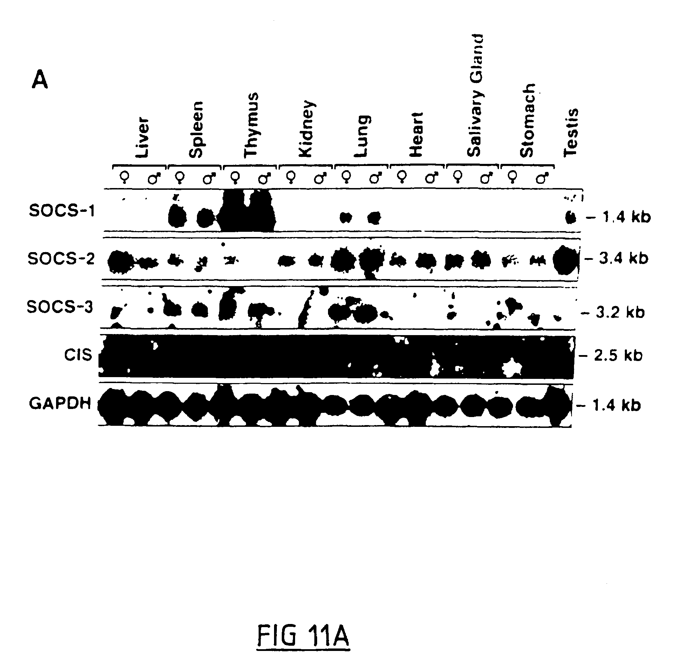

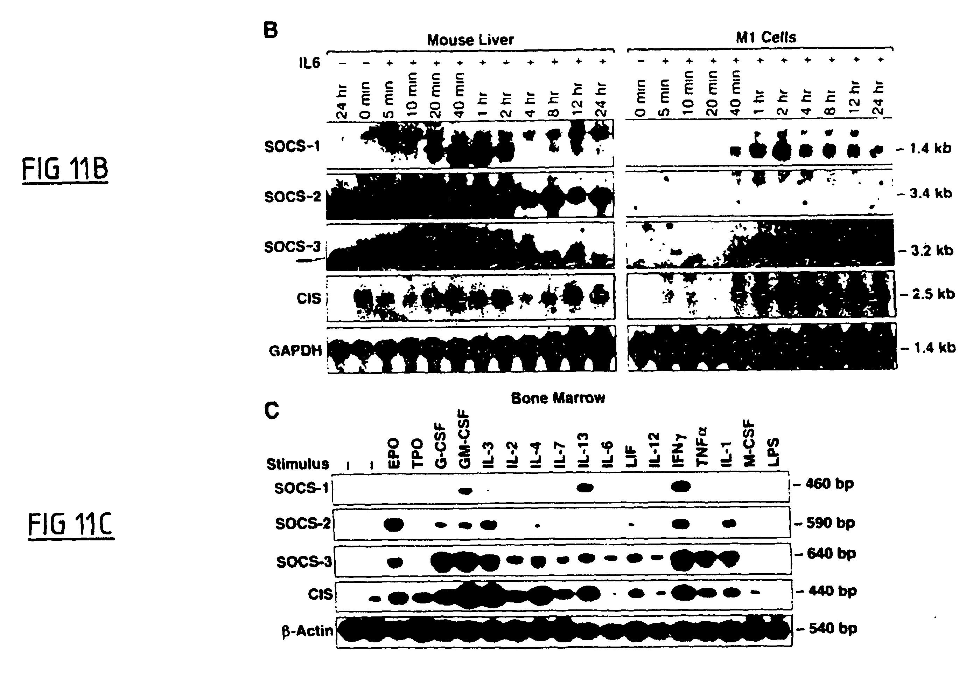

**Figure 11** is a photographic representation showing expression of mRNA for SOCS family members *in vitro* and *in vivo.*
   (A) Northern analysis of mRNA from a range of mouse organs showing constitutive expression of SOCS family members in a limited number of tissues.
   (B) Norther analysis of mRNA from liver and M1 cells showing induction of expression of SOCS family members following exposure to IL-6.
   (C) Reverse transcriptase PCR analysis of mRNA from bone marrow showing induction of expression of SOCS family members by a range of cytokines.

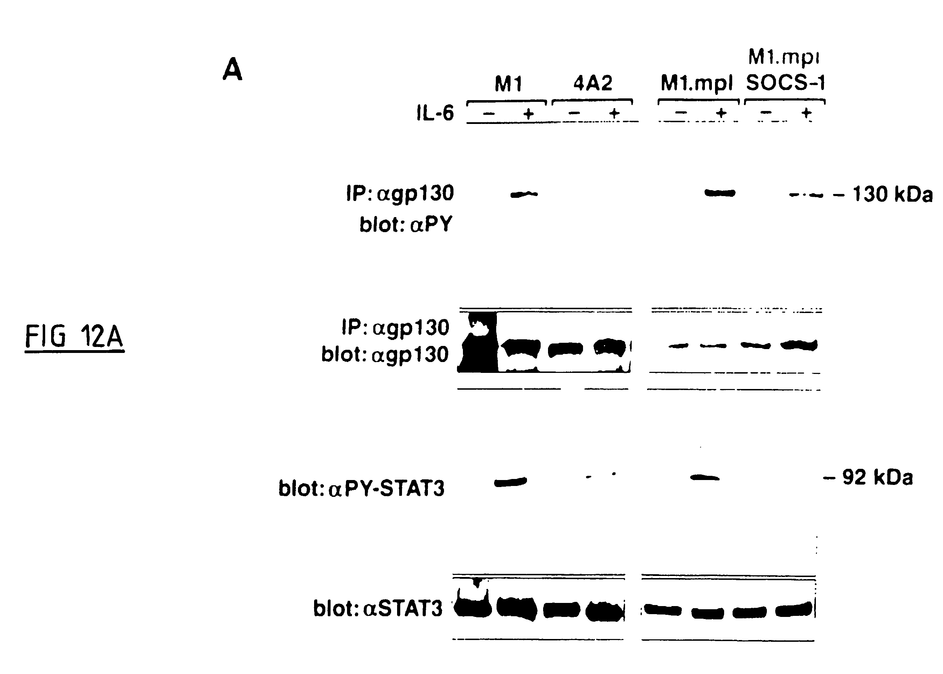

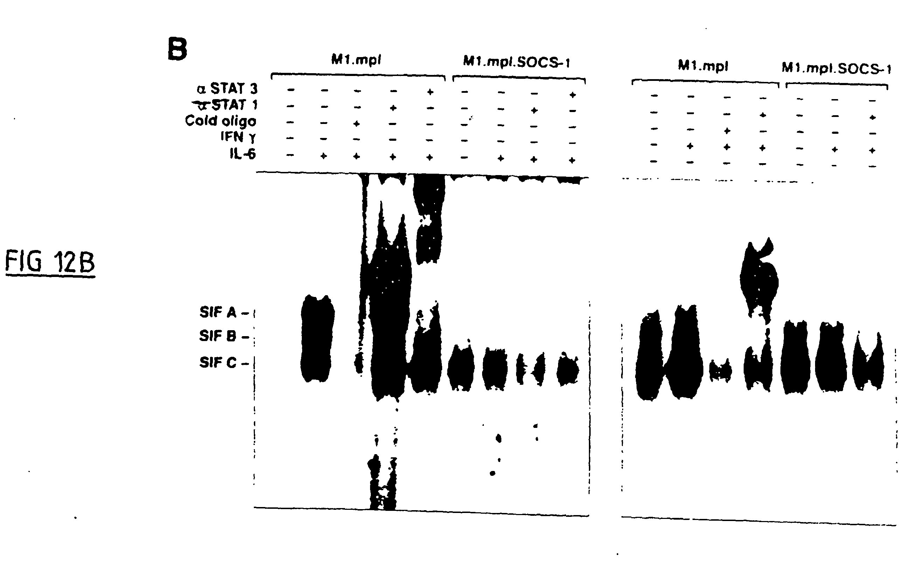

**Figure 12** is a photographic representation showing SOCS 1 suppresses the phosphorylation and activation of gp130 and STAT-3.
   (A) Western blots of extracts from parental M1 cells (M1 and M1.mpl) and M1 cells expressing SOCS1 (4A2 and M1.mpl.SOCS1) stimulated with (+) or without (-) 100 ng/ml IL-6. Top: Extracts immunoprecipitated with antu-gp130 (agp130) and immunoblotted with anti-phosphotyrosine (αPY-STAT3), or for STAT3 (αSTAT3) to demonstrate equal loading of protein. The molecular weights of the bands are shown on the right.
   (B) EMSA of M1.mpl and M1.mpl.SOCS1 cells stimulated with (+) and without (-) 100 ng/ml IL-6 or 100 ng/ml IFNγ. The DNA-binding complexes SIF A, B, and C are indicated at the left.

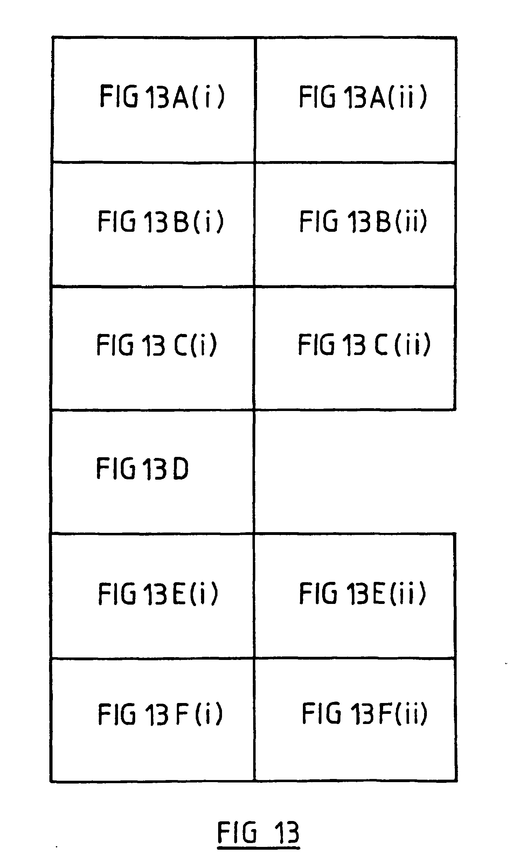

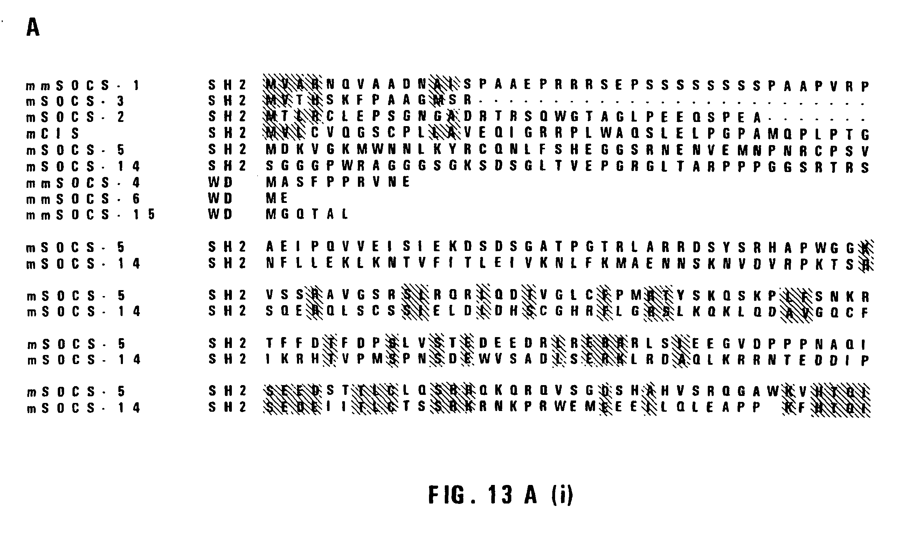

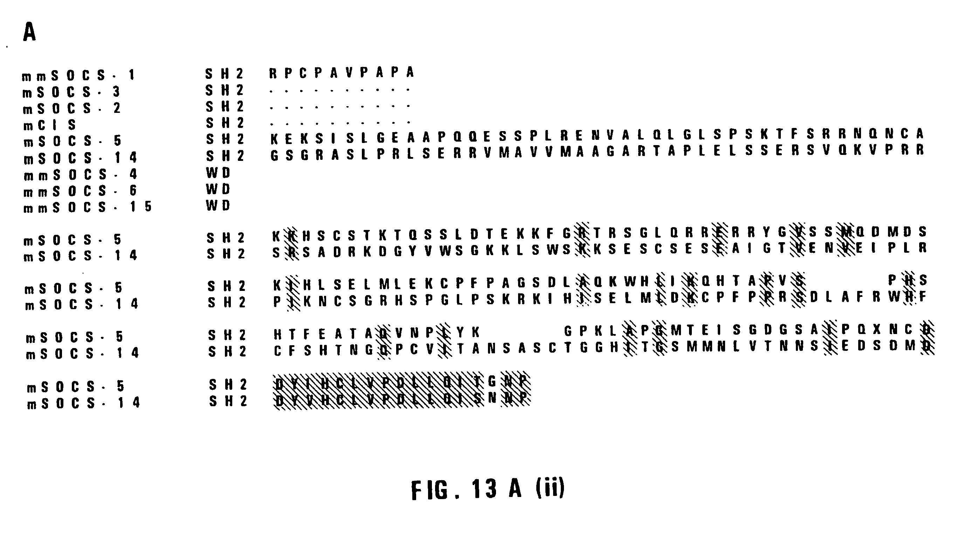

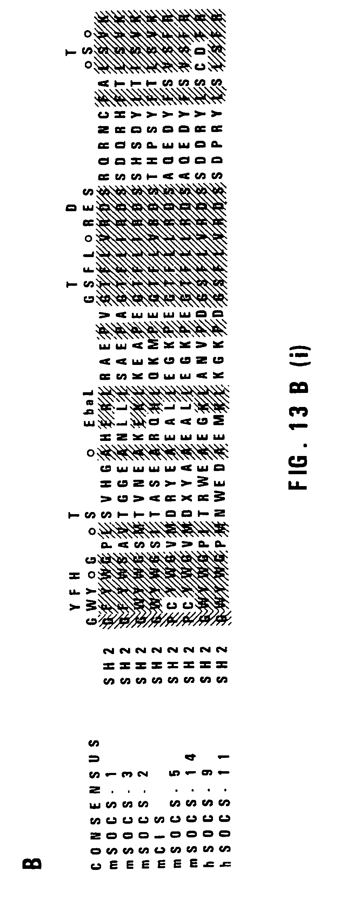

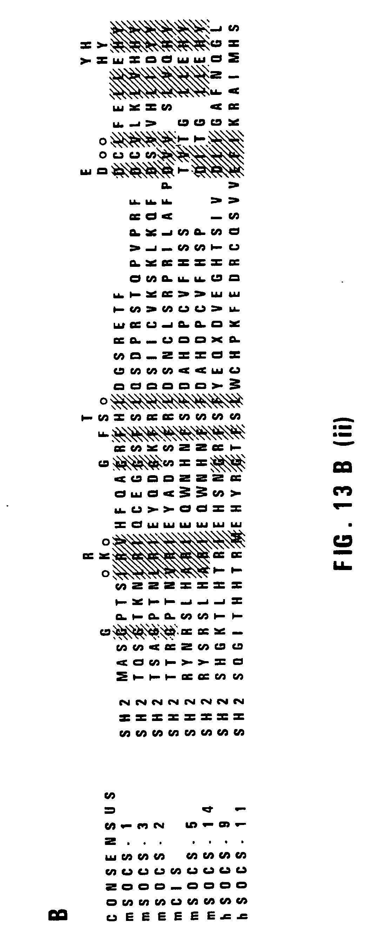

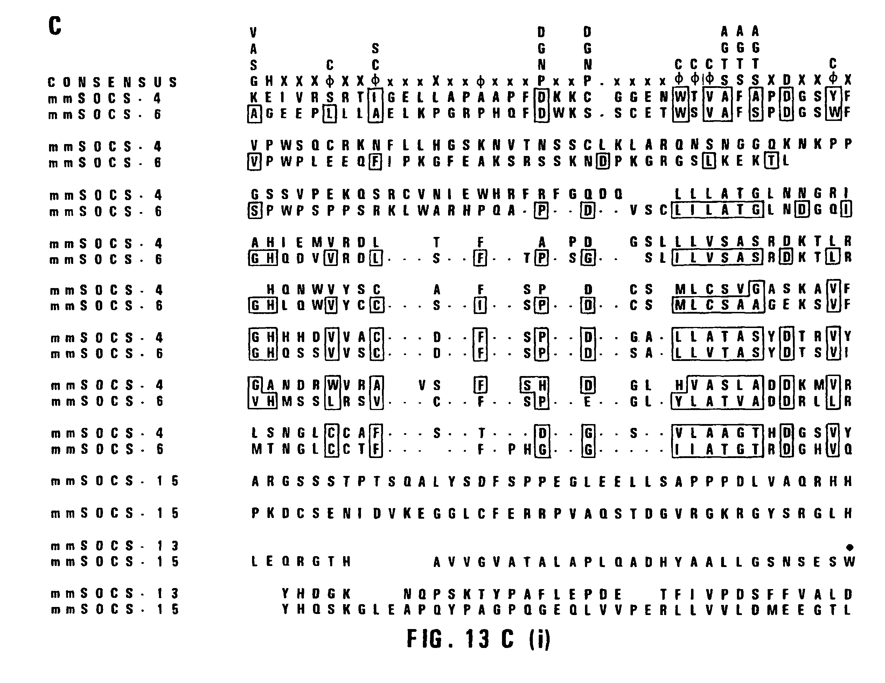

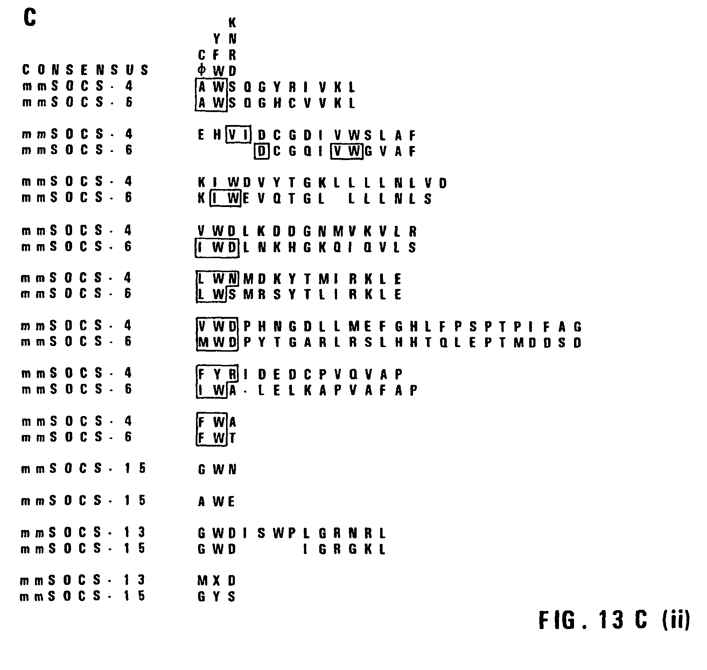

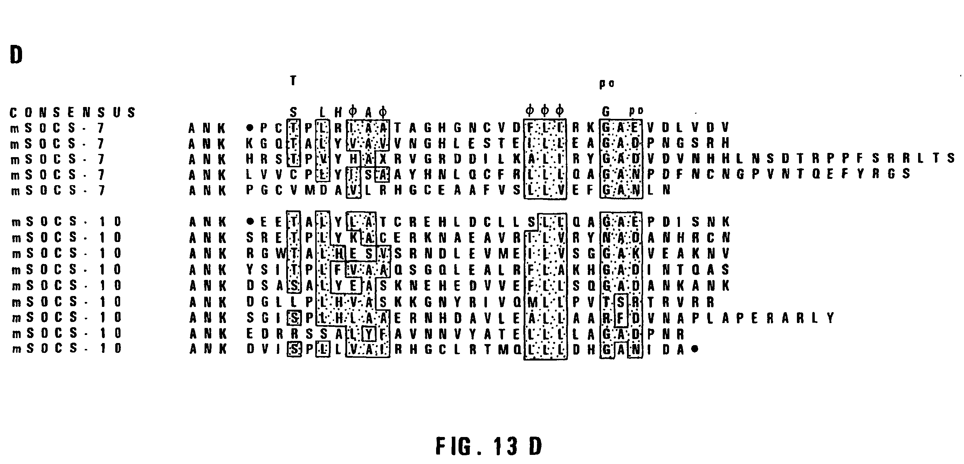

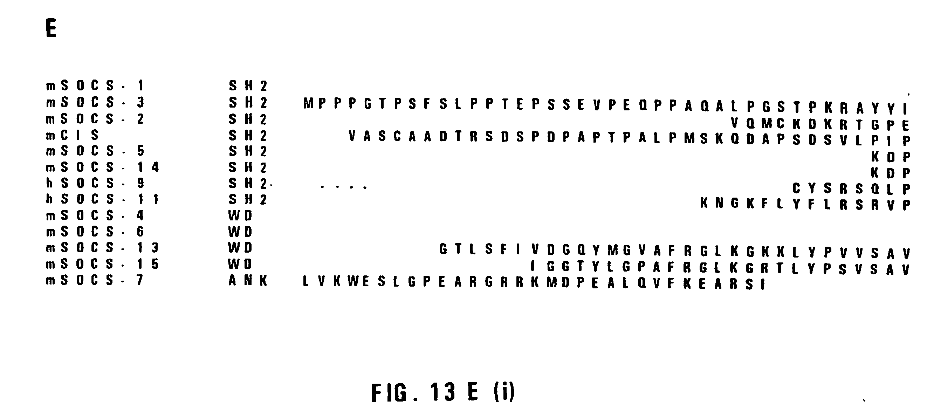

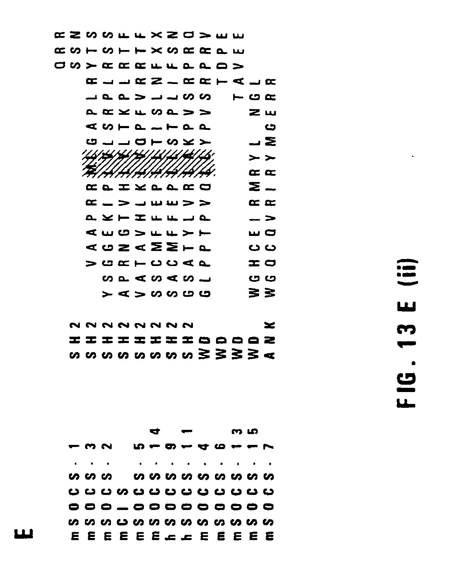

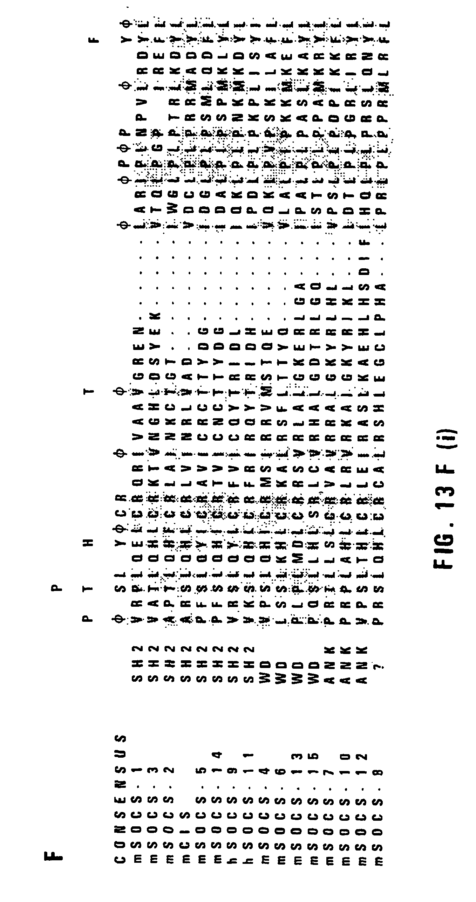

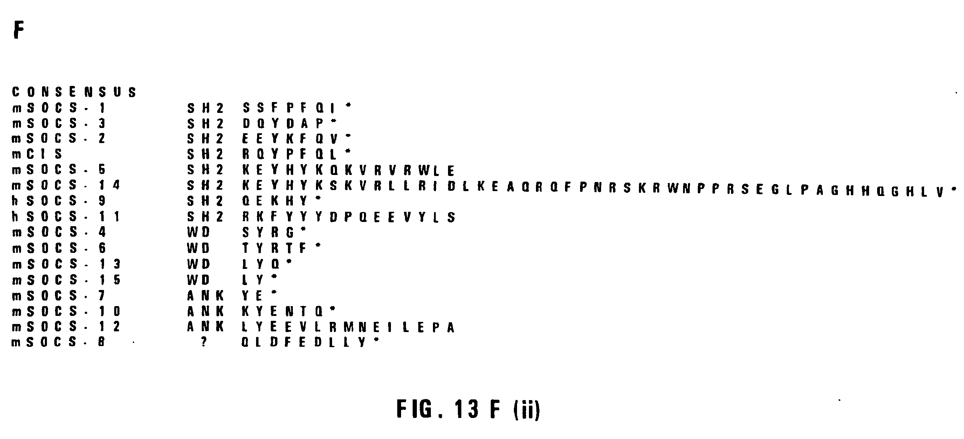

**Figure 13** is a representation of a comparison of the amino acid sequence of the SOCS proteins (A) Schematic representation of structures of SOCS proteins including proteins which contain WD-40 repeats (WSB) and ankyrin repeats (ASB). (B) Alignment of N-terminal regions of SOCS proteins. (C) Alignment of the SH2 domains of CIS, SOCS1, 2, 3, 5, 9, 11 and 14. (D) Alignment of the WD-40 repeats of SOCS4, SOCS6, SOCS13 and SOCS15. (E) Alignment of the ankyrin repeats of SOCS7 and SOCS10. (F) Alignment of the regions between SH2, WD-40 and ankyrin repeats and the SOCS box. (G) Alignment of the SOCS box. In each case the conventional one letter abbreviations for amino acids are used, with X denoting residues of uncertain identity and ○○○ denoting the beginning and the end of contigs. Amino acid sequence obtained from conceptual translation of nucleic acid sequence derived from isolated cDNAs is shown in upper case while amino acid sequence obtained by conceptual translation of ESTs is shown in lower case and is approximate only. Conserved residues, defined as (LIVMA), (FYW), (DE), (QN), (C, S, T), (KRH), (PG) are shaded in the SH2 domain, WD-40 repeats, ankyrin repeats and the SOCS box. For the alignment of SH2 domains, WD-40 repeats and ankyrin repeats a consensus sequence is shown above. In each case this has been derived from examination of a large and diverse set of domains (Neer *et al,* 1994; Bork, 1993).

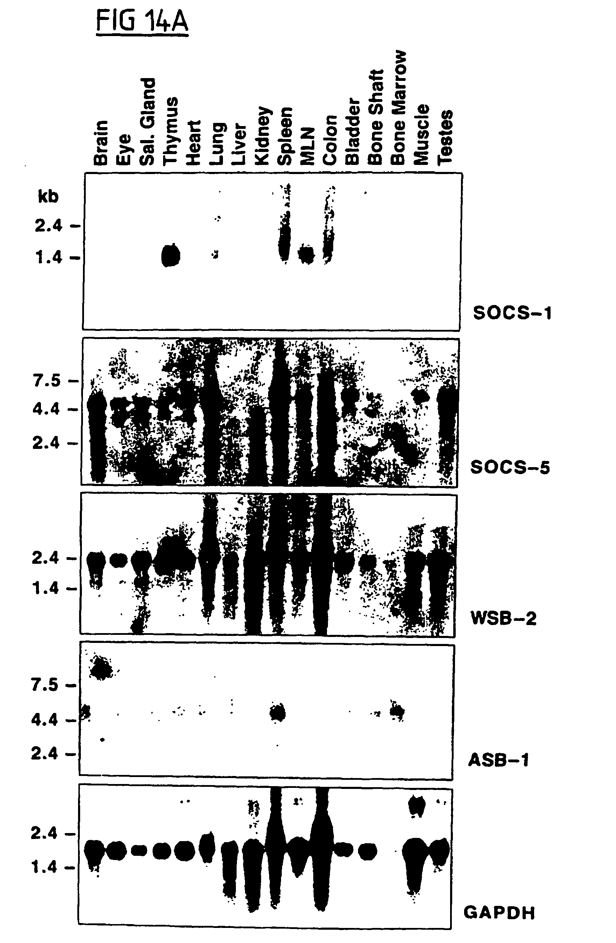

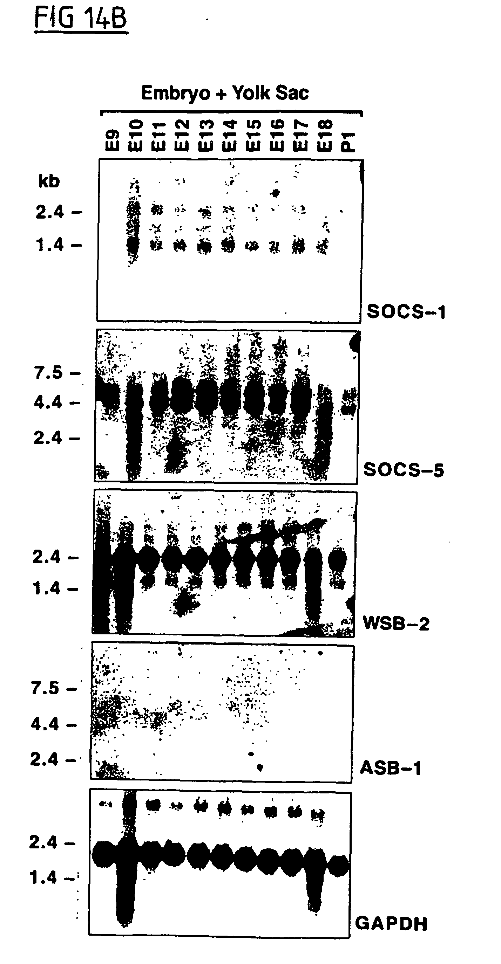

**Figures 14(A)** **and (B)** are photographic representations showing analysis of mRNA expression of mouse SOCS1 and SOCS5 and SOCS containing a WD-40 repeat (WSB2) and ankyrin repeats (ASB 1).

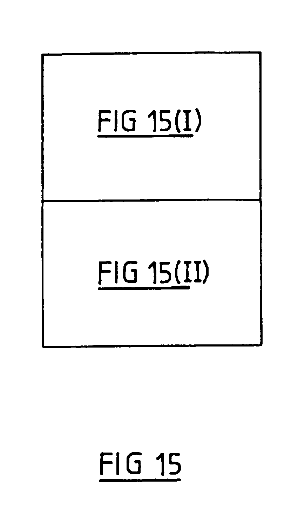

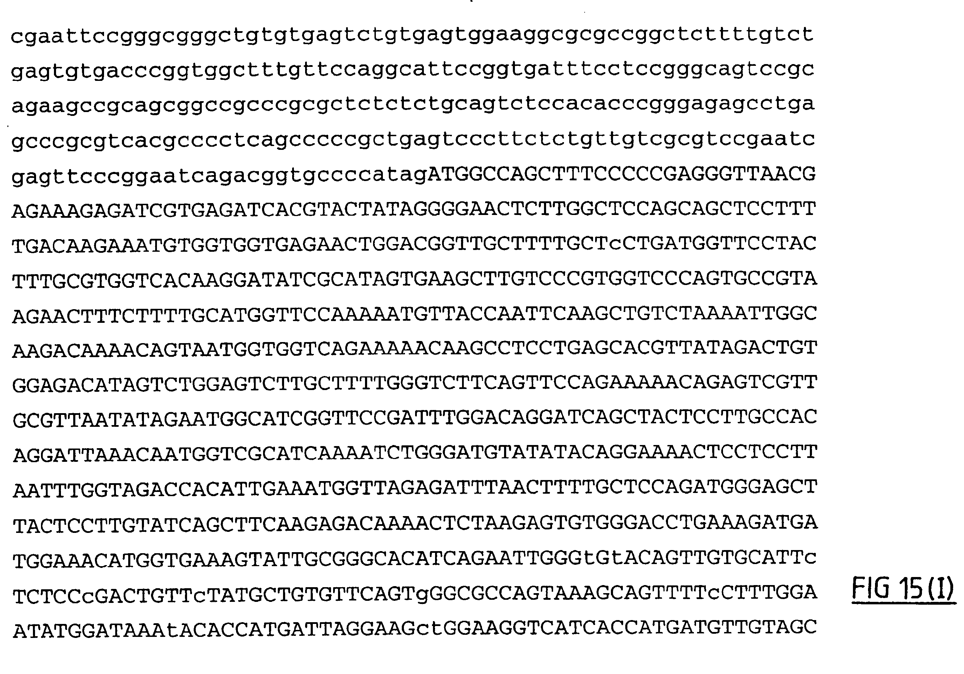

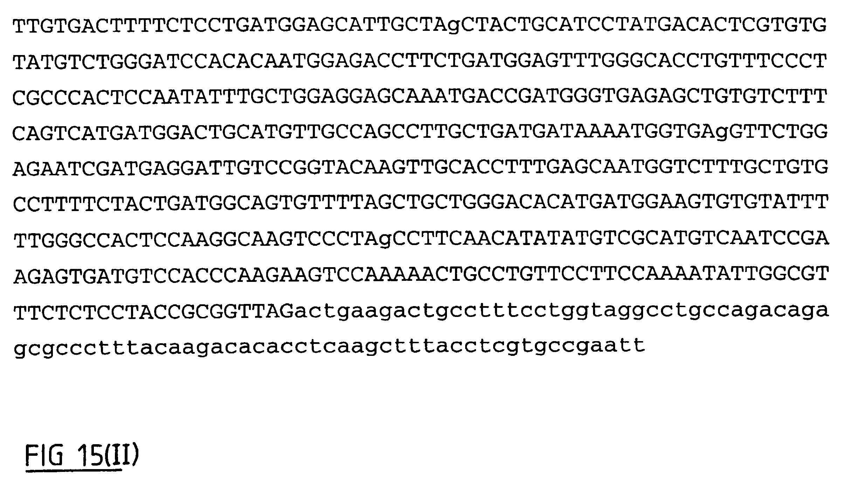

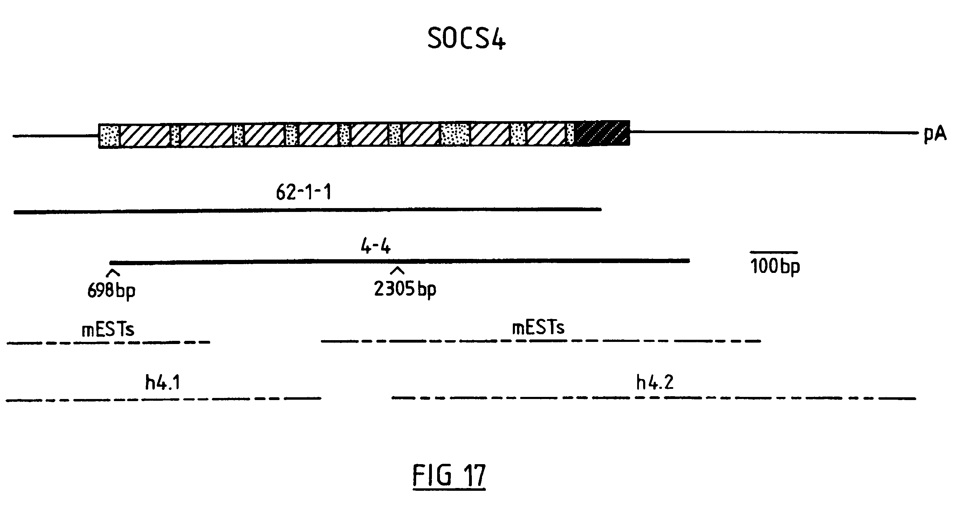

**Figure 15** is a representation showing the nucleotide sequence of the mouse SOCS4 cDNA. The nucleotides encoding the mature coding region from the predicted ATG "start" codon to the stop codon is shown in upper case, while the predicted 5' and 3' untranslated regions are shown in lower case. The relationship of mouse cDNA sequence to mouse and human EST contigs is illustrated in Figure 17.

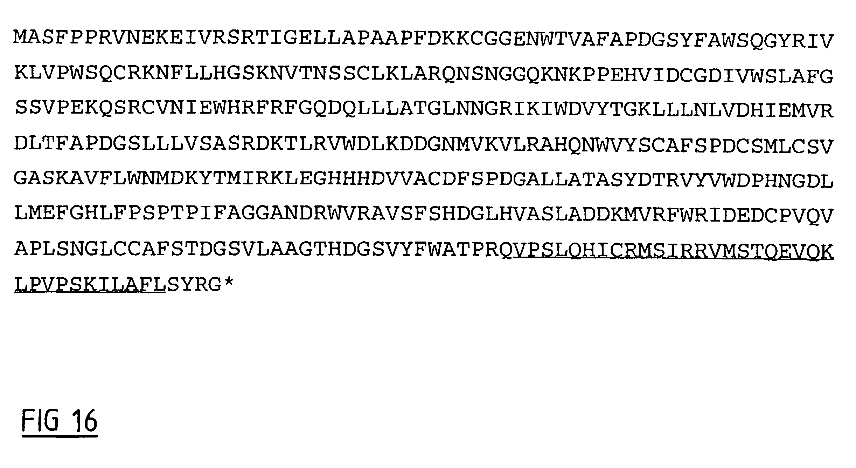

**Figure 16** is a representation showing the predicted amino acid sequence of the mouse SOCS4 protein, derived from the nucleotide sequence in Figure 15. The SOCS box, which also shown in Figure 13, is underlined.

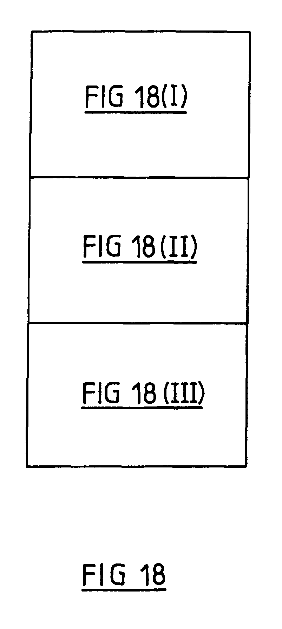

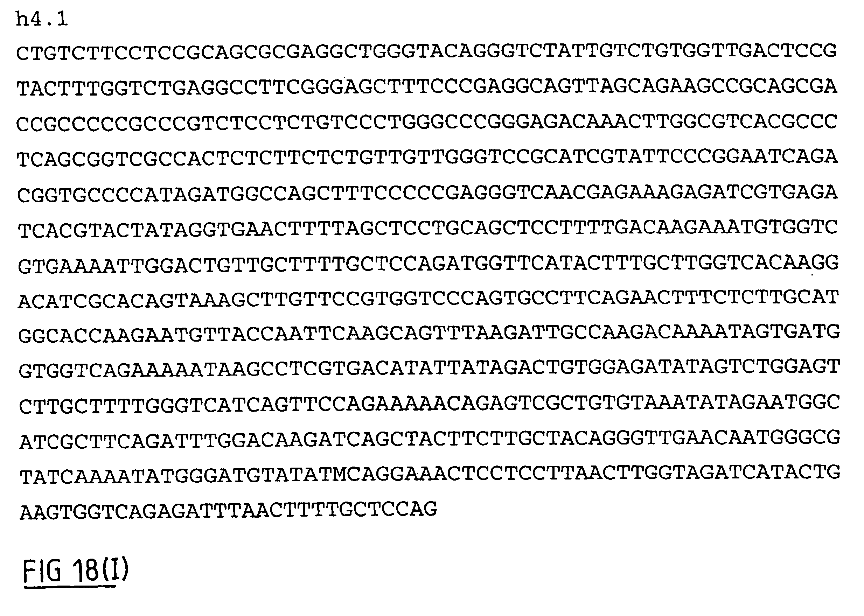

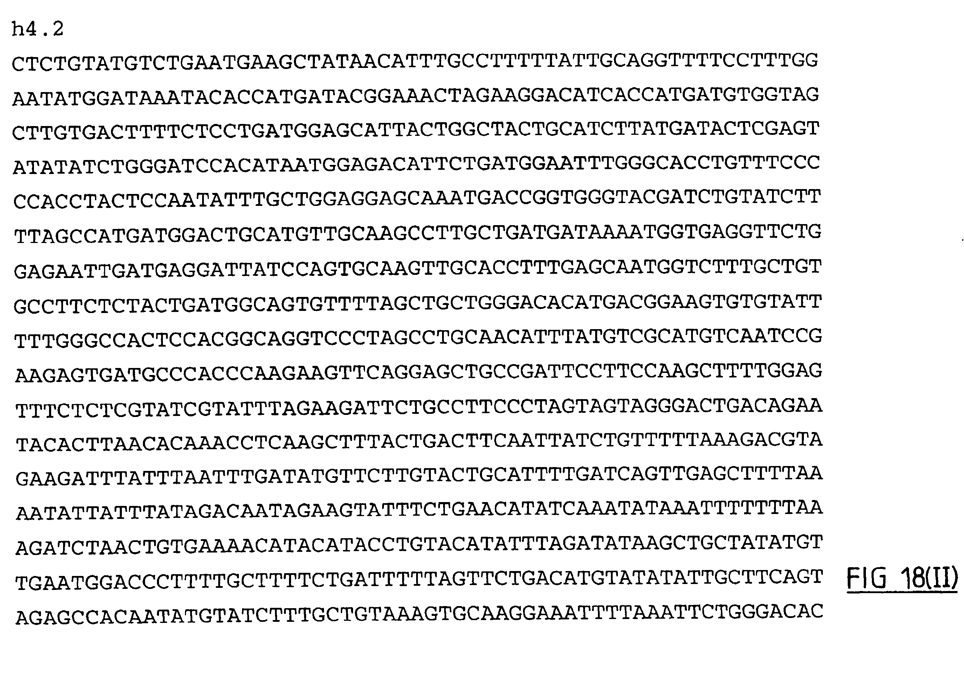

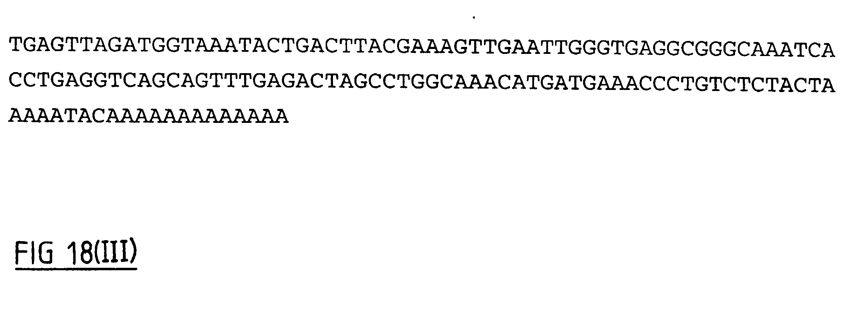

**Figure 18** is a representation showing the nucleotide sequence of human SOCS4 cDNA contigs h4.1 and h4.2, derived from analysis of ESTs listed in Table 4.1. The relationship of these contigs to the mouse cDNA sequence is illustrated in Figure 17.

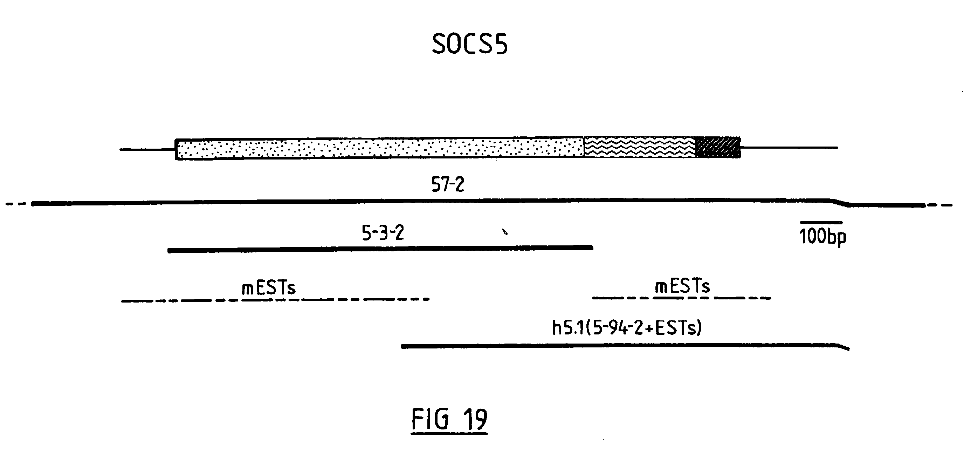

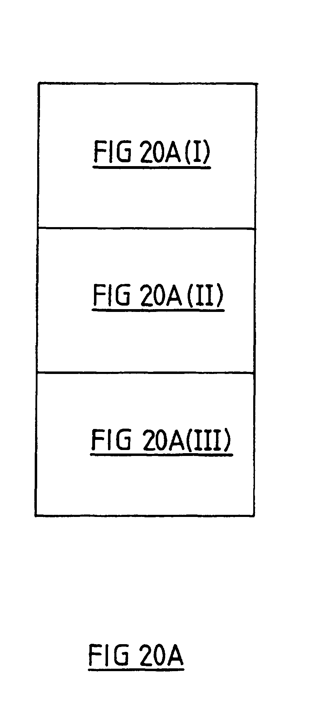

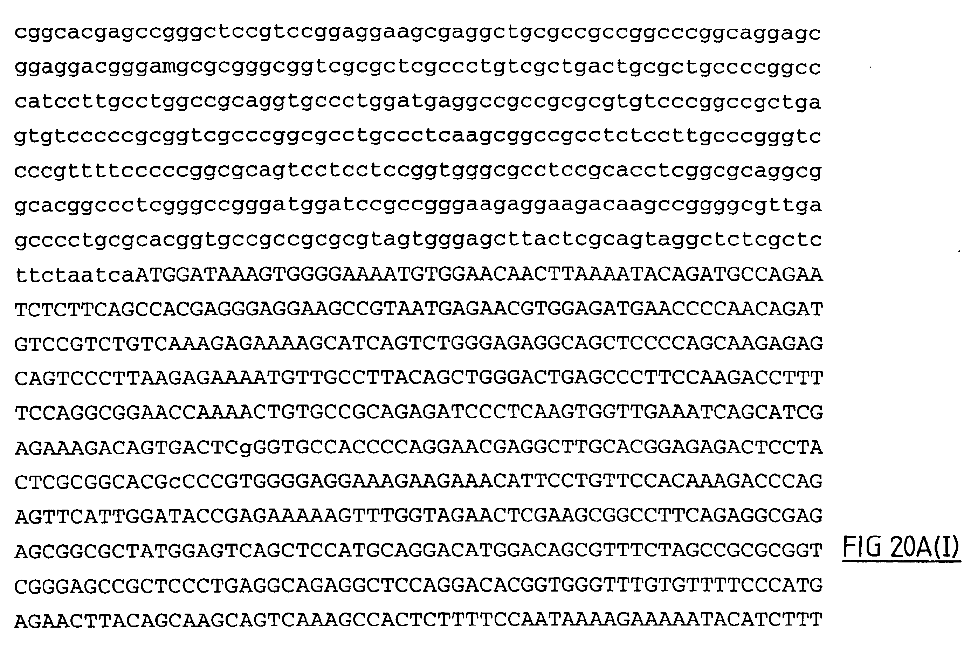

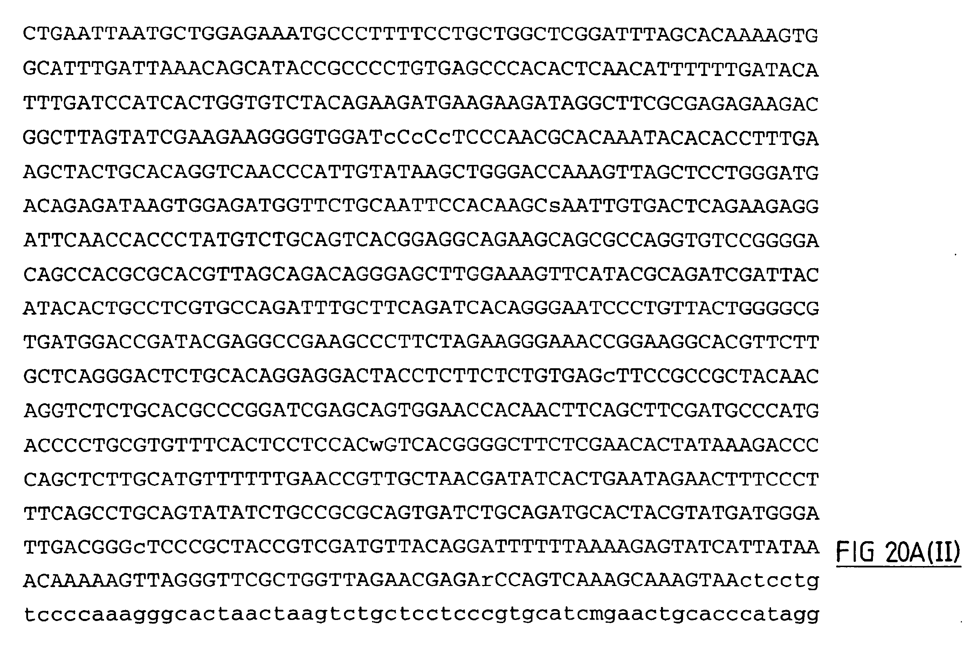

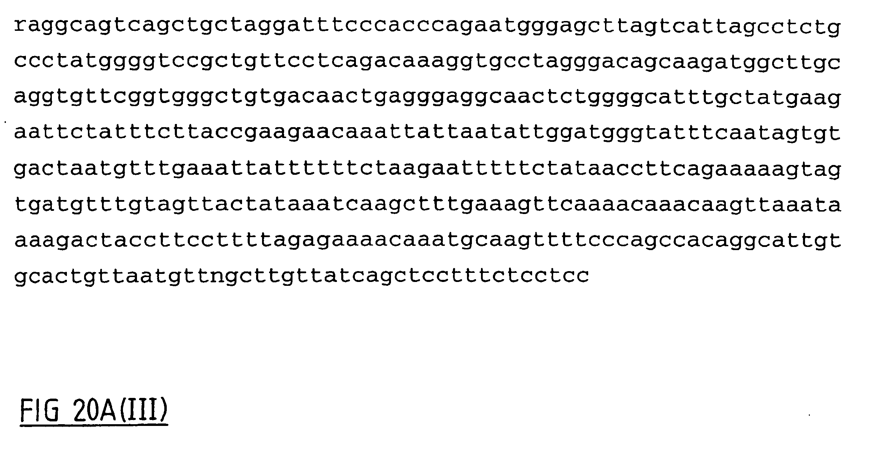

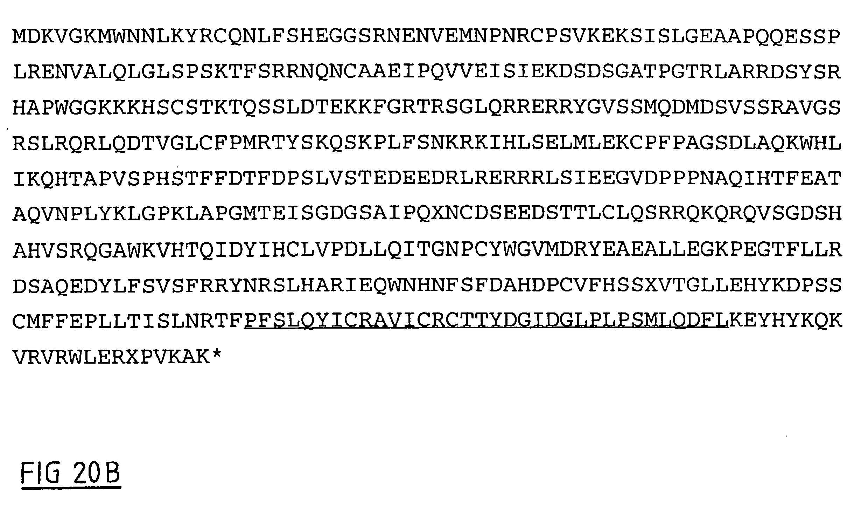

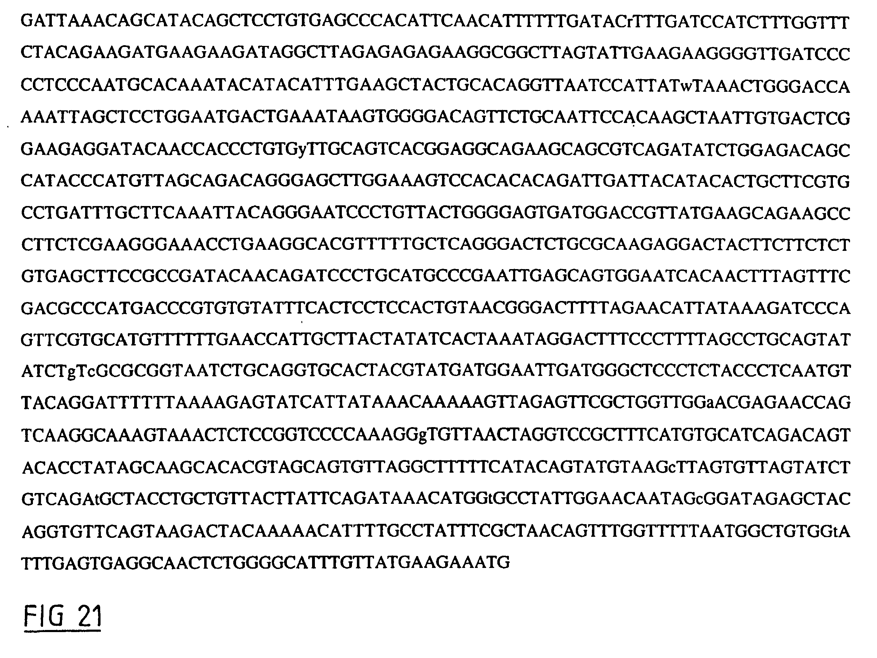

**Figure 19** is a diagrammatic representation showing the relationship of mouse SOCS5 genomic (57-2) and cDNA (5-3-2) clones to contigs derived from analysis of mouse ESTs (Table 5.1) and human cDNA clone (5-94-2) and ESTs (Table 5.2). The nucleotide sequence of the mouse SOCS5 contig is shown in Figure 20, with the sequence of human SOCS5 contig (h5.1) being shown in Figure 21. The deduced amino acid sequence of mouse SOCS5 is shown in Figure 20B. The structure of the protein is shown schematically, with the SH2 domain indicated by ( ) and the SOCS box by ( ). The putative 5' and 3' translated regions are shown by the thin solid line.
**Figure 20A** is a representation showing the nucleotide sequence of the mouse SOCS5 derived from analysis of genomic and cDNA clones. The nucleotides encoding the mature coding region from the predicted ATG "start" codon to the stop codon is shown in upper case, while the predicted 5' and 3' untranslated regions are shown in lower case. The relationship of mouse cDNA sequence to mouse and human EST contigs is illustrated in Figure 19.
**Figure 20B** is a representation of the predicted amino acid sequence of mouse SOCS5 protein, derived from the nucleotide sequence in Figure 20A. The SOCS box, which also shown in Figure 13 is underlined.
**Figure 21** is a representation showing the nucleotide sequence of human SOCS5 cDNA contig h5.1, derived from analysis of cDNA clone 5-94-2 and the ESTs listed in Table 5.2. The relationship of these contigs to the mouse cDNA sequence is illustrated in Figure 19.

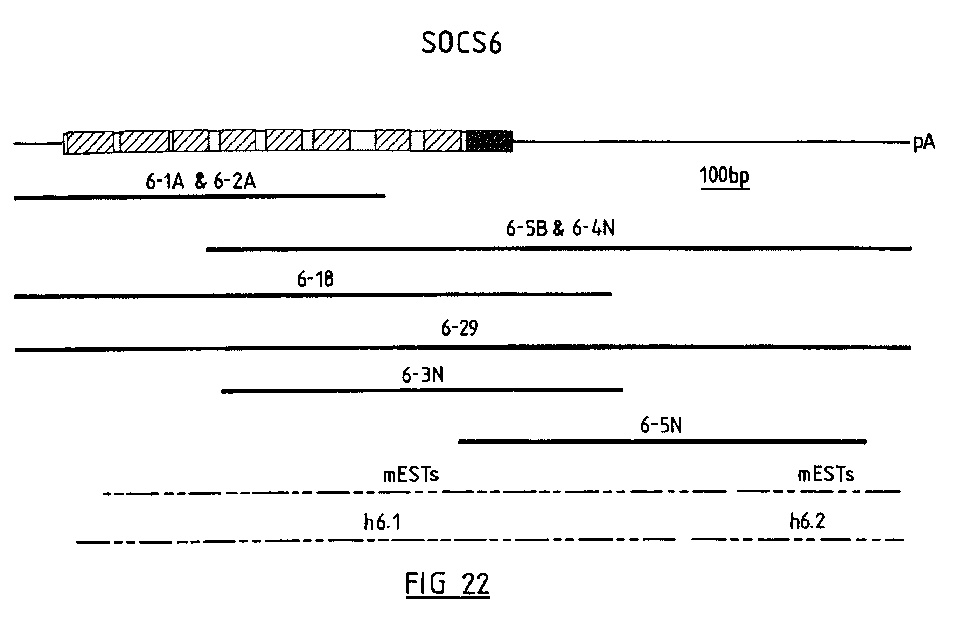

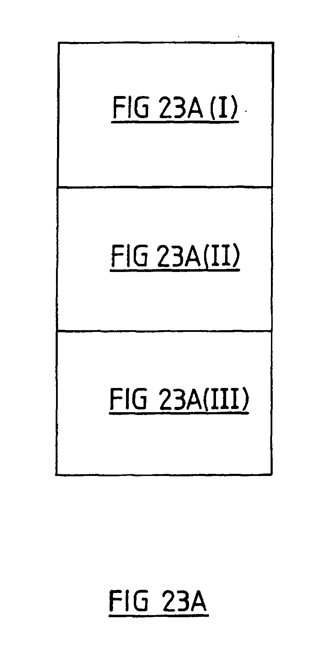

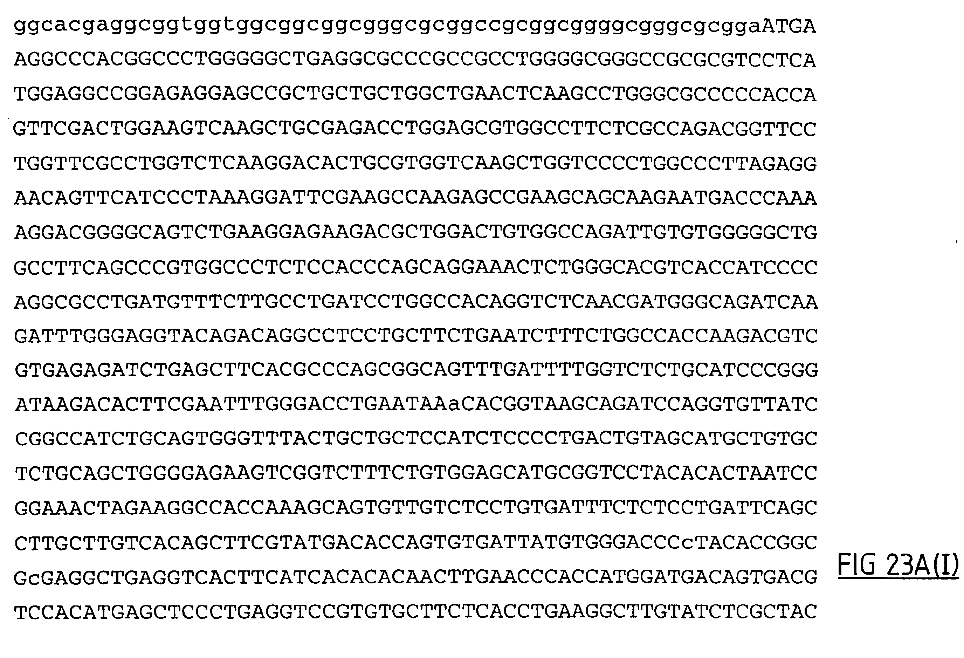

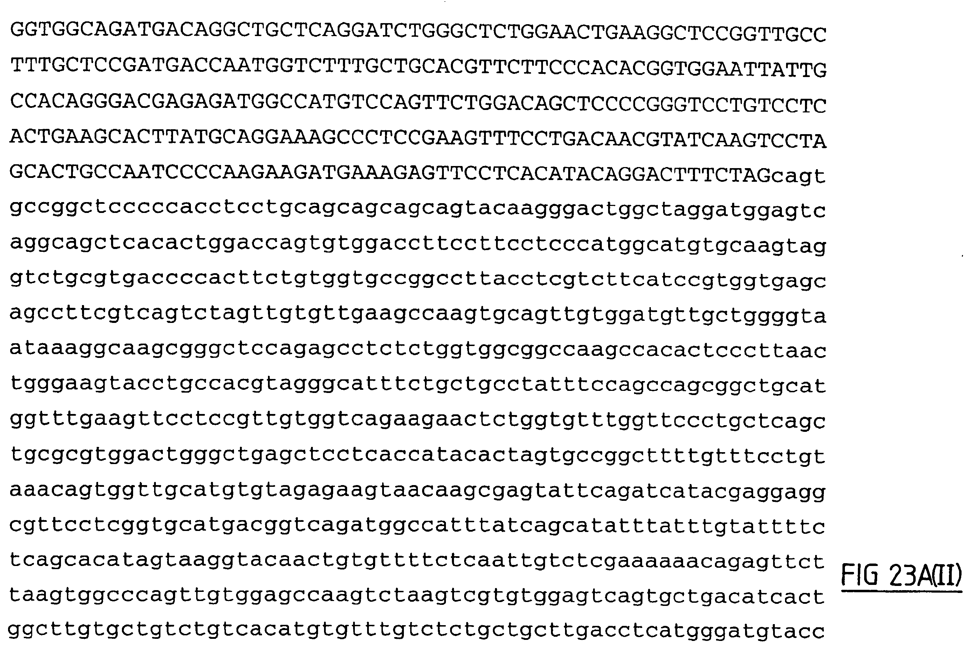

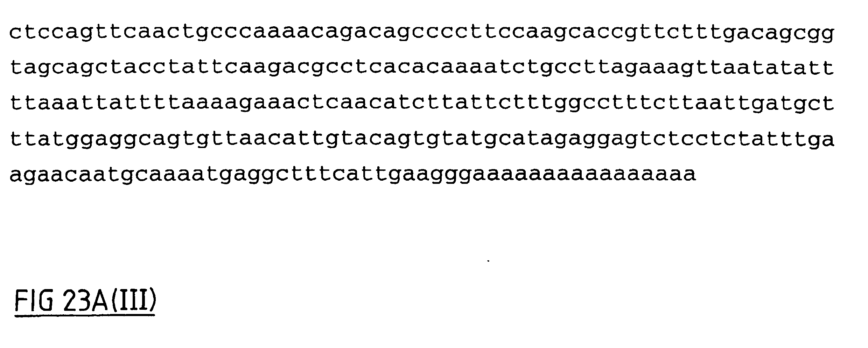

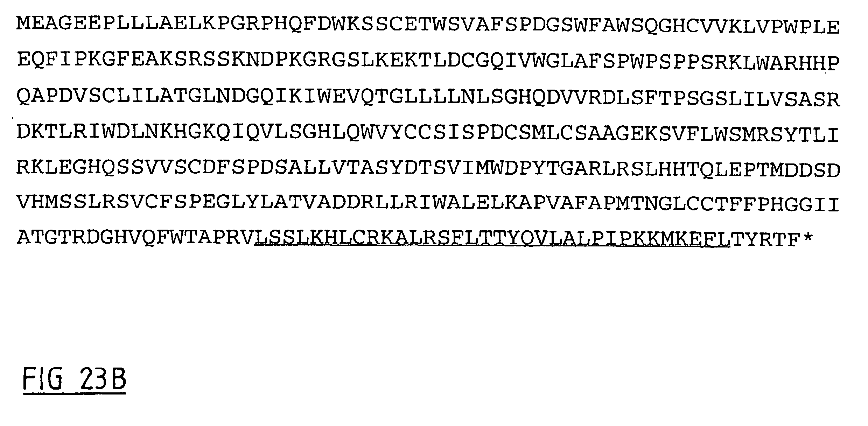

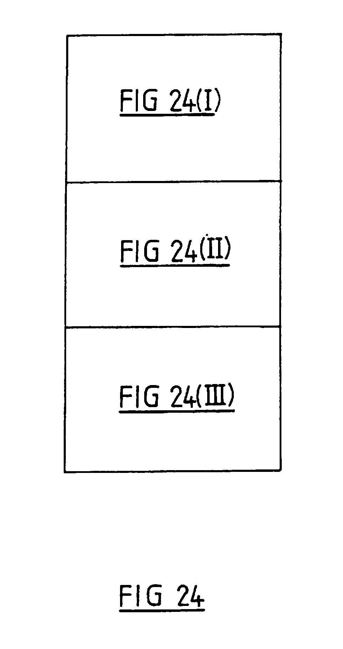

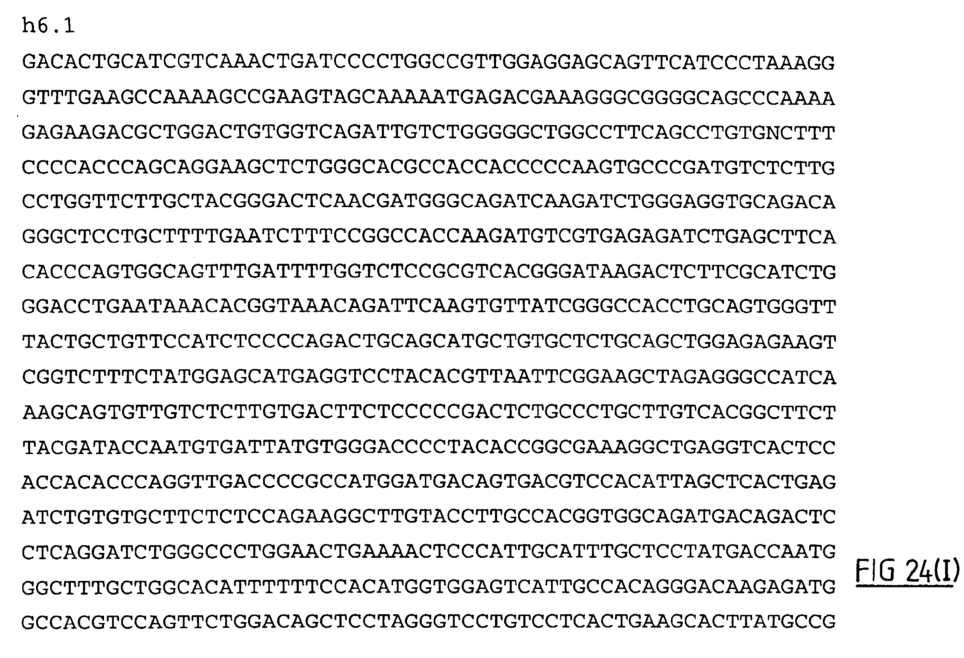

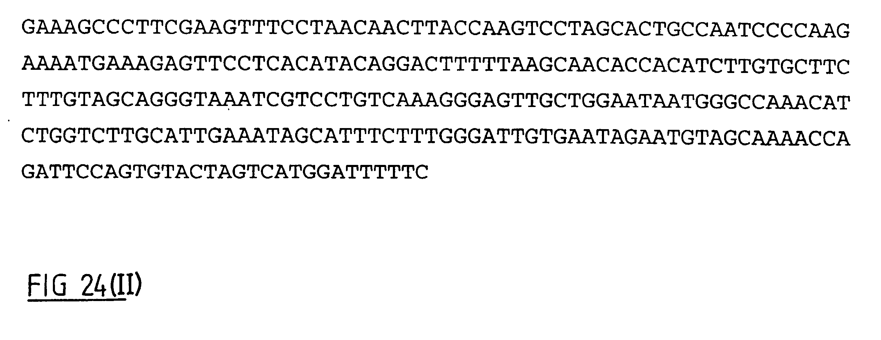

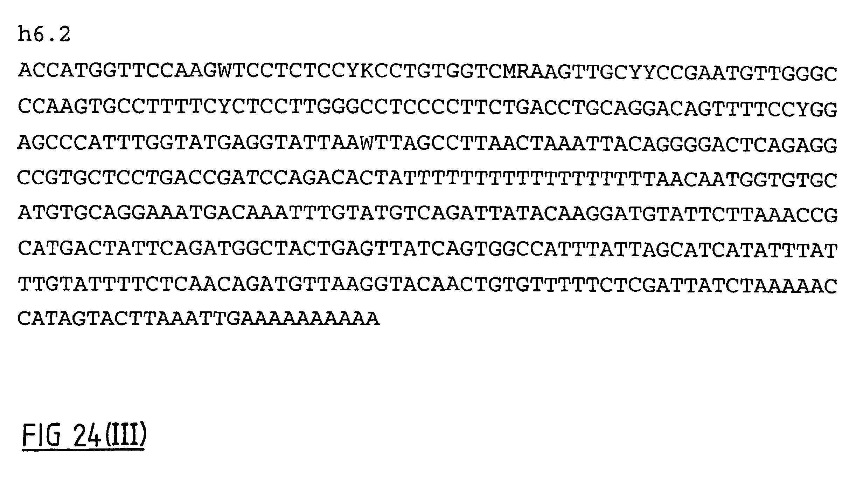

**Figure 22** is a diagrammatic representation showing the relationship of mouse SOCS6 cDNA clones (6-1A, 6-2A, 6-5B, 6-4N, 6-18, 6-29, 6-3N and 6-5N) to contigs derived from analysis of mouse ESTs (Table 6.1) and human ESTs (Table 6.2). The nucleotide sequence of the mouse SOCS-6 contig is shown in Figure 23, with the sequence of human SOCS6 contigs (h6.1 and h6.2) being shown in Figure 24. The deduced amino acid sequence of mouse SOCS6 is shown in Figure 23B. The structure of the protein is shown schematically, while the WD-40 repeats indicated by ( ) and the SOCS box by ( ). The putative S' and 3' untranslated regions are shown by the thin solid line.
**Figure 23A** is a representation showing the nucleotide sequence of the mouse SOCS6 derived from analysis of cDNA clone 64-10A-11. The nucleotides encoding the part of the predicted coding region, ending in the stop codon are shown in upper case, while the predicted 3' untranslated regions are shown in lower case. The relationship of mouse cDNA sequence to mouse and human EST contigs is illustrated in Figure 22.
**Figure 23B** is a representation showing the predicted amino acid sequence of mouse SOCS6 protein, derived from the nucleotide sequence in Figure 23A. The SOCS box, which also shown in Figure 13 is underlined.
**Figure 24** is a representation showing the nucleotide sequence of human SOCS6 cDNA contig h6.1, derived from analysis of cDNA clone 5-94-2 and the ESTs listed in Table 6.2. The relationship of these contigs to the mouse cDNA sequence is illustrated in Figure 22

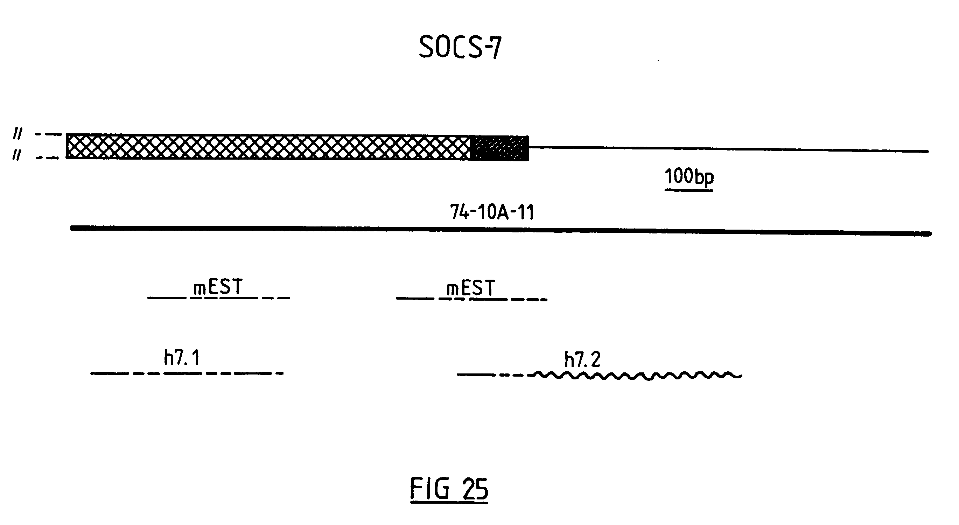

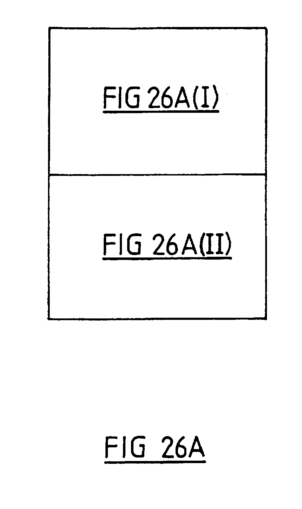

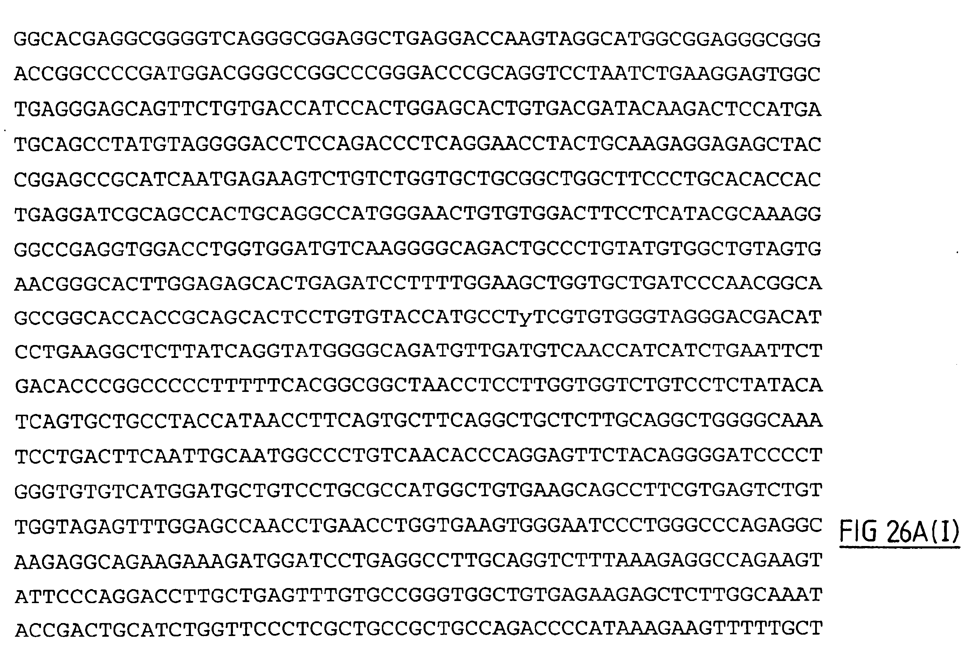

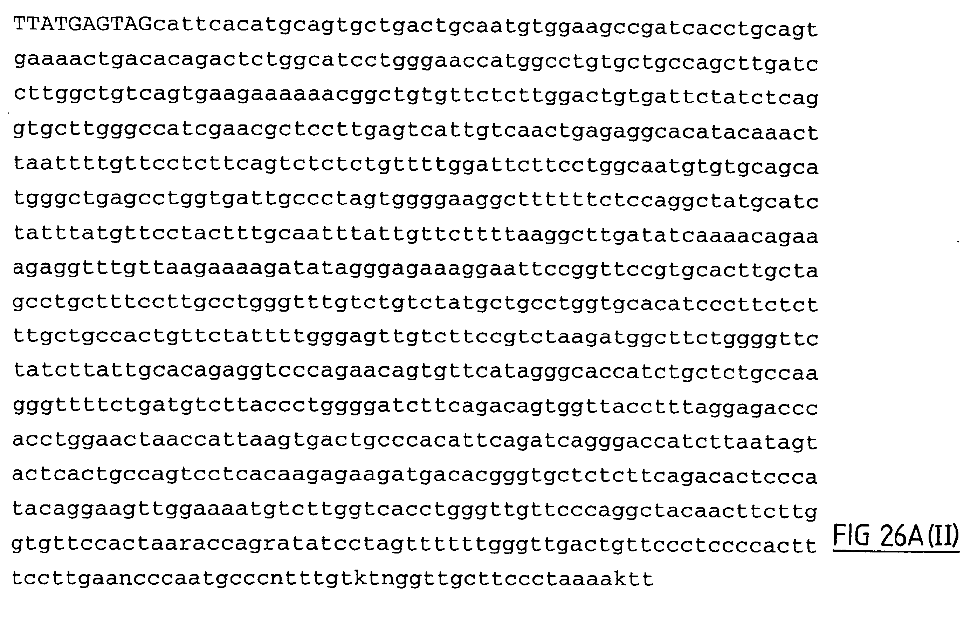

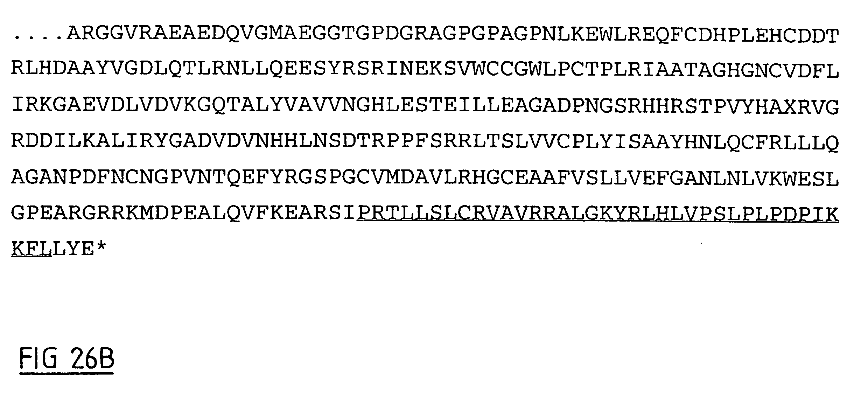

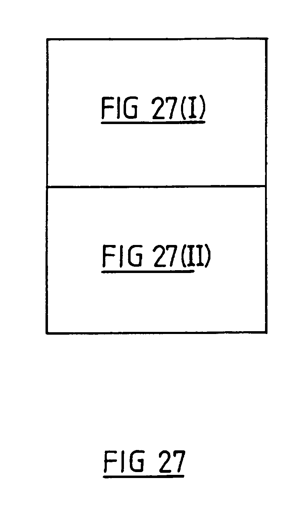

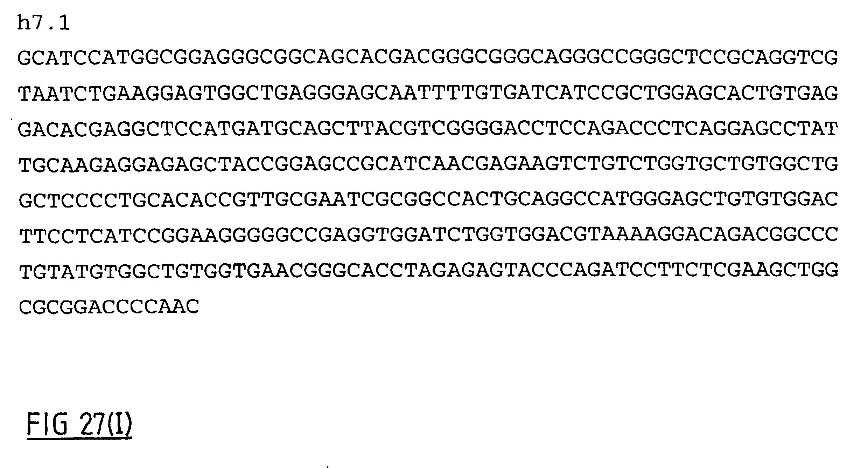

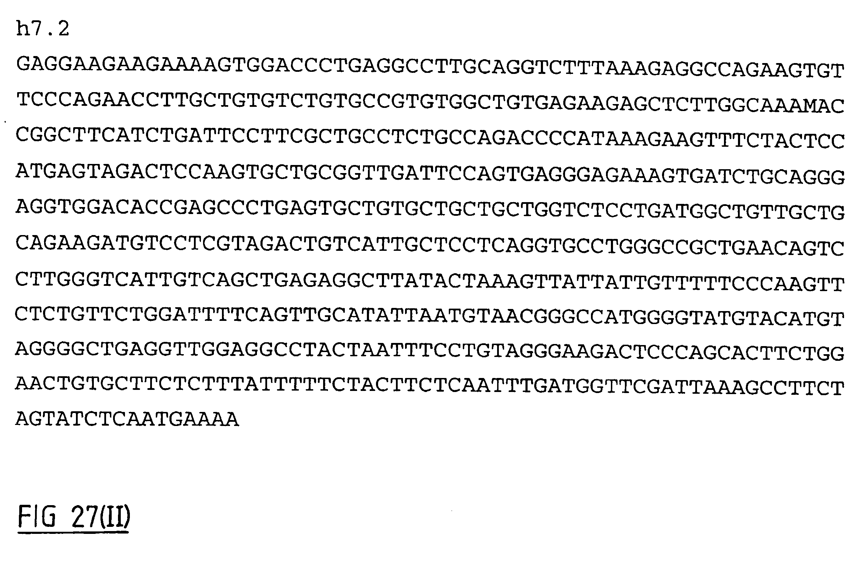

**Figure 25** .is a diagrammatic representation showing the relationship of mouse SOCS7 cDNA clone (74-10A-11) to contigs derived from analysis of mouse ESTs (Table 7.1) and human ESTs (Table 7.2). The nucleotide sequence of the mouse SOCS7 contig is shown in Figure 26 with the sequence of human SOCS7 contigs (h7.1 and h7.2) being shown in Figure 27. The deduced amino acid sequence of mouse SOCS7 is shown in Figure 26B. The structure of the protein is shown schematically, with the ankyrin repeats indicated by ( ) and the SOCS box by ( ). The putative 5' and 3' untranslated regions are shown by the thin solid line in the mouse and by the wavy line in h7.2. Based on analysis of clones isolated to date and ESTs the 3' untranslated regions of mSOCS7 and hSOCS7 share little similarity.
**Figure 26A** is a representation showing the nucleotide sequence of the mouse SOCS7 derived from analysis of cDNA clone 74-10A-11. The nucleotides encoding the part of the predicted coding region, ending in the stop codon are shown in upper case, while the predicted 3' untranslated regions are shown in lower case. The relationship of mouse cDNA sequence to mouse and human EST contigs is illustrated in Figure 25.
**Figure 26B** is a representation showing the predicted amino acid sequence of mouse SOCS7 protein, derived from the nucleotide sequence in Figure 26A. The SOCS box, which also shown in Figure 13 is underlined.
**Figure 27** is a representation showing the nucleotide sequence of human SOCS7 cDNA contig h7.1 and h7.2 derived from analysis of the ESTs listed in Table 7.2. The relationship of these contigs to the mouse cDNA sequence is illustrated in Figure 25.

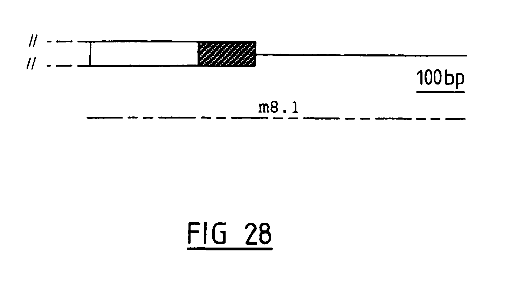

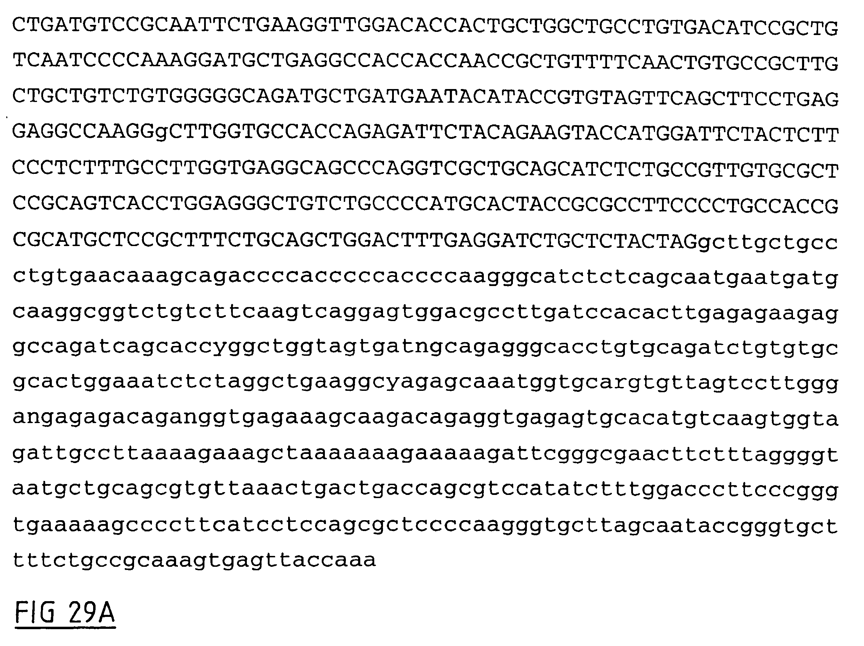

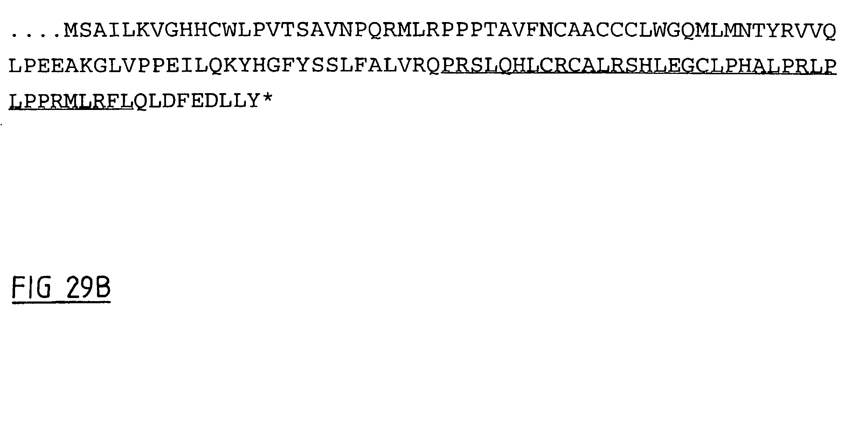

**Figure 28** is a diagrammatic representation of the relationship of sequence derived from analysis of mouse SOCS8 ESTs (Table 8.1 and Figure 29A) to the predicted protein structure of mouse SOCS8. The deduced partial amino acid sequence of mouse SOCS8 is shown in Figure 29B. The structure of the protein is shown schematically with the SOCS box highlighted ( ). The predicted 3' untranslated region is shown by the thin line.
**Figure 29A** is a representation showing the partial nucleotide sequence of mouse SOCS8 cDNA (contig 8.1) derived from analysis of ESTs. The nucleotides encoding the part of the predicted coding region, ending in the STOP codon are shown in upper case, while the predicted 3' untranslated regions are shown in lower case.
**Figure 29B** is a representation showing the partial predicted amino acid sequence of the mouse SOCS8 protein, derived from the nucleotide sequence in Figure 29A. The SOCS box, which also shown in Figure 13 is underlined.

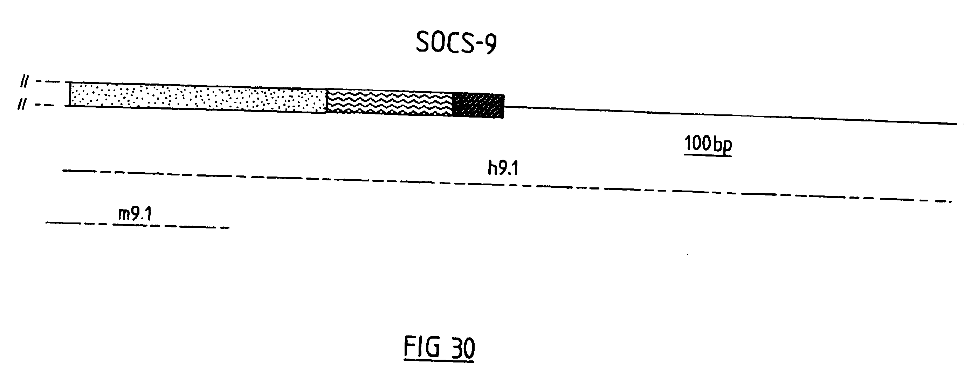

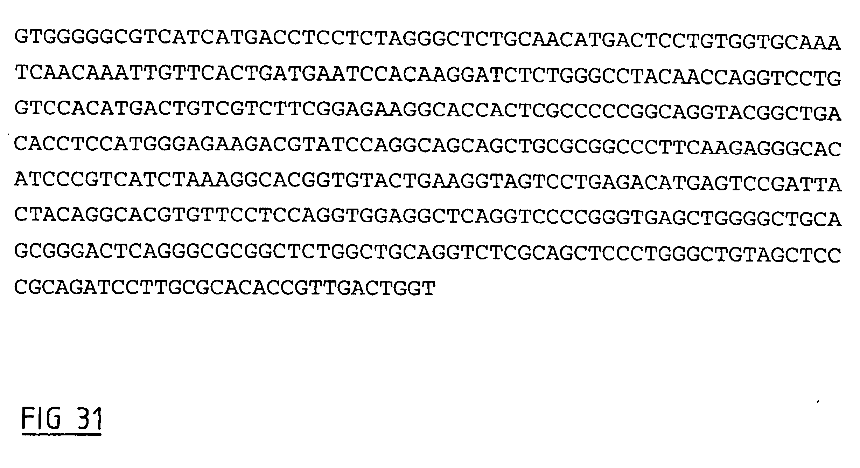

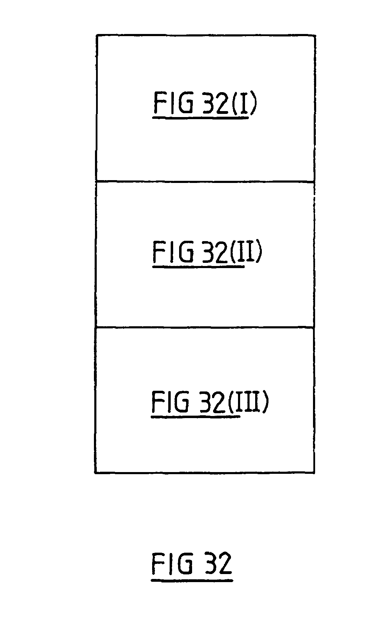

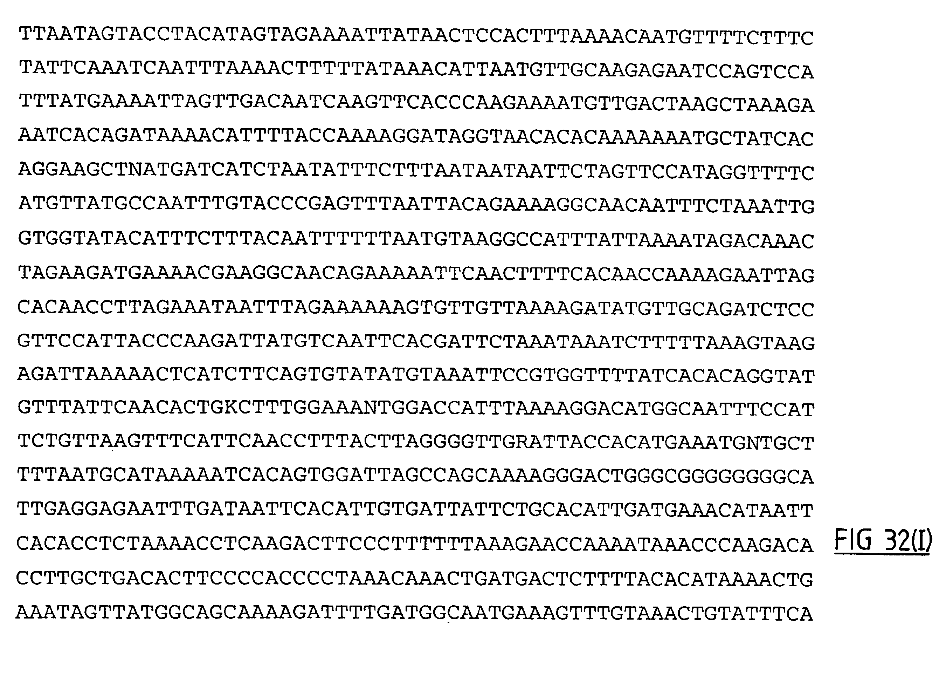

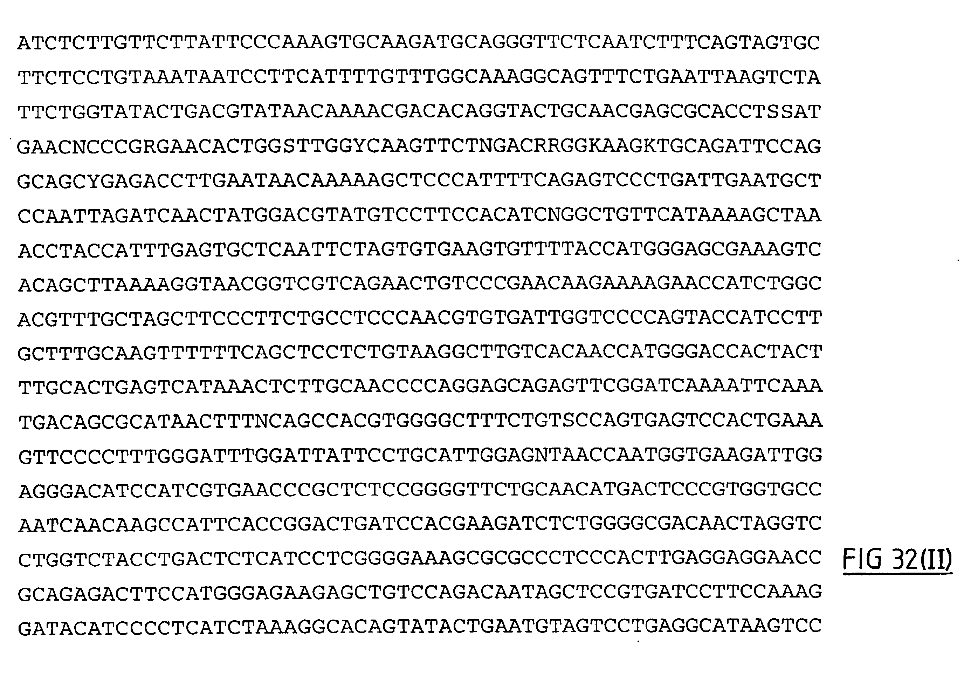

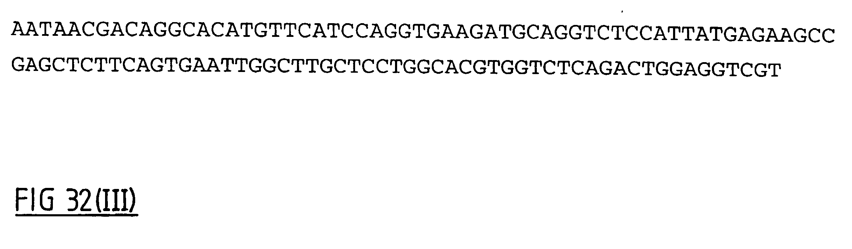

**Figure 30** is a diagrammatic representation showing the relationship of mouse SOCS9 ESTs (Table 9.1) and human SOCS9 ESTs (Table 9.2). The nucleotide sequence of the mouse SOCS9 contig (m9.1) is shown in Figure 31, with the sequence of human SOCS9 contig (h9.1) being shown in Figure 32. The deduced amino acid sequence of human SOCS9 is shown schematically, with the SH2 domain indicated by () and the SOCS box by ( ). The putative 3' untranslated region is shown by the thin solid line.
**Figure 31** is a representation showing the partial nucleotide sequence of mouse SOCS9 cDNA (contig m9.1), derived from analysis of the ESTs listed in Table 9.1. The relationship of these contigs to the mouse cDNA sequence is illustrated in Figure 30.
**Figure 32** is a representation showing the partial nucleotide sequence of human SOCS9 cDNA (contig h9.1), derived from analysis of the ESTs listed in Table 9.2. Although it is clear that contig h9.1 encodes a protein with an SH2 domain and a SOCS box, the quality of the sequence is not high enough to derive a single unambiguous open reading frame. The relationship of these contigs to the mouse cDNA sequence is illustrated in Figure 30.

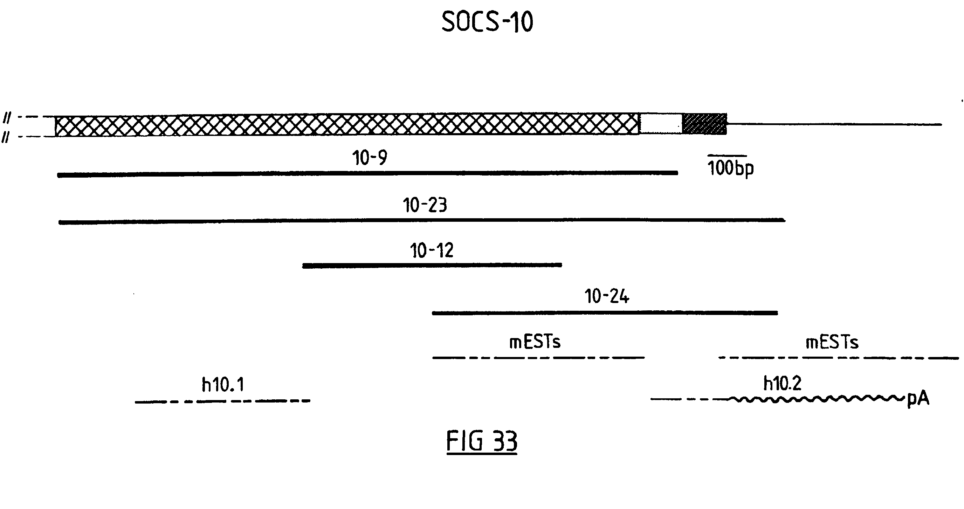

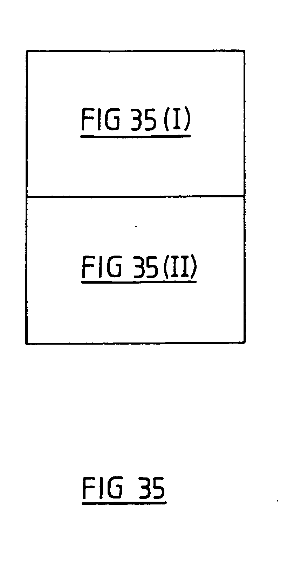

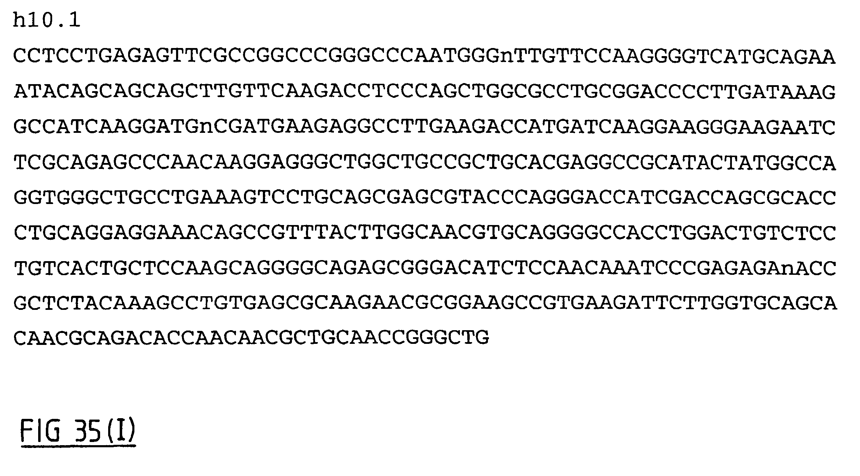

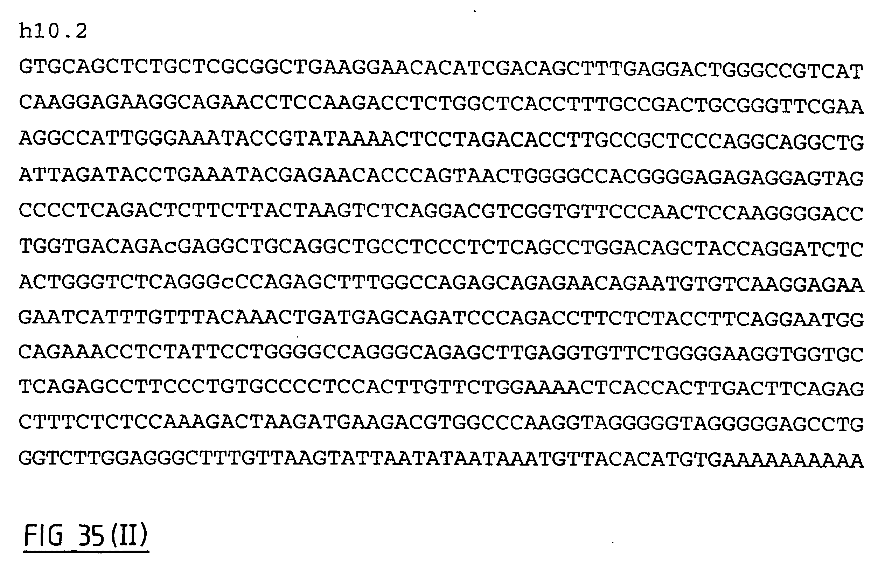

**Figure 33** is a representation showing the relationship of mouse SOCS 10 cDNA clones (10-9, 10-12, 10-23 and 10-24) to contigs derived from analysis of mouse ESTs (Table 10.1) and human ESTs (Table 10.2). The nucleotide sequence of the mouse SOCS 10 contig is shown in Figure 10.2, with the sequence of human SOCS10 contigs (h10.1 and h10.2) being shown in Figure 35. The predicted structure of the protein is shown schematically, with the ankyrin repeats indicated by ( ) and the SOCS box by ( ). The putative 3' untranslated regions is shown by the thin line solid line in the mouse and by the wavy line in h10.2. Based on analysis of clones isolated to date and ESTs the 3' untranslated regions of mSOCS-10 and hSOCS-10 share little similarity.

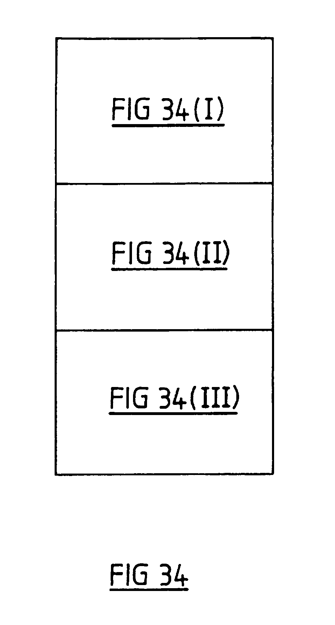

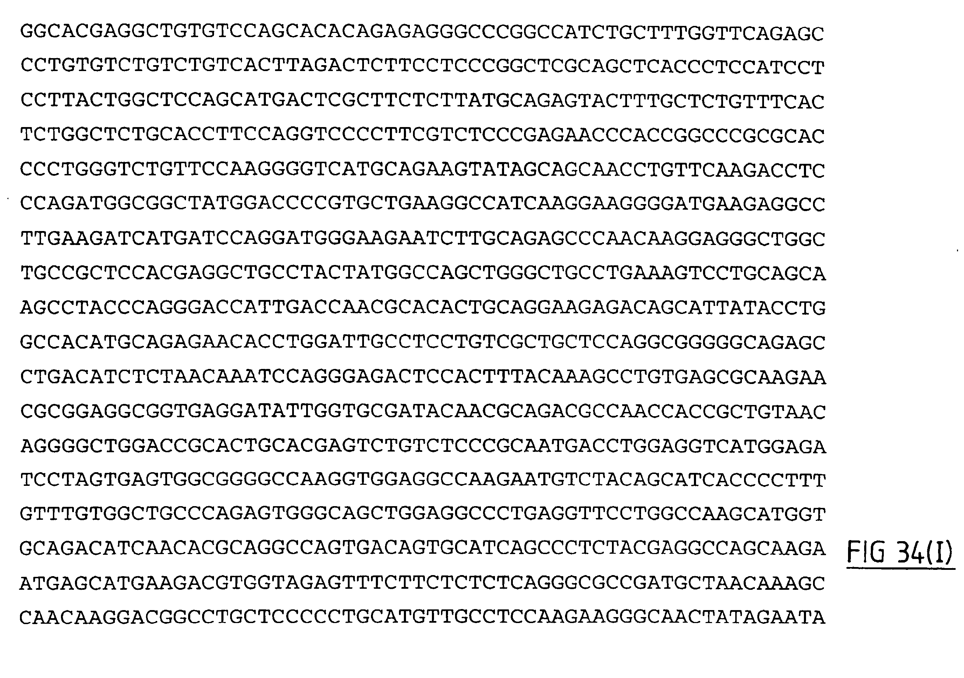

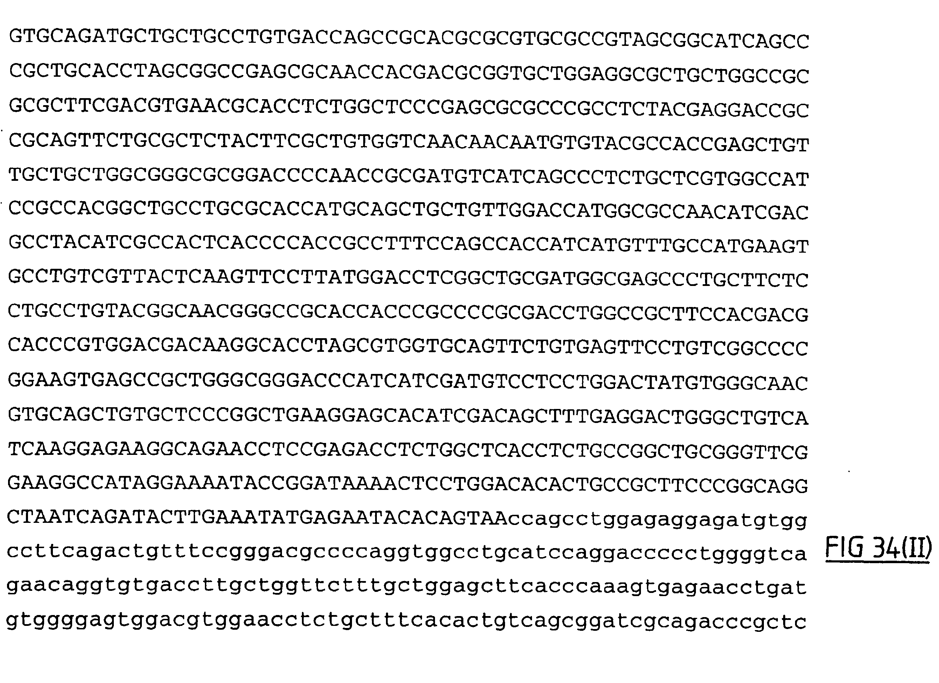

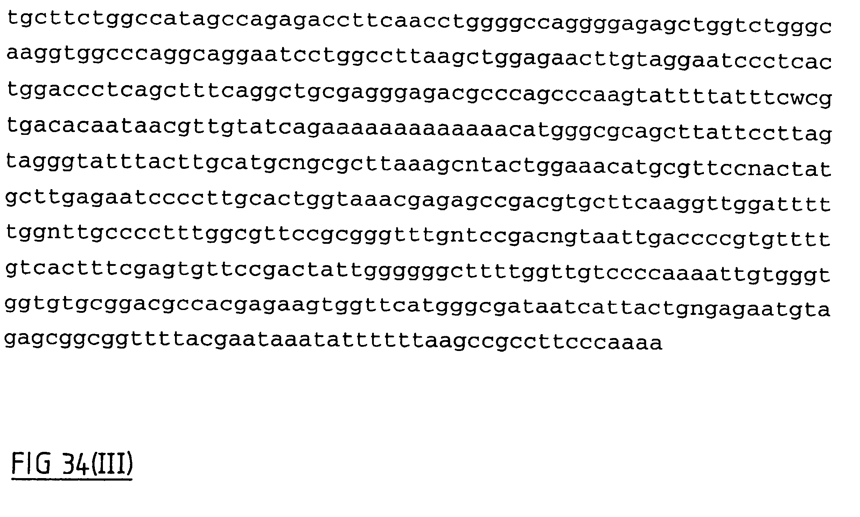

**Figure 34** is a representation showing the nucleotide sequence of the mouse SOCS10 derived from analysis of cDNA clone 10-9, 10-12, 10-23 and 10-24. The nucleotides encoding the part of the predicted coding region, ending in the stop codon are shown in upper case, while the predicted 3' untranslated regions are shown in lower case. Although it is clear that contig m10.1 encodes a protein with a series of ankyrin repeats and a SOCS box, the quality of the sequence is not high enough to derive a single unambiguous open reading frame. The relationship of mouse cDNA sequence to mouse and human EST contigs is illustrated in Figure 33.
**Figure 35** is a representation showing the nucleotide sequence of human SOCS 10 cDNA contig h10.2 and h10.2 derived from analysis of the ESTs listed in Table 10.2. The relationship of these contigs to the mouse cDNA sequence is illustrated in Figure 33.

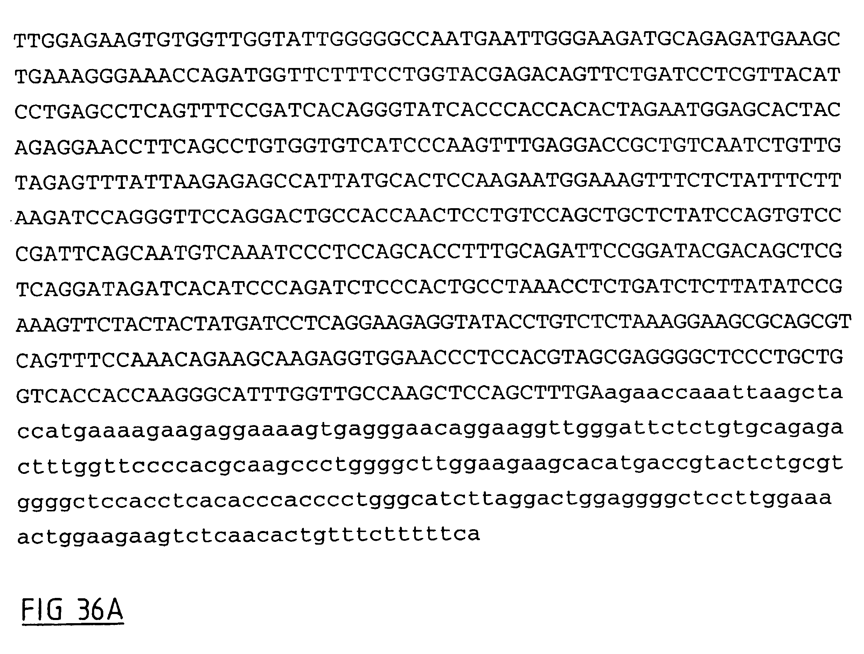

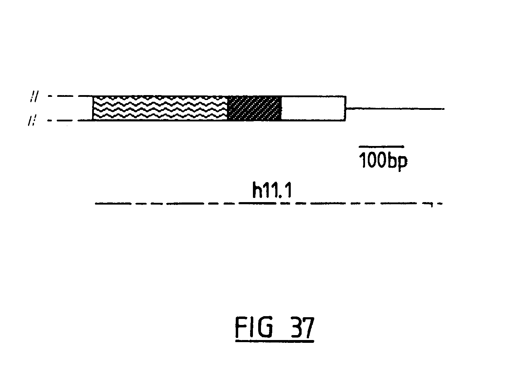

**Figure 36A** is a representation showing the partial nucleotide sequence of the human SOCS 11 cDNA derived from analysis of ESTs listed in Table 11.1 The nucleotides encoding the mature coding region from the predicted ATG "start" codon to the stop codon is shown in upper case, while the predicted 5' and 3' untranslated regions are shown in lower case. The relationship of the partial cDNA sequence, derived from ESTs, to the predicted protein is shown in Figure 37.

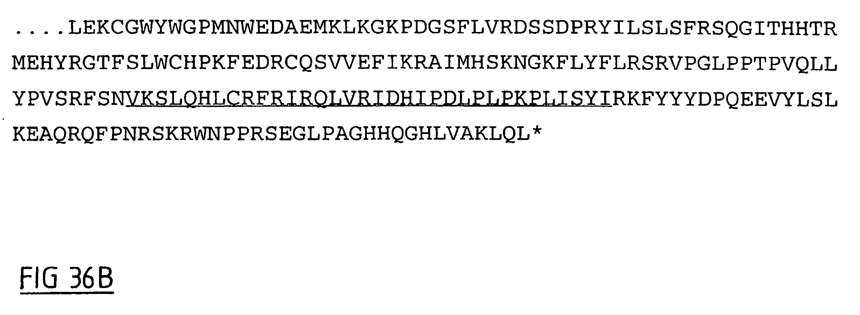

**Figure 36B** is a representation showing the partial predicted amino acid sequence of human SOCS 11 protein, derived from the nucleotide sequence in Figure 36A. The SOCS box, which also shown in Figure 13, is underlined.
**Figure 37** is a diagrammatic representation showing the relationship of sequence derived from analysis of human SOCS-11 ESTs (Table 11.1 and Figure 36A) to the predicted protein structure of human SOCS 11. The deduced partial amino acid sequence of human SOCS 11 is shown in Figure 36B. The structure of the protein is shown schematically with the SH2 domain shown by ( ) and the SOCS box highlighted by ( ). The predicted 3' untranslated region is shown by the thin line.

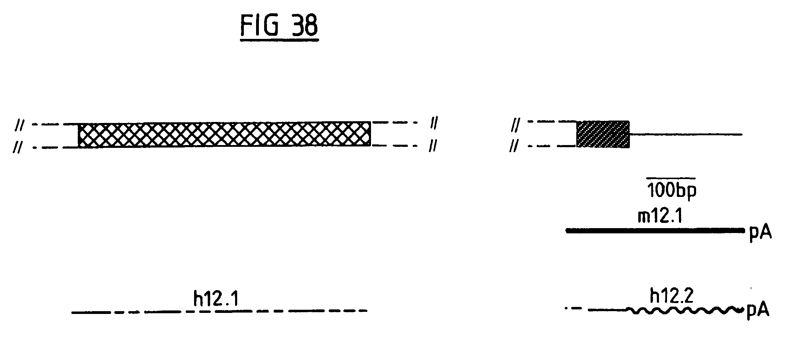

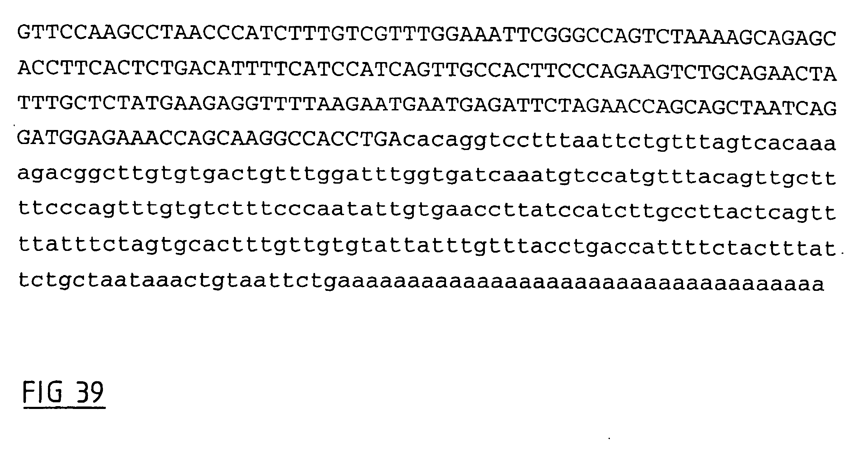

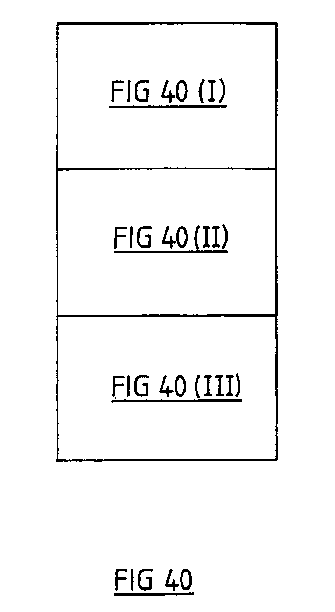

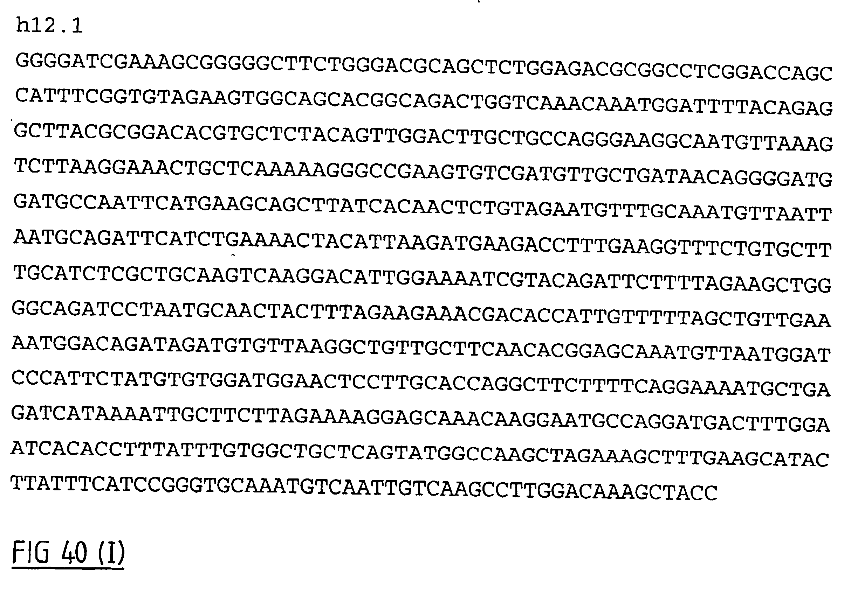

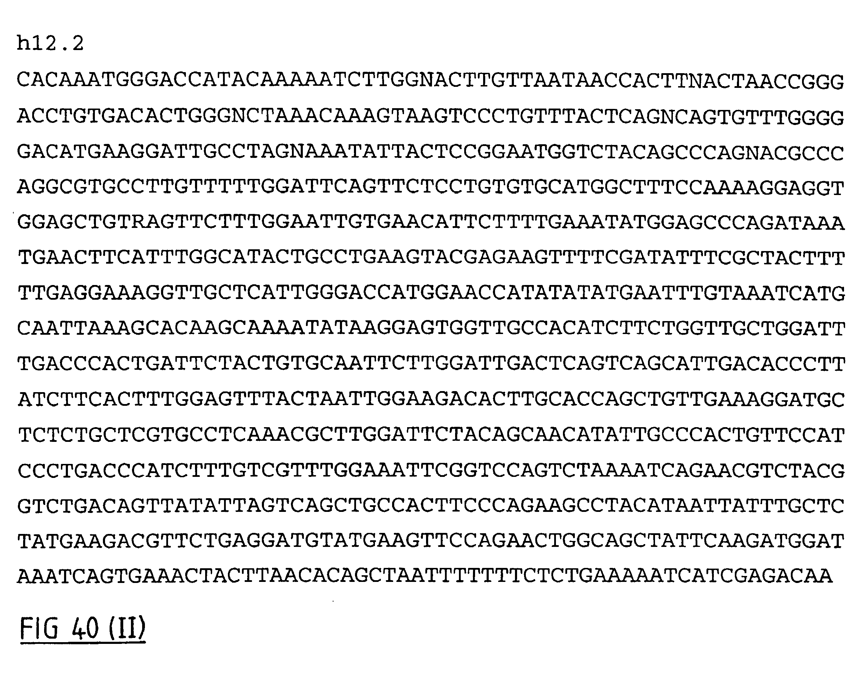

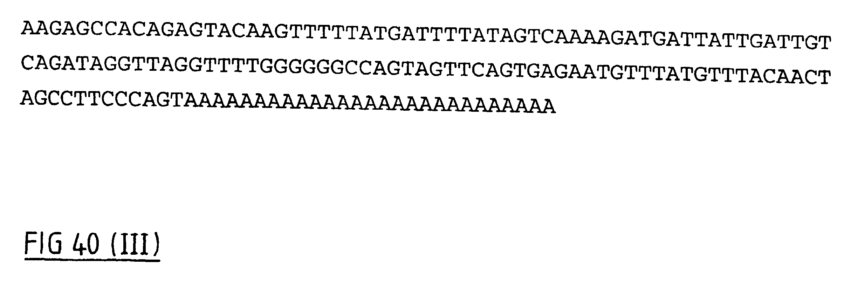

**Figure 38** is a diagrammatic representation showing the relationship of mouse SOCS 12 cDNA clones (12-1) to contigs derived from analysis of mouse ESTs (Table 12.1) and human ESTs (Table 12.2). The nucleotide sequence of the mouse SOCS 12 contig is shown in Figure 12.2, with the sequence of human SOCS12 contigs (h12.1 and h12.2) being shown in Figure 40. The deduced partial amino acid sequence of mouse SOCS12 is shown in Figure 39. The structure of the protein is sown schematically, with the ankyrin repeats indicated by ( ) and the SOCS box by ( ). The putative 3' untranslated region is shown by the thin line solid line in the mouse and by the wavy line in h12.2. Based on analysis of clones isolated to date and ESTs the 3' untranslated regions of mSOCS12 and hSOCS 12 share little similarity.
**Figure 39** is a representation showing the nucleotide sequence of the mouse SOCS 12 derived from analysis of cDNA clone 12-1 and the ESTs listed in Table 12.1. The nucleotides encoding the part of the predicted coding region, including the stop codon are shown in upper case, while the predicted 3' untranslated region is shown in lower case. By homology with human SOCS12 it is clear that contig m12.1 encodes a protein with a series of ankyrin repeats and a SOCS box, the quality of the sequence is not high enough to derive a single unambiguous open reading frame. The relationship of mouse cDNA sequence to mouse and human EST contigs is illustrated in Figure 38.
**Figure 40** is a representation showing the nucleotide sequence of human SOCS 12 cDNA contig h12.1 and h12.2 derived from analysis of the ESTs listed in Table 12.2. The relationship of these contigs to the mouse cDNA sequence is illustrated in Figure 38.

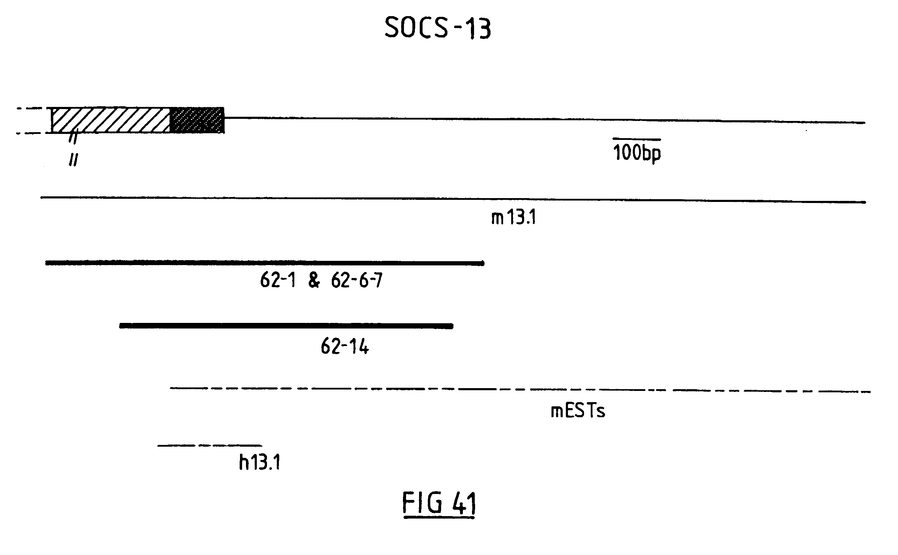

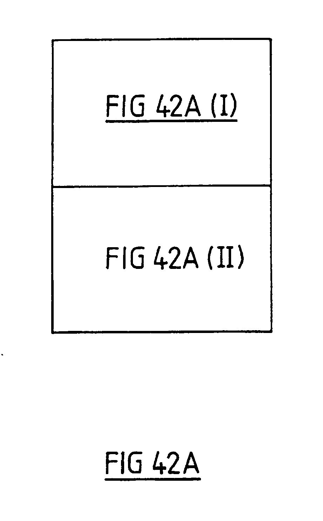

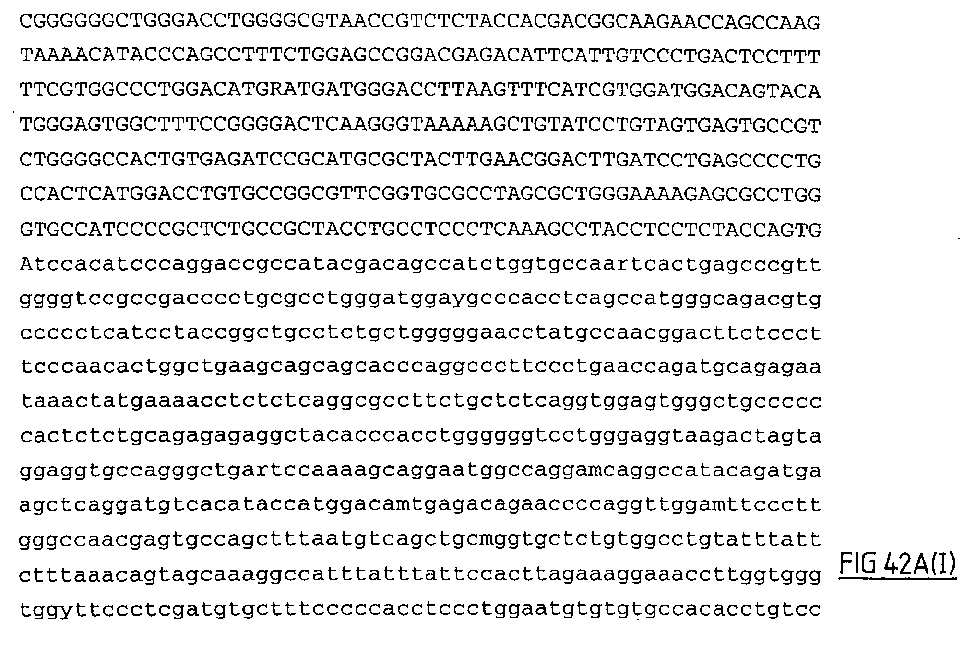

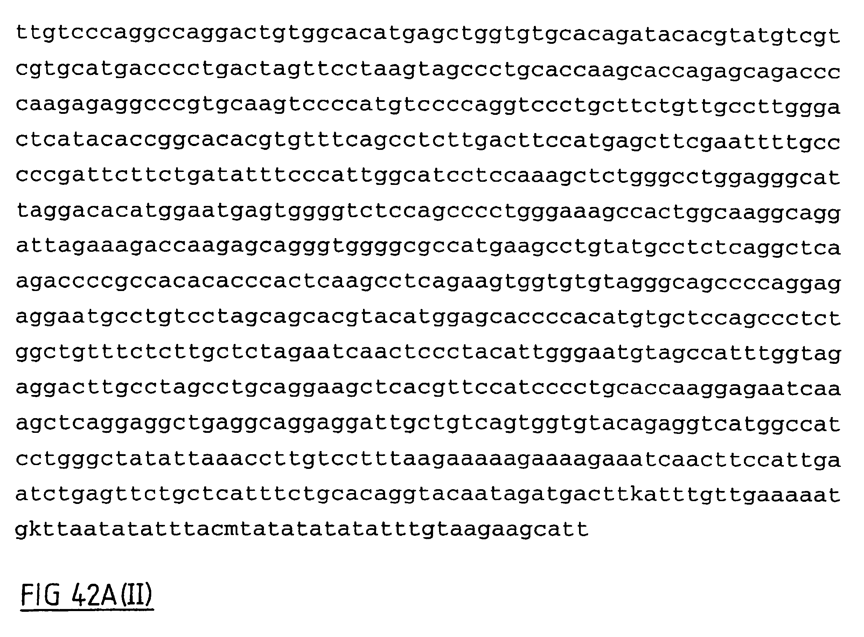

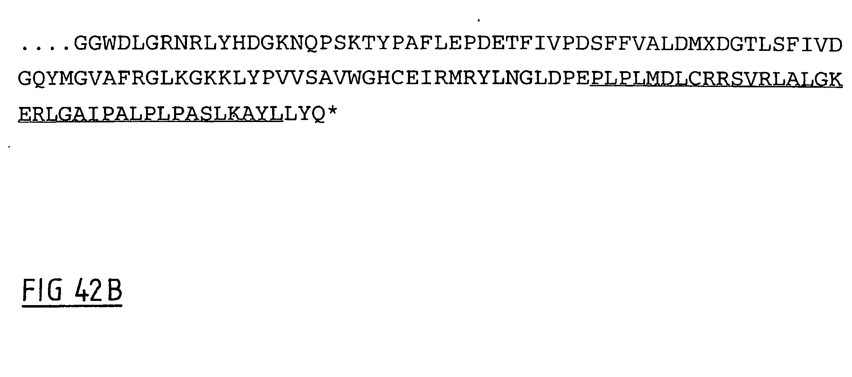

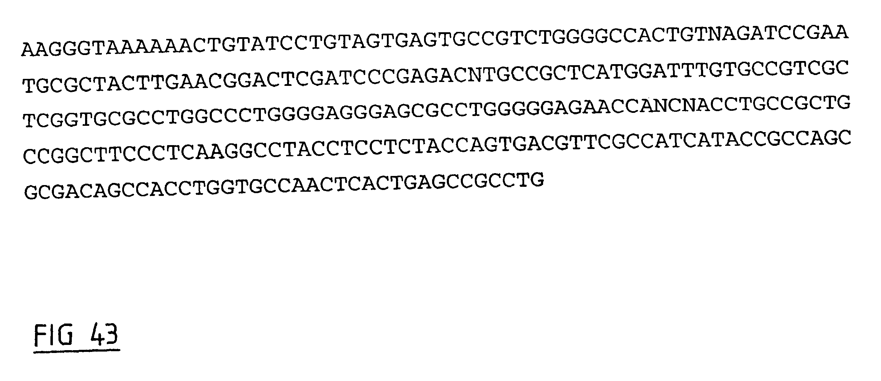

**Figure 41** is a diagrammatic representation showing the relationship of contig m13.1 derived from analysis of mouse SOCS13 cDNA clones (62-1, 62-6-7, 62-14) and mouse ESTs (Table 13.1) to contig h13.1 derived from analysis of human ESTs (Table 13.2). The nucleotide sequence of the mouse SOCS 13 contig is shown in Figure 42, with the sequence of human SOCS13 contig (h13.1) being shown in Figure 43. The deduced amino acid sequence of mouse SOCS 13 is shown in Figure 42B. The structure of the protein is shown schematically, with the WD-40 repeats highlighted by () and the SOCS box highlighted by ( ). The 3' untranslated region is shown by the thin line solid line.
**Figure 42A** is a representation showing the nucleotide sequence of the mouse SOCS 13 derived from analysis of cDNA clones 62-1, 62-6-7 and 62-14. The nucleotides encoding part of the predicted coding region, ending in the stop codon are shown in upper case, while those encoding the predicted 3' untranslated regions are shown in lower case. The relationship of mouse cDNA sequence to mouse and human EST contigs is illustrated in Figure 41.
**Figure 42B** is a representation showing the predicted amino acid sequence of mouse SOCS 13 protein, derived from the nucleotide sequence in Figure 42A. The SOCS box, which also shown in Figure 13 is underlined.
**Figure 43** is a representation showing the nucleotide sequence of human SOCS 13 cDNA contig h13.1 derived from analysis of the ESTs listed in Table 13.2. The relationship of these contigs to the mouse cDNA sequence is illustrated in Figure 41.

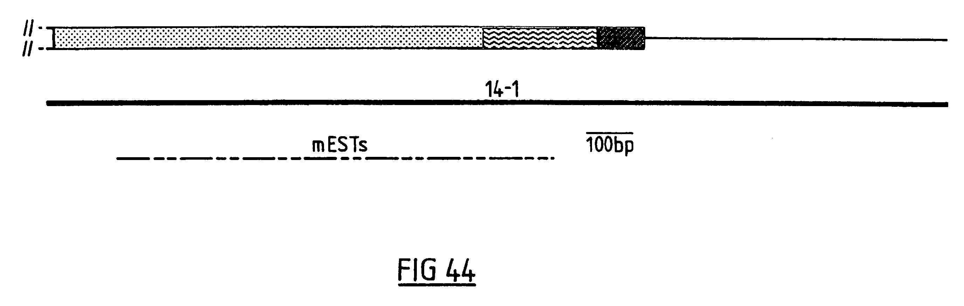

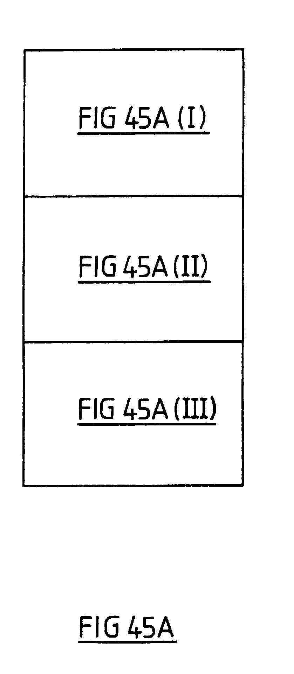

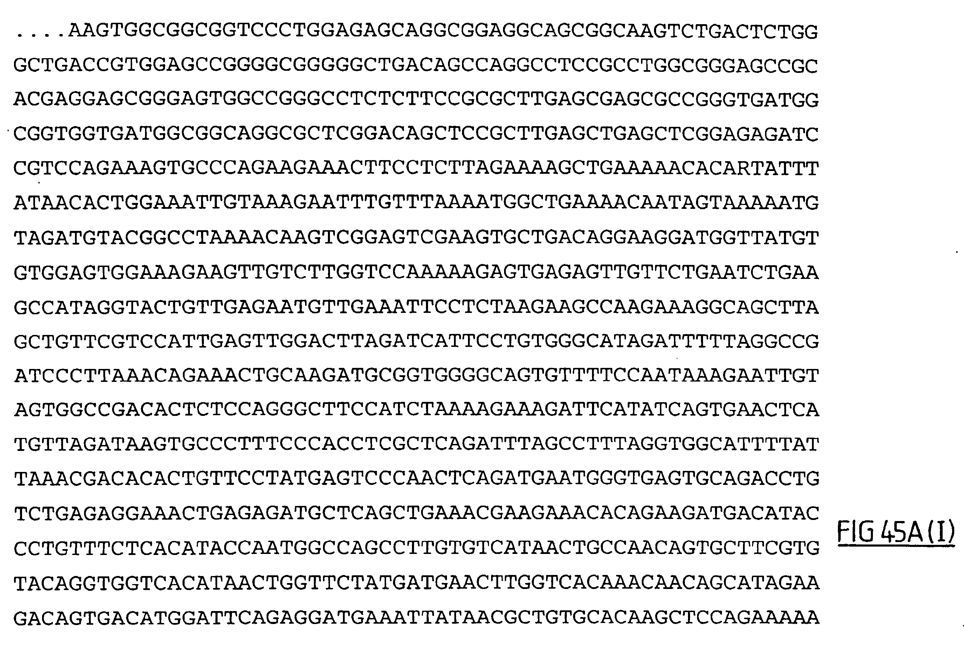

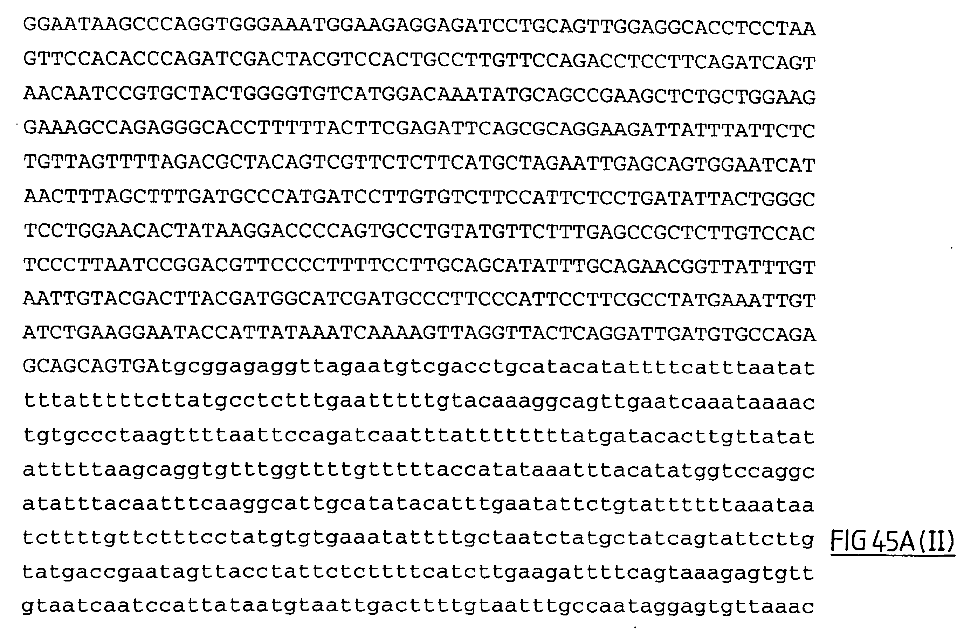

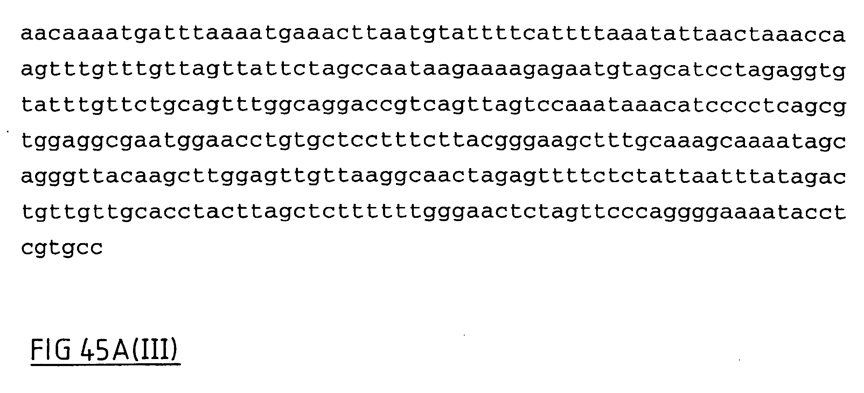

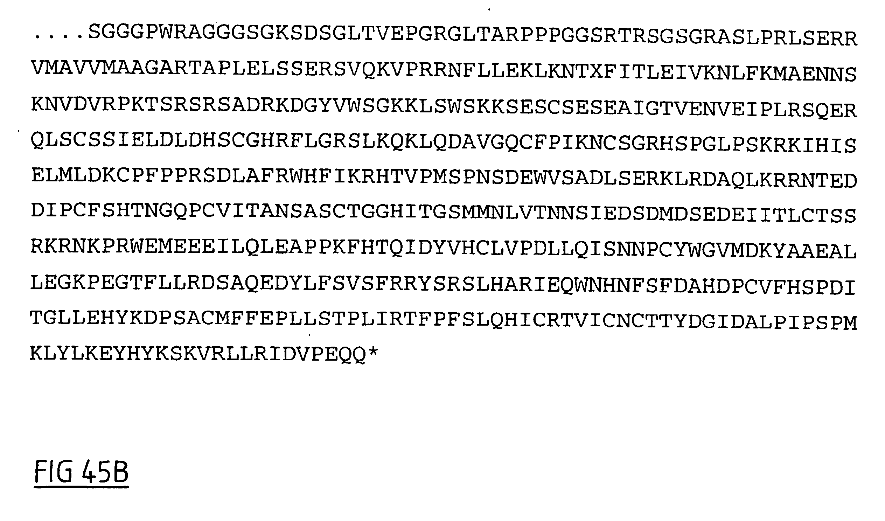

**Figure 44** is a diagrammatic representation showing the relationship of a partial mouse SOCS 14 cDNA clone (14-1) to contigs derived from analysis of mouse ESTs (Table 14.1). The nucleotide sequence of the mouse SOCS 14 contig is shown in Figure 45. The deduced partial amino acid sequence of mouse SOCS 14 is shown in Figure 45B. The structure of the protein is shown schematically, with the SH3 domain indicated by ( ) and the SOCS box by ( ). The putative 3' untranslated region is shown by the thin line.
**Figure 45A** is a representation showing the nucleotide sequence of the mouse SOCS 14 derived from analysis of genomic and cDNA clones. The nucleotides encoding the mature coding region from the predicted ATG "start" codon to the stop codon is shown in upper case, while the predicted 5' and 3' untranslated regions are shown in lower case. The relationship of mouse cDNA sequence to mouse and human EST contigs is illustrated in Figure 44.
**Figure 45B** is a representation showing the predicted amino acid sequence of mouse SOCS 14 protein, derived from the nucleotide sequence in Figure 45B. The SOCS box, which also shown in Figure 13 is underlined.

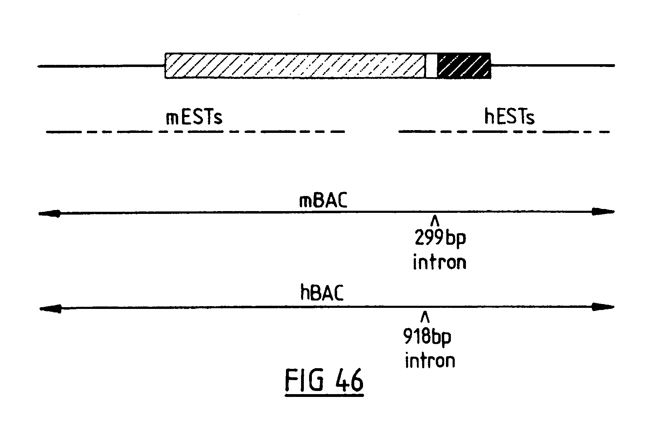

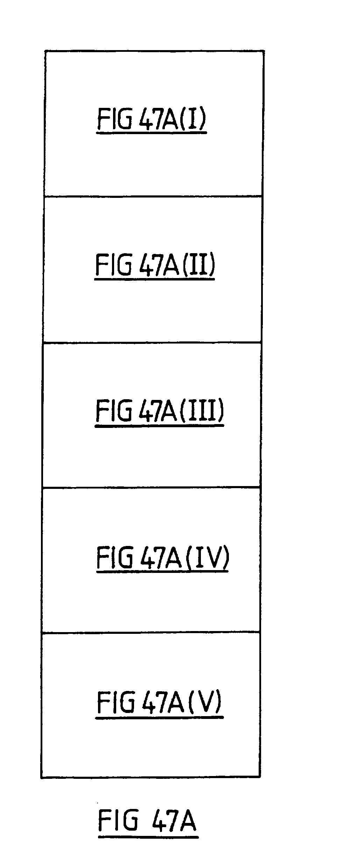

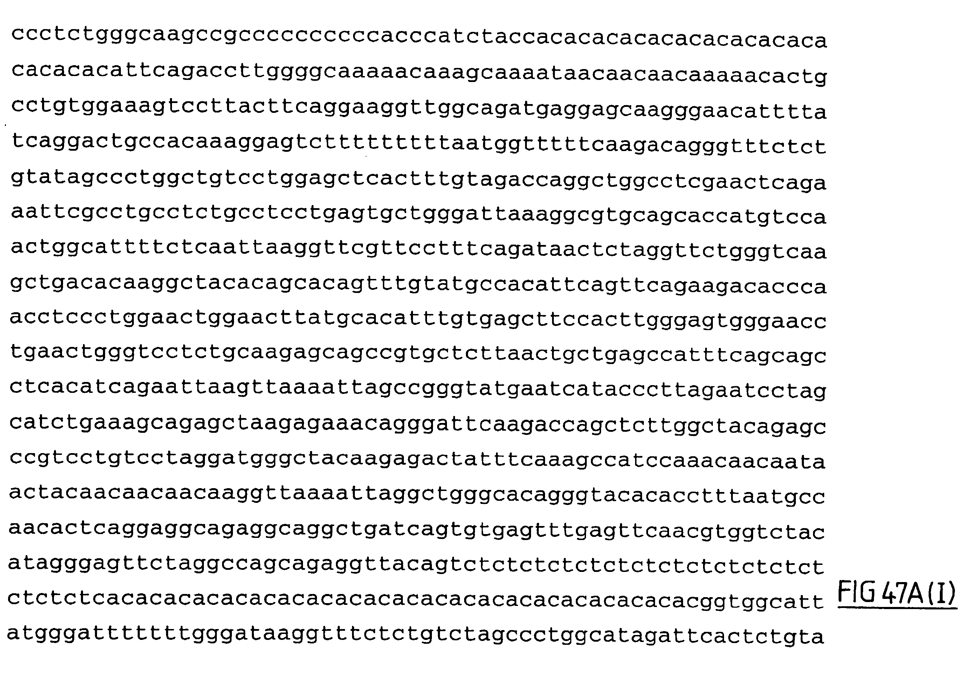

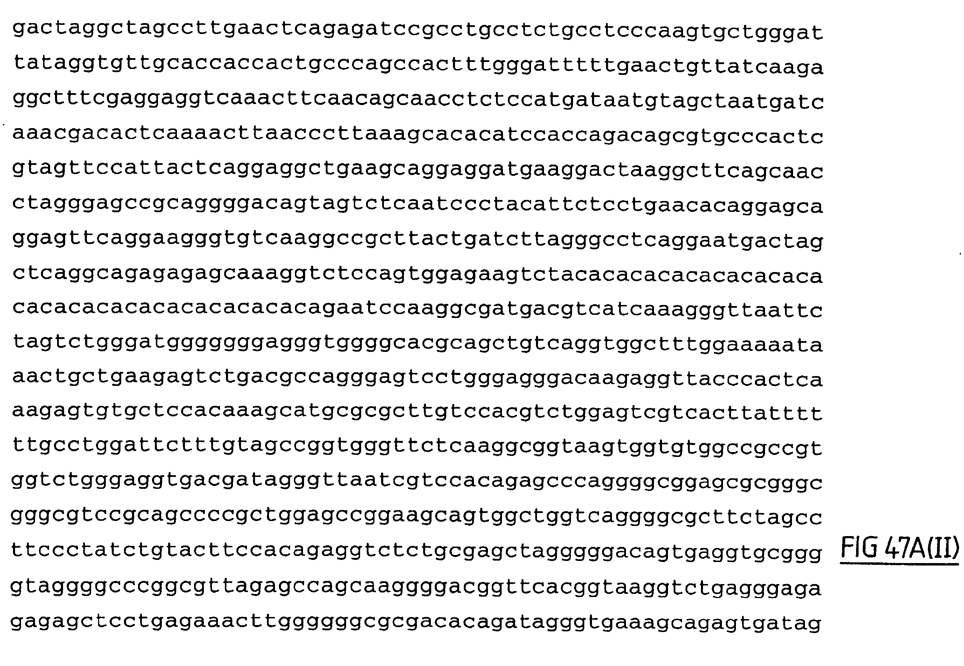

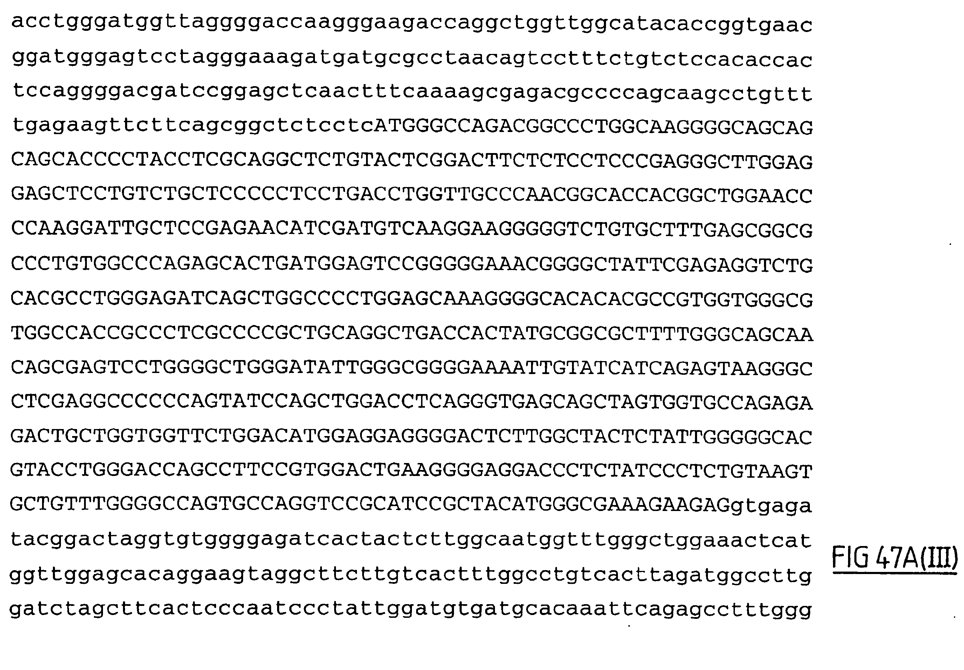

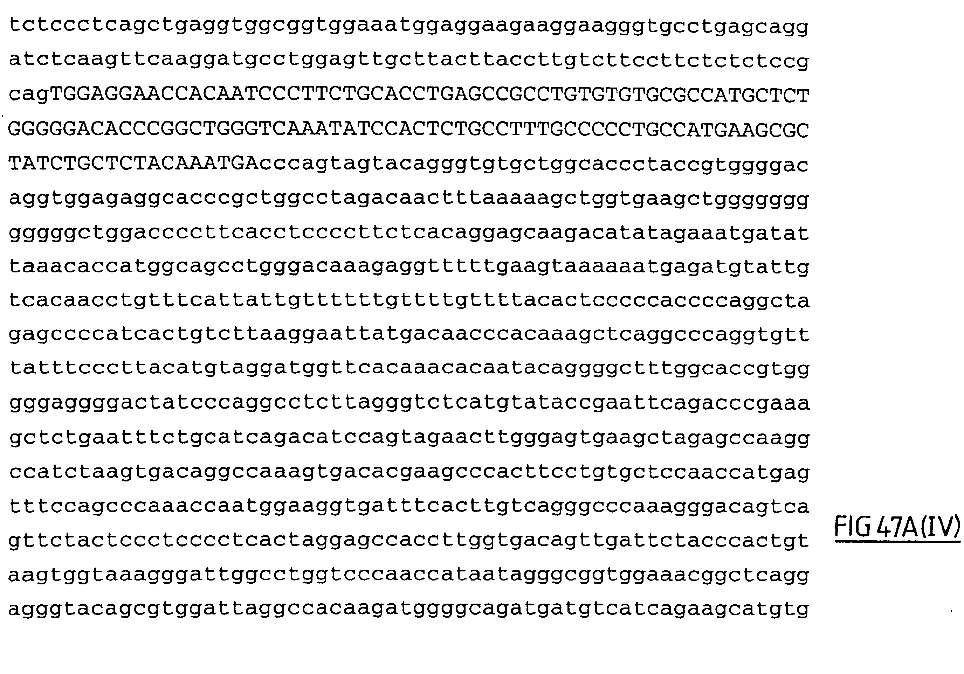

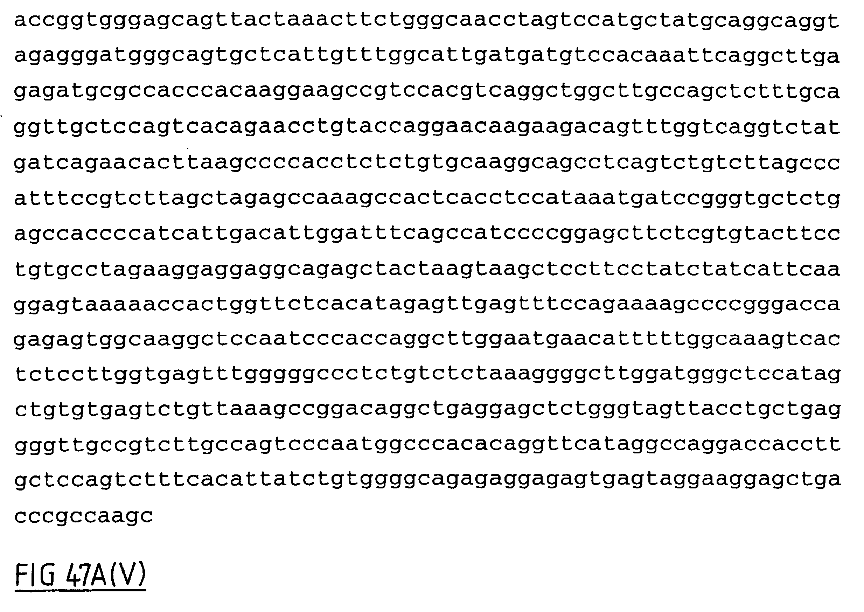

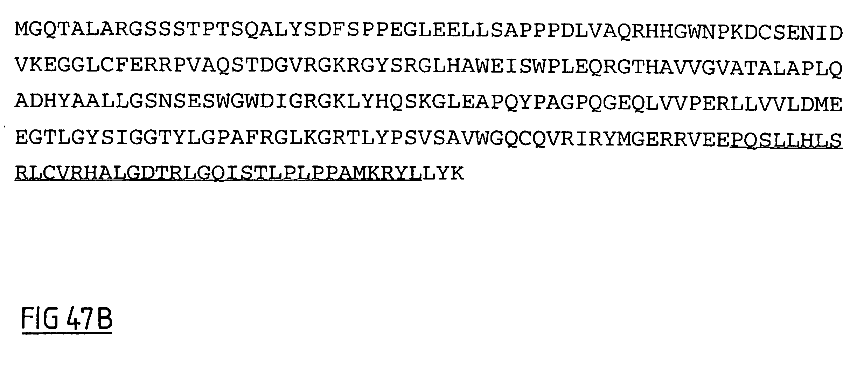

**Figure 46** is a diagrammatic representation showing the relationship of contig m15.1 derived from analysis of mouse BAC and mouse ESTs (Table 15.1) to contig h15.1 derived from analysis of the human BAC and human ESTs (Table 15.2). The nucleotide sequence of the mouse SOCS15 contig is shown in Figure 47, with the sequence of human SOCS 15 contig (h15.1) being shown in Figure 47. The deduced amino acid sequence of mouse SOCS 15 is shown in Figure 47B. The structure of the protein is shown schematically, with the WD-40 repeats highlighted by ( ) and the SOCS box highlighted by ( ). The 5' and 3' untranslated region are shown by the thin line solid line. The introns which interrupt the coding region are shown by ^.
**Figure 47A** is a representation showing the nucleotide sequence covering the mouse SOCS 15 gene derived from analysis the mouse BAC listed in Table 15.1. The nucleotides encoding the predicted coding region, beginning with the ATG and ending in the stop codon are shown in upper case, while those encoding the predicted 5' untranslated region, the introns and the 3' untranslated region are shown in lower case. The relationship of mouse BAC to mouse and human ESTs contigs is illustrated in Figure 46.
**Figure 47B** is a representation showing the predicted amino acid sequence of mouse SOCS 15 protein, derived from the nucleotide sequence in Figure 47A. The SOCS box, which also shown in Figure 13 is underlined.

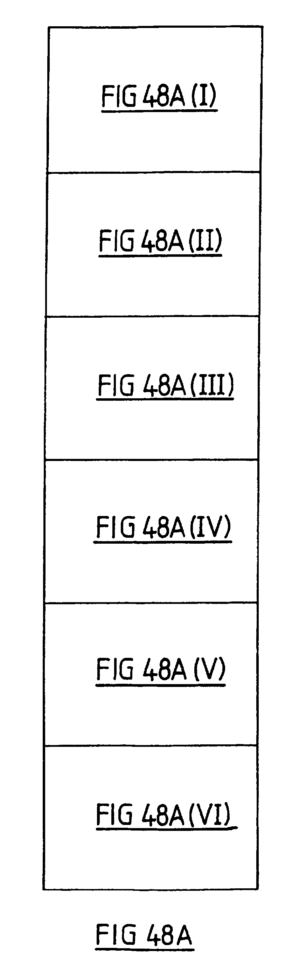

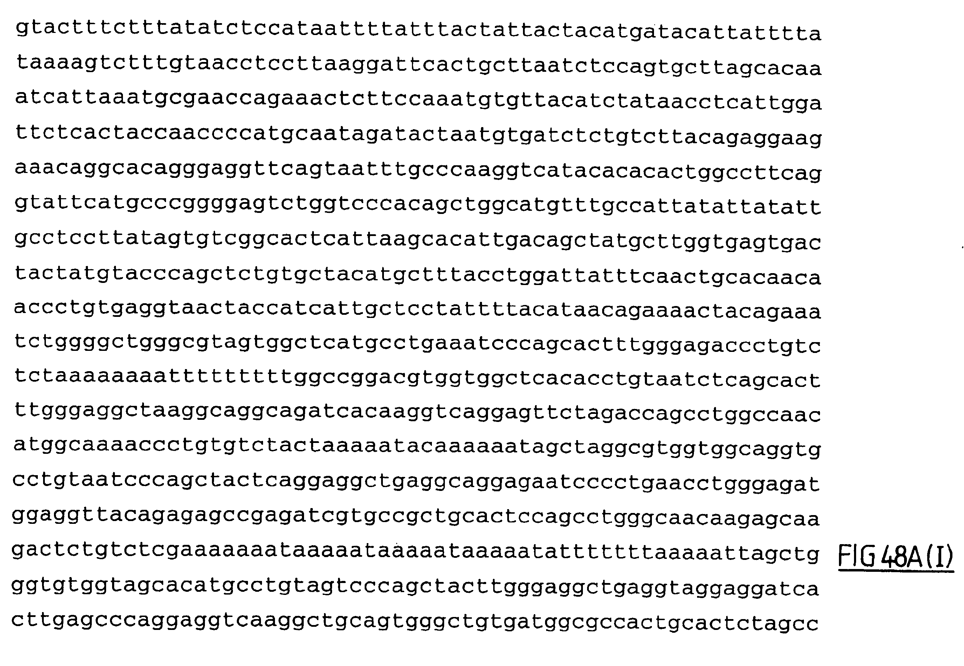

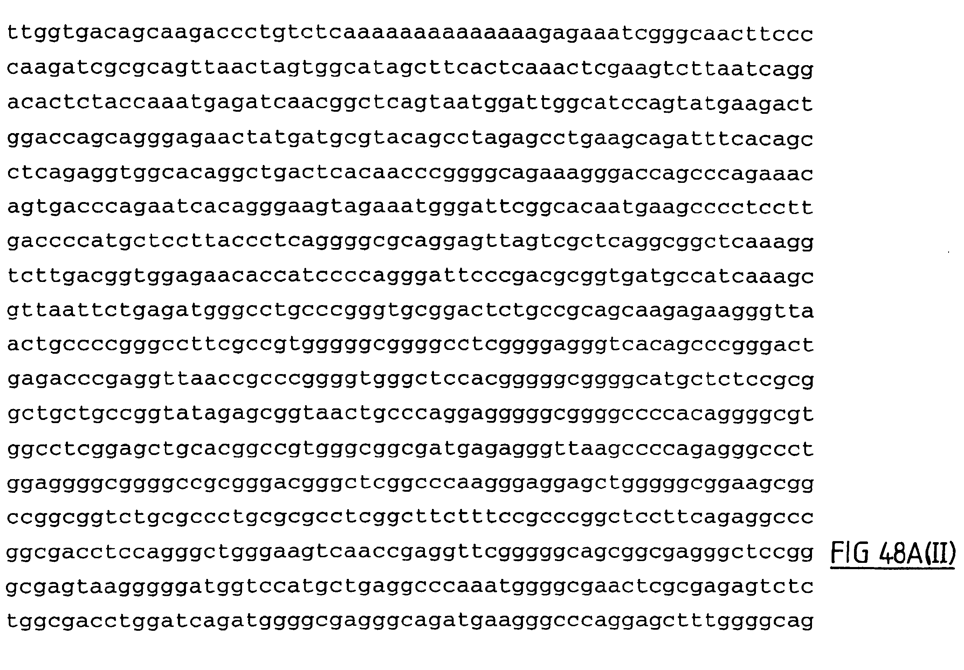

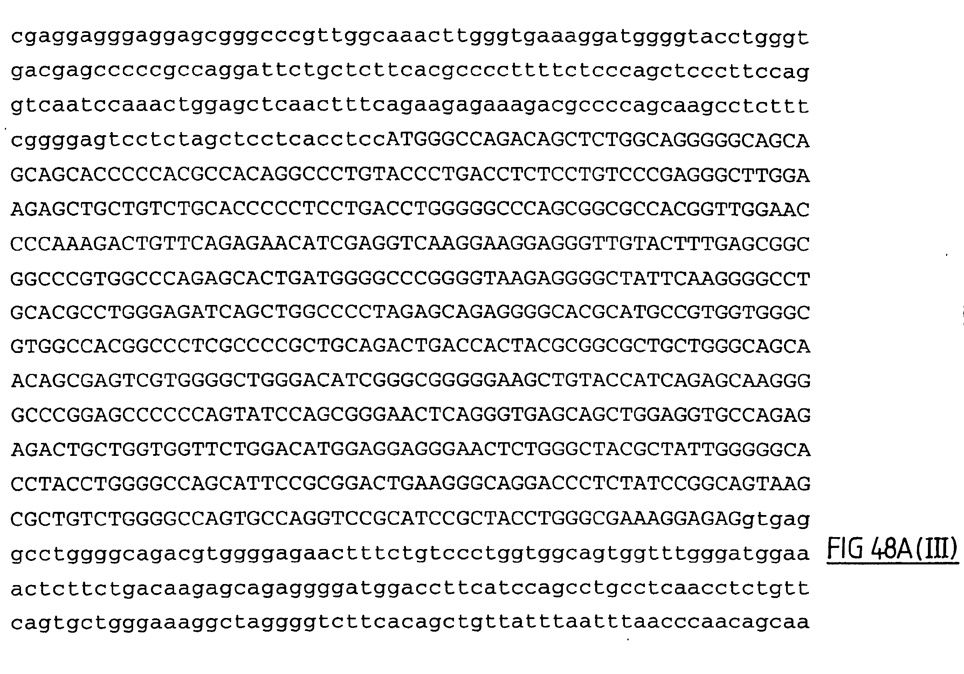

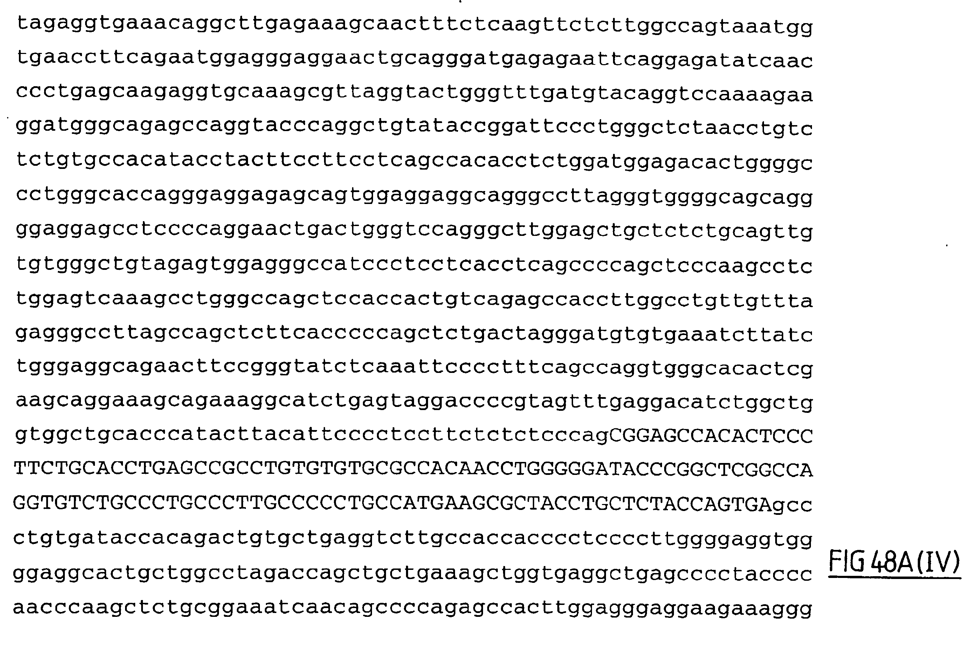

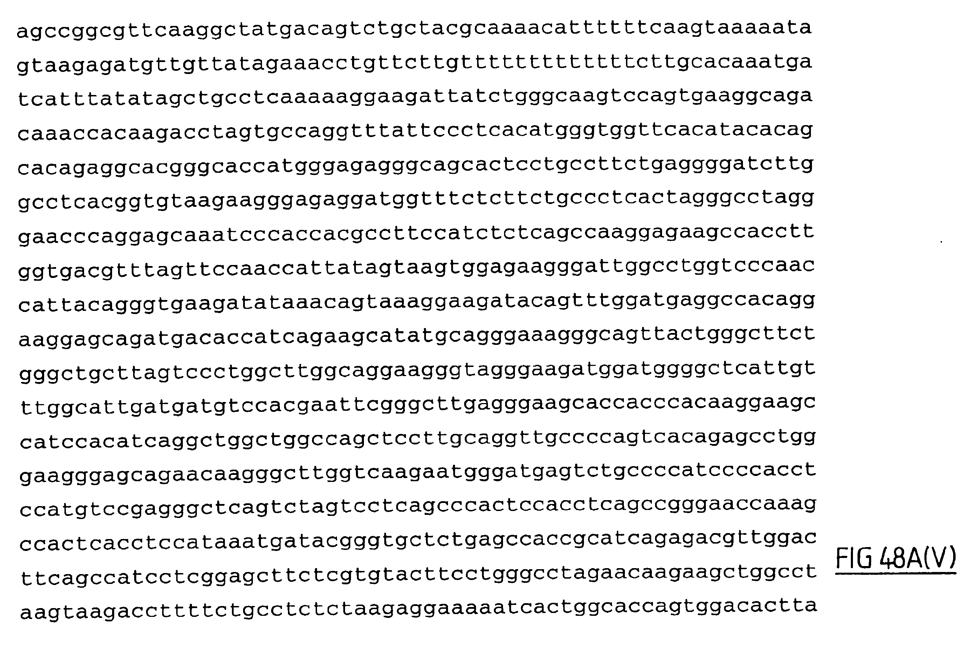

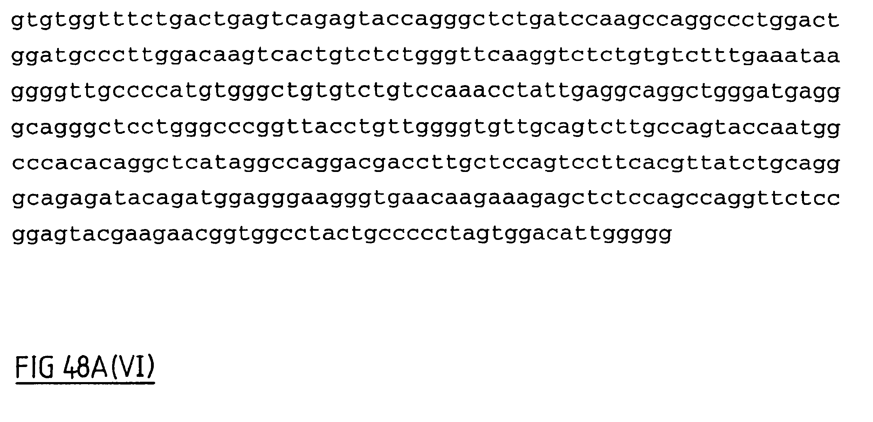

**Figure 48A** is a representation showing the nucleotide sequence covering the human SOCS 15 gene derived from analysis the human BAC listed in Table 15.2. The nucleotides encoding the predicted coding region, beginning with the ATG and ending in the stop codon are shown in upper case, while those encoding the predicted 5' untranslated region, the introns and the 3' untranslated region are shown in lower case. The relationship of the human BAC to mouse and human ESTs contigs is illustrated in Figure 46.

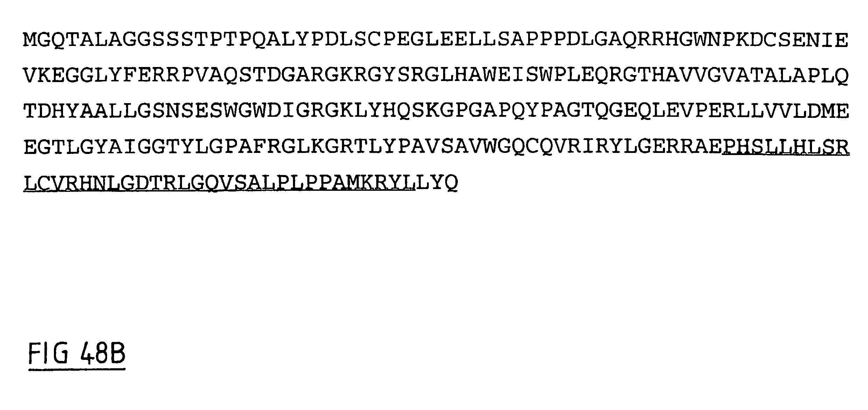

**Figure 48B** is a representation showing the predicted amino acid sequence of human SOCS 15 protein, derived from the nucleotide sequence in Figure 48A. The SOCS box, which also shown in Figure 13 is underlined.

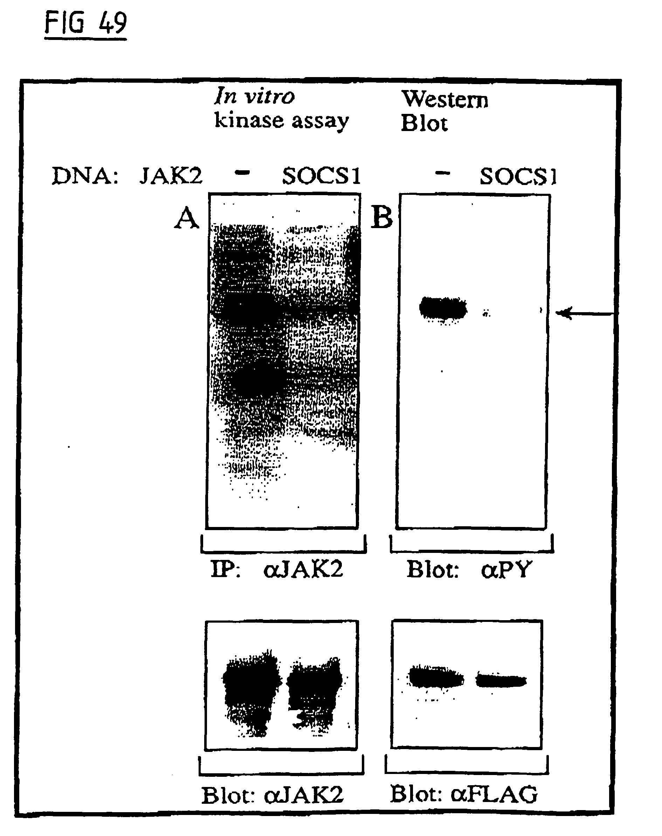

**Figure 49** is a photographic representation showing SOCS 1 inhibition of JAK2 kinase activity. (A) Upper panel. Cos M6 cells were transiently transfected with either Flag-tagged mJAK2 and mSOCS-1 DNA (SOCS1) or Flag-mJAK2 DNA alone (-), lysed, JAK2 proteins immunoprecipitated using anti-JAK2 antibody and subjected to an *in vitro* kinase assay. Lower panel. A portion of the JAK2 immunoprecipitates were Western blotted with anti-JAK2 antibody. (B) Upper panel. Cos M6 cells were transiently transfected with Flag- mJAK2 and Flag- mSOCS-1 DNA or Flag-mJAK2 DNA alone, lysed, JAK2 proteins immunoprecipitated using anti-JAK2 (UBI) and separated by SDS/PAGE gel. Immunoprecipitates were then analysed by Western blot with anti-phosphotyrosine antibody. Lower panel; JAK2 expression. Cos cell lysates were separated by SDS/PAGE gel and analysed by Western blot with anti-FLAG antibody (M2).

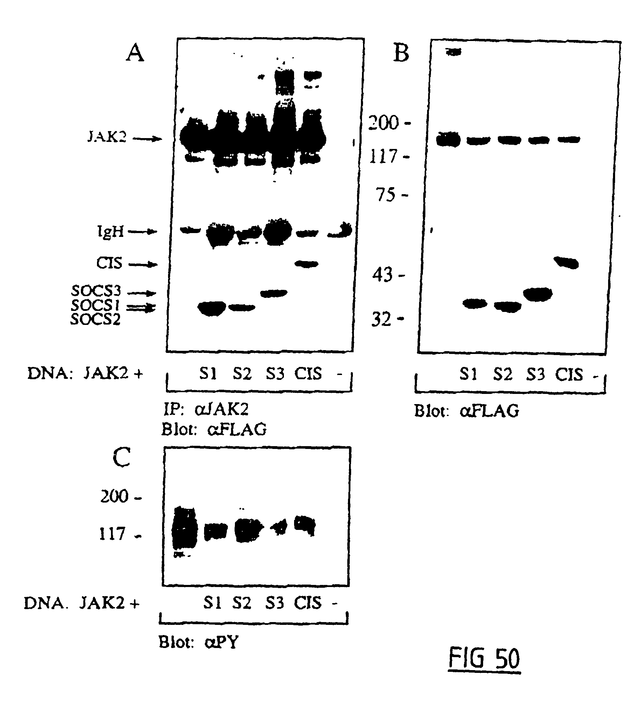

**Figure 50** is a photographic representation showing interaction between JAK2 and SOCS protein. (A) Cos M6 cells were transiently transfected with Flag-tagged mJAK2 and various Flag-tagged SOCS DNAs (SOCS-1;S1, SOCS-2;S2, SOCS-3;S3, CIS) or Flag-mJAK2 alone, lysed, JAK2 proteins immunoprecipitated using anti-JAK2 (UBI) and separated by SDS/PAGE. Immunoprecipitates were then analysed by Western blot with anti-FLAG antibody (M2). (B) Cos cell lysates described in (A) were separated by SDS/PAGE and expression levels of the various proteins were determined by Western blot with anti-FLAG antibody (M2). (C) JAK2 tyrosine phosphorylation. Cos cell lysates described in (A) were separated by SDS/PAGE and proteins analysed by Western blot with anti-phosphotyrosine antibody.

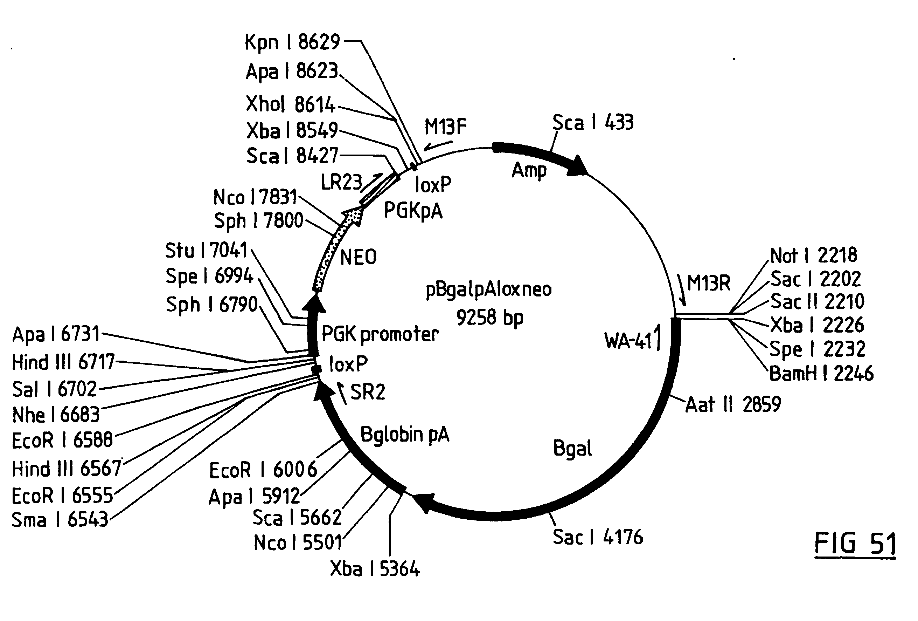

**Figure 51** is a diagrammatic representation of pβgalpAloxneo.

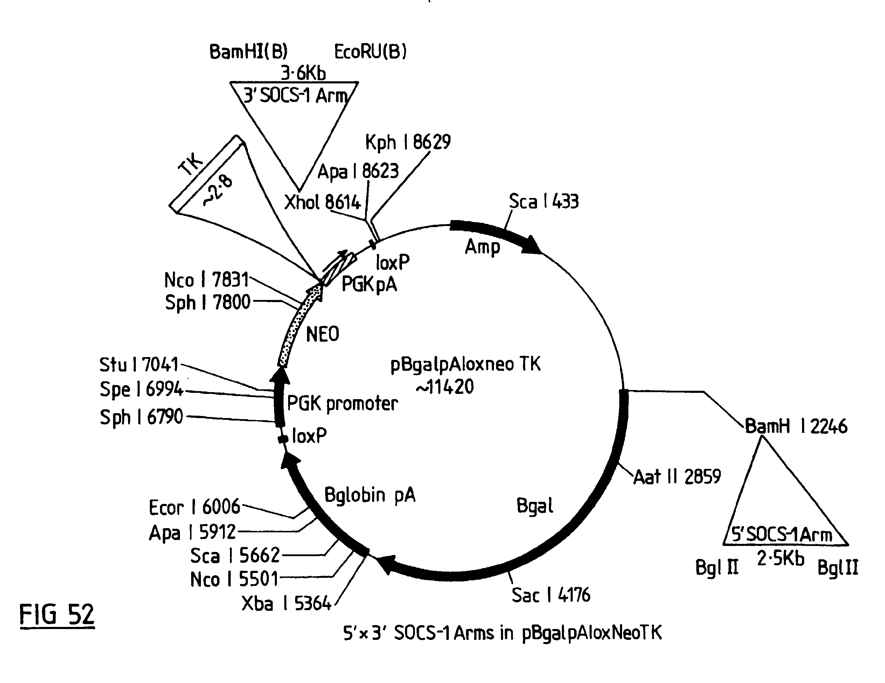

**Figure 52** is a diagrammatic representation of pβgalpAloxneoTK.

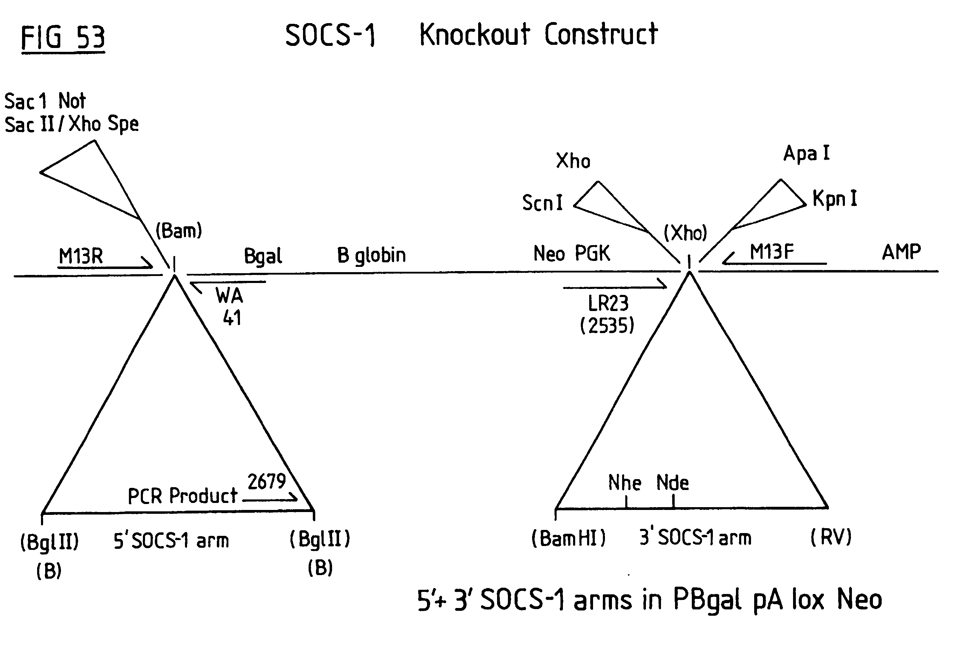

**Figure 53** is a diagrammatic representation of SOCS1 knockout construct.

### DETAILED DESCRIPTION OF PREFERRED EMBODIMENTS

The present invention concerns a new family of modulators of signal transduction. As the initial members of this family suppressed cytokine signalling, the family is referred to as the "suppressors of cytokine signalling" family of "SOCS". The SOCS family is defined by the presence of a C-terminal domain referred to as a "SOCS box". Different classes of SOCS molecules are defined by a motif generally but not exclusively located N-terminal to the SOCS box and which is involved by protein:molecule interaction such as protein:DNA or protein:protein interaction. Particularly preferred motifs are selected from an SH2 domain, WD-40 repeats and ankyrin repeats.

WD-40 repeats were originally recognised in the β-subunit of G-proteins. WD-40 repeats appear to form a β-propeller-like structure and may be involved in protein-protein interactions. Ankyrin repeats were originally recognised in the cytoskeletal protein ankryin.

Members of the SOCS family may be identified by any number of means. For example, SOCS 1 to SOCS3 were identified by their ability to suppress cytokine-mediated signal transduction and, hence, were identified based on activity. SOCS4 to SOCS 15 were identified as nucleotide sequences exhibiting similarity at the level of the SOCS box.

The SOCS box is a conserved motif located in the C-terminal region of the SOCS molecule. In accordance with the present invention, the amino acid sequence of the SOCS box is:

X₁ X₂ X₃ X₄ X₅ X₆ X₇ X₈ X₉ X₁₀ X₁₁ X₁₂ X₁₃ X₁₄ X₁₅ X₁₆ [Xⱼ]ₙ X₁₇ X₁₈ X₁₉ X₂₀ X₂₁ X₂₂ X₂₃ [Xj]ₙ X₂₄ X₂₅ X₂₆ X₂₇ X₂₈

wherein:
X₁ is L, I, V, M, A or P;
X₂ is any amino acid residue;
X₃ is P,T or S;
X₄ is L,I, V, M, A or P;
X₅ is any amino acid;
X₆ is any amino acid;
X₇ is L, I, V, M, A, F, Y or W;
X₈ is C, T or S;
X₉ is R, K or H;
X₁₀ is any amino acid;
X₁₁ is any amino acid;
X₁₂ is L, I, V, M, A or P;
X₁₃ is any amino acid;
X₁₄ is any amino acid;
X₁₅ is any amino acid;
X₁₆ is L, I, V, M, A, P, G, C, T or S;
[Xᵢ]ₙ is a sequence of n amino acids wherein n is from 1 to 50 amino acids and wherein the sequence Xᵢ may comprise the same or different amino acids selected from any amino acid residue;
X_{I7}is L, I, V,M.AorP;
X₁₈ is any amino acid;
X₁₉ is any amino acid;
X₂₀ L, I. V, M, A or P;
X₂₁ is P;
X₂₂ is L, I, V, M, A, P or G;
X₂₃ is P or N;
[Xⱼ]ₙ is a sequence of n amino acids wherein n is from 1 to 50 amino acids and wherein the sequence Xⱼ may comprise the same or different amino acids selected from any amino acid residue;
X₂₄ is L, I, V, M, A or P;
X₂₅ is any amino acid;
X₂₆ is any amino acid;
X₂₇ is Y or F; and
X₂₈ is L, I, V, M, A or P.

As stated above SOCS proteins are divided into separate classes based on the presence of a protein:molecule interacting region such as but not limited to an SH2 domain, WD-40 repeats and ankyrin repeats located N-terminal of the SOCS box. The latter three domains are protein:protein interacting domains.

Examples of SH2 containing SOCS proteins include SOCS1, SOCS2, SOCS3, SOCS5, SOCS9, SOCS11 and SOCS 14. Examples of SOCS containing WD-40 repeats include SOCS4, SOCS6 and SOCS15. Examples of SOCS containing ankyrin repeats include SOCS7, SOCS10 and SOCS12.

The present invention provides *inter alia* nucleic acid molecules encoding SOCS-3 proteins, purified naturally occurring SOCS-3 proteins as well as recombinant forms of SOCS-3 proteins and methods of modulating signal transduction by modulating activity of SOCS-3 proteins or expression of SOCS-3 genes. Preferably, signal transduction is mediated by a cytokine, examples of which include EPO, TPO, G-CSF, GM-CSF, IL-3, IL-2, IL-4, IL-7, IL-13, IL-6, LIF, IL-12, IFNγ, TNFα, IL-1 and/or M-CSF. Particularly preferred cytokines include OSM, IL-6 and LIF.

Accordingly, one aspect of the present invention provides a nucleic acid molecule comprising a nucleotide sequence encoding, or complementary to a sequence encoding, a protein which comprises the amino acid sequence of SEQ ID NO. 8 or an amino acid sequence having at least 70% similarity to SEQ ID No. 8, wherein said protein comprises a SOCS box in its C-terminal region and modulates signal transduction. Optionally the protein may include a protein:molecule interacting domain N-terminal of the SOCS box.

Preferably, the protein:molecule interacting domain is a protein:DNA or protein:protein interacting domain. Most preferably, the protein:molecule interacting domain is an SH2 domain.

As stated above, preferably the subject SOCS modulate cytokine-mediated signal transduction. The present invention extends, however, to SOCS molecules modulating other effector-mediated signal transduction such as mediated by other endogenous or exogenous molecules, antigens, microbes and microbial products, viruses or components thereof, ions, hormones and parasites. Endogenous molecules in this context are molecules produced within the cell carrying the SOCS molecule. Exogenous molecules are produced by other cells or are introduced to the body.

The nucleic acid molecule or SOCS protein is in isolated or purified form. The terms "isolated" and "purified" mean that a molecule has undergone at least one purification step away from other material.

Preferably, the nucleic acid molecule is in isolated form and is DNA such as cDNA or genomic DNA. The DNA may encode the same amino acid sequence as the naturally occurring SOCS or the SOCS may contain one or more amino acid substitutions, deletions and/or additions. The nucleotide sequence may correspond to the genomic coding sequence (including exons and introns) or to the nucleotide sequence in cDNA from mRNA transcribed from the genomic gene or it may carry one or more nucleotide substitutions, deletions and/or additions thereto.

In a preferred embodiment, the present invention provides a nucleic acid molecule comprising a nucleotide sequence as set forth in SEQ ID NO:7 (mSOCS3) or a nucleotide sequence having at least 70% similarity to SEQ ID NO:7 or a nucleic acid molecule capable of hybridizing to SEQ ID NO:7 under medium stringency conditions at 42°C.

Reference herein to a low stringency at 42°C includes and encompasses from at least about 1% v/v to at least about 15% v/v formamide and from at least about 1M to at least about 2M salt for hybridisation and at least about 1M to at least about 2M salt for washing conditions. Alternative stringency conditions may be applied where necessary, such as medium stringency, which includes and encompasses from at least about 16% v/v to at least about 30% v/v formamide and from at least about 0.5M to at least about 0.9M salt for hybridisation, and at least about 0.5M to at least about 0.9M salt for washing conditions, or high stringency, which includes and encompasses from at least about 31% v/v to at least about 50% v/v formamide and from at least about 0.01M to at least about 0.15M salt for hybridisation, and at least about 0.01M to at least about 0.15M salt for washing conditions.

Preferred nucleotide percentage similarities include at least about 80%, at least about 90% or above such as 93%, 95%, 98% or 99%.

Preferred amino acid similarities include at least about 80%, at least about 90%, at least about 95%, at least about 97% or 98% or above.

Similarity may be measured against an entire molecule or a region comprising at least 21 nucleotides or at least 7 amino acids. Preferably, similarity is measured in a conserved region such as SH2 domain or other protein:molecule interacting domains or a SOCS box.

The term "similarity" includes exact identity between sequences or, where the sequence differs, different amino acids that are related to each other at the structural, functional, biochemical and/or conformational levels.

The nucleic acid molecule may be isolated from any animal such as humans, primates, livestock animals (e.g. horses, cows, sheep, donkeys, pigs), laboratory test animals (e.g. mice, rats, rabbits, hamsters, guinea pigs), companion animals (e.g. dogs, cats) or captive wild animals (e.g. deer, foxes, kangaroos).

The terms "derivatives" or its singular form "derivative" whether in relation to a nucleic acid molecule or a protein includes parts, mutants, fragments and analogues as well as hybrid or fusion molecules and glycosylation variants. Derivatives included within the scope of the present invention are as defined in the claims by reference to % sequence similarity or by reference to hybridisation. Particularly useful derivatives comprise single or multiple amino acid substitutions, deletions and/or additions to the SOCS amino acid sequence.

A derivative has functional activity or alternatively act as antagonists or agonists. Homologues of SOCS include the functionally or structurally related molecule from different animal species. Homologues and analogues included within the present invention are those as defined in the claims by reference to % sequence similarity or by reference to hybridisation.

As stated above, the present invention contemplates antagonists of the SOCS-3 which is an antisense oligonucleotide sequence. Useful oligonucleotides are those which have a nucleotide sequence complementary to at least a portion of the protein-coding or "sense" sequence of the nucleotide sequence. These anti-sense nucleotides can be used to effect the specific inhibition of gene expression. The antisense approach can cause inhibition of gene expression apparently by forming an anti-parallel duplex by complementary base pairing between the antisense construct and the targeted mRNA, presumably resulting in hybridisation arrest of translation. Ribozymes and co-suppression molecules may also be used. Antisense and other nucleic acid molecules may first need to be chemically modified to permit penetration of cell membranes and/or to increase their serum half life or otherwise make them more stable for *in vivo* administration. Antibodies may also act as either antagonists or agonists although are more useful in diagnostic applications or in the purification of SOCS proteins. Antagonists and agonists may also be identified following natural product screening or screening of libraries of chemical compounds or may be derivatives or analogues of the SOCS molecules.

Analogues of the SOCS proteins may be used, for example, in the treatment or prophylaxis of cytokine mediated dysfunction such as autoimmunity, immune suppression or hyperactive immunity or other condition including but not limited to dysfunctions in the haemopoietic, endocrine, hepatic and neural systems. Dysfunctions mediated by other signal transducing elements such as hormones or endogenous or exogenous molecules, antigens, microbes and microbial products, viruses or components thereof, ions, hormones and parasites.

Analogues of the proteins contemplated herein include, but are not limited to, modification to side chains, incorporating of unnatural amino acids and/or their derivatives during peptide, polypeptide or protein synthesis and the use of crosslinkers and other methods which impose conformational constraints on the proteinaceous molecule or their analogues.

Examples of side chain modifications contemplated by the present invention include modifications of amino groups such as by reductive alkylation by reaction with an aldehyde followed by reduction with NaBH₄; amidination with methylacetimidate; acylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5-phosphate followed by reduction with NaBH₄.

The guanidine group of arginine residues may be modified by the formation of heterocyclic condensation products with reagents such as 2,3-butanedione, phenylglyoxal and glyoxal.

The carboxyl group may be modified by carbodiimide activation via O-acylisourea formation followed by subsequent derivitisation, for example, to a corresponding amide.

Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides.

Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. A list of unnatural amino acid, contemplated herein is shown in Table 3.

**TABLE 3**

| Non-conventional amino acid | Code | Non-conventional amino acid | Code |
|---|---|---|---|
| α-aminobutyric acid | Abu | L-N-methylalanine | Nmala |
| α-amino-α-methylbutyrate | Mgabu | L-N-methylarginine | Nmarg |
| aminocyclopropane- | Cpro | L-N-methylasparagine | Nmasn |
| carboxylate | | L-N-methylaspartic acid | Nmasp |
| aminoisobutyric acid | Aib | L-N-methylcysteine | Nmcys |
| aminonorbornyl- | Norb | L-N-methylglutamine | Nmgln |
| carboxylate | | L-N-methylglutamic acid | Nmglu |
| cyclohexylalanine | | Chexa L-N-methylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmorn |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-γ-aminobutyrate | Mgabu |
| D-α-methylalanine | Dmala | α-methylcyclohexylalanine | Mchexa |
| D-α-methylarginine | Dmarg | α-methylcylcopentylalanine | Mcpen |
| D-α-methylasparagine | Dmasn | α-methyl-α-napthylalanine | Manap |
| D-α-methylaspartate | Dmasp | α-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Nom |
| D-α-methylisoleucine | Dmile | N-amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbamylethyl)glycine | Ngln |
| D-α-methylomithine | Dmom | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile | N-(imidazolylethyl))glycine | Nhis |
| D-N-methylleucine | Dnmleu | N-(3-indolylyethyl)glycine | Nhtrp |
| D-N-rnethyllysine | Dnmlys | N-methyl-γ-aminobutyrate | Nmgabu |
| N-methylcyclohexylalanine | Nmchexa | D-N-methylmethionine | Dnmmet |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| γ-anunobutyric acid | Gabu | N-(*p*-hydroxyphenyl)glycine | Nhtyr |
| L-t-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-t-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mnva | L-α-methylornithine | Morn |
| L-α-methylphenylalanine | Mphe | L-α-methylproline | Mpro |
| L-α-methylserine | Mser | L-α-methylthreonine | Mthr |
| L-α-methyltryptophan | Mtrp | L-α-methyltymsine | Mtyr |
| L-α-methylvaline | Mval | L-N-methylhomophenylalanine | Nmhphe |
| N-(N-(2,2-diphenylethyl) | Nnbhm | N-(N-(3,3-diphenylpropyl) | Nnbhe |
| carbamylmethyl)glycine | | carbamylmethyl)glycine | |
| 1 -carboxy-1 -(2,2-diphenyl- | Nmbc | | |
| ethylamino)cyclopropane | | | |

Crosslinkers can be used, for example, to stabilise 3D conformations, using homo-bifunctional crosslinkers such as the bifunctional imido esters having (CH₂)ₙ spacer groups with n=1 to n=6, glutaraldehyde, N-hydroxysuccinimide esters and hetero-bifunctional reagents which usually contain an amino-reactive moiety such as N-hydroxysuccinimide and another group specific-reactive moiety such as maleimido or dithio moiety (SH) or carbodiimide (COOH). In addition, peptides can be conformationally constrained by, for example, incorporation of C_{α} and N_{α}-methylamino acids, introduction of double bonds between C_{α} and C_{β} atoms of amino acids and the formation of cyclic peptides or analogues by introducing covalent bonds such as forming an amide bond between the N and C termini, between two side chains or between a side chain and the N or C terminus.

These types of modifications may be important to stabilise the cytokines if administered to an individual or for use as a diagnostic reagent.

Other derivatives include a range of glycosylation variants from a completely unglycosylated molecule to a modified glycosylated molecule. Altered glycosylation patterns may result from expression of recombinant molecules in different host cells.

A method for modulating expression of a SOCS protein in a mammal may comprise contacting a gene encoding a SOCS or a factor/element involved in controlling expression of the SOCS gene with an effective amount of a modulator of SOCS expression for a time and under conditions sufficient to up-regulate or down-regulate or otherwise modulate expression of SOCS. An example of a modulator is a cytokine such as IL-6 or other transcription regulators of SOCS expression.

Expression includes transcription or translation or both.

A method of modulating activity of SOCS in a human may comprise administering to said mammal a modulating effective amount of a molecule for a time and under conditions sufficient to increase or decrease SOCS activity. The molecule may be a proteinaceous molecule or a chemical entity and may also be a derivative of SOCS or a chemical analogue or truncation mutant of SOCS.

A method of inducing synthesis of a SOCS or transcription/translation of a SOCS may comprise contacting a cell containing a SOCS gene with an effective amount of a cytokine capable of inducing said SOCS for a time and under conditions sufficient for said SOCS to be produced. For example, SOCS1 may be induced by IL-6.

A method of modulating levels of a SOCS protein in a cell may comprise contacting a cell containing a SOCS gene with an effective amount of a modulator of SOCS gene expression or SOCS protein activity for a time and under conditions sufficient to modulate levels of said SOCS protein.

A method of modulating signal transduction in a cell containing a SOCS gene may comprise contacting said cell with an effective amount of a modulator of SOCS gene expression or SOCS protein activity for a time sufficient to modulate signal transduction.

A method of influencing interaction between cells wherein at least one cell carries a SOCS gene may comprise contacting the cell carrying the SOCS gene with an effective amount of a modulator of SOCS gene expression or SOCS protein activity for a time sufficient to modulate signal transduction.

A range of mimetics or small molecules may be capable of acting as agonists or antagonists of the SOCS. Such molecules may be obtained from natural product screening such as from coral, soil, plants or the ocean or antarctic environments. Alternatively, peptide, polypeptide or protein libraries or chemical libraries may be readily screened. For example, M1 cells expressing a SOCS do not undergo differentiation in the presence of IL-6. This system can be used to screen molecules which permit differentiation in the presence of IL-6 and a SOCS. A range of test cells may be prepared to screen for antagonists and agonists for a range of cytokines. Such molecules are preferably small molecules and may be of amino acid origin or of chemical origin. SOCS molecules interacting with signalling proteins (eg. JAKS) provide molecular screens to detect molecules which interfere or promote this interaction. Once such screening protocol involves natural product screening.

Accordingly, the present invention contemplates a pharmaceutical composition comprising a SOCS-3 protein as hereinbefore defined or a modulator of SOCS-3 expression or SOCS-3 activity and one or more pharmaceutically acceptable carriers and/or diluents. These components are referred to as the "active ingredients".

The pharmaceutical forms containing active ingredients suitable for injectable use include sterile aqueous solutions (where water soluble) sterile powders for the extemporaneous preparation of sterile injectable solutions. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol and liquid polyethylene glycol, and the like), suitable mixtures thereof, and vegetable oils. The proper fluidity can be maintained, for example, by the use of a coating such as licithin, by the maintenance of the required particle size in the case of dispersion and by the use of superfactants. The preventions of the action of microorganisms can be brought about by various antibacterial and antifungal agents, for example, parabens, chlorobutanol, phenol, sorbic acid, thirmerosal and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

When the active ingredients are suitably protected they may be orally administered, for example, with an inert diluent or with an assimilable edible carrier, or it may be enclosed in hard or soft shell gelatin capsule, or it may be compressed into tablets. For oral therapeutic administration, the active compound may be incorporated with excipients and used in the form of ingestible tablets, buccal tablets, troches, capsules, elixirs, suspensions, syrups, wafers and the like. Such compositions and preparations should contain at least 1 % by weight of active compound. The percentage of the compositions and preparations may, of course, be varied and may conveniently be between about 5 to about 80% of the weight of the unit. The amount of active compound in such therapeutically useful compositions in such that a suitable dosage will be obtained. Preferred compositions or preparations according to the present invention are prepared so that an oral dosage unit form contains between about 0.1 µg and 2000 mg of active compound.

The tablets, troches, pills, capsules and the like may also contain the components as listed hereafter. A binder such as gum, acacia, corn starch or gelatin; excipients such as dicalcium phosphate; a disintegrating agent such as corn starch, potato starch, alginic acid and the like; a lubricant such as magnesium stearate; and a sweetening agent such a sucrose, lactose or saccharin may be added or a flavouring agent such as peppermint, oil of wintergreen or cherry flavouring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compound(s) may be incorporated into sustained-release preparations and formulations.

The present invention also extends to forms suitable for topical application such as creams, lotions and gels.

Pharmaceutically acceptable carriers and/or diluents include any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, use thereof in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore disclosed. A unit dosage form can, for example, contain the principal active compound in amounts ranging from 0.5 µg to about 2000 mg. Expressed in proportions, the active compound is generally present in from about 0.5 µg to about 2000 mg/ml of carrier. In the case of compositions containing supplementary active ingredients, the dosages are determined by reference to the usual dose and manner of administration of the said ingredients. The effective amount may also be conveniently expressed in terms of an amount per kg of body weight. For example, from about 0.01 ng to about 10,000 mg/kg body weight may be administered.

The pharmaceutical composition may also comprise genetic molecules such as a vector capable of transfecting target cells where the vector carries a nucleic acid molecule capable of modulating SOCS expression or SOCS activity. The vector may, for example, be a viral vector. In this regard, a range of gene therapies are enabled by the present invention including isolating certain cells, genetically manipulating and returning the cell to the same subject or to a genetically related or similar subject.

Still another aspect of the present invention is directed to antibodies to SOCS-3 and its derivatives. Such antibodies may be monoclonal or polyclonal and may be selected from naturally occurring antibodies to SOCS-3 or may be specifically raised to SOCS-3 or derivatives thereof. In the case of the latter, SOCS-3 or its derivatives may first need to be associated with a carrier molecule. The antibodies and/or recombinant SOCS-3 or its derivatives of the present invention are particularly useful as therapeutic or diagnostic agents.

For example, SOCS and its derivatives can be used to screen for naturally occurring antibodies to SOCS. These may occur, for example in some autoimmune diseases. Alternatively, specific antibodies can be used to screen for SOCS. Techniques for such assays are well known in the art and include, for example, sandwich assays and ELISA. Knowledge of SOCS levels may be important for diagnosis of certain cancers or a predisposition to cancers or monitoring cytokine mediated cellular responsiveness or for monitoring certain therapeutic protocols.

Antibodies to SOCS-3 of the present invention may be monoclonal or polyclonal. Alternatively, fragments of antibodies may be used such as Fab fragments. Furthermore, the present invention extends to recombinant and synthetic antibodies and to antibody hybrids. A "synthetic antibody" is considered herein to include fragments and hybrids of antibodies. The antibodies of this aspect of the present invention are particularly useful for immunotherapy and may also be used as a diagnostic tool for assessing apoptosis or monitoring the program of a therapeutic regimen.

For example, specific antibodies can be used to screen for SOCS proteins. The latter would be important, for example, as a means for screening for levels of SOCS in a cell extract or other biological fluid or purifying SOCS made by recombinant means from culture supernatant fluid. Techniques for the assays contemplated herein are known in the art and include, for example, sandwich assays and ELISA.

It is within the scope of this invention to include any second antibodies (monoclonal, polyclonal or fragments of antibodies or synthetic antibodies) directed to the first mentioned antibodies discussed above. Both the first and second antibodies may be used in detection assays or a first antibody may be used with a commercially available anti-immunoglobulin antibody. An antibody as contemplated herein includes any antibody specific to any region of SOCS-3.

Both polyclonal and monoclonal antibodies are obtainable by immunization with the enzyme or protein and either type is utilizable for immunoassays. The methods of obtaining both types of sera are well known in the art. Polyclonal sera are less preferred but are relatively easily prepared by injection of a suitable laboratory animal with an effective amount of SOCS, or antigenic parts thereof, collecting serum from the animal, and isolating specific sera by any of the known immunoadsorbent techniques. Although antibodies produced by this method are utilizable in virtually any type of immunoassay, they are generally less favoured because of the potential heterogeneity of the product.

The use of monoclonal antibodies in an immunoassay is particularly preferred because of the ability to produce them in large quantities and the homogeneity of the product. The preparation of hybridoma cell lines for monoclonal antibody production derived by fusing an immortal cell line and lymphocytes sensitized against the immunogenic preparation can be done by techniques which are well known to those who are skilled in the art.

Another aspect of the present invention contemplates a method for detecting SOCS-3 in a biological sample from a subject said method comprising contacting said biological sample with an antibody specific for SOCS-3 as defined herein for a time and under conditions sufficient for an antibody-SOCS-3 complex to form and then detecting said complex.

The presence of SOCS may be accomplished in a number of ways such as by Western blotting and ELISA procedures. A wide range of immunoassay techniques are available as can be seen by reference to US Patent Nos. 4,016,043, 4,424,279 and 4,018,653. These, of course, include both single-site and two-site or "sandwich" assays of the non-competitive types, as well as in the traditional competitive binding assays. These assays also include direct binding of a labelled antibody to a target.

Sandwich assays are among the most useful and commonly used assays and are favoured for use in the present invention. A number of variations of the sandwich assay technique exist, and all are intended to be encompassed by the present invention. Briefly, in a typical forward assay, an unlabelled antibody is immobilized on a solid substrate and the sample to be tested brought into contact with the bound molecule. After a suitable period of incubation, for a period of time sufficient to allow formation of an antibody-antigen complex, a second antibody specific to the antigen, labelled with a reporter molecule capable of producing a detectable signal is then added and incubated, allowing time sufficient for the formation of another complex of antibody-antigen-labelled antibody. Any unreacted material is washed away, and the presence of the antigen is determined by observation of a signal produced by the reporter molecule. The results may either be qualitative, by simple observation of the visible signal, or may be quantitated by comparing with a control sample containing known amounts of hapten. Variations on the forward assay include a simultaneous assay, in which both sample and labelled antibody are added simultaneously to the bound antibody. These techniques are well known to those skilled in the art, including any minor variations as will be readily apparent. In accordance with the present invention the sample is one which might contain SOCS including cell extract, tissue biopsy or possibly serum, saliva, mucosal secretions, lymph, tissue fluid and respiratory fluid. The sample is, therefore, generally a biological sample comprising biological fluid but also extends to fermentation fluid and supernatant fluid such as from a cell culture.

In the typical forward sandwich assay, a first antibody having specificity for the SOCS or antigenic parts thereof, is either covalently or passively bound to a solid surface. The solid surface Is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (e.g. 2-40 minutes or overnight if more convenient) and under suitable conditions (e.g. room temperature to 37°C) to allow binding of any subunit present in the antibody. Following the incubation period, the antibody subunit solid phase is washed and dried and incubated with a second antibody specific for a portion of the hapten. The second antibody is linked to a reporter molecule which is used to indicate the binding of the second antibody to the hapten.

An alternative method involves immobilizing the target molecules in the biological sample and then exposing the immobilized target to specific antibody which may or may not be labelled with a reporter molecule. Depending on the amount of target and the strength of the reporter molecule signal, a bound target may be detectable by direct labelling with the antibody. Alternatively, a second labelled antibody, specific to the first antibody is exposed to the target-first antibody complex to form a target-first antibody-second antibody tertiary complex. The complex is detected by the signal emitted by the reporter molecule.

By "reporter molecule" as used in the present specification, is meant a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (i.e. radioisotopes) and chemiluminescent molecules.

In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, beta-galactosidase and alkaline phosphatase, amongst others. The substrates to be used with the specific enzymes are generally chosen for the production, upon hydrolysis by the corresponding enzyme, of a detectable colour change. Examples of suitable enzymes include alkaline phosphatase and peroxidase. It is also possible to employ fluorogenic substrates, which yield a fluorescent product rather than the chromogenic substrates noted above. In all cases, the enzyme-labelled antibody is added to the first antibody hapten complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of hapten which was present in the sample. "Reporter molecule" also extends to use of cell agglutination or inhibition of agglutination such as red blood cells on latex beads, and the like.

Alternately, fluorescent compounds, such as fluorescein and rhodamine, may be chemically coupled to antibodies without altering their binding capacity. When activated by illumination with light of a particular wavelength, the fluorochrome-labelled antibody adsorbs the light energy, inducing a state to excitability in the molecule, followed by emission of the light at a characteristic colour visually detectable with a light microscope. As in the EIA, the fluorescent labelled antibody is allowed to bind to the first antibody-hapten complex. After washing off the unbound reagent, the remaining tertiary complex is then exposed to the light of the appropriate wavelength the fluorescence observed indicates the presence of the hapten of interest. Immunofluorescene and EIA techniques are both very well established in the art and are particularly preferred for the present method. However, other reporter molecules, such as radioisotope, chemiluminescent or bioluminescent molecules, may also be employed.

Genetic assays such as involving PCR analysis may be used to detect SOCS gene or its derivatives. Alternative methods or methods used in conjunction include direct nucleotide sequencing or mutation scanning such as single stranded conformation polymorphisms analysis (SSCP) as specific oligonucleotide hybridisation, as methods such as direct protein truncation tests.

Since cytokines are involved in transcription of some SOCS molecules, the detection of SOCS provides surrogate markers for cytokines or cytokine activity. This may be useful in assessing subjects with a range of conditions such as those with autoimmune diseases, for example, rheumatoid arthritis, diabetes and stiff man syndrome amongst others.

The nucleic acid molecules of the present invention may be DNA or RNA. When the nucleic acid molecule is in DNA form, it may be genomic DNA or cDNA. RNA forms of the nucleic acid molecules of the present invention are generally mRNA.

Although the nucleic acid molecules of the present invention are generally in isolated form, they may be integrated into or ligated to or otherwise fused or associated with other genetic molecules such as vector molecules and in particular expression vector molecules. Vectors and expression vectors are generally capable of replication and, if applicable, expression in one or both of a prokaryotic cell or a eukaryotic cell. Preferably, prokaryotic cells include *E*. *coli, Bacillus sp* and *Pseudomonas sp.* Preferred eukaryotic cells include yeast, fungal, mammalian and insect cells.

Accordingly, a genetic construct may comprise a vector portion and a mammalian and more particularly a human SOCS gene portion, which SOCS gene portion is capable of encoding a SOCS polypeptide or a functional or immunologically interactive derivative thereof.

Preferably, the SOCS gene portion of the genetic construct is operably linked to a promoter on the vector such that said promoter is capable of directing expression of said SOCS gene portion in an appropriate cell.

In addition, the SOCS gene portion of the genetic construct may comprise all or part of the gene fused to another genetic sequence such as a nucleotide sequence encoding glutathione-S-transferase or part thereof.

Such genetic constructs may be contained in prokaryotic or eukaryotic cells.

The SOCS and its genetic sequence as disclosed herein will be useful in the generation of a range of therapeutic and diagnostic reagents and will be especially useful in the detection of a cytokine involved in a particular cellular response or a receptor for that cytokine. For example, cells expressing SOCS gene such as M1 cells expressing the SOCS1 gene, will no longer be responsive to a particular cytokine such as, in the case of SOCS1, IL-6. The present invention contemplates cells such as M1 cells expressing a SOCS-3 gene as herein defined. Molecules may be used that regulate or potentiate the ability of therapeutic cytokines. For example, molecules which block some SOCS activity, may act to potential therapeutic cytokine activity (e.g. G-CSF).

Soluble SOCS polypeptides may also be particularly useful in the treatment of disease, injury or abnormality involving cytokine mediated cellular responsiveness such as hyperimmunity, immunosuppression, allergies, hypertension and the like.

A further aspect of the present invention contemplates the use of SOCS-3 as hereinbefore defined in the manufacture of a medicament for the treatment of conditions involving cytokine mediated cellular responsiveness.

The present invention further contemplates transgenic mammalian cells expressing a SOCS-3 gene as hereinbefore defined. Such cells are useful indicator cell lines for assaying for suppression of cytokine function. One example is MI cells expressing a SOCS-3 gene. Such cell lines may be useful for screening for cytokines or screening molecules such as naturally occurring molecules from plants, coral, microorganisms or bio-organically active soil or water capable of acting as cytokine antagonists or agonists.

Hybrids may be made between different SOCS from the same or different animal species. For example, a hybrid may be formed between all or a functional part of mouse SOCS1 and human SOCS1. Alternatively, the hybrid may be between all or part of mouse SOCS1 and mouse SOCS2. All such hybrids are particularly useful in developing pleiotropic molecules.

A range of genetic based diagnostic assays may be used for screening for individuals with defective SOCS genes. Such mutations may result in cell types not being responsive to a particular cytokine or resulting in over responsiveness leading to a range of conditions. The SOCS genetic sequence can be readily verified using a range of PCR or other techniques to determine whether a mutation is resident in the gene. Appropriate gene therapy or other interventionist therapy may then be adopted.

The present invention is further described by the following non-limiting Examples.

Examples 1-16 relate to SOCS1, SOCS2 and SOCS3 which were identified on the basis of activity. Examples 17-24 relate to various aspects of SOCS4 to SOCS 15 which were cloned initially on the basis of sequence similarity. Examples 25-36 relate to specific aspects of SOCS4 to SOCS 15, respectively.

### EXAMPLE 1

### CELL CULTURE AND CYTOKINES

The M 1 cell line was derived from a spontaneously arising leukaemia in SL mice [Ichikawa, 1969]. Parental M 1 cells used in this study have been in passage at the Walter and Eliza Hall Institute for Medical Research, Melbourne, Victoria, Australia, for approximately 10 years. M 1 cells were maintained by weekly passage in Dulbecco's modified Eagle's medium (DME) containing 10% (v/v) foetal bovine serum (FCS). Recombinant cytokines are generally available from commercial sources or were prepared by published methods. Recombinant murine LIF was produced in *Escherichia coli* and purified, as previously described [Gearing, 1989]. Purified human oncostatin M was purchased from PeproTech Inc (Rocky Hill, NJ, USA), and purified mouse IFN-γ was obtained from Genzyme Diagnostics (Cambridge, MA, USA). Recombinant murine thrombopoietin was produced as a FLAGTM-tagged fusion protein in CHO cells and then purified.

### EXAMPLE 2

### AGAR COLONY ASSAYS

In order to assay the differentiation of M1 cells in response to cytokines, 300 cells were cultured in 35 mm Petri dishes containing 1 ml of DME supplemented with 20%(v/v) fÏtal calf serum (FCS), 0.3%(w/v) agar and 0.1 ml of serial dilutions of IL-6, LIF, OSM, IFN-γ, tpo or dexamethasone (Sigma Chemical Company, St Louis, MI). After 7 days culture at 37°C in a fully humidified atmosphere, containing 10% (v/v) CO₂ in air, colonies of M1 cells were counted and classified as differentiated if they were composed of dispersed cells or had a corona of dispersed cells around a tightly packed centre.

### EXAMPLE 3

### GENERATION OF RETROVIRAL LIBRARY

A cDNA expression library was constructed from the factor-dependent haemopoietic cell line FDC-P1, essentially as described [Rayner, 1994]. Briefly, cDNA was cloned into the retroviral vector pRUFneo and then transfected into an amphotrophic packaging cell line (PA317). Transiently generated virus was harvested from the cell supernatant at 48 hr posttransfection, and used to infect Y2 ecotropic packaging cells, to generate a high titre virus-producing cell line.

### EXAMPLE 4

### RETROVIRAL INFECTION OF M1 CELLS

Pools of 10⁶ infected Ψ2 cells were irradiated (3000 rad) and cocultivated with 10⁶ M1 cells in DME supplemented with 10%(v/v) FCS and 4 µg/ml Polybrene, for 2 days at 37°C. To select for IL-6-unresponsive clones, retrovirally-infected M1 cells were washed once in DME, and cultured at approximately 2x10⁴ cells/ml in 1 ml agar cultures containing 400 µg/ml geneticin (GibcoBRL, Grand Island, NY) and 100 ng/ml IL-6. The efficiency of infection of M1 cells was 1-2%, as estimated by agar plating the infected cells in the presence of geneticin only.

### EXAMPLE 5

### PCR

Genomic DNA from retrovirally-infected M1 cells was digested with Sac I and 1 µg of phenol/chloroform extracted DNA was then amplified by polymerase chain reaction (PCR). Primers used for amplification of cDNA inserts from the integrated retrovirus were GAG3 (5' CACGCCGCCCACGTGAAGGC 3' [SEQ ID NO:1]), which corresponds to the vector gag sequence approximately 30 bp 5' of the multiple cloning site, and HSVTK (5' TTCGCCAATGACAAGACGCT 3' [SEQ ID NO:2]), which corresponds to the pMClneo sequence approximately 200 bp 3' of the multiple cloning site. The PCR entailed an initial denaturation at 94°C for 5 min, 35 cycles of denaturation at 94°C for 1 min, annealing at 56°C for 2 min, and extension at 72°C for 3 min, followed by a final 10 min extension. PCR products were gel purified and then ligated into the pGEM-T plasmid (Promega, Madison, WI), and sequenced using an ABI PRISM Dye Terminator Cycle Sequencing Kit and a Model 373 Automated DNA Sequencer (Applied Biosystems Inc., Foster City, CA).

### EXAMPLE 6

### CLONING OF cDNAs

Independent cDNA clones encoding mouse SOCS1 were isolated from a murine thymus cDNA library essentially as described (Hilton *et al,* 1994). The nucleotide and predicted amino acid sequences of mouse SOCS1 cDNA were compared to databases using the BLASTN and TFASTA algorithms (Pearson and Lipman, 1988; Pearson, 1990; Altshcul *et al,* 1990). Oligonucleotides were designed from the ESTs encoding human SOCS1 and mouse SOC-1 and SOCS3 and used to probe commercially available mouse thymus and spleen cDNA libraries. Sequencing was performed using an ABI automated sequencer according to the manufacturer's instructions.

### EXAMPLE 7

### SOUTHERN AND NORTHERN BLOT ANALYSES AND RT-PCR

³²P-labelled probes were generated using a random decanucleotide labelling kit (Bresatec, Adelaide, South Australia) from a 600 bp Pst I fragment encoding neomycin phophotransfease from the plasmid pPGKneo, 1070 bp fragment of the SOCS 1 gene obtained by digestion of the 1.4 kbp PCR product with Xho I, SOCS2, SOCS3, CIS and a 1.2 kbp fragment of the chicken glyceraldehyde 3-phosphate dehydrogenase gene [Dugaiczyk, 1983].

Genomic DNA was isolated from cells using a proteinase K-sodium dodecyl sulfate procedure essentially as described. Fifteen micrograms of DNA was digested with either BamH I or Sac I, fractionated on a 0.8%(w/v) agarose gel, transferred to GeneScreenPlus membrane (Du Pont NEN, Boston MA), prehybridised, hybridised with random-primed ³²P-labelled DNA fragments and washed essentially as described [Sambrook, 1989].

Total RNA was isolated from cells and tissues using Trizol Reagent, as recommended by the manufacturer (GibcoBRL,Grand Island, NY). When required polya+ mRNA was purified essentially as described [Alexander, 1995]. Northern blots were prehybridised, hybridized with random-primed 32P-labelled DNA fragments and washed as described [Alexander, 1995].

To assess the induction of SOCS genes by IL-6, mice (C57BL6) were injected intravenously with 5 µg IL-6 followed by harvest of the liver at the indicated timepoints after injection. M1 cells were cultured in the presence of 20 ng/ml IL-6 and harvested at the indicated times. For RT-PCR analysis, bone marrow cells were harvested as described (Metacalf *et al,* 1995) and stimulated for 1 hr at 37°C with 100 ng/ml of a range of cytokines. RT-PCR was performed on total RNA as described (Metcalf *et al,* 1995). PCR products were resolved on an agarose gel and Southern blots were hybridised with probes specific for each SOCS family member. Expression of β-actin was assessed to ensure uniformity of amplification.

### EXAMPLE 8

### DNA CONSTRUCTS AND TRANSFECTION

A cDNA encoding epitope-tagged SOCS1 was generated by subcloning the entire SOCS1 coding region into the pEF-BOS expression vector [Mizushima, 1990], engineered to encode an inframe FLAG epitope downstream of an initiation methionine (pF-SOCS1). Using electroporation as described previously [Hilton, 1994], M1 cells expressing the thrombopoietin receptor (M1.mpl) were transfected with the 20 µg of Aat II-digested pF-SOCS1 expression plasmid and 2 *µ*g of a Sca I-digested plasmid in which transcription of a cDNA encoding puromycin N-acetyl transferase was driven from the mouse phosphoglycerokinase promoter (pPGKPuropA). After 48 hours in culture, transfected cells were selected with 20 µg/ml puromycin (Sigma Chemical Company, St Louis MO), and screened for expression of SOCS1 by Western blotting, using the M2 anti-FLAG monoclonal antibody according to the manafacturer's instructions (Eastman Kodak, Rochester NY). In other experiments M 1 cells were transfected with only the pF-SOCS 1 plasmid or a control and selected by their ability to grow in agar in the presence of 100 ng/ml of IL-6.

### EXAMPLE 9

### IMMUNOPRECIPITATION AND WESTERN BLOTTING

Prior to either immunoprecipitaion or Western blotting, 10⁷ M1 cells or their derivatives were washed twice, resuspended in 1ml of DME, and incubated at 37°C for 30 min. The cells were then stimulated for 4 min at 37°C with either saline or 100 ng/ml IL-6, after which sodium vanadate (Sigma Chemical Co., St Louis, MI) was added to a concentration of 1 mM. Cells were placed on ice, washed once with saline containing 1 mM sodium vanadate, and then solubilised for 5 min on ice with 300 µl 1% (v/v) Triton X-100, 150 mM NaCl, 2 mM EDTA, 50 mM Tris-HCl pH 7.4, containing Complete protease inhibitors (Boehringer Mannheim, Mannheim, Germany) and 1 mM sodium vanadate. Lysates were cleared by centrifugation and quantitated using a Coomassie Protein Assay Reagent (Pierce, Rockford IL).

For immunoprecipitations, equal concentrations of protein extracts (1-2 mg) were incubated for 1 hr or overnight at 4°C with either 4 µg of anti-gp130 antibody (M20; Santa Cruz Biotechnology Inc., Santa Cruz, CA) or 4 µg of anti-phosphotyrosine antibody (4G 10; Upstate Biotechnology Inc., Lake Placid NY), and 15 µl packed volume of Protein G Sepharose (Pharmacia, Uppsala, Sweden) [Hilton *et al*, 1996]. Immunoprecipitates were washed twice in 1 % (v/v) NP40, 150 mM NaCl , 50 mM Tris-HCl pH 8.0, containing Complete protease inhibitors (Boehringer Mannheim, Mannheim, Germany and 1 mM sodium vanadate. The samples were heated for 5 min at 95°C in SDS sample buffer (625 mM Tris-HCl pH 6.8, 0.05% (w/v) SDS, 0.1% (v/v) glycerol, bromophenol blue, 0.125% (v/v) 2-mercaptoethanol), fractionated by SDS-PAGE and immunoblotted as described above.

For Western blotting, 10 µg of protein from a cellular extract or material from an immunoprecipitation reaction was loaded onto 4-15% Ready gels (Bio-Rad Laboratories, Hercules CA), and resolved by sodium dodecyl sulfate polyacrylamide gel electrophoresis (SDS-PAGE). Proteins were transferred to PVDF membrane (Micron Separations Inc., Westborough MA) for 1 hr at 100 V. The membranes were probed with the following primary antibodies; anti-tyrosine phosphorylated STAT3 (1:1000 dilution; New England Biolabs, Beverly, MA); anti-STAT3 (C-20; 1:100 dilution; Santa Cruz Biotechnology Inc., Santa Cruz CA); anti-gp130 (M20, 1:100 dilution; Santa Cruz Biotechnology Inc., Santa Cruz CA); anti-phosphotyrosine (horseradish peroxidase-conjugated RC20, 1:5000 dilution; Transduction Laboratories, Lexington KY); anti-tyrosine phosphorylated MAP kinase and anti-MAP kinase antibodies (1:1000 dilution; New England Biolabs, Beverly, MA). Blots were visualised using peroxidase-conjugated secondary antibodies and Enhanced Chemiluminescence (ECL) reagents according to the manafacturer's instructions (Pierce, Rockford IL).

### EXAMPLE 10

### ELECTROPHORETIC MOBILITY SHIFT ASSAYS

Assays were performed as described [Novak, 1995], using the high affinity SIF (c-sis- inducible factor) binding site m67 [Wakao, 1994]. Protein extracts were prepared from M1 cells incubated for 4-10 min at 37°C in 10 ml serum-free DME containing either saline, 100 ng/ml IL-6 or 100 ng/ml IFN-γ. The binding reactions contained 4-6 µg protein (constant within a given experiment), 5 ng ³²P-labelled m67 oligonucleotide, and 800 ng sonicated salmon sperm DNA. For certain experiments, protein samples were preincubated with an excess of unlabelled m67 oligonucleotide, or antibodies specific for either STAT1 (Transduction Laboratories, Lexington, KY) or STAT3 (Santa Cruz Biotechnology Inc., Santa Cruz CA), as described [Novak, 1995].

Western blots were performed using anti-tyrosine phosphorylated STAT3 or anti-STAT3 (New England Biolabs, Beverly, MA) or anti-gp130 (Santa Cruz Biotechnology Inc.) as described (Nicola *et al,* 1996). EMSA were performed using the m67 oligonucleotide probe, as described (Novak *et al,* 1995).

### EXAMPLE 11

### EXPRESSION CLONING OF A NOVEL SUPPRESSOR OF CYTOKINE SIGNAL TRANSDUCTION

In order to identify cDNAs capable of suppressing cytokine signal transduction, an expression cloning approach was adopted. This strategy centred on M1 cells, a monocytic leukaemia cell line that differentiates into mature macrophages and ceases proliferation in response to the cytokines IL-6, LIF, OSM and IFN-γ, and the steroid dexamethasone. Parental M1 cells were infected with the RUFneo retrovirus, into which cDNAs from the factor-dependent haemopoietic cell line FDC-P1 had been cloned. In this retrovirus, transcription of both the neomycin resistance gene and the cloned cDNA was driven off the powerful constitutive promoter present in the retroviral LTR (Figure 1). When cultured in semi-solid agar, parental M1 cells form large tightly packed colonies. Upon stimulation with IL-6, M 1 cells undergo rapid differentiation, resulting in the formation in agar of only single macrophages or small dispersed clusters of cells. Retrovirally-infected M1 cells that were unresponsive to IL-6 were selected in semi-solid agar culture by their ability to form large, tightly packed colonies in the presence of IL-6 and geneticin. A single stable IL-6-unresponsive clone, 4A2, was obtained after examining 10⁴ infected cells.

A fragment of the neomycin phosphotransferase (neo) gene was used to probe a Southern blot of genomic DNA from clone 4A2 and this revealed that the cell line was infected with a single retrovirus containing a cDNA approximately 1.4 kbp in length (Figure 2). PCR amplification using primers from the retroviral vector which flanked the cDNA cloning site enabled recovery of a 1.4 kbp cDNA insert, which we have named suppressor of cytokine signalling-1, or SOCS 1. This PCR product was used to probe a similar Southern blot of 4A2 genomic DNA and hybridised to two fragments, one which corresponded to the endogenous SOCS 1 gene and the other, which matched the size of the band seen using the neo probe, corresponded to the SOCS 1 cDNA cloned into the integrated retrovirus (Figure 2). The latter was not observed in an M1 cell clone infected with a retrovirus containing an irrelevant cDNA. Similarly, Northern blot analysis revealed that SOCS 1 mRNA was abundant in the cell line 4A2, but not in the control infected M1 cell clone (Figure 2).

### EXAMPLE 12

### SOCS1, SOCS2, SOCS3 AND CIS DEFINE A NEW FAMILY OF SH2-CONTAINING PROTEINS

The SOCS1 PCR product was used as a probe to isolate homologous cDNAs from a mouse thymus cDNA library. The sequence of the cDNAs proved to be identical to the PCR product, suggesting that constitutive or over expression, rather than mutation, of the SOCS 1 protein was sufficient for generating an IL-6-unresponsive phenotype. Comparison of the sequence of SOCS1 cDNA with nucleotide sequence databases revealed that it was present on mouse and rat genomic DNA clones containing the protamine gene cluster found on mouse chromosome 16. Closer inspection revealed that the 1.4 kb SOCS 1 sequence was not homologous to any of the protamine genes, but rather represented a previously unidentified open reading frame located at the extreme 3' end of these clones (Figure 3). There were no regions of discontinuity between the sequences of the SOCS1 cDNA and genomic locus, suggesting that SOCS 1 is encoded by a single exon. In addition to the genomic clone containing the protamine genes, a series of murine and human expressed sequenced tags (ESTs) also revealed large blocks of nucleotide sequence identity to mouse SOCS 1. The sequence information provided by the human ESTs allowed the rapid cloning of cDNAs encoding human SOCS 1.

The mouse and rat SOCS1 gene encodes a 212 amino acid protein whereas the human SOCS 1 gene encodes a 211 amino acid protein. Mouse, rat and human SOCS 1 proteins share 95-99% amino acid identity (Figure 9). A search of translated nucleic acid databases with the predicted amino acid sequence of SOCS1 showed that it was most related to a recently cloned cytokine-inducible immediate early gene product, CIS, and two classes of ESTs. Full length cDNAs from the two classes of ESTs were isolated and found to encode proteins of similar length and overall structure to SOCS1 and CIS. These clones were given the names SOCS2 and SOCS3. Each of the four proteins contains a central SH2 domain and a C-terminal region termed the SOCS motif. The SOCS1 proteins exhibit an extremely high level of amino acid sequence similarity (95-99% identity) amongst different species. However, the forms of the SOCS 1, SOCS2, SOCS3 and CIS from the same animal, while clearly defining a new family of SH2-containing proteins, exhibited a lower amino acid identity. SOCS2 and CIS exhibit approximately 38% amino acid identity, while the remaining members of the family share approximately 25% amino acid identity (Figure 9). The coding region of the genes for SOCS1 and SOC3 appear to contain no introns while the coding region of the genes for SOCS2 and CIS contain one and two introns, respectively.

The Genbank Accession Numbers for the sequences referred to herein are mouse SOCS1 cDNA (U88325), human SOCS 1 cDNA (U88326), mouse SOCS2 cDNA (U88327), mouse SOCS3 cDNA (U88328).

### EXAMPLE 13

### CONSTITUTIVE EXPRESSION OF SOCS1 SUPPRESSES THE ACTION OF A RANGE OF CYTOKINES

To formally establish that the phenotype of the 4A2 cell line was directly related to expression of SOCS 1, and not to unrelated genetic changes which may have occurred independently in these cells, a cDNA encoding an epitope-tagged version of SOCS 1 under the control of the EF1α promoter was transfected into parental M1 cells, and M1 cells expressing the receptor for thrombopoietin, c-mpl (M1.mpl). Transfection of the SOCS 1 expression vector into both cell lines resulted in an increase in the frequency of IL-6 unresponsive M1 cells.

Multiple independent clones of M1 cells expression SOCS1, as detected by Western blot, displayed a cytokine-unresponsive phenotype that was indistinguishable from 4A2. Further, if transfectants were not maintained in puromycin, expression of SOCS 1 was lost over time and cells regained their cytokine responsiveness. In the absence of cytokine, colonies derived from 4A2 and other SOCS 1 expressing clones characteristically grew to a smaller size than colones formed by control M1 cells (Figure 10).

The effect of constitutive SOCS1 expression on the response of M1 cells to a range of cytokines was investigated using the 4A2 cell line and a clone of M1.mpl cells expressing SOCS1 (M1.mpl.SOCS1). Unlike parental M1 cells and M1.mpl cells, the two cell lines expressing SOCS 1 continued to proliferate and failed to form differentiated colonies in response to either IL-6, LIF, OSM, IFN-γ or, in the case of the M1.mpl.SOCS1 cell line, thrombopoietin (Figure 4). For both cell lines, however, a normal response to dexamethasone was observed, suggesting that SOCS1 specifically affected cytokine signal transduction rather than differentiation *per se.* Consistent with these data, while parental M1 cells and M1.mpl cells became large and vacuolated in response to IL-6, 4A2 and M1.mpl.SOCS1 cells showed no evidence of morphological differentiation in response to IL-6 or other cytokines (Figure 5).

### EXAMPLE 14

### SOCS1 INHIBITS A RANGE OF IL-6 SIGNAL TRANSDUCTION PROCESSES, INCLUDING STAT3 PHOSPHORYLATION AND ACTIVATION

Phosphorylation of the cell surface receptor component gp130, the cytoplasmic tyrosine kinase JAK1 and the transcription factor STAT3 is thought to play a central role in IL-6 signal transduction. These events were compared in the parental M1 and M1.mpl cell lines and their SOCS 1-expressing counterparts. As expected, gp130 was phosphorylated rapidly in response to IL-6 in both parental lines, however, this was reduced five- to ten-fold in the cell lines expressing SOCS1 (Figure 6). Likewise, STAT3 phosphorylation was also reduced by approximately ten-fold in response to IL-6 in those cell lines expressing SOCS 1 (Figure 6). Consistent with a reduction in STAT3 phosphorylation, activation of specific STAT DNA binding complexes, as determined by electrophoretic mobility shift assay, was also reduced. Notably, there was a reduction in the formation of SIF-A (containing STAT3), SIF-B (STAT1/STAT3 heterodimer) and SIF-C (containing STAT1), the three STAT complexes induced in M1 cells stimulated with IL-6 (Figure 7). Similarly, constitutive expression of SOCS1 also inhibited IFN-γ-stimulated formation of p91 homodimers (Figure 7). STAT phosphorylation and activation were not the only cytoplasmic processes to be effected by SOCS1 expression, as the phosphorylation of other proteins, including shc and MAP kinase, was reduced to a similar extent (Figure 7).

### EXAMPLE 15

### TRANSCRIPTION OF THE SOCS1 GENE IS STIMULATED BY IL-6 IN VITRO AND IN VIVO

Although SOCS1 can inhibit cytokine signal transduction when constitutively expressed in M1 cells, this does not necessarily indicate that SOCS 1 normally functions to negatively regulate an IL-6 response. In order to investigate this possibility the inventors determined whether transcription of the SOCS 1 gene is regulated in the response of M1 cells to IL-6 and, because of the critical role IL-6 plays in regulating the acute phase response to injury and infection, the response of the liver to intravenous injection of 5 mg IL-6. In the absence of IL-6, SOCS1 mRNA was undetectable in either M 1 cells or in the liver. However, for both cell types, a 1.4 kb SOCS 1 transcript was induced within 20 to 40 minutes by IL-6 (Figure 8). For M1 cells, where the IL-6 was present throughout the experiment, the level of SOCS 1 mRNA remained elevated (Figure 8). In contrast, IL-6 was administered in vivo by a single intravenous injection and was rapidly cleared from the circulation, resulting in a pulse of IL-6 stimulation to the liver. Consistent with this, transient expression of SOCS 1 mRNA was detectable in the liver, peaking approximately 40 minutes after injection and declining to basal levels within 4 hours (Figure 8).

### EXAMPLE 16

### REGULATION OF SOCS GENES

Since CIS was cloned as a cytokine-inducible immediate early gene the inventors examined whether SOCS1, SOCS2 and SOCS3 were similarly regulated. The basal pattern of expression of the four SOCS genes was examined by Northern blot analysis of mRNA from a variety of tissues from male and female C57B 1/6 mice (Figure 11 A). Constitutive expression of SOCS 1 was observed in the thymus and to a lesser extend in the spleen and the lung. SOCS2 expression was restricted primarily to the testis and in some animals the liver and lung; for SOCS3 a low level of expression was observed in the lung, spleen and thymus, while CIS expression was more widespread, including the testis, heart, lung, kidney and, in some animals, the liver.

The inventors sought to determine whether expression of the four SOCS genes was regulated by IL-6. Northern blots of mRNA prepared from the livers of untreated and IL-6-injected mice, or from unstimulated and IL-6-stimulated M1 cells, were hybridised with labelled fragments of SOCS1, SOCS2, SOCS3 and CIS cDNAs (Figure 11B). Expression of all four SOCS genes was increased in the liver following IL-6 injection, however the kinetics of induction appeared to differ. Expression of SOCS1 and SOCS3 was transient in the liver, with mRNA detectable after 20 minutes of IL-6 injection and declining to basal levels within 4 hours for SOCS and 8 hours for SOCS3. Induction of SOCS2 and CIS mRNA in the liver followed similar initial kinetics to that of SOCS1, but was maintained at an elevated level for at least 24 hours. A similar induction of SOCS gene mRNA was observed in other organs, notably the lung and the spleen. In contrast, in M1 cells, while SOCS 1 and CIS mRNA were induced by IL-6, no induction of either SOCS2 or SOCS3 expression was detected. This result highlights cell type-specific differences in the expression of the genes of SOCS family members in response to the same cytokine.

In order to examine the spectrum of cytokines that was capable of inducing transcription of the various members of the SOCS gene family, bone marrow cells were stimulated for an hour with a range of cytokines, after which mRNA was extracted and cDNA was synthesised. PCR was then used to assess the expression of SOCS1, SOCS2, SOCS3 and CIS (Figure 11C). In the absence of stimulation, little or no expression of any of the SOCS genes was detectable in bone marrow by PCR. Stimulation of bone marrow cells with a broad array of cytokines appeared capable of up regulating mRNA for one or more members of the SOCS family. IFNγ, for example, induced expression of all four SOCS genes, while erythropoietin, granulocyte colony-stimulating factor, granulocyte-macrophage colony stimulating factor and interleukin-3 induced expression of SOCS2, SOCS3 and CIS. Interestingly, tumor necrosis factor alpha, macrophage colony-stimulating factor and interleukin-1, which act through receptors that do not fall into the type I cytokine receptor class also appeared capable of inducing expression of SOCS3 and CIS, suggesting that SOCS proteins may play a broader role in regulating signal transduction.

As constitutive expression of SOCS 1 inhibited the response of M1 cells to a range of cytokines, the inventors examined whether phosphorylation of the cell surface receptor component gp130 and the transcription factor STAT3, which are though to play a central role in IL-6 signal transduction, were affected. These events were compared in the parental M1 and M1.mpl cell lines and their SOCS 1-expressing counterparts. As expected, gp130 was phyosphorylated rapidly in response to IL-6 in both parental lines, however, this was reduced in the cell lines expressing SOCS 1 (Figure 12A). Likewise, STAT3 phosphorylation was also reduced in response to IL-6 in those cell lines expressing SOCS 1 (Figure 12A). Consistent with a reduction in STAT3 phosphorylation, activation of specific STAT/DNA binding complexes, as determined by electrophoretic mobility shift assay, was also reduced. Notably, there was a failure to form SIF-A (containing STAT3) and SIF-B(STAT1/STAT3 heterodimer), the major STAT complexes induced in M1 cells stimulated with IL-6 (Figure 12B). Similarly, constitutive expression of SOCS 1 also inhibited IFNγ-stimulating formation of SIF-C (STAT1 homodimer, Figure 12B). These experiments are consistent with the proposal that SOCS1 inhibits signal transduction upstream of receptor and STAT phosphorylation, potentially at the level of the JAK kinases.

The ability of SOCS1 to inhibit signal transduction and ultimately the biological response to cytokines suggest that, like the,SH2-containing phosphatase SHP-1 [Ihle *et al,* 1994; Yi *et al,* 1993], the SOCS proteins may play a central role in controlling the intensity and/or duration of a cell's response to a diverse range of extracellular stimuli by suppressing the signal transduction process. The evidence provided here indicates that the SOCS family acts in a classical negative feedback loop for cytokine signal transduction. Like other genes such as OSM, expression of genes encoding the SOCS proteins is induced by cytokines through the activation of STATs. Once expressed, it is proposed that the SOCS proteins inhibit the activity of JAKs and so reduce the phosphorylation of receptors and STATs, thereby suppressing signal transduction and any ensuing biological response. Importantly, inhibition of STAT activation will, over time, lead to a reduction in SOCS gene expression, allowing cells to regain responsiveness to cytokines.

### COMPARATIVE EXAMPLE 17

### DATABASE SEARCHES

The NCBI genetic sequence database (Genbank), which encompasses the major database of expressed sequence tags (ESTs) and TIGR database of human expressed sequence tags, were searched for sequences with similarity to a concensus SOCS box sequence using the TFASTA and MOTIF/PATTERN algorithms [Pearson, 1990; Cockwell and Giles, 1989]. Using the software package SRS [Etzold *et al,* 1996], ESTs that exhibited similarity to the SOCS box (and their partners derived from sequencing the other end of cDNAs) were retrieved and assembled into contigs using Autoassembler (Applied Biosystems, Foster City, CA). Consensus nucleotide sequences derived from overlapping ESTs were then used to search the various databases using BLASTN [Altschul *et al,* 1990]. Again, positive ESTs were retrieved and added to the contig. This process was repeated until no additional ESTs could be recovered. Final consensus nucleotide sequences were then translated using Sequence Navigator (Applied Biosystems, Foster City, CA).

The ESTs encoding the new SOCS proteins are as follows: **human SOCS4** (EST81149, EST180909, EST182619, ya99H09, ye70co4, yh53c09, yh77g11, yh87h05, yi45h07, yj04e06, yq12h06, yq56a06, yq60e02, yq92g03, yq97h06, yr90f01, yt69c03. yv30a08, yv55fU7, yv57h09, yv87h02, yv98e11, yw68d10, yw82a03, yx08a07, yx72h06, yx76b09, yy37h08, yy66b02, za81f08, zb18f07, zc06e08, zd14g06, zd5lhl2, zd52b09, ze25g11, ze69f02, zf54f03, zh96e07. zv66h12, zs83a08 and zs83g08). **mouse SOCS-4** (mc65f04, mf42e06, mp10c10, mr81g09. and mt19h12). **human SOCS-5** (EST15B103, EST15B105, EST27530 and zf50f01). **mouse SOCS-5** (mc55a01, mh98f09. my26h12 and ve24e06). **human SOCS-6** (yf61e08, yf93a09, yg05f12, yg41f04, yg45c02, yh11f10, yh13b05. zc35a12, ze02h08. z109a03, z169e10, zn39d08 and zo39e06). **mouse SOCS-6** (mc04c05, md48a03, mf31d03, mh26b07, mh78e11, mh88h09, mh94h07, mi27h04 and mj29c05, mp66g04, mw75g03, va53b05, vb34h02, vc55d07, vc59e05, vc67d03, vc68d10, vc97h01, vc99c08, vd07h03, vd08c01, vd09b12, vd19b02, vd29a04 and vd46d06). **human SOCS-7** (STS WI30171, EST00939, EST12913, yc29b05, yp49f10, zt10f03 and zx73g04). **mouse SOCS-7** (mj39a01 and vi52h07). **mouse SOCS-8** (mj6e09 and vj27a029). **human SOCS-9** (CSRL-82f2-u, EST114054, yy06b07, yy06g06, zr40c09, zr72h01, yx92c08, yx93b08 and hfe0662). **mouse SOCS-9** (me65d05). **human SOCS-10** (aa48h10, zp35h01, zp97h12, zq08h01, zr34g05, EST73000 and HSDHEI005). **mouse SOCS-10** (mb14d12, mb40f06, mg89b11, mq89e12, mp03g12 and vh53c11). **human SOCS-11** (zt24h06 and zr43b02). **human SOCS-13** (EST59161). **mouse SOCS-13** (ma39a09. me60c05, mi78g05, mk10c11, mo48g12, mp94a01, vb57c07 and vh07c11). **human SOCS-14** (mi75e03, vd29h11 and vd53g07).

### COMPARATIVE EXAMPLE 18

### cDNA CLONING

Based on the concensus sequences derived from overlapping ESTs, oligonucleotides were designed that were specific to various members of the SOCS family. As described above, oligonucleotides were labelled and used to screen commerically available genomic and cDNA libraries cloned with λ bacteriophage. Genomic and/or cDNA clones covering the entire coding region of mouse SOCS4, mouse SOCS5 and mouse SOCS6 were isolated. The entire gene for SOCS 15 is on the human 12p13 BAC (Genbank Accession Number HSU47924) and the mouse chromosome 6 BAC (Genbank Accession Number AC002393). Partial cDNAs for mouse SOCS7, SOCS9, SOCS10, SOCS11. SOCS12, SOCS 13 and SOCS 14 were also isolated.

### COMPARATIVE EXAMPLE 19

### NORTHERN BLOTS AND rtPCR

Northern blots were performed as described above. The sources of hybridisation probes were as follows; (i) the entire coding region of the mouse SOCS1 cDNA, (ii) a 1059 bp PCR product derived from coding region of SOCS5 upstream of the SH2 domain, (iii) the entire coding region of the mouse SOCS6 cDNA, (iv) a 790 bp PCR, product derived from the coding region of a partial SOCS7 cDNA and (v) a 1200 bp Pst I fragment of the chicken glyceraldehyde 3-phosphate dehydrogenase (GAPDH) cDNA.

### COMPARATIVE EXAMPLE 20

### ADDITIONAL MEMBERS OF SOCS FAMILY

SOCS1, SOCS2 and SOCS3 are members of the SOCS protein family identified in Examples 1-16. Each contains a central SH2 domain and a conserved motif at the C-terminus, named the SOCS box. In order to isolate further members of this protein family, various DNA databases were searched with the amino acid sequence corresponding to conserved residues of the SOCS box. This search revealed the presence of human and mouse ESTs encoding twelve further members of the SOCS protein family (Figure 13). Using this sequence information cDNAs encoding SOCS4, SOCS5, SOCS6, SOCS7, SOCS9, SOCS 10, SOCS 11, SOCS12, SOCS13, SOCS 14 and SOCS15 have been isolated. Further analysis of contigs derived from ESTs and cDNAs revealed that the SOCS proteins could be placed into three groups according to their predicted structure N-terminal of the SOCS box. The three groups are those with (i) SH2 domains, (ii) WD-40 repeats and (iii) ankyrin repeats.

### EXAMPLE 21

### SOCS PROTEIN WITH SH2 DOMAINS

Eight SOCS proteins with SH2 domains have been identified. These include SOCS 1, SOCS2 and SOCS3, SOCS5, SOCS9, SOCS11 and SOCS14 (Figure 13). Full length cDNAs were isolated for mouse SOCS5 and SOCS14 and partial clones encoding mouse SOCS9 and SOCS 14. Analysis of primary amino acid sequence and genomic structure suggest that pairs of these proteins (SOCS 1 and SOCS3, SOCS2 and CIS, SOCS5 and SOCS 14 and SOCS9 and SOCS11) are most closely related (Figure 13). Indeed, the SH2 domains of SOCS5 and SOCS14 are almost identical (Figure 13B), and unlike CIS, SOCS1. SOCS2 and SOCS3, SOCS5 and SOCS14 have an extensive, though less well conserved, N-terminal region preceding their SH2 domains (Figure 13A).

### COMPARATIVE EXAMPLE 22

### SOCS PROTEINS WITH WD-40 REPEATS

Four SOCS proteins with WD-40 repeats were identified. As with the SOCS proteins with SH2 domains, pairs of these proteins appeared to be closely related. Full length cDNAs of mouse SOCS4 and SOCS6 were isolated and shown to encode proteins containing eight WD-40 repeats N-terminal of the SOCS box (Figure 13) and SOCS4 and SOCS6 share 65% amino acid similarity. SOCS 15 was recognised as an open reading frame upon sequencing BACs from human chromosome 12p13 and the syntenic region of mouse chromosome 6 [Ansari-Lari *et al*, 1997]. In the human, chimp and mouse, SOCS 15 is encoded by a gene with two coding exons that lies within a few hundred base pairs of the 3' end of the triose phosphate isomerase (TPI) gene, but which is encoded on the opposite strand to TPI (9). In addition to a C-terminal SOCS box, the SOCS15 protein contains four WD-40 repeats. Interestingly, within the EST databases, there is a sequence of a nematode, an insect and a fish relative of SOCS 15. SOCS 15 appears most closely related to SOCS 13.

### COMPARATIVE EXAMPLE 23

### SOCS PROTEINS WITH ANKYRIN REPEATS

Three SOCS proteins with ankyrin repeats were identified. Analysis of partial cDNAs of mouse SOCS7, SOCS 10 and SOCS 12 demonstrated the presence of multiple ankyrin repeats.

### COMPARATIVE EXAMPLE 24

### EXPRESSION PATTERN OF SOCS PROTEINS

The expression of mRNA from representative members of each class of SOCS proteins - SOCS1 and SOCS5 from the SH2 domain group, SOCS6 from the WD-40 repeat group and SOCS7 from the ankyrin repeat group was examined. As shown above, SOCS1 mRNA is found in abundance in the thymus and at lower levels in other adult tissues.

Since transcription of the SOCS1 gene is induced by cytokines, the inventors sought to determine whether levels of SOCS5. SOCS6 and SOCS7 mRNA increased upon cytokine stimulation. In the livers of mice injected with IL-6, SOCS 1 mRNA is detectable after 20 min and decreases to background levels within 2 hours. In contrast, the kinetics of SOCS5 mRNA expression are quite different, being only detectable 12 to 24 hours after IL-6 injection. SOCS6 mRNA appears to be expressed constitutively while SOCS7 mRNA was not detected in the liver either before injection of IL-6 or at any time after injection.

Expression of these genes was also examined after cytokine stimulation of the factor-dependent cell line FDCP-1 engineered to express bcl-w. Again, while SOCS6 mRNA was expressed constitutively.

### COMPARATIVE EXAMPLE 25

### SOCS4

Mouse and human SOCS4 were recognized through searching EST databases using the SOCS box consensus (Figure 13). Those ESTs derived from mouse and human SOCS4 cDNAs are tabulated below (Tables 4.1 and 4.2). Using sequence information derived from mouse ESTs several oligonucleotides were designed and used to screen, in the conventional manner, a mouse thymus cDNA library cloned into λ-bacteriophage. Two cDNAs encoding mouse SOCS4 were isolated and sequenced in their entirety (Figure 15) and shown to overlap the mouse ESTs identified in the database (Table 4.1 and Figure 17). These cDNAs include a region of 5' untranslated region, the entire mouse SOCS4 coding region and a region of 3' untranslated region (Figure 17). Analysis of the sequence confirms that the SOCS4 cDNA encodes a SOCS Box at its C-terminus and a series of 8 WD-40 repeats before the SOCS Box (Figures 17 and 16). The relationship of the two sequence contigs of human SOCS4 (h4.1 and h4.2) to the experimentally determined mouse SOCS4 cDNA sequence is shown in Figure 17. The nucleotide sequence of the two human contigs is listed in Figure 18.

SEQ ID NO:13 and 14 represent the nucleotide sequence of murine SOCS4 and the corresponding amino acid sequence. SEQ ID NOs: 15 and 16 are SOCS4 cDNA human contigs h4.1 and h4.2, respectively.

### COMPARATIVE EXAMPLE 26

### SOCS5

Mouse and human SOCS5 were recognized through searching EST databases using the SOCS box consensus. (Figure 13). Those ESTs derived from mouse and human SOCSS cDNAs are tabulated below (Tables 5.1 and 5.2). Using sequence information derived from mouse and human ESTs, several oligonucleotides were designed and used to screen, in the conventional manner, a mouse thymus cDNA library, a mouse genomic DNA library and a human thymus cDNA library cloned into λ-bacteriophage . A single genomic DNA clone (57-2) and (5-3-2) cDNA clone encoding mouse SOCS5 were isolated and sequenced in their entirety and shown to overlap with the mouse ESTs identified in the database (Figures 19 and 20A). The entire coding region, in addition to a region of 5' and 3' untranslated regions of mouse SOCS5 appears to be encoded on a single exon (Figure 19). Analysis of the sequence (Figure 20) confirms that SOCS5 genomic and cDNA clones encode a protein with a SOCS box at its C-terminus in addition to an SH2 domain (Figure 19 and 20B). The relationship of the human SOCS5 contig (h5.1; Figure 21) derived from analysis of cDNA clone 5-94-2 and the human SOCS5 ESTs (Table 5.2) to the mouse SOCS5 DNA sequence is shown in Figure 19. The nucleotide sequence and corresponding amino acid sequence of murine SOCS5 are shown in SEQ ID NOs: 17 and 18, respectively. The human SOCS5 nucleotide sequence is shown in SEQ ID NO: 19.

### COMPARATIVE EXAMPLE 27

### SOCS6

Mouse and human SOCS6 were recognized through searching EST databases using the SOCS box consensus (Figure 13). Those ESTs derived from mouse and human SOCS6 cDNAs are tabulated below (Tables 6.1 and 6.2). Using sequence information derived from mouse ESTs, several oligonucleotides were designed and use to screen, in the conventional manner, a mouse thymus cDNA library. Eight cDNA clones (6-1A, 6-2A, 6-5B, 6-4N, 6-18, 6-29, 6-3N, 6-5N) cDNA clone encoding mouse SOCS6 were isolated and sequenced in their entirety and shown to overlap with the mouse ESTs identified in the database (Figures 22 and 23A). Analysis of the sequence (Figure 23) confirms that the mouse SOCS6 cDNA clones encode a protein with a SOCS box at its C-terminus in addition to a eight WD-40 repeats (Figures 22 and 23B). The relationship of the human SOCS-6 contigs (h6.1 and h6.2 ; Figure 24) derived from analysis of human SOCS6 ESTs (Table 6.2) to the mouse SOCS6 DNA sequence is shown in Figure 22. The nucleotide and corresponding amino acid sequences of murine SOCS6 are shown in SEQ ID NOs: 20 and 21, respectively. SOCS6 human contigs h6.1 and h6.2 are shown in SEQ ID NOs: 22 and 23, respectively.

### COMPARATIVE EXAMPLE 28

### SOCS7

Mouse and human SOCS7 were recognized through searching EST databases using the SOCS box consensus (Figure 13). Those ESTs derived from mouse and human SOCS-7 cDNAs are tabulated below (Tables 7.1 and 7.2). Using sequence information derived from mouse ESTs, several oligonucleotides were designed and use to screen, in the conventional manner, a mouse thymus cDNA library. One cDNA clone (74-10A-11) cDNA clone encoding mouse SOCS7 was isolated and sequenced in its entirety and shown to overlap with the mouse ESTs identified in the database (Figures 25 and 26A). Analysis of the sequence (Figure 26) suggests that mouse SOCS7 encodes a protein with a SOCS box at its C-terminus, in addition to several ankyrin repeats (Figure 25 and 26B). The relationship of the human SOCS7 contigs (h7.1 and h7.2 ; Figure 27) derived from analysis of human SOCS7 ESTs (Table 7.2) to the mouse SOCS7 DNA sequence is shown in Figure 25. The nucleotide and corresponding amino acid sequences of murine SOCS7 are shown in SEQ ID NOs: 24 and 25, respectively. The nucleotide sequence of SOCS7 human contigs h7.1 and h7.2 are shown in SEQ ID NOs: 26 and 27, respectively.

### COMPARATIVE EXAMPLE 29

### SOCS8.

ESTs derived from mouse SOCS8 cDNAs are tabulated below (Table 8.1). As described for other members of the SOCS family, it is possible to isolate cDNAs for mouse SOCS8 using sequence information derived from mouse ESTs. The relationship of the ESTs to the predicted coding region of SOCS8 is shown in Figure 28. With the nucleotide sequence obtained from the ESTs shown in Figure 29A and the partial amino acid sequence of SOCS8 shown in Figure 29B. The nucleotide sequence and corresponding amino acid sequences for murine SOCS8 are shown in SEQ ID NOs:28 and 29, respectively.

### COMPARATIVE EXAMPLE 30

### SOCS9

Mouse and human SOCS-9 were recognized through searching EST databases using the SOCS box consensus (Figure 13). Those ESTs derived from mouse and human SOCS9 cDNAs are tabulated below (Tables 9.1 and 9.2). The relationship of the mouse SOCS9 contigs (m9.1; Figure 9.2) derived from analysis of the mouse SOCS9 EST (Table 9.1) to the human SOCS-9 DNA contig (h9.1; Figure 32) derived from analysis of human SOCS9 ESTs (Table 9.2) is shown in Figure 31. Analysis of the sequence (Figure 32) indicates that the human SOCS9 cDNA encodes a protein with a SOCS box at its C-terminus, in addition to an SH2 domain (Figure 30). The nucleotide sequence of muring SOCS9 cDNA is shown in SEQ ID NO:30. The nucleotide sequence of human SOCS9 cDNA is shown in SEQ ID NO:31.

### COMPARATIVE EXAMPLE 31

### SOCS10

Mouse and human SOCS10 were recognized through searching EST databases using the SOCS box consensus (Figure 13). Those ESTs derived from mouse and human SOCS10 cDNAs are tabulated below (Table 10.1 and 10.2). Using sequence information derived from mouse ESTs, several oligonucleotides were designed and use to screen, in the conventional manner, a mouse thymus cDNA library. Four cDNA clones (10-9, 10-12, 10-23 and 10-24) encoding mouse SOCS10 were isolated, sequenced in their entirety and shown to overlap with the mouse and human ESTs identified in the database (Figures 33 and 34). Analysis of the sequence (Figure 34) indicates that the mouse SOCS10 cDNA clone is not full length but that it does encode a protein with a SOCS box at its C-terminus, in addition to several ankyrin repeats (Figure 33). The relationship of the human SOCS 10 contigs (h10.1 and h10.2 ; Figure 35) derived from analysis of human SOCS10 ESTs (Table 10.2) to the mouse SOCS 10 DNA sequence is shown in Figure 33. Comparison of mouse cDNA clones and ESTs with human ESTs suggests that the 3' untranslated regions of mouse and human SOCS10 differ significantly. The nucleotide sequence of murine SOCS10 is shown in SEQ ID NO:32 and the nucleotide sequence of SOCS10 human contigs h10.1 and h10.2 are shown in SEQ ID NOs:33 and 34, respectively.

### COMPARATIVE EXAMPLE 32

### SOCS11

Human SOCS11 were recognized through searching EST databases using the SOCS box consensus (Figure 13). Those ESTs derived from human SOCS 11 cDNAs are tabulated below (Table 11.1 and 11.2). The relationship of the human SOCS11 contigs (h11.1; Figure 36A, B), derived from analysis ESTs (Table 11.2) to the predicted encoded protein, is shown in Figure 37. Analysis of the sequence indicates that the human SOCS 11 cDNA encodes a protein with a SOCS box at its C-terminus, in addition to an SH2 domain (Figure 37 and 36B). The nucleotide sequence and corresponding amino acid sequence of human SOCS 11 are represented in SEQ ID NOs:35 and 36, respectively.

### COMPARATIVE EXAMPLE 33

### SOCS12

Mouse and human SOCS-12 were recognized through searching EST databases using the SOCS box consensus (Figure 13). Those ESTs derived from mouse and human SOCS 12 cDNAs are tabulated below (Tables 12.1 and 12.2). Using sequence information derived from mouse ESTs, several oligonucleotides were designed and use to screen, in the conventional manner, a mouse thymus cDNA library. Four cDNA clones (10-9, 10-12, 10-23 and 10-24) encoding mouse SOCS 12 were isolated, sequenced in their entirety and shown to overlap with the mouse and human ESTs identified in the database (Figures 38 and 39). Analysis of the sequence (Figure 39 and 40) indicates that the SOCS12 cDNA clone encodes a protein with a SOCS box at its C-terminus, in addition to several ankyrin repeats (Figure 38). The relationship of the human SOCS12 contigs (h12.1 and h12.2; Figure 40) derived from analysis of human SOCS 12 ESTs (Table 12.2) to the mouse SOCS 12 DNA sequence is shown in Figure 38. Comparison of mouse cDNA clones and ESTs with human ESTs suggests that the 3' untranslated regions of mouse and human SOCS12 differ significantly. The nucleotide sequence of SOCS12 is shown in SEQ ID NO:37. The nucleotide sequence of human SOCS12 contigs h12.1 and h12.2 are shown in SEQ ID NOs:38 and 39, respectively.

### COMPARATIVE EXAMPLE 34

### SOCS13

Mouse and human SOCS-13 were recognized through searching EST databases using the SOCS box consensus (Figure 13). Those ESTs derived from mouse and human SOCS13 cDNAs are tabulated below (Tables 13.1 and 13.2). Using sequence information derived from mouse ESTs, several oligonucleotides were designed and use to screen, in the conventional manner, a mouse thymus and a mouse embryo cDNA library. Three cDNA clones (62-1, 62-6-7 and 62-14) encoding mouse SOCS 13 were isolated, sequenced in their entirety and shown to overlap with the mouse ESTs identified in the database (Figure 41 and 42A). Analysis of the sequence (Figure 42) indicates that the mouse SOCS13 cDNA encodes a protein with a SOCS box at its C-terminus, in addition to a potential WD-40 repeat (Figure 41 and 42B). The relationship of the human SOCS13 contigs (h13.1 and h13.2 ; Figure 43) derived from analysis of human SOCS 13 ESTs (Table 13.2) to the mouse SOCS 13 DNA sequence is shown in Figure 41. The nucleotide sequence and corresponding amino acid sequence of murine SOCS13 and shown in SEQ ID NOs:40 and 41, respectively. The nucleotide sequence of human SOCS 13 contig h13.1 is shown in SEQ ID NO:42.

### COMPARATIVE EXAMPLE 35

### SOCS14

Mouse and human SOCS-14 were recognized through searching EST databases using the SOCS box consensus (Figure 13). Those ESTs derived from mouse and human SOCS14 cDNAs are tabulated below (Tables 14.1 and 14.2). Using sequence information derived from mouse and human ESTs, several oligonucleotides were designed and use to screen, in the conventional manner, a mouse thymus cDNA library, a mouse genomic DNA library and a human thymus cDNA library cloned into λ-bacteriophage. A single genomic DNA clone (57-2) and (5-3-2) cDNA clone encoding mouse SOCS14 were isolated and sequenced in their entirety and shown to overlap with the mouse ESTs identified in the database (Figures 44 and 45A). The entire coding region, in addition to a region of 5' and 3' untranslated regions, of mouse SOCS 14 appears to be encoded on a single exon (Figure 44). Analysis of the sequence (Figure 45) confirms that SOCS 14 genomic and cDNA clones encode a protein with a SOCS box at its C-terminus in addition to an SH2 domain (Figure 44 and 45B). The relationship of the human SOCS14 contig (h14.1; Figure 14.3) derived from analysis of cDNA clone 5-94-2 and the human SOCS 14 ESTs (Table 14.2) to the mouse SOCS14 DNA sequence is shown in Figure 44.

The nucleotide sequence and corresponding amino acid sequence of murine SOCS 14 are shown in SEQ ID NOs: 43 and 44, respectively.

### COMPARATIVE EXAMPLE 36

### SOCS15

Mouse and human SOCS15 were recognized through searching DNA databases using the SOCS box consensus (Figure 13). Those ESTs derived from mouse and human SOCS15 cDNAs are tabulated below (Tables 15.1 and 15.2), as are a mouse and human BAC that contain the entire mouse and human SOCS-15 genes. Using sequence information derived from the ESTs and the BACs it is possible to predict the entire amino acid sequence of SOCS15 and as described for the other SOCS genes it is feasible to design specific oligonucleotide probes to allow cDNAs to be isolated. The relationship of the BACs to the ESTs is shown in Figure 46 and the nucleotide and predicted amino acid sequence of the SOCS-15, derived from the mouse and human BACs is shown in Figures 47 and 48. The nucleotide sequence and corresponding amino acid sequence of murine SOCS 15 are shown in SEQ ID NOs:46 and 47, respectively. The nucleotide and corresponding amino acid sequence of human SOCS 15 are shown in SEQ ID NO:48 and 49, respectively.

### EXAMPLE 37

### SOCS INTERACTION WITH JAK2 KINASE

These Examples show interaction between SOCS and JAK2 kinase. Interaction is mediated via the SH2 domain of SOCS 1, 2, 3 and CIS. The interaction resulted in inhibition of JAK2 kinase activity by SOCS1 (Figure 49). General interaction between JAK2 and SOCS1, 2, 3, and CIS is shown in Figure 50.

The following methods are employed:

**Immunoprecipitation**: Cos 6 cells were transiently transfected by electroporation and cultured for 48 hours. Cells were then lysed on ice in lysis buffer (50 mM Tris/HCL, pH 7.5, 150 mM NaCl, 1% v/v Triton-X-100, 1 mM EDTA, 1 mM Naf, 1 mM Na₃VO₄) with the addition of complete protease inhibitors (Boehringer Mannheim), centrifuged at 4°C (14,000 x g, 10 min) and the supernatant retained for immunoprecipitation. JAK2 proteins were immunoprecipitated using 5 µl anti-JAK2 antibody (UBI). Antigen-antibody complexes were recovered using protein A-Sepharose (30 µl of a 50% slurry).

**Western blotting:** Immunoprecipitates were analysed by sodium dodecyl sulphate (SDS) - polyacrylamide gel electrophoresis (PAGE) under reducing conditions. Protein was then electrophoretically transferred to nitrocellulose, blocked overnight in 10% w/v skim-milk and washed in PBS/0. % v/v Tween-20 (Sigma) (wash buffer) prior to incubation with either anti-phosphotyrosine antibody (4G10) (1:5000, UBI), anti-FLAG antibody (1.6 µg/ml) or anti-JAK2 antibody (1:2000, UBI) diluted in wash buffer/1% w/v BSA for 2 hr. Nitrocellulose blots were washed and primary antibody detected with either peroxidase-conjugated sheep anti-rabbit immunoglobulin (1:5000, Silenus) or peroxidase-conjugated sheep anti-mouse immunoglobulin (1:5000, Silenus) diluted in wash buffer/1% w/v BSA. Blots were washed and antibody binding visualised using the enhanced chemiluminescence (ECL) system (Amersham, UK) according to the manufacturers' instructions.

***In-vitro* kinase assay**: An *in vitro* kinase assy was performed to assess intrinsic JAK2 kinase catalytic activity. JAK2 protein were immunopreciptated as described, washed twice in kinase assay buffer (50 mM NaCl, 5 mM MgCl₂, 5 mM MnCl2, 1 mM NaF, 1 mM Na₃VO₄, 10 mM HEPES, pH 7.4) and suspended in an equal volume of kinase buffer containing 0.25 *µ*Ci/ml (γ-³²P)-ATP (30 min, room temperature). Excess (γ- P)-ATP was removed and the immunoprecipitates analysed by SDS/PAGE under reducing conditions. Gels were subjected to a mild alkaline hydrolysis by treatment with 1 M KOH (55°C, 2 hours) to remove phosphoserine and phosphothreonine. Radioactive bands were visualised with IMAGEQUANT. software on a PhosphorImage system (Molecular Dynamics, Sunnyvale, CA, USA).

### COMPARATIVE EXAMPLE 38

### MAKING SOCS-1 KNOCKOUT CONSTRUCTS

Diagrams of plasmid constructs and knockout constructs are shown in Figures 51-53. The genomic SOCS-1 clone 95-11-10 was digested with the restriction enzymes BamH1 and EcoR1 to obtain a 3.6Kb DNA fragment 3' of the coding region (SOCS-1 exon), which was used as the 3' arm in the SOCS-1 knockout vectors. The ends of this fragment were then blunted. This fragment was then ligated into the following vectors:
pBgalpAloxNeo
and pBgalpAloxNeoTK
which had been linearized at the unique Xhol site and then blunted. This ligation resulted in the formation of the following vectors:
3'SOCS-1 arm in pBgalpAloxNeo
and 3'SOCS-1 arm in pBgalpAloxNeoTK

The 5' arm of the SOCS-1 knockout vectors was constructed by using PCR to generate a 2.5Kb PCR product from the genomic SOCS-1 clone 95-11-10 just 5' of the SOCS-1 coding region (SOCS-1 exon). The oligo's used to generate this product were:
5' oligo (sense) (2465)
   AGCT AGA TCT GGA CCC TAC AAT GGC AGC [SEQ ID NO:49]
3' oligo (antisense) (2466)
   AGCT AG ATC TGC CAT CCT ACT CGA GGG GCC AGC TGG [SEQ ID NO:50]

The PCR product was then digested with the restriction enzyme BglII, to generate BglII ends to the PCR product. This 5' SOCS-1 PCR product,with BglII, ends was then ligated as follows: 3'SOCS-1 arm in pBgalpAloxNeo and 3'SOCS-1 arm in pBgalpAloxNeoTK, which had been linearized with the unique restriction enzyme BamH1. This resulted in the following vectors being formed:
5'&3'SOCS-1 arms in pBgalpAloxNeo
and 5'&3'SOCS-1 arms in pBgalpAloxNeoTK

These were the final SOCS-1 knockout constructs. Both these constructs lacked the entire SOCS-1 coding region (SOCS-1 EXON), being replaced with portions of the Bgal, B globin polyA, PGK promoter, neomycin and PGK polyA sequences. The 5'&3'SOCS-1 arms in pBgalpAloxNeoTK vector also contained the tymidine kinase gene sequence, between the neomycin and PGK poly A sequences.
The vectors: 5'&3'SOCS-1 arms in pBgalpAloxNeo
and 5'&3'SOCS-1 arms in pBgalpAloxNeoTK
were linearized with the unique restriction enzyme Not1 and then transfected into Embryonic stem cells by electroporation. Clones which were resistant to neomycin were selected and analysed by southern blot to determine if they contained the correctly integrated SOCS-1 targeting sequence. In order to determine if correct integration had occurred, genomic DNA from the neomycin resistant clones was digested with the restriction enzyme EcoR1. The digested DNA was then blotted onto nylon filters and probed with a 1.5Kb EcoR1/Hind III DNA fragment, which was further 5' of the 5'arm sequence used in the knockout constructs. The band sizes expected for correct integration were:
Wild type SOCS-1 allele 5.4Kb
SOCS-1 knockout allele 8.2Kb in 5'&3'SOCS-1 arms in pBgalpAloxNeo
or 11Kb in 5'&3'SOCS-1 arms in pBgalpAloxNeoTK transfomed cells.

**Table 4.1**

| **Summary of ESTs derived from mouse SOCS.4 cDNAs** | | | | | | |
|---|---|---|---|---|---|---|
| **SOCS** | **Species** | **EST name** | **End** | **EST no** | **Library source** | **Contig** |
| SOCS-4 | Mouse | mc65f04 | 5' | EST0549700 | d13.5-14.5 mouse embryo | m4.1 |
| | | mf42e06 | 5' | EST0593477 | d13.5-14.5 mouse embryo | m4.1 |
| | | mp10c10 | 5' | EST0747905 | d 8.5 mouse embryo | m4.1 |
| | | mr81g09 | 5' | EST0783081 | d13 embryo | m4.1 |
| | | mt19h12 | 5' | EST0816531 | spleen | m4.1 |

**Table 4.2**

| **Summary of ESTs derived from human SOCS-4 cDNAs** | | | | | | |
|---|---|---|---|---|---|---|
| **SOCS** | **Species** | **EST name** | **End** | **EST no** | **Library source** | **Contig** |
| SOCS-4 | Human | 27b5 | 5' | EST0534081 | retina | h4.2 |
| | | 30d2 | 5' | EST0534315 | retina | h4.2 |
| | | J0159F | 5' | EST0461188 | foetal heart | h4.2 |
| | | J3802F | 5' | EST0461428 | foetal heart | h4.2 |
| | | EST19523 | 5' | EST0958884 | retina | h4.2 |
| | | EST81149 | 5' | EST1011015 | placenta | h4.2 |
| | | EST180909 | 5' | EST0951375 | Jurkat T-lymphocyte | h4.2 |
| | | EST182619 | 5' | EST0953220 | Jurkat T-lymphocyte | h4.1 |
| | | ya99h09 | 3' | EST0103262 | placenta | h4.2 |
| | | ye70c04 | 5' | EST0172673 | foeat] liver/spleen | h4.2 |
| | | yh53c09 | 5' | EST0197390 | placenta | h4.2 |
| | | | 3' | EST0197391 | | h4.2 |
| | | yh77g11 | 5' | EST0203418 | placenta | h4.2 |
| | | | 3' | EST0203419 | | h4.1 |
| | | yh87h05 | 5' | EST0204888 | placenta | h4.1 |
| | | | 3' | EST0204773 | | h4.1 |
| | | yi45h07 | 5' | EST0246604 | placenta | h4.2 |
| | | yj04e06 | 5' | EST0258541 | placenta | h4.1 |
| | | | 3' | EST0258285 | | h4.1 |
| | | yq12h06 | 5' | EST0309968 | foetal liver spleen | h4.2 |
| | | yq56a06 | 3' | EST0346924 | foetal liver spleen | h4.2 |
| | | yq60e02 | 5' | EST0347259 | foetal liver spleen | h4.2 |
| | | | 3' | EST0347209 | | h4.2 |
| | | yq92g03 | 5' | EST0355932 | foetal liver spleen | h4.2 |
| | | | 3' | EST0355884 | | h4.2 |
| | | yq97h06 | 5' | EST0357618 | foetal liver spleen | h4.2 |
| | | | 3' | EST0357416 | | h4.2 |
| | | yr90f01 | 5' | EST0372402 | foetal liver spleen | h4.2 |
| | | yt69c03 | 5' | EST0338395 | foetal liver spleen | h4.2 |
| | | | 3' | EST0338303 | | h4.2 |
| | | yv30a08 | 3' | EST0458506 | foetal liver spleen | h4.2 |
| | | yv55f07 | 5' | EST0465391 | foetal liver spleen | h4.2 |
| | | | 3' | EST0463331 | | h4.2 |
| | | yv57h09 | 5' | EST0464336 | foetal liver spleen | h4.2 |
| | | | 3' | EST0458765 | | h4.2 |
| | | yv87h02 | 5' | EST0388085 | melanocyte | h4.2 |
| | | yv98e11 | 5' | EST0400679 | melanocyte | h4.2 |
| | | | 3' | EST0400680 | | h4.2 |
| | | yw68d10 | 5' | EST0441370 | placenta (8-9 wk) | h4.2 |
| | | yw82a03 | 5' | EST0463005 | placenta (8-9 wk) | h4.2 |
| | | | 3' | EST0433678 | | h4.1 |
| | | yx08a07 | 3' | EST0407016 | melanoocyte | h4.1 |
| | | yx72h06 | 5' | EST0435158 | melanoocyte | h4.2 |
| | | | 3' | EST0422871 | melanoocyte | h4.1 |
| | | yx76b09 | 5' | EST0434011 | melanoocyte | h4.2 |
| | | yy37h08 | 5' | EST0451704 | melanoocyte | h4.2 |
| | | yy66b02 | 5' | EST0505446 | multiple sclerosis lesion | h4.2 |
| | | za81f08 | 5' | EST0511777 | foetal lung | h4.2 |
| | | zb18f07 | 3' | EST0485315 | foetal lung | h4.1 |
| | | zc06e08 | 5' | EST0540473 | parathyroid tumor | h4.1 |
| | | | 3' | EST0540354 | | h4,1 |
| | | zd14g06 | 3' | EST0564666 | foetal heart | h4.1 |
| | | zd51h12 | 3' | EST0578099 | foetal heart | h4.1 |
| | | zd52b09 | 5' | EST0582012 | foetal heart | h4.1 |
| | | | 3' | EST0581958 | | h4.1 |
| | | ze25g11 | 3' | EST0679543 | foetal heart | h4.1 |
| | | ze69f02 | 5' | EST0635563 | retina | h4,2 |
| | | | 3' | EST0635472 | | h4.1 |
| | | zf54f03 | 5' | EST0680111 | retina | h4.2 |
| | | zh96e07 | 5' | EST0616241 | foetal liver spleen | h4.2 |
| | | | 3' | EST0615745 | | h4.2 |
| | | zv66h12 | 5' | EST1043265 | 8-9w foetus | h4.2 |
| | | zs83a08 | 5' | EST0920072 | germinal centre B cell | h4.1 |
| | | | 3' | EST0920016 | | h4.1 |
| | | zs83g08 | 5' | EST0920121 | germinal centre B cell | h4.1 |
| | | | 3' | EST0920122 | | h4.1 |

**Table 5.1**

| **Summary of ESTs derived from mouse SOCS-5 cDNAs** | | | | | | |
|---|---|---|---|---|---|---|
| **SOCS** | **Species** | **EST name** | **End** | **EST no** | **Library source** | **Contig** |
| SOCS-5 | Mouse | mc55a01 | 5' | EST0541556 | d13.5-14.5 mouse embryo | m5.1 |
| | | mh98f09 | 5' | EST0638237 | placenta | m5.1 |
| | | my26h12 | 5' | EST0859939 | mixed organs | m5.1 |
| | | ve24e06 | 5' | EST0819106 | heart | m5.1 |

**Table 5.2**

| **Summary of ESTs derived from human SOCS-5 cDNAs** | | | | | | |
|---|---|---|---|---|---|---|
| **SOCS** | **Species** | **EST name** | **End** | **EST no** | **Library source** | **Contig** |
| SOCS-5 | Human | EST15B103 | ? | EST0258029 | adipose tissue | h5.1 |
| | | EST15B105 | ? | EST0258028 | adipose tissue | h5.1 |
| | | EST27530 | 5' | EST0965892 | cerebellum | h5.1 |
| | | zf50f01 | 5' | EST0679820 | retina | h5.1 |

**Table 6.1**

| **Summary of ESTs derived from mouse SOCS-6 cDNAs** | | | | | | |
|---|---|---|---|---|---|---|
| **SOCS** | **Species** | **EST name** | **End** | **EST no** | **Library source** | **Contig** |
| SOCS-6 | Mouse | mco4c05 | 5' | EST0525832 | d19.5 embryo | m6.1 |
| | | md48a03 | 5' | EST0566730 | d13.5-14.5 embryo | m6.1 |
| | | mf31d03 | 5' | EST0675970 | d13.5-14.5 embryo | m6.1 |
| | | mh26b07 | 5' | EST0628752 | d13.5-14.5 placenta | m6.1 |
| | | mh78e11 | 5' | EST0637608 | d13.5-14.5 placenta | m6.1 |
| | | mh88h09 | 5' | EST0644383 | d13.5-14.5 placenta | m6.1 |
| | | mh94h07 | 5' | EST0638078 | d13.5-14.5 placenta | m6.1 |
| | | mi27h04 | 5' | EST0644252 | d13.5-14.5 embryo | m6.1 |
| | | mj29c05 | 5' | EST0664093 | d13.5-14.5 embryo | m6.1 |
| | | mp66g04 | 5' | EST0757905 | thymus | m6.1 |
| | | mw75g03 | 5' | EST0847938 | liver | m6.1 |
| | | va53b05 | 5' | EST0901540 | d12.5 embryo | m6.1 |
| | | vb34h02 | 5' | EST0930132 | lymph node | m6.1 |
| | | vc55d07 | 3' | EST1057735 | 2 cell embryo | m6.1 |
| | | vc59e05 | 3' | EST1058201 | 2 cell embryo | m6.1 |
| | | vc67d03 | 3' | EST1057849 | 2 cell embryo | m6.1 |
| | | vc68d10 | 3' | EST1058663 | 2 cell embryo | m6.1 |
| | | vc97h01 | 3' | EST1059343 | 2 cell embryo | m6.1 |
| | | vc99c08 | 3' | EST1059410 | 2 cell embryo | m6.1 |
| | | vd07h03 | 3' | EST1058173 | 2 cell embryo | m6.1 |
| | | vd08c01 | 3' | EST1058275 | 2 cell embryo | m6.1 |
| | | vd09b12 | 3' | EST1058632 | 2 cell embryo | m6.1 |
| | | vd19b02 | 3' | EST1059723 | 2 cell embryo | m6.1 |
| | | vd29a04 | 3' | ? none found | | m6.1 |
| | | vd46d06 | 3' | ? none found | | m6.1 |

**Table 6.2**

| **Summary of ESTs derived from human SOCS-5 cDNAs** | | | | | | |
|---|---|---|---|---|---|---|
| **SOCS** | **Species** | **EST name** | **End** | **EST no** | **Library source** | **Contig** |
| SOCS-6 | Human | | | | | |
| | | yf61e08 | 5' | EST0184387 | d73 infant brain | h6.1 |
| | | yf93a09 | 5' | EST0186084 | d73 infant brain | h6.1 |
| | | yg05f12 | 5' | EST0191486 | d73 infant brain | h6.1 |
| | | yg41f04 | 5' | EST0195017 | d73 infant brain | h6.1 |
| | | yg45c02 | 5' | EST0185308 | d73 infant brain | h6.1 |
| | | yh11f10 | 5' | EST0236705 | d73 infant brain | h6.1 |
| | | yh13b05 | 5' | EST0237191 | d73 infant brain | h6.1 |
| | | | 3' | EST0236958 | | h6.2 |
| | | zc35a12 | 5' | EST0555518 | senescent fibroblasts | h6.1 |
| | | ze02h08 | 5' | EST0603826 | foetal heart | h6.1 |
| | | | 3' | EST0603718 | | h6.2 |
| | | z109a03 | 5' | EST0773936 | pregnant uterus | h6.1 |
| | | | 3' | EST0773892 | | h6.1 |
| | | z169e10 | 5' | EST0683363 | colon | h6.1 |
| | | zn39d08 | 5' | EST0718885 | endothelial cell | h6.1 |
| | | zo39e06 | 5' | EST0785947 | endothelial cell | h6.1 |

**Table 7.1**

| **Summary of ESTs derived from mouse SOCS-7 cDNAs** | | | | | | |
|---|---|---|---|---|---|---|
| SOCS | Species | EST name | End | EST no | Library source | Contig |
| SOCS-7 | Mouse | mj39a01 | 5' | EST0665627 | d13.5/14.5 embryo | m7.1 |
| | | vi52h07 | 5' | EST1267404 | d7.5 embryo | m7.1 |

**Table 7.2**

| **Summary of ESTs derived from human SOCS-5 cDNAs** | | | | | | |
|---|---|---|---|---|---|---|
| **SOCS** | **Species** | **EST name** | **End** | **EST no** | **Library source** | **Contig** |
| SOCS-7 | HUMAN | STS WI-30171 | | (G21563) | Chromosome 2 | h7.2 |
| | | EST00939 | 5' | EST0000906 | hippocampus | h7.1 |
| | | EST12913 | 3' | EST0944382 | uterus | h7.2 |
| | | yc29b05 | 3' | EST0128727 | liver | h7.2 |
| | | yp49f10 | 3' | EST0301914 | retina | h7.2 |
| | | zt10f03 | 5' | EST0922932 | germinal centre B cell | h7.2 |
| | | | 3' | EST0921231 | | h7.1 |
| | | zx73g04 | 3' | EST1102975 | ovarian tumour | h7.1 |

**Table 8.1**

| **Summary of ESTs derived from mouse SOCS-8 cDNAs** | | | | | | |
|---|---|---|---|---|---|---|
| **SOCS** | **Species** | **EST name** | **End** | **EST no** | **Library source** | **Contig** |
| SOCS-8 | Mouse | mj16e09 | r1 | EST0666240 | d13.5/14.5 embryo | m8.1 |
| | | vj27a029 | rl | EST1155973 | heart | m8.1 |

**Table 9.1**

| **Summary of ESTs derived from mouse SOCS-9 cDNAs** | | | | | | |
|---|---|---|---|---|---|---|
| **SOCS** | **Species** | **EST name** | **End** | **EST no** | **Library source** | **Contig** |
| | Mouse | me65d05 | 5' | EST0585211 | d 13.5/14.5 embryo | m9.1 |

**Table 9.2**

| **Summary of ESTs derived from human SOCS-5 cDNAs** | | | | | | |
|---|---|---|---|---|---|---|
| **SOCS** | **Species** | **EST name** | **End** | **EST no** | **Library source** | **Contig** |
| SOCS-9 | Human | CSRL-83f2-u | | (B06659) | chromsome 11 | h9.1 |
| | | EST114054 | 5' | EST0939759 | placenta | h9.1 |
| | | yy06b07 | 3' | EST0434504 | melanocyte | h9.1 |
| | | yy06g06 | 5' | EST0443783 | melanocyte | h9.1 |
| | | zr40c09 | 5' | EST0832461 | melanocyte, heart, uterus | h9.1 |
| | | zr72h01 | 5' | EST0892025 | melanocyte, heart, uterus | h9.1 |
| | | | 3' | EST0892026 | | h9.1 |
| | | yx92c08 | 5' | EST0441160 | melanocyte | h9.1 |
| | | yx93b08 | 5' | EST0441260 | melanocyte | h9.1 |
| | | hfe0662 | 5' | EST0889611 | foetal heart | h9.1 |

**Table 10.1**

| **Summary of ESTs derived from mouse SOCS-10 cDNAs** | | | | | | |
|---|---|---|---|---|---|---|
| **SOCS** | **Species** | **EST name** | **End** | **EST no** | **Library source** | **Contig** |
| | Mouse | mb14d12 | 5' | EST0549887 | d19.5 embryo | m10.1 |
| | | mb40f06 | 5' | EST0515064 | d19.5 embryo | m10.1 |
| | | mg89b11 | 5' | EST0630631 | d13.5-14.5 embryo | m10.1 |
| | | mq89e12 | 5' | EST0776015 | heart | m10.1 |
| | | mp03g12 | 5' | EST0741991 | heart | m10.1 |
| | | vh53c11 | 5' | EST1154634 | mammary gland | m10.1 |

**Table 10.2**

| **Summary of ESTs derived from human SOCS-5 cDNAs** | | | | | | |
|---|---|---|---|---|---|---|
| **SOCS** | **Species** | **EST name** | **End** | **EST no** | **Library source** | **Contig** |
| SOCS-10 | Human | aa48h10 | 3' | EST1135220 | germinal centre B cell | h10.2 |
| | | zp35h01 | 3' | EST0819137 | muscle | h10.2 |
| | | zp97h12 | 5' | EST0835442 | muscle | h10.2 |
| | | | 3' | EST0831211 | | h10.2 |
| | | zq08h01 | 5' | EST0835907 | muscle | h10.1 |
| | | zr34g05 | 5' | EST0834251 | melanocyte, heart, uterus | h10.2 |
| | | | 3' | EST0834440 | | h10.2 |
| | | EST73000 | 5 | EST1004491 | ovary | h10.2 |
| | | HSDHEI005 ? | | EST0013906 | heart | h10.2 |

**Table 11.1**

| **Summary of ESTs derived from human SOCS-5 cDNAs** | | | | | | |
|---|---|---|---|---|---|---|
| **SOCS** | **Species** | **EST name** | **End** | **EST no** | **Library source** | **Contig** |
| SOCS-11 | Human | zt24h06 | r1 | EST0925023 | ovarian tumor | 11.1 |
| | | zr43b02 | rl | EST0873006 | melanocyte, heart, uterus | 11.1 |
| | | | s1 | EST0872954 | | 11.1 |

**Table 12.1**

| **Summary of ESTs derived from mouse SOCS-12 cDNAs** | | | | | | |
|---|---|---|---|---|---|---|
| **SOCS** | **Species** | **EST name** | **End** | **EST no** | **Library source** | **Contig** |
| SOCS-12 | Mouse | EST03803 | 5' | EST1054173 | day 7.5 emb ectoplacental cone | m12.1 |
| | | mt18f02 | 5' | EST0817652 | 3NbMS spleen | m12.1 |
| | | mz60g10 | 5' | EST0890872 | lymph node | m12.1 |
| | | va05c11 | 5' | EST0909449 | lymph node | m12.1 |

**Table 12.2**

| **Summary of ESTs derived from human SOCS-5 cDNAs** | | | | | | |
|---|---|---|---|---|---|---|
| **SOCS** | **Species** | **EST name** | **End** | **EST no** | **Library source** | **Contig** |
| SOCS-12 | Human | STS-SHGC-13867 | | | Chromosome 2 | h12.2 |
| | | EST177695 | 5' | EST0948071 | Jurkat cells | h12.1 |
| | | EST64550 | 5' | EST0997367 | Jurkat cells | h12.1 |
| | | EST76868 | 5' | EST1007291 | pineal body | h12.2 |
| | | PMY2369 | 5' | EST1115998 | KG-1 | h12.1 |
| | | yb38f04 | 5' | EST0108807 | foetal spleen | h12.1 |
| | | | 3' | | | h12.2 |
| | | yg74e12 | 5' | EST0224407 | d73 brain | h12.1 |
| | | yh13g04 | 5' | EST0237226 | d73 brain | h12.1 |
| | | | 3' | EST0236992 | | h12.2 |
| | | yh48b06 | 5' | yh48b06 | placenta | h12.2 |
| | | yh53a05 | 5' | EST0197282 | placenta | h12.2 |
| | | | 3' | EST0197486 | | h12.2 |
| | | yn48h09 | 5' | EST0278258 | brain | h12.2 |
| | | | 3' | EST0278259 | | h12.2 |
| | | yn90a09 | 3' | EST0302557 | brain | h12.2 |
| | | yo08f03 | 5' | EST0301790 | brain | h12.2 |
| | | | 3' | EST0302059 | | h12.2 |
| | | yo11e01 | 3' | ? none found | | h12.2 |
| | | yo63b12 | 5' | EST0303606 | breast | h12.2 |
| | | | 3' | EST0304085 | | h12.2 |
| | | yq56g02 | 3' | EST0346935 | foetal liver spleen | h12.1 |
| | | zh57c04 | 3' | EST0594201 | foetal liver spleen | h12.2 |
| | | zh79h01 | 3' | EST0598945 | foetal liver spleen | h12.2 |
| | | zh99a11 | 3' | EST0618570 | foetal liver spleen | h12.2 |
| | | zo92h12 | 5' | EST0803392 | ovarian cancer | h12.1 |
| | | | 3' | EST0803393 | | h12.2 |
| | | zs48c01 | 5' | EST0925714 | germinal centre B cell | h12.1 |
| | | | 3' | EST0925530 | | h12.2 |
| | | zs45h02 | 3' | EST0932296 | germinal centre B cell | h12.2 |

**Table 13.1**

| **Summary of ESTs derived from mouse SOCS-13 cDNAs** | | | | | | |
|---|---|---|---|---|---|---|
| **SOCS** | **Species** | **EST name** | **End** | **EST no** | **Library source** | **Contig** |
| SOCS-13 | Mouse | ma39c09 | 5' | EST0517875 | day 19.5 embryo | m13.1 |
| | | me60c05 | 5' | EST0584950 | day 13.5114.5 embryo | ml3.1 |
| | | mi78g05 | 5' | EST0653834 | day 19.5 embryo | m13.1 |
| | | mk10c11 | 5' | EST0735158 | day 19.5 embryo | m13.1 |
| | | mo48g12 | 5' | EST0745111 | day 10.5 embryo | ml3.1 |
| | | mp94a01 | 5' | EST0762827 | thymus | m13.1 |
| | | vb57c07 | 5' | EST1028976 | day 11.5 embryo | ml3.1 |
| | | vh07c11 | 5' | EST1117269 | mammary gland | m13.1 |

**Table 13.2**

| **Summary of ESTs derived from human SOCS-13 cDNAs** | | | | | | |
|---|---|---|---|---|---|---|
| **SOCS** | **Species** | **EST name** | **End** | **EST no** | **Library source** | **Contig** |
| SOCS-13 | Human | EST59161 | 5' | EST0992726 | infant brain | h13.1 |

**Table 14.1**

| **Summary of ESTs derived from mouse SOCS.14 cDNAs** | | | | | | |
|---|---|---|---|---|---|---|
| **SOCS** | **Species** | **EST name** | **End** | **EST no** | **Library source** | **Contig** |
| SOCS-14 | mouse | mi75e03 | 5' | EST0651892 | d19.5 embryo | m14.1 |
| | | vd29h11 | 5' | EST1067080 | 2 cell embryo | m14.1 |
| | | vd53g07 | 5' | EST1119627 | 2 cell embryo | m14.1 |

**Table 15.1**

| **Summary of ESTs derived from mouse SOCS-15 cDNAs** | | | | | | |
|---|---|---|---|---|---|---|
| **SOCS** | **Species** | **EST name** | **End** | **EST no** | **Library source** | **Contig** |
| SOCS-15 | Mouse | mh29b05 | 5' | EST0628834 | placenta | m15.1 |
| | | mh98h09 | 5' | EST0638243 | placenta | m15.1 |
| | | ml45a02 | 5' | EST0687171 | testis | m15.1 |
| | | mu43a10 | 5' | EST851588 | thymus | m15.1 |
| | | my38c09 | 5' | EST878461 | pooled organs | m15.1 |
| | | vj37h07 | 5' | EST1174791 | diaphragm | m15.1 |
| | | AC002393 | | | Chromosome 6 BAC | m15.1 |

**Table 15.2**

| **Summary of ESTs derived from human SOCS-15 cDNAs** | | | | | | |
|---|---|---|---|---|---|---|
| **SOCS** | **Species** | **EST name** | **End** | **EST no** | **Library source** | **Contig** |
| SOCS-15 | Human | EST98889 | 5' | EST1026568 | thyroid | h15.1 |
| | | ne48bo5 | 3' | EST1138057 | colon tumour | h15.1 |
| | | yb12h12 | 5' | EST0098885 | placenta | h15.1 |
| | | | 3' | EST0098886 | | h15.1 |
| | | HSU47924 | | | Chromosome 12 BAC | h15.1 |

### BIBLIOGRAPHY:

Alexander WS, Metcalf D and Dunn AR (1995). Embo Journal 14, 5569-78.
Altschul, S.F. Gish, W. Miller, W. Myers, E.W. & Lipman, D.J. (1990) J. Mol. Biol. 215, 403-10.
Ansari-Lari, M.A., Shen, Y., Munzy, D.M., Lee, W. and Gibbs, R.A. (1997) Genome. Res. 7, 268-280.
Bazan JF (1990). [Review]. Immunology Today 11, 350-4.
Bork, P. (1993) Proteins: Struct. Funct. Genet. 17, 363-374.
Cockwell, L.Y. and Giles, I.G. (1989) Comp. Appl. Biosci. 5, 227-232.
Cutler RL, Liu L, Damen JE and Krystal G (1993). Journal of Biological Chemistry 268, 21463-5.
Darnell J Jr., Kerr IM and Stark GR (1994). Science 264, 1415-21.
David M, Petricoin E3, Benjamin C, Pine R, Weber MJ and Larner AC (1995). Science 269, 1721-3.
David M, Wong L, Flavell R, Thompson SA, Wells A, Larner AC and Johnson GR (1996). Journal of Biological Chemistry 271, 9185-8.
Dugaiczyk A, Haron JA, Stone EM, Dennison OE, Rothblum KN and Schwartz RJ (1983). Biochemistry 22, 1605-13.
Durbin JE, Hackenmiller R, Simon MC and Levy DE (1996). Cell 84, 443-50.
Etzold, T., Ulyanov, A. and Argos, P. (1996) Methods Enzymol. 266, 114-28.
Gearing DP, Nicola NA, Metcalf D, Foote S, Willson TA, Gough NM and Williams L (1989). BioTechnology 7, 1157-1161.
Gupta S, Yan H, Wong LH, Ralph S, Krolewski J and Schindler C (1996). Embo Journal 15, 1075-84.
Hilton DJ (1994). An introduction to cytokine receptors, p8-16 in Guidebook to Cytokines and Their Receptors, Eds: N. A. Nicola. Oxford University Press: Oxford.
Hilton DJ, Hilton AA, Raicevic A, Rakar S, Harrison-Smith M, Gough NM, Begley CG, Metcalf D, Nicola NA and Willson TA (1994). Embo Journal 13, 4765-75.
Hilton DJ, Watowich SS, Katz L and Lodish HF (1996). J. Biol. Chem. 271, 4699-4708.
Ichikawa Y (1969). Journal of Cellular Physiology 74, 223-34.
Ihle JN (1995). Nature 377, 591-4.
Ihle JN, Witthuhn BA, Quelle FW, Yamamoto K and Silvennoinen O (1995). Annual Review of Immunology 13, 369-98.
Kaplan MH, Schindler U, Smiley ST and Grusby MJ (1996a). Immunity 4, 313-9.
Kaplan MH, Sun YL, Hoey T and Grusby MJ (1996b). Nature 382, 174-179.
Levy DE and Stark GR (1996). Molecular & Cellular Biology 16, 369-75.
Metcalf D, Wilson TA, Hilton DJ, DiRago L and Mifsud S. (1995) Leukaemia 9, 1556-1564.
Meraz MA, White JM, Sheehan KC, Bach EA, Rodig SJ, Dighe AS, Kaplan DH, Riley JK, Greenlund AC, Campbell D, Carver-Moore K, DuBois RN, Clark R, Aguet M and Schreiber RD (1996). Cell 84, 431-42.
Mizushima S and Nagata S (1990). Nucleic Acids Research 18, 5322.
Murakami M, Narazaki M, Hibi M, Yawata H, Yasukawa K, Hamaguchi M, Taga T and Kishimoto T (1991). Proc. Natl. Acad. Sci. USA 88, 11349-11353.
Neer, E.J., Schmidt, C.J., Nambudripad, R. and Smith, T.F. (1994) Nature 371, 297-300.
Nicola NA( (1994). Guidebook to Cytokines and Their Receptors. Oxford University Press: Oxford.
Nicola, NV, Viney E, Hilton DJ, Roberts B and Wilson T. (1996) Growth Factors 13, 141-149.
Novak U, Harpur AG, Paradiso L, Kanagasundaram V, Jaworowski A, Wilks AF and Hamilton JA (1995). Blood 86, 2948-56.
Pearson WR and Lipman DJ. (1988) Proc. Natl. Acad. Sci. USA 85, 2444-8.
Pearson WR. (1990) Methods Enzymol. 183, 63-98.
Rayner JR and Gonda TJ (1994). Molecular & Cellular Biology 14, 880-7.
Sambrook J, Fritsch EF and Maniatis T (1989). Molecular Cloning, A Laboratory Manual. Cold Spring Harbour Laboratory Press, Cold Spring Harbour USA.
Sato N, Sakamaki K, Terada N, Arai K and Miyajima A (1993). Embo Journal 12, 4181-9.
Shimoda K, van Deursen J, Sangster MY, Sarawar SR, Carson RT, Tripp RA, Chu C, Quelle FW, Nosaka T, Vignali DA, Doherty PC, Grosveld G, Paul WE and Ihle JN (1996). Nature 380, 630-3.
Shual K, Ziemiecki A, Wilks AF, Harpur AG, Sadowski HB, Gilman MZ and Darnell JE (1993). Nature 366, 580-3.
Sprang SR and Bazan JF (1993). Curr. Opin. Structural Biol. 3, 815-827.
Takeda K, Tanaka T, Shi W, Matsumoto M, Minami M, Kashiwamura S, Nakanishi K, Yoshida N, Kishimoto T and Akira S (1996). Nature 380, 627-30.
Thierfelder WE, Vandeursen JM, Yamamoto K, Tripp RA, Sarawar SR, Carson RT, Sangster MY, Vignali DDA, Doherty PC, Grosveld GC and Ihle JN (1996). Nature 382, 171-174.
Wakao H, Gouilleux F and Groner B (1994). Embo Journal 13, 2182-91.
Wen Z, Zhong Z and Darnell J Jr. (1995). Cell 82, 241-50.
Yi T, Mui AL, Krystal G and Ihle JN (1993). Molecular & Cellular Biology 13, 7577-86.
Yoshimura A, Ohkubo T, Kiguchi T, Jenkins NA, Gilbert DJ, Copeland NG, Hara T and Miyajima A (1995). Embo Journal 14, 2816-26.

## Claims

1. A nucleic acid molecule comprising a nucleotide sequence encoding, or complementary to a sequence encoding, a protein which comprises the amino acid sequence of SEQ ID NO. 8 or an amino acid sequence having at least 70% similarity to SEQ ID No. 8, wherein said protein comprises a SOCS box in its C-terminal region and modulates signal transduction.

2. A nucleic acid molecule according to claim 1 wherein the nucleotide sequence is as set forth in SEQ ID NO.7, or has at least 70% similarity to SEQ ID NO.7 or is capable of hybridizing to SEQ ID No. 7 under medium stringency conditions at 42°C.

3. An isolated protein comprising the amino acid sequence of SEQ ID NO. 8 or an amino acid sequence having at least 70% similarity to SEQ ID No. 8, wherein the protein comprises a SOCS box in its C-terminal region and modulates signal transduction.

4. A nucleic acid molecule according to claim 1 or claim 2 or a protein according to claim 3, wherein the protein comprises an SH2 domain in a region N-terminal of the SOCS box.

5. A nucleic acid molecule according to any one of claims 1, 2 or 4 or a protein according to any one of claims 3 or 4, wherein the signal transduction is mediated by a cytokine or other endogenous molecule, a hormone, a microbe or a microbial product, a parasite, an antigen or other effector molecule.

6. A nucleic acid molecule or a protein according to claim 5 wherein the protein modulates cytokine-mediated signal transduction.

7. A nucleic acid molecule or a protein according to claim 6 wherein the signal transduction is mediated by one or more of the cytokines G-CSF, IL-6 and/or LIF.

8. An *in vitro* method of modulating levels of a SOCS-3 protein in a cell said method comprising contacting a cell containing a SOCS-3 gene with an effective amount of a modulator of SOCS-3 gene expression as defined in any one of claims 1, 2 or 4 to 7 or of SOCS-3 protein activity as defined in any one of claims 3 to 7 for a time and under conditions sufficient to modulate levels of said SOCS-3 protein.

9. An *in vitro* method of modulating signal transduction in a cell containing a SOCS-3 gene comprising contacting said cell with an effective amount of a modulator of SOCS-3 gene expression as defined in any one of claims 1, 2 or 4 to 7 or of SOCS-3 protein activity as defined in any one of claims 3 to 7 for a time sufficient to modulate signal transduction.

10. An *in vitro* method of influencing interaction between cells wherein at least one cell carries a SOCS-3 gene, said method comprising contacting the cell carrying the SOCS-3 gene with an effective amount of a modulator of SOCS-3 gene expression as defined in any one of claims 1, 2 or 4 to 7 or of SOCS-3 protein activity as defined in any one of claims 3 to 7 for a time sufficient to modulate signal transduction.

11. A method according to any one of claims 8 to 10 wherein signal transduction is mediated by a mediator as defined in any one of claims 5 to 7.

12. A method according to any one of claims 8 to 11 wherein the SOCS-3 protein is as defined in any one of claims 3 to 7 and/or the SOCS-3 gene comprises a nucleic acid molecule as defined in any one of claims 1, 2 and 4 to 7.

13. An antisense oligonucleotide having a nucleotide sequence complementary to at least a portion of the "sense" sequence of a nucleic acid molecule as defined in any one of claims 1, 2 or 4 to 7, said antisense oligonucleotide being an antagonist of SOCS-3 capable of inhibiting SOCS-3 gene expression.

14. A pharmaceutical composition comprising a protein as defined in any one of claims 3 to 7 and one or more pharmaceutically acceptable carriers and/or diluents.

15. A vector comprising a nucleic acid molecule as defined in any one of claims 1, 2 or 4 to 7.

16. A transgenic mammalian cell line expressing a nucleic acid molecule as defined in any one of claims 1, 2 or 4 to 7.

17. Use of a protein as defined in any one of claims 3 to 7 in the manufacture of a medicament for the treatment of hyperimmunity, immunosuppression, allergies and hypertension.

18. Use of a modulator of the expression of a SOCS-3 gene comprising a nucleotide sequence as defined in any one of claims 1, 2 or 4 to 7 or of the activity of a SOCS-3 protein as defined in any one of claims 3 to 7, in the manufacture of a medicament for treatment of hyperimmunity, immunosuppression, allergies and hypertension.

19. An antibody or fragment thereof, that is specific for a protein according to any one of claims 3 to 7.

20. An antibody or fragment thereof according to claim 19 wherein said antibody is a polyclonal antibody, a monoclonal antibody, a synthetic antibody or a recombinant antibody.

21. An antibody or fragment thereof according to claim 19 or claim 20 wherein said antibody is labelled with a reporter molecule.

22. An antibody or fragment thereof that is specific for an antibody or fragment according to any one of claims 19 to 21 and that is optionally labelled with a reporter molecule.

23. A method for detecting a protein according to any one of claims 3 to 7 in a sample, said method comprising contacting said sample with an antibody or fragment thereof according to any one of claims 19 to 21 for a time and under conditions sufficient for a complex to form between said protein and said antibody or antibody fragment and then detecting said complex.

24. A method according to claim 23 wherein the sample is a biological sample.

25. A method according to claim 24 wherein the biological sample is a cell extract.

26. A method according to claim 25 wherein the biological sample is a biological fluid.

27. A method according to claim 26 wherein the biological fluid is selected from serum, saliva, mucosal secretions, lymph, tissue fluid and respiratory fluid.

28. A method according to claim 23 wherein the sample is fermentation fluid or supernatant fluid such as from a cell culture.

29. A method according to any one of claims 23 to 28 wherein the method is an immunoassay.

30. A method according to claim 29 wherein the immunoassay is an ELISA.

31. A method according to claim 29 wherein the method is a sandwich assay.

32. A method according to claim 29 wherein the immunoassay is a Western blot.

33. A method according to any one of claims 23 to 32 which is quantitative.

34. Use of an antibody or fragment thereof according to any one of claims 19 to 22 for purifying a protein according to any one of claims 3 to 7.

## Patentansprüche

1. Nukleinsäuremolekül, umfassend eine Nukleotidsequenz, die ein Protein kodiert, oder komplementär ist zu einer ein Protein kodierenden Sequenz, wobei das Protein die Aminosäuresequenz gemäß SEQ ID NO:8 oder eine Aminosäuresequenz mit wenigstens 70% Ähnlichkeit zu SEQ ID NO:8 umfasst, wobei das Protein eine SOCS-Box in seiner C-terminalen Region umfasst und Signaltransduktion moduliert.

2. Nukleinsäuremolekül gemäß Anspruch 1, wobei die Nukleotidsequenz der in SEQ ID NO:7 angegebenen Nukleotidsequenz entspricht, oder wenigstens 70% Ähnlichkeit mit SEQ ID NO:7 aufweist oder in der Lage ist, unter Bedingungen mittlerer Stringenz bei 42°C mit SEQ ID NO:7 zu hybridisieren.

3. Isoliertes Protein, umfassend die Aminosäuresequenz gemäß SEQ ID NO:8 oder eine Aminosäuresequenz mit wenigstens 70% Ähnlichkeit zu SEQ ID NO:8, wobei das Protein in seiner C-terminalen Region eine SOCS-Box umfasst und Signaltransduktion moduliert.

4. Nukleinsäuremolekül gemäß Anspruch 1 oder Anspruch 2 oder Protein gemäß-Anspruch 3, wobei das Protein eine SH2-Domäne in einer zur SOCS-Box N-terminal gelegenen Region umfasst.

5. Nukleinsäuremolekül gemäß einem der Ansprüche 1, 2 oder 4 oder Protein gemäß einem der Ansprüche 3 oder 4, wobei die Signaltransduktion über ein Zytokin oder ein anderes endogenes Molekül, ein Hormon, eine Mikrobe oder ein Mikrobenprodukt, einen Parasiten, ein Antigen oder ein anderes Effektormolekül vermittelt wird.

6. Nukleinsäuremolekül oder Protein gemäß Anspruch 5, wobei das Protein durch Zytokin vermittelte Signaltransduktion moduliert.

7. Nukleinsäuremolekül oder Protein gemäß Anspruch 6, wobei die Signaltransduktion durch eines oder mehrere der Zytokine G-CSF, IL-6 und/oder LIF vermittelt wird.

8. In vitro-Verfahren zur Modulation der Konzentrationen eines SOCS-3-Proteins in einer Zelle, wobei man bei diesem Verfahren eine Zelle, die ein SOCS-3-Gen enthält, mit einer wirksamen Menge eines Modulators der SOCS-3-Genexpression wie in einem der Ansprüche 1, 2 oder 4 bis 7 definiert oder der SOC-3-Proteinaktivität wie in einem der Ansprüche 3 bis 7 definiert für einen Zeitraum und unter Bedingungen in Kontakt bringt, die für eine Modulation der Konzentrationen des SOCS-3-Proteins ausreichend sind.

9. In vitro-Verfahren zur Modulation der Signaltransduktion in einer ein SOCS-3-Gen enthaltenden Zelle, wobei man die Zelle mit einer wirksamen Menge eines Modulators der SOCS-3-Genexpression wie in einem der Ansprüche 1, 2 oder 4 bis 7 definiert oder der SOC-3-Proteinaktivität wie in einem der Ansprüche 3 bis 7 definiert für einen Zeitraum in Kontakt bringt, der ausreichend ist, um eine Signaltransduktion zu modulieren.

10. In vitro-Verfahren zur Beeinflussung der Wechselwirkung zwischen Zellen, wobei wenigstens eine Zelle ein SOCS-3-Gen aufweist, wobei man bei diesem Verfahren die das SOCS-3-Gen aufweisende Zelle mit einer wirksamen Menge eines Modulators der SOCS-3-Genexpression wie in einem der Ansprüche 1, 2 oder 4 bis 7 definiert oder der SOC-3-Proteinaktivität wie in einem der Ansprüche 3 bis 7 definiert für einen Zeitraum in Kontakt bringt, der ausreichend ist, um eine Signaltransduktion zu modulieren.

11. Verfahren gemäß einem der Ansprüche 8 bis 10, wobei die Signaltransduktion durch einen Mediator gemäß der Definition in einem der Ansprüche 5 bis 7 vermittelt wird.

12. Verfahren gemäß einem der Ansprüche 8 bis 11, wobei das SOCS-3-Protein der Definition in einem der Ansprüche 3 bis 7 entspricht und/oder das SOCS-3-Gen ein Nukleinsäuremolekül gemäß der Definition in einem der Ansprüche 1, 2 und 4 bis 7 umfasst.

13. Antisense-Oligonukleotid mit einer Nukleotidsequenz, die komplementär ist zu wenigstens einem Teil der "Sense"-Sequenz eines Nukleinsäuremoleküls gemäß der Definition in einem der Ansprüche 1, 2 oder 4 bis 7, wobei das Antisense-Oligonukleotid ein Antagonist von SOCS-3 ist, der in der Lage ist, die SOCS-3-Genexpression zu inhibieren.

14. Pharmazeutische Zusammensetzung, umfassend ein Protein gemäß der Definition in einem der Ansprüche 3 bis 7 und einen oder mehrere pharmazeutisch akzeptable Träger und/oder Verdünnungsmittel.

15. Vektor, umfassend ein Nukleinsäuremolekül gemäß der Definition in einem der Ansprüche 1, 2 oder 4 bis 7.

16. Transgene Säuger-Zelllinie, die ein Nukleinsäuremolekül gemäß der Definition in einem der Ansprüche 1, 2 oder 4 bis 7 exprimiert.

17. Verwendung eines Proteins gemäß der Definition in einem der Ansprüche 3 bis 7 zur Herstellung eines Medikaments zur Behandlung von Hyperimmunität, Immunsuppression, Allergien und Hypertonie.

18. Verwendung eines Modulators der Expression eines SOCS-3-Gens umfassend eine Nukleotidsequenz gemäß der Definition in einem der Ansprüche 1, 2 oder 4 bis 7 oder der Aktivität eines SOCS-3-Proteins gemäß der Definition in einem der Ansprüche 3 bis 7 zur Herstellung eines Medikaments zur Behandlung von Hyperimmunität, Immunsuppression, Allergien und Hypertonie.

19. Antikörper oder Fragment davon, der/das für ein Protein gemäß einem der Ansprüche 3 bis 7 spezifisch ist.

20. Antikörper oder Fragment davon nach Anspruch 19, wobei der Antikörper ein polyklonaler Antikörper, ein monoklonaler Antikörper, ein synthetischer Antikörper oder ein rekombinanter Antikörper ist.

21. Antikörper oder Fragment davon nach Anspruch 19 oder Anspruch 20, wobei der Antikörper mit einem Reporter-Molekül markiert ist.

22. Antikörper oder Fragment davon, der/das spezifisch ist für einen Antikörper oder ein Fragment gemäß einem der Ansprüche 19 bis 21 und optional mit einem Reporter-Molekül markiert ist.

23. Verfahren zum Nachweis eines Proteins gemäß einem der Ansprüche 3 bis 7 in einer Probe, wobei man die Probe mit einem Antikörper oder einem Fragment davon gemäß einem der Ansprüche 19 bis 21 für einen Zeitraum und unter Bedingungen, die ausreichend sind für die Bildung eines Komplexes zwischen dem Protein und dem Antikörper oder dem Antikörperfragment, in Kontakt bringt und anschließend den Komplex nachweist.

24. Verfahren gemäß Anspruch 23, wobei die Probe eine biologische Probe ist.

25. Verfahren gemäß Anspruch 24, wobei die biologische Probe ein Zellextrakt ist.

26. Verfahren gemäß Anspruch 25, wobei die biologische Probe eine biologische Flüssigkeit ist.

27. Verfahren gemäß Anspruch 26, wobei die biologische Flüssigkeit ausgewählt ist unter Serum, Speichel, Mukosa-Sekretionen, Lymphe, Gewebeflüssigkeit und respiratorischer Flüssigkeit.

28. Verfahren gemäß Anspruch 23, wobei die Probe Fermentationsfiüssigkeit oder Überstandsflüssigkeit beispielsweise von einer Zellkultur ist.

29. Verfahren gemäß einem der Ansprüche 23 bis 28, wobei das Verfahren ein Immunassay ist.

30. Verfahren gemäß Anspruch 29, wobei der Immunassay ein ELISA ist.

31. Verfahren gemäß Anspruch 29, wobei das Verfahren ein Sandwich-Assay ist.

32. Verfahren gemäß Anspruch 29, wobei der Immunassay ein Western-Blot ist.

33. Verfahren gemäß einem der Ansprüche 23 bis 32, wobei das Verfahren quantitativ ist.

34. Verwendung eines Antikörpers oder eines Fragments gemäß einem der Ansprüche 19 bis 22 zur Aufreinigung eines Proteins gemäß einem der Ansprüche 3 bis 7

## Revendications

1. Molécule d'acide nucléique comprenant une séquence de nucléotides codant, ou complémentaire d'une séquence codant, une protéine qui comprend la séquence d'acides aminés de SEQ ID N° 8 ou une séquence d'acides aminés ayant au moins 70 % de similarité avec SEQ ID N° 8, ladite protéine comprenant une boîte SOCS dans sa région C-terminale et modulant la transduction de signal.

2. Molécule d'acide nucléique selon la revendication 1 dans laquelle la séquence de nucléotides est comme indiquée dans SEQ ID N° 7, ou a au moins 70 % de similarité avec SEQ ID N° 7 ou est capable de s'hybrider à SEQ ID N° 7 dans des conditions de stringence moyennes à 42 °C.

3. Protéine isolée comprenant la séquence d'acides nucléiques de SEQ ID N° 8 ou une séquence d'acides aminés ayant au moins 70 % de similarité avec SEQ ID N° 8, la protéine comprenant une boîte SOCS dans sa région C-terminale et modulant la transduction de signal.

4. Molécule d'acide nucléique selon la revendication 1 ou la revendication 2 ou protéine selon la revendication 3, la protéine comprenant un domaine SH2 dans une région N-terminale de la boîte SOCS.

5. Molécule d'acide nucléique selon l'une quelconque des revendications 1, 2 ou 4, ou protéine selon l'une quelconque des revendications 3 ou 4, la transduction de signal étant médiée par une cytokine ou une autre molécule endogène, une hormone, un microbe ou un produit microbien, un parasite, un antigène ou une autre molécule effectrice.

6. Molécule d'acide nucléique ou protéine selon la revendication 5, la protéine modulant la transduction de signal médiée par cytokine.

7. Molécule d'acide nucléique ou protéine selon la revendication 6, la transduction de signal étant médiée par une ou plusieurs des cytokines G-CSF, IL-6 et/ou LIF.

8. Procédé *in vitro* pour moduler les niveaux d'une protéine SOCS-3 dans une cellule, ledit procédé comprenant l'étape consistant à mettre en contact une cellule contenant un gène SOCS-3 avec une quantité efficace d'un modulateur de l'expression du gène SOCS-3 comme défini dans l'une quelconque des revendications 1, 2 ou 4 à 7 ou de l'activité de la protéine SOCS-3 comme définie dans l'une quelconque des revendications 3 à 7 pendant une durée et dans des conditions suffisantes pour moduler les niveaux de ladite protéine SOCS-3.

9. Procédé *in vitro* pour moduler la transduction de signal dans une cellule contenant un gène SOCS-3, comprenant l'étape consistant à mettre en contact ladite cellule avec une quantité efficace d'un modulateur de l'expression du gène SOCS-3 comme défini dans l'une quelconque dés revendications 1, 2 ou 4 ou 4 à 7 ou de l'activité de la protéine SOCS-3 comme définie dans l'une quelconque des revendications 3 à 7 pendant une durée suffisante pour moduler la transduction de signal.

10. Procédé *in vitro* pour influencer l'interaction entre des cellules, au moins une cellule portant un gène SOCS-3, ledit procédé comprenant l'étape consistant à mettre en contact la cellule portant le gène SOCS-3 avec une quantité efficace d'un modulateur de l'expression du gène SOCS-3 comme défini dans l'une quelconque des revendications 1, 2 ou 4 à 7 ou de l'activité de la protéine SOCS-3 comme définie dans l'une quelconque des revendications 3 à 7 pendant une durée suffisante pour moduler la transduction de signal.

11. Procédé selon l'une quelconque des revendications 8 à 10 dans lequel la transduction de signal est médiée par un médiateur comme défini dans l'une quelconque des revendications 5 à 7.

12. Procédé selon l'une quelconque des revendications 8 à 11 dans lequel la protéine SOCS-3 est comme définie dans l'une quelconque des revendications 3 à 7 et/ou le gène SOCS-3 comprend une molécule d'acide nucléique comme définie dans l'une quelconque des revendications 1, 2 et 4 à 7.

13. Oligonucléotide anti-sens ayant une séquence de nucléotides complémentaire d'au moins une partie de la séquence « sens » d'une molécule d'acide nucléique comme définie dans l'une quelconque des revendications 1, 2 ou 4 à 7, ledit oligonucléotide anti-sens étant un antagoniste de SOCS-3 capable d'inhiber l'expression du gène SOCS-3.

14. Composition pharmaceutique comprenant une protéine comme définie dans l'une quelconque des revendications 3 à 7 et un ou plusieurs véhicules et/ou diluants pharmaceutiquement acceptables.

15. Vecteur comprenant une molécule d'acide nucléique comme définie dans l'une quelconque des revendications 1, 2 ou 4 à 7.

16. Lignée de cellules mammifères transgéniques exprimant une molécule d'acide nucléique comme définie dans l'une quelconque des revendications 1, 2 ou 4 à 7.

17. Utilisation d'une protéine comme définie dans l'une quelconque des revendications 3 à 7 dans la fabrication d'un médicament pour le traitement de l'hyperimmunité, de l'immunosuppression, des allergies et de l'hypertension.

18. Utilisation d'un modulateur de l'expression d'un gène SOCS-3 comprenant une séquence de nucléotides comme définie dans l'une quelconque des revendications 1, 2 ou 4 à 7 ou de l'activité d'une protéine SOCS-3 comme définie dans l'une quelconque des revendications 3 à 7, dans la fabrication d'un médicament pour le traitement de l'hyperimmunité, de l'immunosuppression, des allergies et de l'hypertension.

19. Anticorps ou fragment d'anticorps qui est spécifique d'une protéine selon l'une quelconque des revendications 3 à 7.

20. Anticorps ou fragment d'anticorps selon la revendication 19, ledit anticorps étant un anticorps polyclonal, un anticorps monoclonal, un anticorps synthétique ou un anticorps recombinant.

21. Anticorps ou fragment d'anticorps selon la revendication 19 ou la revendication 20, ledit anticorps étant marqué avec une molécule de rapporteur.

22. Anticorps ou fragment d'anticorps qui est spécifique d'un anticorps ou d'un fragment d'anticorps selon l'une quelconque des revendications 19 à 21 et qui est facultativement marqué avec une molécule rapporteur.

23. Procédé pour détecter une protéine selon l'une quelconque des revendications 3 à 7 dans un échantillon, ledit procédé comprenant les étapes consistant à mettre en contact ledit échantillon avec un anticorps ou fragment d'anticorps selon l'une quelconque des revendications 19 à 21 pendant une durée et dans des conditions suffisantes pour qu'un complexe se forme entre ladite protéine et ledit anticorps ou ledit fragment d'anticorps puis à détecter ledit complexe.

24. Procédé selon la revendication 23 dans lequel l'échantillon est un échantillon biologique.

25. Procédé selon la revendication 24 dans lequel l'échantillon biologique est un extrait cellulaire.

26. Procédé selon la revendication 25 dans lequel l'échantillon biologique est un fluide biologique.

27. Procédé selon la revendication 26 dans lequel le fluide biologique est choisi parmi le sérum, la salive, des sécrétions muqueuses, la lymphe, du fluide tissulaire et du fluide respiratoire.

28. Procédé selon la revendication 23 dans lequel l'échantillon est un fluide de fermentation ou un fluide surnageant de culture cellulaire.

29. Procédé selon l'une quelconque des revendications 23 à 28, le procédé étant un immunodosage.

30. Procédé selon la revendication 29, l'immunodosage étant un dosage ELISA.

31. Procédé selon la revendication 29, le procédé étant un test en sandwich.

32. Procédé selon la revendication 29, l'immunodosage étant un buvardage de Western.

33. Procédé selon l'une quelconque des revendications 23 à 32 qui est quantitatif.

34. Utilisation d'un anticorps ou d'un fragment d'anticorps selon l'une quelconque des revendications 19 à 22 pour purifier une protéine selon l'une quelconque des revendications 3 à 7.
